(19) Europäisches Patentamt
European Patent Office
Office européen des brevets

(11) EP 4 313 962 B1

(12) EUROPEAN PATENT SPECIFICATION

(45) Date of publication and mention of the grant of the patent:
**22.07.2026 Bulletin 2026/30**

(21) Application number: **22778907.0**

(22) Date of filing: **29.03.2022**

(51) International Patent Classification (IPC):
***C07D 417/12*** *(2006.01)* ***C07D 409/12*** *(2006.01)*
***C07D 471/04*** *(2006.01)* ***C07D 495/14*** *(2006.01)*
***C07D 519/00*** *(2006.01)* ***C07D 495/04*** *(2006.01)*
***A61K 45/06*** *(2006.01)* ***A61P 35/00*** *(2006.01)*

(52) Cooperative Patent Classification (CPC):
**A61K 45/06; A61P 35/00; C07D 409/12; C07D 417/12; C07D 471/04; C07D 495/04; C07D 495/14; C07D 519/00**

(86) International application number:
**PCT/CN2022/083557**

(87) International publication number:
**WO 2022/206725 (06.10.2022 Gazette 2022/40)**

# (54) NOVEL COMPOUNDS USEFUL AS STING AGONISTS AND USES THEREOF

NEUE VERBINDUNGEN ALS STING-AGONISTEN UND VERWENDUNGEN DAVON

NOUVEAUX COMPOSÉS UTILES EN TANT QU'AGONISTES STING ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**30.03.2021 PCT/CN2021/083783**
**29.04.2021 PCT/CN2021/090945**
**09.08.2021 PCT/CN2021/111479**
**05.11.2021 PCT/CN2021/128942**
**11.11.2021 PCT/CN2021/130097**
**16.12.2021 PCT/CN2021/138806**
**30.12.2021 PCT/CN2021/143043**
**12.01.2022 PCT/CN2022/071548**

(43) Date of publication of application:
**07.02.2024 Bulletin 2024/06**

(73) Proprietor: **Jacobio Pharmaceuticals Co., Ltd.**
**Beijing 100176 (CN)**

(72) Inventors:
- **YE, Yang**
  **Beijing 100176 (CN)**
- **LI, Xiuwei**
  **Beijing 100176 (CN)**
- **YANG, Guiqun**
  **Beijing 100176 (CN)**
- **YAN, Fashun**
  **Beijing 100176 (CN)**
- **WANG, Yanping**
  **Beijing 100176 (CN)**
- **LONG, Wei**
  **Beijing 100176 (CN)**

(74) Representative: **Maikowski & Ninnemann Patentanwälte Partnerschaft mbB**
**Postfach 15 09 20**
**10671 Berlin (DE)**

(56) References cited:
**WO-A1-2019/195063**
**WO-A1-2019/195124**
**WO-A1-2021/014365**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 313 962 B1

## Description

### Cross Reference To Related Applications

The application claims the benefit of PCT application Ser. No. PCT/CN2021/083783 filed on Mar. 30, 2021; PCT application Ser. No. PCT/CN2021/090945 filed on Apr. 29, 2021; PCT application Ser. No. PCT/CN2021/111479 filed on Aug. 9, 2021; PCT application Ser. No. PCT/CN2021/128942 filed on Nov. 5, 2021; PCT application Ser. No. PCT/CN2021/130097 filed on Nov. 11, 2021; PCT application Ser. No. PCT/CN2021/138806 filed on Dec. 16, 2021; PCT application Ser. No. PCT/CN2021/143043 filed on Dec. 30, 2021; and PCT application Ser. No. PCT/CN2022/071548 filed on Jan. 12, 2022.

### Technical Field

The present invention relates to compounds and derivatives thereof and that may be useful as STING (Stimulator of Interferon Genes) agonists that activate the STING pathway. The present invention also relates to compositions comprising such compounds, processes for the synthesis of such compounds, and uses of such compounds to induce immune responses, to induce STING-dependent type I interferon production, and/or to treat a cell proliferation disorder, such as cancer.

### Background Art

The immune system has evolved to recognize and neutralize different types of threats in order to maintain the homeostasis of the host, and it is generally broken down into two arms: adaptive and innate. The adaptive immune system is specialized to recognize as foreign those antigens not naturally expressed in the host and to mount an anti-antigen response through the coordinated actions of many leukocyte subsets. The hallmark of adaptive immune responses is their ability to provide "memory" or long-lasting immunity against the encountered antigen. While this specific and long-lasting effect is critical to host health and survival, the adaptive immune response requires time to generate a full-blown response.

The innate immune system compensates for this time delay and is specialized to act quickly against different insults or danger signals. It provides the first line of defense against bacteria, viruses, parasites and other infectious threats, but it also responds strongly to certain danger signals associated with cellular or tissue damage. The innate immune system has no antigen specificity but does respond to a variety of effector mechanisms. Opsonization, phagocytosis, activation of the complement system, and production of soluble bioactive molecules such as cytokines or chemokines are all mechanisms by which the innate immune system mediates its response. By responding to these damage-associated molecular patterns (DAMPs) or pathogen-associated molecular patterns (PAMPs) described above, the innate immune system is able to provide broad protection against a wide range of threats to the host.

Free cytosolic DNA and RNA are among these PAMPs and DAMPs. It has recently been demonstrated that the main sensor for cytosolic DNA is cGAS (cyclic GMP-AMP synthase). Upon recognition of cytosolic DNA, cGAS catalyzes the generation of the cyclic-dinucleotide 2'3'-cGAMP, an atypical second messenger that strongly binds to the ER-transmembrane adaptor protein STING. A conformational change is undergone by cGAMP-bound STING, which translocates to a perinuclear compartment and induces the activation of critical transcription factors IRF-3 and NF-κB. This leads to a strong induction of type I interferons and production of pro-inflammatory cytokines such as IL-6, TNF-α and IFN-y.

The importance of type I interferons and pro-inflammatory cytokines on various cells of the immune system has been very well established. In particular, these molecules strongly potentiate T-cell activation by enhancing the ability of dendritic cells and macrophages to uptake, process, present and cross-present antigens to T-cells. The T-cell stimulatory capacity of these antigen-presenting cells is augmented by the up-regulation of critical co-stimulatory molecules, such as CD80 or CD86. Finally, type I interferons can rapidly engage their cognate receptors and trigger the activation of interferon-responsive genes that can significantly contribute to adaptive immune cell activation.

From a therapeutic perspective, type I interferons are shown to have antiviral activities by directly inhibiting human hepatitis B virus and hepatitis C virus replication, and by stimulating immune responses to virally infected cells. Compounds that can induce type I interferon production are used in vaccines, where they act as adjuvants, enhancing specific immune responses to antigens and minimizing side effects by reducing dosage and broadening the immune response.

In addition, interferons, and compounds that can induce interferon production, have potential use in the treatment of human cancers. Such molecules are potentially useful as anti-cancer agents with multiple pathways of activity. Interferons can inhibit human tumor cell proliferation directly and may be synergistic with various approved chemotherapeutic agents. Type I interferons can significantly enhance anti-tumor immune responses by inducing activation of both the adaptive and innate immune cells. Finally, tumor invasiveness may be inhibited by interferons by modulating enzyme

expression related to tissue remodeling.

WO2019195124A1 discloses compounds that may be useful as inductors of type I interferon production, specifically as STING active agents.

In view of the potential of type I interferons and type I interferon-inducing compounds as anti-viral and anti-cancer agents, there remains a need for new agents that can induce potent type I interferon production. With the growing body of data demonstrating that the cGAS-STING cytosolic DNA sensory pathway has a significant capacity to induce type Iinterferons, the development of STING activating agents is rapidly taking an important place in today's anti-tumor therapy landscape.

Thus, there remains a need in the art for developing compounds with novel structure, good biological activity and high druggability, and methods for preventing or treating disease and disorders, such as cancer. The compounds of the present invention fulfill this need.

**Summary of Invention**

The present invention relates to novel compounds useful as STING agonists and to their use in the treatment of cell proliferation disorders. The present disclosure includes compounds of general formula (I), compounds of general formula (II), compounds of general formula (III), compounds of general formula (IV), compounds of general formula (V), and pharmaceutically acceptable salts thereof. These compounds and their pharmaceutically acceptable salts may be useful as agents to induce immune responses, to induce STING-dependent type I interferon production, and/or to treat a cell proliferation disorder.

The present disclosure relates to novel compounds of general structure as Formula I, II, III, IV, V, or a pharmaceutically acceptable salt:

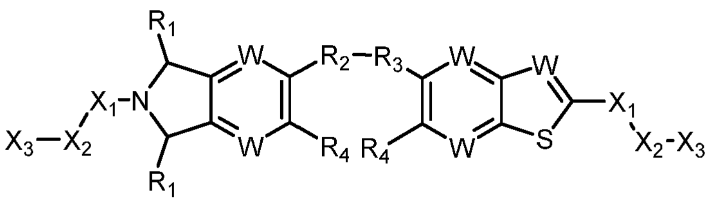

I

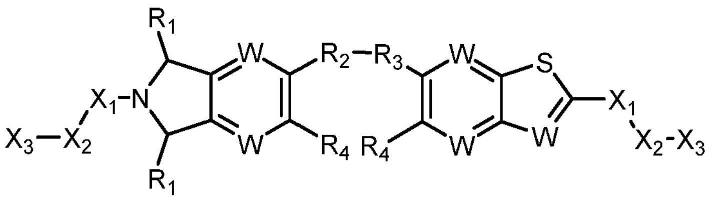

II

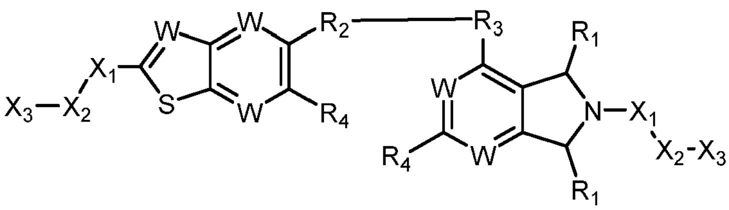

III

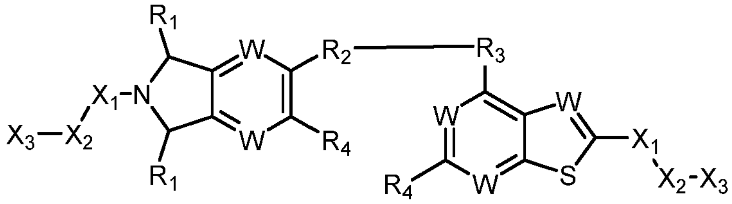

IV

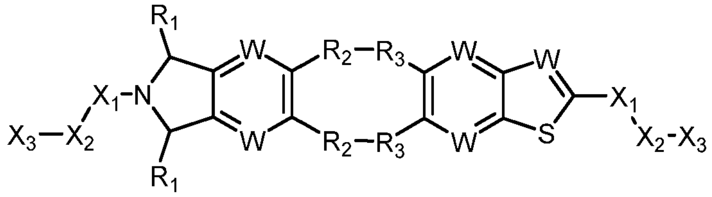

V

wherein

each W is independently selected from $CR_1$ or N;
each $R_1$ is independently selected from H, deuterium, halogen, $OR^6$, $N(R^6)_2$, $COOR^6$, or $C(O)N(R^6)_2$, CN or $C_1$-$C_6$ alkyl; wherein the $C_1$-$C_6$ alkyl is optionally substituted with one or more deuterium, halogen, $OR^6$, $N(R^6)_2$, $COOR^6$, or $C(O)N(R^6)_2$;
$R_2$ and $R_3$ are independently selected from O-($C_1$-$C_4$ alkylene or haloalkylene), $C_1$-$C_5$ alkylene or haloalkylene, $N(R^6)$-($C_1$-$C_4$ alkylene or haloalkylene), -$T_a$-$C_1$-$C_6$ alkyl-$T_b$-, -$T_a$-N(Rs)-$T_b$, -$T_a$-O-$T_b$, -$T_a$-$PEG_n$-O-$T_b$, -$T_a$-S-S-$T_b$, -$T_a$-S-S-S-$T_b$, -$T_a$-N($R_s$)-N(Rs)-$T_b$-, - $T_a$-$C_2$-$C_6$alkenyl-$T_b$-, -$T_a$-$C_2$-$C_6$alkynyl-$T_b$-, -$T_a$-C(=O)-$T_b$-, -$T_a$-C(=$CH_2$)-$T_b$-, -$T_a$-C(=O)-C(=O)- $T_b$-, -$T_a$-C(=O)-($C_1$-$C_6$alkyl)-C(=O)-$T_b$-, -$T_a$-C(=O)-($C_3$-$C_{12}$cycloalkyl)-C(=O)-$T_b$-,-$T_a$-C(=O)-($C_1$-$C_6$alkyl)-($C_3$-$C_{12}$cycloalkyl)-($C_1$-$C_6$alkyl)-C(=O)-$T_b$ -, -$T_a$-C(=O)-(3- to 12-membered heterocycloalkyl)-C(=O)-$T_b$-, -$T_a$-C(=O)-( $C_1$-$C_6$alkyl)-(3- to 12-membered heterocycloalkyl)-($C_1$-$C_6$alkyl)-C(=O)-$T_b$-, -$T_a$-C(=S)-$T_b$-, -$T_a$-S(=O)$_2$-$T_b$-, -$T_a$-S(=O)-$T_b$-, -$T_a$-P(=O)(-ORs)-$T_b$-, -$T_a$-($C_3$-$C_{12}$cycloalkyl)-$T_b$-, -$T_{a}$-($C_6$-$C_{12}$ aryl)-$T_b$-, -$T_a$-(3- to 12-membered heterocycloalkyl)-$T_b$-, or -$T_a$-(5- to 12-membered heteroaryl)-$T_b$-, wherein the $C_1$-$C_6$alkyl, $C_2$-$C_6$ alkenyl, $C_2$-$C_6$ alkynyl, $C_3$-$C_{12}$ cycloalkyl, $C_6$-$C_{12}$ aryl, 3- to 12-membered heterocycloalkyl, or 5- to 12-membered heteroaryl is optionally substituted with one or more deuterium, halo, -ORs, -N(Rs)$_2$, or -C(=O)ORs;
$PEG_n$ is $(-OCH_2CH_2-)_n$, n = 1-8;
$T_a$ and $T_b$ each independently are absent, -N(Rs)-, -O-, $C_1$-$C_6$ alkyl, -N(Rs)-( $C_1$-$C_6$alkyl)-, -( $C_1$-$C_6$ alkyl)-N(Rs)-, -N(Rs)-( $C_1$-$C_6$alkyl)-N(Rs)-, -O-($C_1$-$C_6$ alkyl)-, -($C_1$-$C_6$alkyl)-O-, or -O- ($C_1$-$C_6$ alkyl)-O-; wherein the $C_1$-$C_6$ alkyl is optionally substituted with one or more halogen; and
each Rs independently is H, deuterium or $C_1$-$C_6$ alkyl optionally substituted with one or more halogen;
each $R_4$ is independently selected from the group consisting of H, deuterium, halogen, CN, $OR^6$, $N(R^6)_2$, $COOR^6$, $C(O)N(R^6)_2$, $SO_2R^6$, $C_1$-$C_6$ alkyl, $C_1$-$C_6$ haloalkyl, $C_1$-$C_6$ alkyl substituted by $OR^6$, $C_2$-$C_6$ alkenyl, $C_2$-$C_6$ haloalkenyl, $C_2$-$C_6$ alkenyl substituted by $OR^6$, $C_2$-$C_6$ alkynyl, $C_2$-$C_6$ haloalkynyl, $C_2$-$C_6$ alkynyl substituted by $OR^6$, $C_3$-$C_6$ cycloalkyl, and a 3- to 6-membered heterocyclic ring including 1 to 2 ring members selected from the group consisting of O, S, and $N(R^6)$;
each $R^6$ is independently selected from the group consisting of -H, deuterium, halogen, -$NH_2$, -CN, -OH, -$N_3$, -$NO_2$, carboxyl, $C_{1-6}$alkyl, $C_{1-6}$alkoxy, $C_{2-6}$alkenyl, $C_{2-6}$alkynyl, -$C_{6-10}$aryl, -$C_{5-10}$heteroaryl, $C_{3-10}$heterocyclic ring or $C_{3-10}$carbocyclic ring; and each of which is independently optionally substituted with deuterium, halogen, -$NH_2$, -CN, -OH, -$NO_2$, carbonyl, =O, oxo, carboxyl, $C_{1-6}$alkoxy, or $C_{1-6}$alkyl; and each of the heteroaryl and heterocyclic ring contains at least one heteroatoms selected from N, O or S;
each $X_1$ is independently selected from the group consisting of C=O, -$CH_2$-, -CHF-, and -$CF_2$-;
each $X_2$ is independently selected from $(C(R^8)_2)_{(1-3)}$, -$NR^8(C(R^8)_2)_{(1-3)}$, -$NH(C(R^8)_2)_{(1-3)}$, -N($C_{1-6}$alkyl) $(C(R^8)_2)_{(1-3)}$ or -N(halo$C_{1-6}$alkyl) $(C(R^8)_2)_{(1-3)}$; wherein each $R^8$ is independently selected from the group consisting of H, deuterium, halogen, $C_1$-$C_6$ alkyl, CN, $OR^6$, $N(R^6)_2$, $C_1$-$C_6$ haloalkyl, $C_3$-$C_6$ cycloalkyl, $C_1$-$C_6$ alkyl substituted by $OR^6$, and $C_1$-$C_6$ alkyl substituted by $N(R^6)_2$; optionally 2 $R^8$ on different carbon atoms may be taken together, along with the atoms to which they are attached, to form a 3- to 6-membered fused ring; and optionally 2 $R^8$ on a single carbon atom may be taken together, along with the atom to which they are attached, to form a 3- to 6-membered spirocycle;
each $X_3$ is independently selected from the group consisting of $COOR^6$, $C(O)SR^6$, $C(S)OR^6$, $SO_2R^6$, $C(O)N(R^9)_2$,

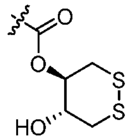

and CN; and each $R^9$ is independently selected from H, deuterium, $COOR^6$, $SO_2R^6$, $(CH_2)_{1-3}$-C(=O)$OR^6$, $OR^6$, $SR^6$, $NH_2$, NH($C_1$-$C_6$ alkyl), N($C_1$-$C_6$ alkyl)$_2$, O($C_1$-$C_6$ alkyl), O($C_6$-$C_{10}$ aryl), O($C_1$-$C_6$ alkyl)-$OR^6$, S($C_1$-$C_6$alkyl), S($C_6$-$C_{10}$ aryl), S(=O)$_2R^6$, S(=O)$_2OR^6$, P(=O)($R^6$)$_2$, $C_1$-$C_6$alkyl, $C_2$-$C_6$ alkenyl, $C_2$-$C_6$ alkynyl, $C_3$-$C_8$ cycloalkyl, $C_6$-$C_{10}$aryl, 3-8 membered heterocycloalkyl, or 3-10 membered heteroaryl.

The present invention provides a compound of Formula I-1 as claimed in claim 1:

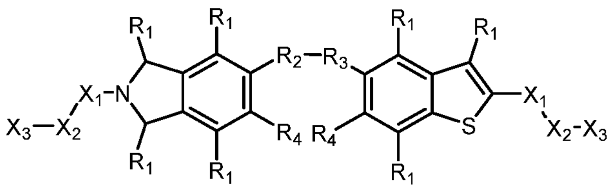

I-1,

wherein

each $R_1$ is independently selected from H, deuterium, or halogen;
$R_2$ and $R_3$ are independently selected from the group consisting of O-($C_1$-$C_4$ alkylene), -$T_a$-$C_1$-$C_6$alkyl-$T_b$-, or -$T_a$-O-$T_b$, wherein the $C_1$-$C_6$alkyl is optionally substituted with one or more deuterium, or halo;
$T_a$ and $T_b$ each independently are absent, -O-, $C_1$-$C_6$ alkyl, -O-($C_1$-$C_6$ alkyl)-, or -($C_1$-$C_6$alkyl)-O-;
each $R_4$ is independently selected from the group consisting of H, deuterium, halogen, or $OR^6$;
each $R^6$ is independently selected from the group consisting of -H, deuterium, or $C_{1-6}$alkyl, and each of which is independently optionally substituted with deuterium, or -OH;
each $X_1$ is C=O;
each $X_2$ is independently selected from -(C($R^8$)$_2$)$_{(1-3)}$, -$NR^8$(C($R^8$)$_2$)$_{(1-3)}$, or -NH(C($R^8$)$_2$)$_{(1-3)}$; wherein each $R^8$ is independently selected from the group consisting of H, deuterium, or $C_1$-$C_6$ alkyl; and
each $X_3$ is $COOR^6$, $SO_2R^6$,

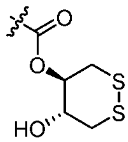

or CN.

In some embodiments of Formula I-1 each $R_1$ is independently selected from the group consisting of H, deuterium, F, Cl or Br.

In some embodiments, each $R_1$ independently is hydrogen or halogen.

In some embodiments, each $R_1$ independently is hydrogen or F.

In some embodiments, each $R_1$ independently is deuterium.

In some embodiments of Formula 1-1, $R_2$-$R_3$ is selected from selected from



,

or

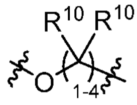

,

wherein:
each $R^{10}$ is independently hydrogen or $C_{1-2}$ alkyl.

In some embodiments, each $R^{10}$ is independently hydrogen or methyl.

In some embodiments of Formula I-1, $R_2$-$R_3$ is selected from the group consisting of $-O(CH_2)_2-$, $-O(CH_2)_3-$, $-O(CH_2)_4-$, $-O(CH_2)_2O-$, $-O(CH_2)_3O-$, $-O(CH_2)_4O-$, $-OCH_2CH(CH_3)CH_2O-$, $-OCH(CH_3)CH_2CH(CH_3)O-$ , $-O(CH_2)_4O-$, or $-O(CH_2)_5O-$.

In some embodiments of Formula 1-1, each $R_4$ is independently selected from the group consisting of H, deuterium, Br,or $OR^6$.

In some embodiments of Formula 1-1, each $R_4$ is independently selected from the group consisting of H, deuterium, Br, OH, or $OC_1$-$C_3$ alkyl.

In some embodiments of Formula 1-1, each $R_4$ independently is selected from the group consisting of H, deuterium, Br, OH, or $OCH_3$.

In some embodiments of Formula 1-1, each $R^6$ is independently selected from the group consisting of -H, deuterium, or $C_{1-}C_3$alkyl; and each of which is independently optionally substituted with deuterium, or -OH.

In some embodiments of Formula 1-1, each $R^6$ is independently selected from the group consisting of -H, deuterium, methyl, ethyl, propyl, isopropyl, ethylene; and each of which is independently optionally substituted with deuterium, or -OH.

Each $X_1$ is C=O.

In some embodiments, each $X_2$ independently is $-CH_2CH_2-$.

In some embodiments of Formula 1-1, each $X_2$ is $CH_2CHR^8$, where $R^8$ is selected from the group consisting of H, deuterium, or $C_1$-$C_3$ alkyl.

In some embodiments of Formula 1-1, each $X_2$ is $CH_2CHR^8$, wherein $R^8$ is selected from the group consisting of H, deuterium, $CH_3$, $CH_2CH_3$, $CH_2CH_2CH_3$, and $CH(CH_3)_2$, .

In some embodiments of Formula 1-1, each $X_3$ is independently selected from the group consisting of COOH, and

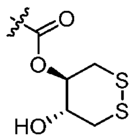

.

In some embodiments of the preceding Formula I-1 defined herein, the compound is selected from the compounds described in Table 1 and pharmaceutically acceptable salts thereof.

In some embodiments of the preceding Formula I-1 defined herein, the compound is the compounds described in Table 1.

Table 1

| | |
|---|---|
| 1. | 4-(5-(3-((2-(3-carboxypropanoyl)-6-methoxybenzo[b]thiophen-5-yl)oxy)propoxy)-6-methoxyiso indolin-2-yl)-4-oxobutanoic acid |
| 2. | 4-(5-(3-((2-(3-carboxypropanoyl)-4-chloro-6-methoxyisoindolin-5-yl)oxy)propoxy)-4-fluoro-6-methoxybenzo [b]thiophen-2-yl)-4-oxobutanoic acid |
| 3. | 4-(5-(3-((2-(3-carboxypropanoyl)-4-chloro-6-methoxybenzo[b]thiophen-5-yl)oxy)propoxy)-4-flu oro-6-methoxvisoindolin-2-yl)-4-oxobutanoic acid |
| 4. | 4-(5-(3-((2-(3-carboxypropanoyl)-4-fluoro-6-methoxybenzo[b]thiophen-5-yl)oxy)propoxy)-4-flu oro-6-methoxvisoindolin-2-yl)-4-oxobutanoic acid |
| 5. | sodium (S)-4-(5-(3-((2-((S)-3-carboxylatobutanoyl)-6-methoxybenzo[b]thiophen-5-yl)oxy)propoxy)-6-m ethoxyisoindolin-2-yl)-2-methyl-4-oxobutanoate |

(continued)

| | |
|---|---|
| 6. | sodium (S)-4-(5-(3-((2-((S)-3-carboxylatobutanoyl)-4-fluoro-6-methoxybenzo[b]thiophen-5-yl)oxy)prop oxy)-6-methoxvisoindolin-2-yl)-2-methyl-4-oxobutanoate |
| 7. | sodium 4-(5-(3-((2-(3-carboxylatopropanoyl)-6-hydroxybenzo[b]thiophen-5-yl)oxy)propoxy)-6-methoxy isoindolin-2-yl)-4-oxobutanoate |
| 9. | 4-(5-(3-((6-bromo-2-(3-carboxypropanoyl)benzo[b]thiophen-5-yl)oxy)propoxy)-6-methoxyisoin dolin-2-yl)-4-oxobutanoic acid |
| 10. | 4-(5-(3-((2-(3-carboxypropanoyl)-4-fluoro-6-methoxyisoindolin-5-yl)oxy)propoxy)-6-methoxyb enzo[b]thiophen-2-yl)-4-oxobutanoic acid |
| 12. | 4-(5-(3-((2-(4-ethoxy-4-oxobutanoyl)-6-methoxybenzo[b]thiophen-5-yl)oxy)propoxy)-6-methox yisoindolin-2-yl)-4-oxobutanoic acid |
| 14. | 4-(5-(3-((2-(3-carboxypropanoyl)-4-chloro-6-methoxyisoindolin-5-yl)oxy)propoxy)-6-methoxyb enzo[b]thiophen-2-yl)-4-oxobutanoic acid |
| 17. | (S)-4-(5-(3-((2-((S)-3-carboxybutanoyl)-4-fluoro-6-methoxybenzo[b]thiophen-5-yl)oxy)propoxy )-4-fluoro-6-methoxvisoindolin-2-yl)-2-methyl-4-oxobutanoic acid |
| 18. | 4-(5-(3-((2-((2-carboxyethyl)carbamoyl)-6-methoxybenzo[b]thiophen-5-yl)oxy)propoxy)-6-meth oxvisoindolin-2-yl)-4-oxobutanoic acid |
| 20. | 4-(5-(3-((2-(3-carboxypropanoyl)-4-fluoro-6-methoxybenzo[b]thiophen-5-yl)oxy)propoxy)-6-me thoxvisoindolin-2-yl)-4-oxobutanoic acid |
| 22. | 4-(5-(3-((2-(3-carboxybutanoyl)-6-methoxybenzo[b]thiophen-5-yl)oxy)propoxy)-6-methoxyisoi ndolin-2-yl)-2-methyl-4-oxobutanoic acid |
| 23. | (S)-4-(5-(3-((2-((S)-3-carboxybutanoyl)-6-methoxybenzo[b]thiophen-5-yl)oxy)propoxy)-6-meth oxyisoindolin-2-yl)-2-methyl-4-oxobutanoic acid |
| 24. | (S)-4-(5-(3-((2-(3-carboxypropanoyl)-6-methoxyisoindolin-5-yl)oxy)propoxy)-6-methoxybenzo[ b]thiophen-2-yl)-2-methyl-4-oxobutanoic acid |
| 25. | (S)-4-(5-(3-((2-(3-carboxypropanoyl)-6-methoxybenzo[b]thiophen-5-yl)oxy)propoxy)-6-methox yisoindolin-2-yl)-2-methyl-4-oxobutanoic acid |
| 26. | (S)-4-(5-(2-((2-((S)-3-carboxybutanoyl)-6-methoxybenzo[b]thiophen-5-yl)oxy)ethoxy)-6-metho xyisoindolin-2-yl)-2-methyl-4-oxobutanoic acid |
| 27. | (S)-4-(5-(4-((2-((S)-3-carboxybutanoyl)-6-methoxybenzo[b]thiophen-5-yl)oxy)butoxy)-6-metho xyisoindolin-2-yl)-2-methyl-4-oxobutanoic acid |
| 28. | (S)-4-(5-((5-((2-((S)-3-carboxybutanoyl)-6-methoxybenzo[b]thiophen-5-yl)oxy)pentyl)oxy)-6-m ethoxyisoindolin-2-yl)-2-methyl-4-oxobutanoic acid |
| 34. | (2S)-4-(5-((4-((2-((S)-3-carboxybutanoyl)-6-methoxybenzo[b]thiophen-5-yl)oxy)pentan-2-yl)ox y)-6-methoxyisoindolin-2-yl)-2-methyl-4-oxobutanoic acid |
| 35. | (S)-4-(5-(3-((2-((S)-3-carboxybutanoyl)-6-methoxybenzo[b]thiophen-5-yl)oxy)-2,2-dimethylpro poxy)-6-methoxyisoindolin-2-yl)-2-methyl-4-oxobutanoic acid |
| 37. | 4-(5-(3-((2-(3-carboxypropanoyl)-6-methoxyisoindolin-5-yl)oxy)propoxy)-6-methoxybenzo[b]th iophen-2-yl)-2,2-dimethyl-4-oxobutanoic acid |
| 38. | 4-(5-(3-((2-(3-carboxypropanoyl)-6-methoxybenzo[b]thiophen-5-yl)oxy)propoxy)-6-methoxyiso indolin-2-yl)-2,2-dimethyl-4-oxobutanoic acid |
| 39. | 4-(5-(3-((2-(3-carboxy-3-methylbutanoyl)-6-methoxybenzo[b]thiophen-5-yl)oxy)propoxy)-6-me thoxyisoindolin-2-yl)-2,2-dimethyl-4-oxobutanoic acid |
| 43. | (S)-4-(5-(3-((2-((S)-3-carboxybutanoyl)-4-fluoro-6-methoxybenzo[b]thiophen-5-yl)oxy)propoxy )-6-methoxyisoindolin-2-yl)-2-methyl-4-oxobutanoic acid |

(continued)

| | |
|---|---|
| 44. | (S)-4-(5-(3-((2-((S)-3-carboxybutanoyl)-4-chloro-6-methoxybenzo[b]thiophen-5-yl)oxy)propoxy )-6-methoxyisoindolin-2-yl)-2-methyl-4-oxobutanoic acid |
| 45. | (S)-4-(5-(3-((4-bromo-2-((S)-3-carboxybutanoyl)-6-methoxybenzo[b]thiophen-5-yl)oxy)propoxy )-6-methoxyisoindolin-2-yl)-2-methyl-4-oxobutanoic acid |
| 47. | (S)-4-(5-(3-((2-((S)-3-carboxybutanoyl)-6-hydroxybenzo[b]thiophen-5-yl)oxy)propoxy)-6-meth oxyisoindolin-2-yl)-2-methyl-4-oxobutanoic acid |
| 48. | (S)-4-(5-(3-((2-((S)-3-carboxybutanoyl)-4,7-difluoro-6-methoxybenzo[b]thiophen-5-yl)oxy)prop oxy)-6-methoxyisoindolin-2-yl)-2-methyl-4-oxobutanoic acid |
| 49. | (S)-4-(5-(3-((2-((S)-3-carboxybutanoyl)-4,7-dichloro-6-methoxybenzo[b]thiophen-5-yl)oxy)prop oxy)-6-methoxyisoindolin-2-yl)-2-methyl-4-oxobutanoic acid |
| 51. | (S)-4-(5-(3-((2-((S)-3-carboxybutanoyl)-4-fluoro-6-methoxyisoindolin-5-yl)oxy)propoxy)-6-met hoxybenzo [b]thiophen-2-yl)-2-methyl-4-oxobutanoic acid |
| 52. | (S)-4-(5-(3-((2-((S)-3-carboxybutanoyl)-4-chloro-6-methoxyisoindolin-5-yl)oxy)propoxy)-6-met hoxybenzo [b]thiophen-2-yl)-2-methyl-4-oxobutanoic acid |
| 53. | (S)-4-(5-(3-((4-bromo-2-((S)-3-carboxybutanoyl)-6-methoxyisoindolin-5-yl)oxy)propoxy)-6-met hoxybenzo [b]thiophen-2-yl)-2-methyl-4-oxobutanoic acid |
| 58. | (S)-4-(5-(3-((2-((S)-3-carboxybutanoyl)-4,7-difluoro-6-methoxyisoindolin-5-yl)oxy)propoxy)-6-methoxybenzo[b]thiophen-2-yl)-2-methyl-4-oxobutanoic acid |
| 59. | (S)-4-(5-(3-((2-((S)-3-carboxybutanoyl)-6-methoxybenzo[b]thiophen-5-yl)oxy)propoxy)-7-chlor o-4-fluoro-6-methoxyisoindolin-2-yl)-2-methyl-4-oxobutanoic acid |
| 60. | (S)-4-(5-(3-((2-((S)-3-carboxybutanoyl)-4,7-dichloro-6-methoxyisoindolin-5-yl)oxy)propoxy)-6-methoxybenzo[b]thiophen-2-yl)-2-methyl-4-oxobutanoic acid |
| 61. | (S)-4-(5-(3-((2-((S)-3-carboxybutanoyl)-4-chloro-6-methoxybenzo[b]thiophen-5-yl)oxy)propoxy )-4-fluoro-6-methoxyisoindolin-2-yl)-2-methyl-4-oxobutanoic acid |
| 62. | (S)-4-(5-(3-((2-((S)-3-carboxybutanoyl)-4-chloro-6-methoxyisoindolin-5-yl)oxy)propoxy)-4-fluo ro-6-methoxybenzo[b]thiophen-2-yl)-2-methyl-4-oxobutanoic acid |
| 63. | (S)-4-(5-(3-((2-((S)-3-carboxybutanoyl)-4-chloro-6-methoxybenzo[b]thiophen-5-yl)oxy)propoxy )-4-chloro-6-methoxyisoindolin-2-yl)-2-methyl-4-oxobutanoic acid |
| 67. | (S)-4-(5-(3-((2-((S)-3-carboxybutanoyl)-4,7-difluoro-6-methoxyisoindolin-5-yl)oxy)propoxy)-7-chloro-6-methoxybenzo[b]thiophen-2-yl)-2-methyl-4-oxobutanoic acid |
| 68. | (S)-4-(5-(3-((2-((S)-3-carboxybutanoyl)-4,7-difluoro-6-methoxyisoindolin-5-yl)oxy)propoxy)-7-fluoro-6-methoxybenzo[b]thiophen-2-yl)-2-methyl-4-oxobutanoic acid |
| 69. | (S)-4-(5-(3-((2-((S)-3-carboxybutanoyl)-4-chloro-7-fluoro-6-methoxyisoindolin-5-yl)oxy)propox y)-7-fluoro-6-methoxybenzo[b]thiophen-2-yl)-2-methyl-4-oxobutanoic acid |
| 70. | (S)-4-(6-(3-((2-((S)-3-carboxybutanoyl)-4,7-difluoro-6-methoxybenzo[b]thiophen-5-yl)oxy)prop oxy)-4-fluoro-5-methoxyisoindolin-2-yl)-2-methyl-4-oxobutanoic acid |
| 71. | (S)-4-(6-(3-((2-((S)-3-carboxybutanoyl)-7-chloro-4-fluoro-6-methoxybenzo[b]thiophen-5-yl)oxy )propoxy)-4-fluoro-5-methoxyisoindolin-2-yl)-2-methyl-4-oxobutanoic acid |
| 72. | (S)-4-(6-(3-((2-((S)-3-carboxybutanoyl)-4,7-dichloro-6-methoxybenzo[b]thiophen-5-yl)oxy)prop oxy)-4-fluoro-5-methoxyisoindolin-2-yl)-2-methyl-4-oxobutanoic acid |
| 73. | (S)-4-(5-(3-((2-((S)-3-carboxybutanoyl)-4-chloro-7-fluoro-6-methoxyisoindolin-5-yl)oxy)propox y)-7-chloro-6-methoxybenzo[b]thiophen-2-yl)-2-methyl-4-oxobutanoic acid |
| 74. | (S)-4-(5-(3-((2-((S)-3-carboxybutanoyl)-4,7-dichloro-6-methoxyisoindolin-5-yl)oxy)propoxy)-7-fluoro-6-methoxybenzo[b]thiophen-2-yl)-2-methyl-4-oxobutanoic acid |
| 75. | (S)-4-(5-(3-((2-((S)-3-carboxybutanoyl)-7-chloro-4-fluoro-6-methoxyisoindolin-5-yl)oxy)propox y)-7-fluoro-6-methoxybenzo[b]thiophen-2-yl)-2-methyl-4-oxobutanoic acid |

(continued)

| | |
|---|---|
| 76. | (S)-4-(6-(3-((2-((S)-3-carboxybutanoyl)-4,7-difluoro-6-methoxybenzo[b]thiophen-5-yl)oxy)prop oxy)-4-chloro-5-methoxyisoindolin-2-yl)-2-methyl-4-oxobutanoic acid |
| 77. | (S)-4-(6-(3-((2-((S)-3-carboxybutanoyl)-4-chloro-7-fluoro-6-methoxybenzo[b]thiophen-5-yl)oxy )propoxy)-4-chloro-5-methoxyisoindolin-2-yl)-2-methyl-4-oxobutanoic acid |
| 78. | (S)-4-(5-(3-((2-((S)-3-carboxybutanoyl)-4-fluoro-6-hydroxyisoindolin-5-yl)oxy)propoxy)-4-fluo ro-6-methoxybenzo[b]thiophen-2-yl)-2-methyl-4-oxobutanoic acid |
| 79. | (S)-4-(5-(3-((2-((S)-3-carboxybutanoyl)-4-chloro-6-methoxybenzo[b]thiophen-5-yl)oxy)propoxy )-4-fluoro-6-hydroxyisoindolin-2-yl)-2-methyl-4-oxobutanoic acid |
| 80. | (S)-4-(5-(3-((2-((S)-3-carboxybutanoyl)-4-chloro-6-hydroxyisoindolin-5-yl)oxy)propoxy)-4-fluo ro-6-methoxybenzo[b]thiophen-2-yl)-2-methyl-4-oxobutanoic acid |
| 81. | (S)-4-(5-(3-((2-((S)-3-carboxybutanoyl)-4-chloro-6-hydroxyisoindolin-5-yl)oxy)propoxy)-4-chlo ro-6-methoxybenzo[b]thiophen-2-yl)-2-methyl-4-oxobutanoic acid |
| 86. | (S)-4-(5-(3-((2-((S)-3-carboxybutanoyl)-4,7-difluoro-6-methoxyisoindolin-5-yl)oxy)propoxy)-4-chloro-7-fluoro-6-methoxybenzo[b]thiophen-2-yl)-2-methyl-4-oxobutanoic acid |
| 87. | (S)-4-(5-(3-((2-((S)-3-carboxybutanoyl)-4-chloro-7-fluoro-6-methoxybenzo[b]thiophen-5-yl)oxy )propoxy)-4-chloro-7-fluoro-6-methoxyisoindolin-2-yl)-2-methyl-4-oxobutanoic acid |
| 88. | (S)-4-(5-(3-((2-((S)-3-carboxybutanoyl)-4,7-difluoro-6-methoxybenzo[b]thiophen-5-yl)oxy)prop oxy)-4,7-difluoro-6-methoxyisoindolin-2-yl)-2-methyl-4-oxobutanoic acid |
| 89. | (S)-4-(5-(3-((2-((S)-3-carboxybutanoyl)-4,7-difluoro-6-methoxyisoindolin-5-yl)oxy)propoxy)-7-chloro-4-fluoro-6-methoxybenzo[b]thiophen-2-yl)-2-methyl-4-oxobutanoic acid |
| 90. | (S)-4-(5-(3-((2-((S)-3-carboxybutanoyl)-4,7-dichloro-6-methoxybenzo[b]thiophen-5-yl)oxy)prop oxy)-4,7-difluoro-6-methoxyisoindolin-2-yl)-2-methyl-4-oxobutanoic acid |
| 91. | (S)-4-(5-(3-((2-((S)-3-carboxybutanoyl)-4,7-dichloro-6-methoxybenzo[b]thiophen-5-yl)oxy)prop oxy)-4-chloro-7-fluoro-6-methoxyisoindolin-2-yl)-2-methyl-4-oxobutanoic acid |
| 92. | (S)-4-(5-(3-((2-((S)-3-carboxybutanoyl)-4-chloro-7-fluoro-6-methoxyisoindolin-5-yl)oxy)propox y)-7-chloro-4-fluoro-6-methoxybenzo[b]thiophen-2-yl)-2-methyl-4-oxobutanoic acid |
| 93. | (S)-4-(5-(3-((2-((S)-3-carboxybutanoyl)-4,7-dichloro-6-methoxyisoindolin-5-yl)oxy)propoxy)-4-chloro-7-fluoro-6-methoxybenzo[b]thiophen-2-yl)-2-methyl-4-oxobutanoic acid |
| 94. | (S)-4-(5-(3-((2-((S)-3-carboxybutanoyl)-4-chloro-7-fluoro-6-methoxybenzo[b]thiophen-5-yl)oxy )propoxy)-7-chloro-4-fluoro-6-methoxyisoindolin-2-yl)-2-methyl-4-oxobutanoic acid |
| 104. | (1R,2R)-2-(5-(3-((2-((S)-3-carboxybutanoyl)-6-methoxyisoindolin-5-yl)oxy)propoxy)-6-methox ybenzo[b] thiophene-2-carbonyl)cyclopropane-1-carboxylic acid |
| 105. | (1R,2R)-2-(5-(3-((2-((1R,2R)-2-carboxycyclopropane-1-carbonyl)-6-methoxybenzo[b]thiophen-5-yl)oxy) propoxy)-6-methoxyisoindoline-2-carbonyl)cyclopropane-1-carboxylic acid |
| 106. | (1R,2R)-2-(5-(3-((2-((S)-3-carboxybutanoyl)-6-methoxybenzo[b]thiophen-5-yl)oxy)propoxy)-6-methoxyisoindoline-2-carbonyl)cyclopropane-1-carboxylic acid |
| 111. | (S)-4-(4-(3-((2-((S)-3-carboxybutanoyl)-6-methoxybenzo[b]thiophen-5-yl)oxy)propoxy)-6-meth oxyisoindolin-2-yl)-2-methyl-4-oxobutanoic acid |
| 112. | (S)-4-(5-(3-((2-((S)-3-carboxybutanoyl)-6-methoxybenzo[b]thiophen-4-yl)oxy)propoxy)-6-meth oxyisoindolin-2-yl)-2-methyl-4-oxobutanoic acid |
| 123. | (S)-4-(5-(3-((6-bromo-2-((S)-3-carboxybutanoyl)benzo[b]thiophen-5-yl)oxy)propoxy)-6-methox yisoindolin-2-yl)-2-methyl-4-oxobutanoic acid |
| 130. | (S)-4-(5-(3-((6-bromo-2-((S)-3-carboxybutanoyl)isoindolin-5-yl)oxy)propoxy)-6-methoxybenzo[ b]thiophen-2-yl)-2-methyl-4-oxobutanoic acid |
| 146. | (S)-4-(5-(3-((2-((S)-3-carboxybutanoyl)-4-fluoro-6-hydroxybenzo[b]thiophen-5-yl)oxy)propoxy) -4-fluoro-6-methoxyisoindolin-2-yl)-2-methyl-4-oxobutanoic acid |

(continued)

| | |
|---|---|
| 147. | (S)-4-(5-(3-((2-((S)-3-carboxybutanoyl)-4-chloro-6-hydroxybenzo[b]thiophen-5-yl)oxy)propoxy )-4-fluoro-6-methoxyisoindolin-2-yl)-2-methyl-4-oxobutanoic acid |
| 148. | (S)-4-(5-(3-((2-((S)-3-carboxybutanoyl)-4-chloro-6-methoxyisoindolin-5-yl)oxy)propoxy)-4-fluo ro-6-hydroxybenzo[b]thiophen-2-yl)-2-methyl-4-oxobutanoic acid |
| 149. | (S)-4-(5-(3-((2-((S)-3-carboxybutanoyl)-4-chloro-6-hydroxybenzo[b]thiophen-5-yl)oxy)propoxy )-4-chloro-6-methoxyisoindolin-2-yl)-2-methyl-4-oxobutanoic acid |
| 150. | (S)-4-(6-(3-((2-((S)-3-carboxybutanoyl)-6-hydroxybenzo[b]thiophen-5-yl)oxy)propoxy)-4-fluoro -5-methoxyisoindolin-2-yl)-2-methyl-4-oxobutanoic acid |
| 151. | (S)-4-(6-(3-((2-((S)-3-carboxybutanoyl)-7-fluoro-6-hydroxybenzo[b]thiophen-5-yl)oxy)propoxy) -4-fluoro-5-methoxyisoindolin-2-yl)-2-methyl-4-oxobutanoic acid |
| 152. | (S)-4-(6-(3-((2-((S)-3-carboxybutanoyl)-7-chloro-6-hydroxybenzo[b]thiophen-5-yl)oxy)propoxy )-4-fluoro-5-methoxyisoindolin-2-yl)-2-methyl-4-oxobutanoic acid |
| 153. | (S)-4-(5-(3-((2-((S)-3-carboxybutanoyl)-7-chloro-6-methoxyisoindolin-5-yl)oxy)propoxy)-7-fluo ro-6-hydroxybenzo[b]thiophen-2-yl)-2-methyl-4-oxobutanoic acid |
| 154. | (S)-4-(5-(3-((2-((S)-3-carboxybutanoyl)-4,7-difluoro-6-methoxyisoindolin-5-yl)oxy)propoxy)-7-chloro-6-hydroxybenzo[b]thiophen-2-yl)-2-methyl-4-oxobutanoic acid |
| 155. | (S)-4-(5-(3-((2-((S)-3-carboxybutanoyl)-4,7-difluoro-6-methoxyisoindolin-5-yl)oxy)propoxy)-7-fluoro-6-hydroxybenzo[b]thiophen-2-yl)-2-methyl-4-oxobutanoic acid |
| 156. | (S)-4-(5-(3-((2-((S)-3-carboxybutanoyl)-4-chloro-7-fluoro-6-methoxyisoindolin-5-yl)oxy)propox y)-7-fluoro-6-hydroxybenzo[b]thiophen-2-yl)-2-methyl-4-oxobutanoic acid |
| 157. | (S)-4-(6-(3-((2-((S)-3-carboxybutanoyl)-4,7-difluoro-6-hydroxybenzo[b]thiophen-5-yl)oxy)prop oxy)-4-fluoro-5-methoxyisoindolin-2-yl)-2-methyl-4-oxobutanoic acid |
| 158. | (S)-4-(6-(3-((2-((S)-3-carboxybutanoyl)-7-chloro-4-fluoro-6-hydroxybenzo[b]thiophen-5-yl)oxy )propoxy)-4-fluoro-5-methoxyisoindolin-2-yl)-2-methyl-4-oxobutanoic acid |
| 159. | (S)-4-(6-(3-((2-((S)-3-carboxybutanoyl)-4,7-dichloro-6-hydroxybenzo[b]thiophen-5-yl)oxy)prop oxy)-4-fluoro-5-methoxyisoindolin-2-yl)-2-methyl-4-oxobutanoic acid |

In some embodiments, the compound is an isotopic derivative (e.g., isotopically labeled compound) of any one of the compounds of the formulae disclosed herein.

In some embodiments, the compound is an isotopic derivative of any one of the compounds described in Table 1 and pharmaceutically acceptable salts thereof.

In some embodiments, the compound is an isotopic derivative of any one of the compounds described in Table 1 and pharmaceutically acceptable salts thereof.

In some embodiments, the isotopic derivative is a deuterium labeled compound.

In some embodiments, the compound is a deuterium labeled compound of any one of the compounds described in Table 1 and pharmaceutically acceptable salts thereof.

In some embodiments, the compound is a deuterium labeled compound of any one of the compounds described in Table 1 and pharmaceutically acceptable salts thereof.

METHODS OF PREPRATION

The compounds of the present invention can be prepared in a number ways well known to one skilled in the art of organic synthesis using the methods described below or variations thereon as appreciated by those skilled in the art.

The methods of synthesis described herein after are intended as an illustration of the invention. Where the preparation of starting compounds is not described, they are commercially obtainable or may be prepared analogously to known compounds or methods described herein. Compounds of any of the formulae desctribed herein may be synthesized by reference to methods illustrated in the following schemes. As shown herein, the end compound is a product having the same structural formula depicted as any of formulaes. It will be understood that any compound of the formulaes may be prepared by the suitable selection of reagents with appropriate substitution. Solvents, temperature, pressures, and other reaction conditions may be readily selected by one of ordinary skill in the art. Protecting groups are manipulated according to standard methods of organic synthesis (T. W. Green and P. G. M. Wuts (1999) Protective Groups

in Organic Synthesis, 3th edition, John Wiley & Sons). These groups are removed at certain stage of the compound synthesis using the methods that are apparent to those skilled in the art.

By way of example, a suitable protecting group for an amino or alkylamino group is, for example, an acyl group, for example an alkanoyl group such as acetyl, an alkoxycarbonyl group, for example a methoxycarbonyl, ethoxycarbonyl, or t-butoxycarbonyl group, an arylmethoxycarbonyl group, for example benzyloxycarbonyl, or an aroyl group, for example benzoyl. The deprotection conditions for the above protecting groups necessarily vary with the choice of protecting group. Thus, for example, an acyl group such as an alkanoyl or alkoxycarbonyl group or an aroyl group may be removed by, for example, hydrolysis with a suitable base such as an alkali metal hydroxide, for example lithium or sodium hydroxide. Alternatively an acyl group such as a tert-butoxycarbonyl group may be removed, for example, by treatment with a suitable acid as hydrochloric, sulfuric or phosphoric acid or trifluoroacetic acid and an arylmethoxycarbonyl group such as a benzyloxycarbonyl group may be removed, for example, by hydrogenation over a catalyst such as palladium on carbon, or by treatment with a Lewis acid for example boron tris(trifluoroacetate). A suitable alternative protecting group for a primary amino group is, for example, a phthaloyl group which may be removed by treatment with an alkylamine, for example dimethylaminopropylamine, or with hydrazine.

A suitable protecting group for a hydroxy group is, for example, an acyl group, for example an alkanoyl group such as acetyl, an aroyl group, for example benzoyl, or an arylmethyl group, for example benzyl. The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium, sodium hydroxide or ammonia. Alternatively an arylmethyl group such as a benzyl group may be removed, for example, by hydrogenation over a catalyst such as palladium on carbon.

A suitable protecting group for a carboxy group is, for example, an esterifying group, for example a methyl or an ethyl group which may be removed, for example, by hydrolysis with a base such as sodium hydroxide, or for example a tert-butyl group which may be removed, for example, by treatment with an acid, for example an organic acid such as trifluoroacetic acid, or for example a benzyl group which may be removed, for example, by hydrogenation over a catalyst such as palladium on carbon.

Once a compound of the present disclosure has been synthesized by any one of the processes defined herein, the processes may then further comprise the additional steps of: (i) removing any protecting groups present; (ii) converting the compound of the present disclosure into another compound of the present disclosure; (iii) forming a pharmaceutically acceptable salt, hydrate thereof.

For illustrative purposes, Schemes 1 and 2 show a general synthetic method for preparing the compounds described herein. For a more detailed description of the individual reaction steps, see the Examples section below. Those skilled in the art will appreciate that other synthetic routes may be used to synthesize the compounds. In addition, many of the compounds prepared by the methods described below can be further modified in light of this disclosure using conventional chemistry well known those skilled in the art.

General routes to compounds illustrated in the invention is described in Scheme 1 and 2, where the W, $R_1$, $X_1$, $X_2$, $X_3$, $R_2$, $R_3$, and $R_4$, etc. substituents are defined previously in the text or a functional group that can be converted to the desired final substituent.

In the following Methods and Schemes, LG represents a leaving group, which may be a halide or triflate group. The variables included in the Methods and Schemes have the meanings provided; exemplary catalysts are defined in the Abbreviations (below).

Scheme 1:

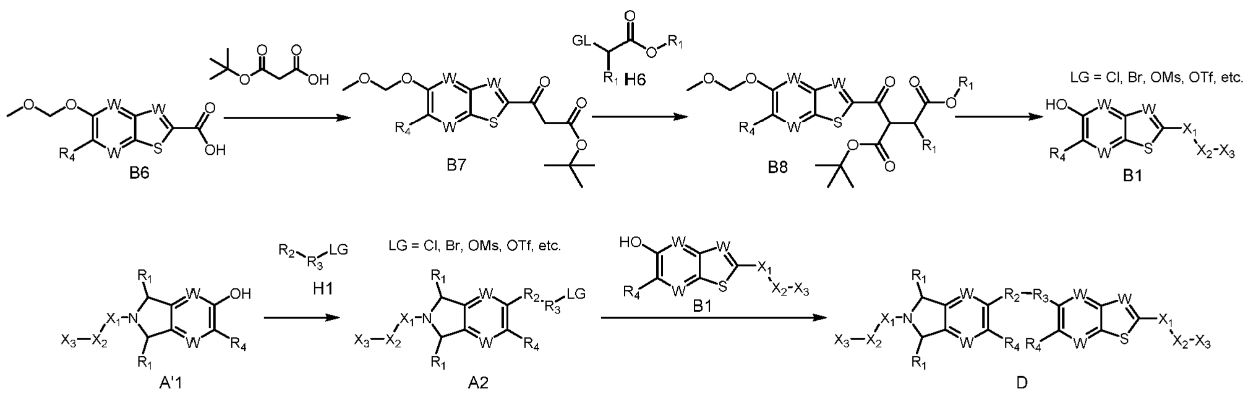


B6 reacts with 3-(tert-butoxy)-3-oxopropanoic acid in the presence of CDI, $MgCl_2$ and basic conditions to afford B7. B7 reacts with H6 in the presence of LDA or $K_2CO_3$ to afford B8. B8 reacts with TFA to obtain B1.

A'1 and H1 with an substitution reaction under basic conditions to afford A2.

A2 with B1 with an substitution reaction under basic conditions to afford D.

Scheme 2:

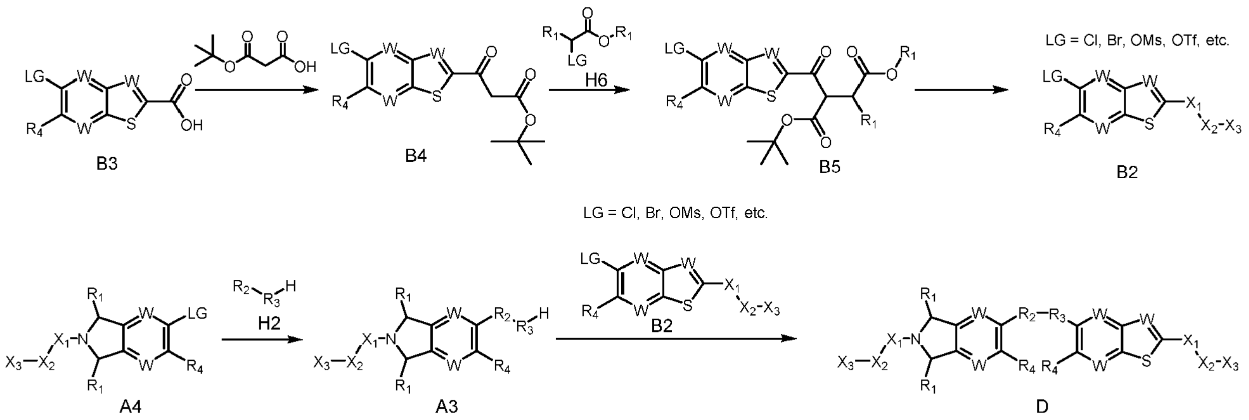


B3 reacts with 3-(tert-butoxy)-3-oxopropanoic acid in the presence of CDI, $MgCl_2$ and basic conditions to afford B4. B4 reacts with H6 in the presence of basic conditions to afford B5. B5 reacts with TFA to remove tert butyl to obtain B2.

A4 reacts with h2 in the presence of NaH to afford A3.

A3 reacts with B2 in the presence of a palladium catalyst to afford D.

The present invention also provides a pharmaceutical composition comprising at least one compound or pharmaceutically acceptable salt thereof of Formula I-1 and at least one pharmaceutically acceptable carrier. Furthermore, in the composition, the said compound or pharmaceutically acceptable salt thereof of Formula I-1 is in a weight ratio to the said carrier within the range from about 0.0001 to about 10.

The pharmaceutical composition of the this invention may further conprise at least one of additional active agents selected from STING agonist compounds, anti-viral compounds, antigens, adjuvants, CTLA-4 and PD-1 pathway antagonists and other immunomodulatory agents, lipids, liposomes, peptides, anti-cancer agents, and chemotherapeutic agents, etc.

The present invention additionally provided using at least one compound or pharmaceutically acceptable salt thereof of Formula 1-1, or pharmaceutical composition described above, which is for the the manufacture of a medicament.

At least one compound or pharmaceutically acceptable salt thereof of Formula 1-1, and/or a pharmaceutical composition described herein, which is for use in therapy.

At least one compound or pharmaceutically acceptable salt thereof of Formula 1-1, and/or a pharmaceutical composition described herein, which is for use in inducing an immune response , inducing STING-dependent type I interferon production , inducing a STING-dependent cytokine production, or treating a cell proliferation disorder in a subject.

In some embodiments, the cell proliferation disorder is cancer, cancer metastasis, cardiovascular disease, an immunological disorder, fibrosis, or an ocular disorder.

At least one compound or pharmaceutically acceptable salt thereof of Formula 1-1, and/or a pharmaceutical composition described herein, which is for use as a STING agonists.

At least one compound or pharmaceutically acceptable salt thereof of Formula 1-1, and/or a pharmaceutical composition described herein, which is for use as a medicament.

The present disclosure additionally describes a method of inducing an immune response in a subject, said method comprising administering to the patient a therapeutically effective amount of at least one compound of Formula I, I-1, II,III,IV or V or pharmaceutically acceptable salt thereof, or the pharmaceutical composition described above.

The present disclosure additionally describes a method of inducing a STING-dependent type I interferon production in a subject, said method comprising administering to the patient a therapeutically effective amount of at least one compound of Formula I, I-1, II,III,IV or V or pharmaceutically acceptable salt thereof, or the pharmaceutical composition described above.

The present disclosure additionally describes a method of inducing a STING-dependent cytokine production in a subject, said method comprising administering to the patient a therapeutically effective amount of at least one compound of Formula I, I-1, II,III,IV or V or pharmaceutically acceptable salt thereof, or the pharmaceutical composition described above.

The present disclosure additionally describes a method of treating a cell proliferation disorder in a subject, said

method comprising administering to the patient a therapeutically effective amount of at least one compound of Formula I, I-1, II,III,IV or V or pharmaceutically acceptable salt thereof, or the pharmaceutical composition described above.

In some embodiments, the cell proliferation disorder is cancer, cancer metastasis, cardiovascular disease, an immunological disorder, fibrosis, or an ocular disorder.

The compounds disclosed herein may be STING agonists. These compounds are potentially useful in treating diseases or disorders including cell proliferation disorders. Cell-proliferation disorders include cancers, benign papillomatosis, gestational trophoblastic diseases, and benign neoplastic diseases, such as skin papilloma (warts) and genital papilloma.

In specific embodiments, the disease or disorder to be treated is a cell proliferation disorder. In certain embodiments, the cell proliferation disorder is cancer. In particular embodiments, the cancer is selected from brain and spinal cancers, cancers of the head and neck, leukemia and cancers of the blood, skin cancers, cancers of the reproductive system, cancers of the gastrointestinal system, liver and bile duct cancers, kidney and bladder cancers, bone cancers, lung cancers, malignant mesothelioma, sarcomas, lymphomas, glandular cancers, thyroid cancers, heart tumors, germ cell tumors, malignant neuroendocrine (carcinoid) tumors, midline tract cancers, and cancers of unknown primary (i.e., cancers in which a metastasized cancer is found but the original cancer site is not known). In particular embodiments, the cancer is present in an adult patient; in additional embodiments, the cancer is present in a pediatric patient. In particular embodiments, the cancer is AIDS-related.

In specific embodiments, the cancer is selected from brain and spinal cancers. In particular embodiments, the cancer is selected from the group consisting of anaplastic astrocytomas, glioblastomas, astrocytomas, and estheosioneuroblastomas (also known as olfactory blastomas). In particular embodiments, the brain cancer is selected from the group consisting of astrocytic tumor (e.g., pilocytic astrocytoma, subependymal giant-cell astrocytoma, diffuse astrocytoma, pleomorphic xanthoastrocytoma, anaplastic astrocytoma, astrocytoma, giant cell glioblastoma, glioblastoma, secondary glioblastoma, primary adult glioblastoma, and primary pediatric glioblastoma), oligodendroglial tumor (e.g., oligodendroglioma, and anaplastic oligodendroglioma), oligoastrocytic tumor (e.g., oligoastrocytoma, and anaplastic oligoastrocytoma), ependymoma (e.g., myxopapillary ependymoma, and anaplastic ependymoma); medulloblastoma, primitive neuroectodermal tumor, schwannoma, meningioma, atypical meningioma, anaplastic meningioma, pituitary adenoma, brain stem glioma, cerebellar astrocytoma, cerebral astorcytoma/malignant glioma, visual pathway and hypothalmic glioma, and primary central nervous system lymphoma. In specific instances of these embodiments, the brain cancer is selected from the group consisting of glioma, glioblastoma multiforme, paraganglioma, and suprantentorial primordial neuroectodermal tumors (sP ET).

In specific embodiments, the cancer is selected from cancers of the head and neck, including nasopharyngeal cancers, nasal cavity and paranasal sinus cancers, hypopharyngeal cancers, oral cavity cancers (e.g., squamous cell carcinomas, lymphomas, and sarcomas), lip cancers, oropharyngeal cancers, salivary gland tumors, cancers of the larynx (e.g., laryngeal squamous cell carcinomas, rhabdomyosarcomas), and cancers of the eye or ocular cancers. In particular embodiments, the ocular cancer is selected from the group consisting of intraocular melanoma and retinoblastoma.

In specific embodiments, the cancer is selected from leukemia and cancers of the blood. In particular embodiments, the cancer is selected from the group consisting of myeloproliferative neoplasms, myelodysplastic syndromes, myelodysplastic/myeloproliferative neoplasms, acute myeloid leukemia (AML), myelodysplastic syndrome (MDS), chronic myelogenous leukemia (CML), myeloproliferative neoplasm (MPN), post-MPN AML, post- MDS AML, del(5q)-associated high risk MDS or AML, blast-phase chronic myelogenous leukemia, angioimmunoblastic lymphoma, acute lymphoblastic leukemia, Langerans cell histiocytosis, hairy cell leukemia, and plasma cell neoplasms including plasmacytomas and multiple myelomas. Leukemias referenced herein may be acute or chronic.

In specific embodiments, the cancer is selected from skin cancers. In particular embodiments, the skin cancer is selected from the group consisting of melanoma, squamous cell cancers, and basal cell cancers.

In specific embodiments, the cancer is selected from cancers of the reproductive system. In particular embodiments, the cancer is selected from the group consisting of breast cancers, cervical cancers, vaginal cancers, ovarian cancers, prostate cancers, penile cancers, and testicular cancers. In specific instances of these embodiments, the cancer is a breast cancer selected from the group consisting of ductal carcinomas and phyllodes tumors. In specific instances of these embodiments, the breast cancer may be male breast cancer or female breast cancer. In specific instances of these embodiments, the cancer is a cervical cancer selected from the group consisting of squamous cell carcinomas and adenocarcinomas. In specific instances of these embodiments, the cancer is an ovarian cancer selected from the group consisting of epithelial cancers.

In specific embodiments, the cancer is selected from cancers of the gastrointestinal system. In particular embodiments, the cancer is selected from the group consisting of esophageal cancers, gastric cancers (also known as stomach cancers), gastrointestinal carcinoid tumors, pancreatic cancers, gallbladder cancers, colorectal cancers, and anal cancer. In instances of these embodiments, the cancer is selected from the group consisting of esophageal squamous cell carcinomas, esophageal adenocarcinomas, gastric adenocarcinomas, gastrointestinal carcinoid tumors, gastrointestinal stromal tumors, gastric lymphomas, gastrointestinal lymphomas, solid pseudopapillary tumors of the

pancreas, pancreatoblastoma, islet cell tumors, pancreatic carcinomas including acinar cell carcinomas and ductal adenocarcinomas, gallbladder adenocarcinomas, colorectal adenocarcinomas, and anal squamous cell carcinomas.

In specific embodiments, the cancer is selected from liver and bile duct cancers. In particular embodiments, the cancer is liver cancer (also known as hepatocellular carcinoma). In particular embodiments, the cancer is bile duct cancer (also known as cholangiocarcinoma); in instances of these embodiments, the bile duct cancer is selected from the group consisting of intrahepatic cholangiocarcinoma and extrahepatic cholangiocarcinoma.

In specific embodiments, the cancer is selected from kidney and bladder cancers. In particular embodiments, the cancer is a kidney cancer selected from the group consisting of renal cell cancer, Wilms tumors, and transitional cell cancers. In particular embodiments, the cancer is a bladder cancer selected from the group consisting of urethelial carcinoma (a transitional cell carcinoma), squamous cell carcinomas, and adenocarcinomas.

In specific embodiments, the cancer is selected from bone cancers. In particular embodiments, the bone cancer is selected from the group consisting of osteosarcoma, malignant fibrous histiocytoma of bone, Ewing sarcoma, chordoma (cancer of the bone along the spine).

In specific embodiments, the cancer is selected from lung cancers. In particular embodiments, the lung cancer is selected from the group consisting of non-small cell lung cancer, small cell lung cancers, bronchial tumors, and pleuropulmonary blastomas.

In specific embodiments, the cancer is selected from malignant mesothelioma. In particular embodiments, the cancer is selected from the group consisting of epithelial mesothelioma and sarcomatoids.

In specific embodiments, the cancer is selected from sarcomas. In particular embodiments, the sarcoma is selected from the group consisting of central chondrosarcoma, central and periosteal chondroma, fibrosarcoma, clear cell sarcoma of tendon sheaths, and Kaposi's sarcoma.

In specific embodiments, the cancer is selected from lymphomas. In particular embodiments, the cancer is selected from the group consisting of Hodgkin lymphoma (e.g., Reed-Sternberg cells), non-Hodgkin lymphoma (e.g., diffuse large B-cell lymphoma, follicular lymphoma, mycosis fungoides, Sezary syndrome, primary central nervous system lymphoma), cutaneous T-cell lymphomas, primary central nervous system lymphomas.

In specific embodiments, the cancer is selected from glandular cancers. In particular embodiments, the cancer is selected from the group consisting of adrenocortical cancer (also known as adrenocortical carcinoma or adrenal cortical carcinoma), pheochromocytomas, paragangliomas, pituitary tumors, thymoma, and thymic carcinomas.

In specific embodiments, the cancer is selected from thyroid cancers. In particular embodiments, the thyroid cancer is selected from the group consisting of medullary thyroid carcinomas, papillary thyroid carcinomas, and follicular thyroid carcinomas.

In specific embodiments, the cancer is selected from germ cell tumors. In particular embodiments, the cancer is selected from the group consisting of malignant extracranial germ cell tumors and malignant extragonadal germ cell tumors. In specific instances of these embodiments, the malignant extragonadal germ cell tumors are selected from the group consisting of nonseminomas and seminomas.

In specific embodiments, the cancer is selected from heart tumors. In particular embodiments, the heart tumor is selected from the group consisting of malignant teratoma, lymphoma, rhabdomyosacroma, angiosarcoma, chondrosarcoma, infantile fibrosarcoma, and synovial sarcoma.

In specific embodiments, the cell-proliferation disorder is selected from benign papillomatosis, benign neoplastic diseases and gestational trophoblastic diseases. In particular embodiments, the benign neoplastic disease is selected from skin papilloma (warts) and genital papilloma. In particular embodiments, the gestational trophoblastic disease is selected from the group consisting of hydatidiform moles, and gestational trophoblastic neoplasia (e.g., invasive moles, choriocarcinomas, placental -site trophoblastic tumors, and epithelioid trophoblastic tumors).

As used herein, the terms "treatment" and "treating" refer to all processes in which there may be a slowing, interrupting, arresting, controlling, or stopping of the progression of a disease or disorder described herein. The terms do not necessarily indicate a total elimination of all disease or disorder symptoms.

The terms "administration of and or "administering" a compound should be understood to include providing a compound described herein, or a pharmaceutically acceptable salt thereof, and compositions of the foregoing to a subject.

The amount of a compound administered to a subject is an amount sufficient to induce an immune response and/or to induce STING-dependent type I interferon production in the subject. In an embodiment, the amount of a compound can be an "effective amount" or "therapeutically effective amount," such that the subject compound is administered in an amount that will elicit, respectively, a biological or medical (i.e., intended to treat) response of a tissue, system, animal, or human that is being sought by a researcher, veterinarian, medical doctor, or other clinician. An effective amount does not necessarily include considerations of toxicity and safety related to the administration of a compound.

An effective amount of a compound will vary with the particular compound chosen (e.g., considering the potency, efficacy, and/or half-life of the compound); the route of administration chosen; the condition being treated; the severity of the condition being treated; the age, size, weight, and physical condition of the subject being treated; the medical history of

the subject being treated; the duration of the treatment; the nature of a concurrent therapy; the desired therapeutic effect; and like factors and can be routinely determined by the skilled artisan.

The term "subject" (alternatively referred to herein as "patient") as used herein refers to an animal, preferably a mammal, most preferably a human, who has been the object of treatment, observation or experiment.

As used herein, the term "immune response" relates to any one or more of the following: specific immune response, non-specific immune response, both specific and nonspecific response, innate response, primary immune response, adaptive immunity, secondary immune response, memory immune response, immune cell activation, immune cell proliferation, immune cell differentiation, and cytokine expression. In certain embodiments, a compound of general Formula 1-1, or a pharmaceutically acceptable salt of the foregoing, is administered in conjunction with one or more additional therapeutic agents including anti -viral compounds, vaccines intended to stimulate an immune response to one or more predetermined antigens, adjuvants, CTLA-4 and PD-1 pathway antagonists and other immunomodulatory agents, lipids, liposomes, peptides, anti-cancer agents, and chemotherapeutic agents, etc.

The term "halogen", as used herein, unless otherwise indicated, means fluoro, chloro, bromo or iodo. The preferred halogen groups include F, Cl and Br. The terms "halo$C_{1-6}$alkyl", "halo$C_{2-6}$alkenyl", "halo$C_{2-6}$alkynyl" and "halo$C_{1-6}$alkoxy" mean a $C_{1-6}$alkyl, $C_{2-6}$alkenyl, $C_{2-6}$alkynyl or $C_{1-6}$alkoxy in which one or more (in particular, 1, 2 or 3) hydrogen atoms have been replaced by halogen atoms, especially fluorine or chlorine atoms. In some embodiment, preferred are fluoro$C_{1-6}$alkyl, fluoro$C_{2-6}$alkenyl, fluoro$C_{2-6}$alkynyl and fluoro$C_{1-6}$alkoxy groups, in particular fluoro$C_{1-3}$alkyl, for example, $CF_3$, $CHF_2$, $CH_2F$, $CH_2CH_2F$, $CH_2CHF_2$, $CH_2CF_3$ and fluoro$C_{1-3}$alkoxy groups, for example, $OCF_3$, $OCHF_2$, $OCH_2F$, $OCH_2CH_2F$, $OCH_2CHF_2$ or $OCH_2CF_3$, and most especially $CF_3$, $OCF_3$ and $OCHF_2$.

As used herein, unless otherwise indicated, alkyl includes saturated monovalent hydrocarbon radicals having straight, branched or cyclic moieties. For example, alkyl radicals include methyl, ethyl, propyl, isopropyl, cyclcopropyl, n-butyl, isobutyl, sec-butyl, t-butyl, cyclcobutyl, n-pentyl, 3- (2-methyl) butyl, 2-pentyl, 2-methylbutyl, neopentyl, cyclcopentyl, n-hexyl, 2-hexyl, 2-methylpentyl and cyclohexyl. Similary, $C_{1-8}$, as in $C_{1-8}$alkyl is defined to identify the group as having 1, 2, 3, 4, 5, 6, 7 or 8 carbon atoms in a linear or branched arrangement.

Alkylene means a difunctional group obtained by removal of a hydrogen atom from an alkyl group that is defined above. For example, methylene (i.e., $-CH_2-$), ethylene (i.e., $-CH_2-CH_2-$ or $-CH(CH_3)-$) and propylene (i.e., $-CH_2-CH_2-CH_2-$, $-CH(-CH_2-CH_3)-$ or $-CH_2-CH(CH_3)-$).

As used herein, the term "alkenyl" refers to a monovalent straight or branched chain, unsaturated aliphatic hydrocarbon radical having a number of carbon atoms in the specified range and including one or more double bonds.

As used herein, the term "alkenylene" refers to a bivalent straight chain, unsaturated aliphatic hydrocarbon radical having a number of carbon atoms in the specified range and including one or more double bonds.

As used herein, the term "alkynyl" refers to a monovalent straight or branched chain, unsaturated aliphatic hydrocarbon radical having a number of carbon atoms in the specified range and including one or more triple bonds.

As used herein, the term "alkynylene" refers to a bivalent straight chain, unsaturated aliphatic hydrocarbon radical having a number of carbon atoms in the specified range and including one or more triple bonds.

As used herein, the term "alkoxy" as used herein, alone or in combination, includes an alkyl group connected to the oxy connecting atom. The term "alkoxy" also includes alkyl ether groups, where the term 'alkyl' is defined above, and 'ether' means two alkyl groups with an oxygen atom between them. Examples of suitable alkoxy groups include methoxy, ethoxy, n-propoxy, i-propoxy, n-butoxy, s-butoxy, t-butoxy, methoxymethane (also referred to as 'dimethyl ether'), and methoxy ethane (also referred to as 'ethyl methyl ether').

The term "aryl", as used herein, unless otherwise indicated, by itself or as part of another substituent refers to a monocyclic or polycyclic aromatic hydrocarbon. The preferred aryls are mono cyclic or bicyclic 6-10 membered aromatic ring systems. Phenyl and naphthyl are preferred aryls. The most preferred aryl is phenyl.

The term "heterocyclic", "heterocyclyl", or "heterocyclic", as used herein, unless otherwise indicated, by itself or as part of another substituent refers to unsubstituted and substituted mono- or polycyclic non-aromatic, partially unsaturated or fully saturated ring system containing one or more heteroatoms. Preferred heteroatoms include N, O, and S, including N-oxides, sulfur oxides, and dioxides. Preferably the ring is three to eight membered and is either fully saturated or has one or more degrees of unsaturation. Multiple degrees of substitution, preferably one, two or three, are included within the present definition.

Examples of such heterocyclic groups include azetidinyl, pyrrolidinyl, piperidinyl, piperazinyl, oxopiperazinyl, oxopiperidinyl, oxoazepinyl, azepinyl, tetrahydrofuranyl, dioxolanyl, tetrahydroimidazolyl, tetrahydrothiazolyl, tetrahydrooxazolyl, tetrahydropyranyl, morpholinyl, thiomorpholinyl, thiamorpholinyl sulfoxide, thiamorpholinyl sulfone and oxadiazolyl.

The term "heteroaryl", as used herein, unless otherwise indicated, by itself or as part of another substituent refers to an aromatic ring system containing carbon(s) and at least one heteroatom. Heteroaryl may be monocyclic or polycyclic, substituted or unsubstituted. A monocyclic heteroaryl group may have 1 to 4 heteroatoms in the ring, while a polycyclic heteroaryl may contain 1 to 10 hetero atoms. A polycyclic heteroaryl ring may contain fused, spiro or bridged ring junction, for example, bycyclic heteroaryl is a polycyclic heteroaryl. Bicyclic heteroaryl rings may contain from 8 to 12 member

atoms. Monocyclic heteroaryl rings may contain from 5 to 8 member atoms (cabons and heteroatoms). Examples of heteroaryl groups include thienyl, furanyl, imidazolyl, isoxazolyl, oxazolyl, pyrazolyl, pyrrolyl, thiazolyl, thiadiazolyl, triazolyl, pyridyl, pyridazinyl, indolyl, azaindolyl, indazolyl, benzimidazolyl, benzofuranyl, benzothienyl, benzisoxazolyl, benzoxazolyl, benzopyrazolyl, benzothiazolyl, benzothiadiazolyl, benzotriazolyl adeninyl, quinolinyl or isoquinolinyl.

The term "carbocyclic" refers to a substituted or unsubstituted monocyclic ring, bicyclic ring, bridged ring, fused ring, sipiro ring non-aromatic ring system onle containing carbon atoms. Examplary "carbocyclic" groups include cyclopropyl, cyclobutyl, cyclopentyl, cyclohexyl and so on.

The term "cycloalkyl" as used herein, unless otherwise indicated, by itself or as part of another substituent refers to a substituted or unsubstituted monocyclic, bicyclic or polycyclic non-aromatic saturated or partially unsatureated hydrocarbon group, which optionally includes an alkylene linker through which the cycloalkyl may be attached. Examplary "cycloalkyl" groups includes cyclopropyl, cyclobutyl, cyclopentyl, cyclohexyl and so on.

The term "carbonyl", "-C=O", "C=O", "-CO", "-C(O) ", and "CO" refer to the group

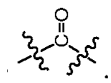

The term "oxo"refers to the radical =O.

Whenever the term "alkyl" or "aryl" or either of their prefix roots appear in a name of a substituent (e.g., aralky or dialkylamino) , unless otherwise indicated, by itself or as part of another substituent, it shall be interpreted as including those limitations given above for "alkyl" and "aryl". Designated numbers of carbon atoms (e.g., $C_{1-6}$) shall refer independently to the number of carbon atoms in an alkyl moiety or to the alkyl portion of a larger substituent in which alkyl appears as its prefix root.

As used herein, the term "fused ring" refers to a cyclic group formed by substituents on separate atoms in a straight or branched alkane, or to a cyclic group formed by substituents on separate atoms in another ring.

As used herein, the term "spirocycle" or "spirocyclic ring" refers to a pendant cyclic group formed by substituents on a single atom.

The term "ring systems" as used herein, unless otherwise indicated, include but not limite to a carbocyclic ring, a heterocyclic ring, a heteroaromatic ring, etc., may also include only a heterocyclic ring, and/or a heteroaromatic ring, specifically includes which rings need to be determined according to the context, but anyway the "ring systems" do not include the cycloalkyl based on a $C_{1-6}$ alkyl or $C_{1-3}$ alkyl ogroup, and do not include the cycloalkoxy based on a $C_{1-6}$ alkoxy or $C_{1-3}$ alkoxy group.

Unless expressly stated to the contrary, all ranges cited herein are inclusive; i.e., the range includes the values for the upper and lower limits of the range as well as all values in between. As an example, temperature ranges, percentages, ranges of equivalents described herein include the upper and lower limits of the range and any value in the continuum there between. Numerical values provided herein, and the use of the term "about", may include variations of $\pm$ 1%, $\pm$ 2%, $\pm$3%, $\pm$ 4%, $\pm$ 5%, $\pm$ 10%, $\pm$ 15%, and $\pm$ 20% and their numerical equivalents.

As used herein, the term "one or more" item includes a single item selected from the list as well as mixtures of two or more items selected from the list.

Wherein the term "substituted" refers to a group mentioned above in which one or more (preferably 1-6, more preferably 1-3) hydrogen atoms are each independently replaced with the same or different substituent(s). Typical substituents include X, $C_{1-6}$alkyl, $C_{1-6}$alkoxy, $C_{3-20}$ cycloalkyl, $-OR_{13}$, $SR_{13}$, =O, =S, $-C(O)R_{13}$, $-C(S)R_{13}$, $=NR_{13}$, $-C(O)OR_{13}$, $-C(S)OR_{13}$, $-NR_{13}R_{14}$, $-C(O)NR_{13}R_{14}$, cyano, nitro, $-S(O)_2R_{13}$, $-OS(O_2)OR_{13}$, $-OS(O)_2R_{13}$, or $-OP(O)(OR_{13})(OR_{14})$; wherein each X is independently a halogen (F, Cl, Br or I), and $R_{13}$ and $R_{14}$ is independently selected from -H, $C_{1-6}$ alkyl and $C_{1-6}$ haloalkyl. In some embodiments, the substituent(s) is independently selected from the group consisting of -F, -Cl, -Br, -I, -OH, trifluromethoxy, ethoxy, propyloxy, iso-propyloxy, n-butyloxy, isobutyloxy, t-butyloxy, $-SCH_3$ , $-SC_2H_5$ , formaldehyde group, $-C(OCH_3)$, cyano, nitro,$CF_3$ ,$-OCF_3$, amino, dimethylamino, methyl thio, sulfonyl and acetyl. Particularly preferred substituent(s) is -F, -Cl or -Br.

"$R_1$" "$X_1$" "$X_2$" or "$X_3$"of Formula I-1 can be the same or different.

Compounds described herein can exist in isotope-labeled or -enriched form containing one or more atoms having an atomic mass or mass number different from the atomic mass or mass number most abundantly found in nature. Isotopes can be radioactive or non-radioactive isotopes. Isotopes of atoms such as hydrogen, carbon, sulfur, fluorine, chlorine, and iodine include $^{2}H$, $^{3}H$, $^{13}C$, $^{14}C$, $^{15}N$, $^{18}O$, $^{35}S$, $^{18}F$, $^{36}Cl$, and $^{125}I$. Compounds that contain other isotopes of these and/or other atoms are within the scope of this invention. In some embodiments, one or more hydrogen atoms of any of the compounds described herein can be substituted with deuterium to provide the corresponding deterium-labeled or -enriched compounds.

Where the plural form (e.g. compounds, salts) is used, this includes the singular (e.g. a single compound, a single salt). "A compound" does not exclude that ( e.g. in a pharmaceutical formulation) more than one compound of the Formula

I-1 (or a salt thereof) is present, the "a" merely representing the indefinite article. "A" can thus preferably be read as "one or more", less preferably alternatively as "one".

The term "composition", as used herein, is intended to encompass a product comprising the specified ingredients in the specified amounts, as well as any product which results, directly or indirectly, from combinations of the specified ingredients in the specified amounts. Accordingly, pharmaceutical compositions containing the compounds of the present invention as the active ingredient as well as methods of preparing the instant compounds are also part of the present invention. Furthermore, some of the crystalline forms for the compounds may exist as polymorphs and as such are intended to be included in the present invention. In addition, some of the compounds may form solvates with water (i.e., hydrates) or common organic solvents and such solvates are also intended to be encompassed within the scope of this invention.

The compounds of the present invention may also be present in the form of pharmaceutically acceptable salts. For use in medicine, the salts of the compounds of this invention refer to non-toxic "pharmaceutically acceptable salts". The pharmaceutically acceptable salt forms include pharmaceutically acceptable acidic/anionic or basic/cationic salts. The pharmaceutically acceptable acidic/anionic salt generally takes a form in which the basic nitrogen is protonated with an inorganic or organic acid. Representative organic or inorganic acids include hydrochloric, hydrobromic, hydriodic, perchloric, sulfuric, nitric, phosphoric, acetic, propionic, glycolic, lactic, succinic, maleic, fumaric, malic, tartaric, citric, benzoic, mandelic, methanesulfonic, hydroxyethanesulfonic, benzenesulfonic, oxalic, pamoic, 2-naphthalenesulfonic, p-toluenesulfonic, cyclohexanesulfamic, salicylic, saccharinic or trifluoroacetic. Pharmaceutically acceptable basic/cationic salts include aluminum, calcium, chloroprocaine, choline, diethanolamine, ethylenediamine, lithium, magnesium, potassium, sodium and zinc.

It is intended that the definition of any substituent or variable at a particular location in a molecule be independent of its definitions elsewhere in that molecule. It is understood that substituents and substitution patterns on the compounds of this invention can be selected by one of ordinary skill in the art to provide compounds that are chemically stable and that can be readily synthesized by techniques know in the art as well as those methods set forth herein.

Compounds described herein, namely the compounds of Formula I-1 may contain asymmetrically substituted carbon atoms (or chiral centers) in the R or S configuration. The present invention includes racemic mixtures, relative and absolute stereoisomers, and mixtures of relative and absolute stereoisomers.

The compounds described herein, when specifically designated as the R- or S- isomer, either in a chemical name or in a drawing, should be understood as an enriched R-isomer or S-isomer, respectively. For example, in any of the embodiments described herein, such enriched R- or S-designated isomer can be substantially free (e.g., with less than 5%, less than 1%, or non-detectable, as determined by chiral HPLC) of the other isomer for the respective chiral center. The enriched R- or S-isomers can be prepared by methods exemplified in this application, such as by using a chiral auxiliary such as R- or S-tert-butylsulfinamide in the synthetic process. Other methods for prepaing the enriched R- or S-isomers herein include chiral HPLC purifications of a stereoisomeric mixture, such as a racemic mixture. General methods for separating stereoisomers (such as enantiomers and/or diastereomers) using HPLC are known in the art.

Compounds of Formula I-1 may have different stereoisomeric forms. For example, any asymmetric carbon atom may be present in the (R)-, (S)- or (R,S)-configuration, preferably in the (R)- or (S)-configuration. Substituents at a double bond or especially a ring may be present cis-( = Z-) or trans (= E-) form. The compounds may thus be present as mixtures of stereoisomers or preferably as pure stereoisomers, preferably as pure diastereomers or pure enantiomers.

The present invention includes compounds described can contain one or more asymmetric centers and may thus give rise to diastereomers and optical isomers. The present invention includes all such possible diastereomers as well as their racemic mixtures, their substantially pure resolved enantiomers, all possible geometric isomers, and pharmaceutically acceptable salts thereof.

The above Formula I-1 is shown without a definitive stereochemistry at certain positions. The present invention includes all stereoisomers of Formula I-1 and pharmaceutically acceptable salts thereof. Further, mixtures of stereoisomers as well as isolated specific stereoisomers are also included. During the course of the synthetic procedures used to prepare such compounds, or in using racemization or epimerization procedures known to those skilled in the art, the products of such procedures can be a mixture of stereoisomers.

When a tautomer of the compound of Formula I-1 exists, the present invention includes any possible tautomers and pharmaceutically acceptable salts thereof, and mixtures thereof, except where specifically stated otherwise.

When the compound of Formula I-1 and pharmaceutically acceptable salts thereof exist in the form of solvates or polymorphic forms, the present invention includes any possible solvates and polymorphic forms. A type of a solvent that forms the solvate is not particularly limited so long as the solvent is pharmacologically acceptable. For example, water, ethanol, propanol, acetone can be used.

The term "pharmaceutically acceptable salts" refers to salts prepared from pharmaceutically acceptable non-toxic bases or acids. When the compound of the present invention is acidic, its corresponding salt can be conveniently prepared from pharmaceutically acceptable non-toxic bases, including inorganic bases and organic bases. When the compound of the present invention is basic, its corresponding salt can be conveniently prepared from pharmaceutically

acceptable non-toxic acids, including inorganic and organic acids. Since the compounds of Formula I-1 are intended for pharmaceutical use they are preferably provided in substantially pure form, for example at least 60% pure, more suitably at least 75% pure, especially at least 98% pure (% are on a weight for weight basis).

The pharmaceutical compositions of the present invention comprise a compound represented by Formula I-1 (or a pharmaceutically acceptable salt thereof) as an active ingredient, a pharmaceutically acceptable carrier and optionally other therapeutic ingredients or adjuvants. The compositions include compositions suitable for oral, rectal, topical, and parenteral (including subcutaneous, intramuscular, and intravenous) administration, although the most suitable route in any given case will depend on the particular host, and nature and severity of the conditions for which the active ingredient is being administered. The pharmaceutical compositions may be conveniently presented in unit dosage form and prepared by any of the methods well known in the art of pharmacy.

In practice, the compounds represented by Formula 1-1, or pharmaceutically acceptable salts thereof, of this invention can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). Thus, the pharmaceutical compositions of the present invention can be presented as discrete units suitable for oral administration such as capsules, cachets or tablets each containing a predetermined amount of the active ingredient. Further, the compositions can be presented as a powder, as granules, as a solution, as a suspension in an aqueous liquid, as a non-aqueous liquid, as an oil-in-water emulsion, or as a water-in- oil liquid emulsion. In addition to the common dosage forms set out above, the compound represented by Formula 1-1, or a pharmaceutically acceptable salt thereof, may also be administered by controlled release means and/or delivery devices. The compositions may be prepared by any of the methods of pharmacy. In general, such methods include a step of bringing into association the active ingredient with the carrier that constitutes one or more necessary ingredients. In general, the compositions are prepared by uniformly and intimately admixing the active ingredient with liquid carriers or finely divided solid carriers or both. The product can then be conveniently shaped into the desired presentation.

Thus, the pharmaceutical compositions of this invention may include a pharmaceutically acceptable carrier, and a compound or a pharmaceutically acceptable salt of Formula 1-1. The compounds of Formula 1-1, or pharmaceutically acceptable salts thereof, can also be included in pharmaceutical compositions in combination with one or more additional therapeutically active agents.

The additional active agent(s) may be one or more agents selected from the group consisting of STING agonist compounds, anti-viral compounds, antigens, adjuvants, anti -cancer agents, CTLA-4, LAG-3 and PD-1 pathway antagonists, lipids, liposomes, peptides, cytotoxic agents, chemotherapeutic agents, immunomodulatory cell lines, checkpoint inhibitors, vascular endothelial growth factor (VEGF) receptor inhibitors, topoisomerase II inhibitors, smoothen inhibitors, alkylating agents, anti-tumor antibiotics, anti-metabolites, retinoids, and immunomodulatory agents including anti -cancer vaccines. It will be understood that such additional active agent(s) may be provided as a pharmaceutically acceptable salt. It will be understood the descriptions of the above additional active agents may be overlapping. It will also be understood that the treatment combinations are subject to optimization, and it is understood that the best combination to use of the compounds of general Formula I-1 or pharmaceutically acceptable salts of the foregoing, and one or more additional active agents will be determined based on the individual patient needs.

A compound disclosed herein may be used in combination with one or more other active agents, including other anti-cancer agents that are used in the prevention, treatment, control, amelioration, or reduction of risk of a particular disease or condition (e.g., cell proliferation disorders). In one embodiment, a compound disclosed herein is combined with one or more other anti -cancer agents for use in the prevention, treatment, control amelioration, or reduction of risk of a particular disease or condition for which the compounds disclosed herein are useful. Such other active agents may be administered, by a route and in an amount commonly used therefor, contemporaneously or sequentially with a compound of the present disclosure.

The pharmaceutical carrier employed can be, for example, a solid, liquid, or gas. Examples of solid carriers include lactose, terra alba, sucrose, talc, gelatin, agar, pectin, acacia, magnesium stearate, and stearic acid. Examples of liquid carriers are sugar syrup, peanut oil, olive oil, and water. Examples of gaseous carriers include carbon dioxide and nitrogen. In preparing the compositions for oral dosage form, any convenient pharmaceutical media may be employed. For example, water, glycols, oils, alcohols, flavoring agents, preservatives, coloring agents may be used to form oral liquid preparations such as suspensions, elixirs and solutions; while carriers such as starches, sugars, microcrystalline cellulose, diluents, granulating agents, lubricants, binders, disintegrating agents may be used to form oral solid preparations such as powders, capsules and tablets. Because of their ease of administration, tablets and capsules are the preferred oral dosage units whereby solid pharmaceutical carriers are employed. Optionally, tablets may be coated by standard aqueous or nonaqueous techniques.

A tablet containing the composition of this invention may be prepared by compression or molding, optionally with one or more accessory ingredients or adjuvants. Compressed tablets may be prepared by compressing, in a suitable machine, the active ingredient in a free-flowing form such as powder or granules, optionally mixed with a binder, lubricant,

inert diluent, surface active or dispersing agent. Molded tablets may be made by molding in a suitable machine, a mixture of the powdered compound moistened with an inert liquid diluent. Each tablet preferably contains from about 0.05mg to about 5g of the active ingredient and each cachet or capsule preferably containing from about 0.05mg to about 5g of the active ingredient. For example, a formulation intended for the oral administration to humans may contain from about 0.5mg to about 5g of active agent, compounded with an appropriate and convenient amount of carrier material which may vary from about 5 to about 95 percent of the total composition. Unit dosage forms will generally contain between from about lmg to about 2g of the active ingredient, typically 25mg, 50mg, 100mg, 200mg, 300mg, 400mg, 500mg, 600mg, 800mg, or 1000mg.

Pharmaceutical compositions of the present invention suitable for parenteral administration may be prepared as solutions or suspensions of the active compounds in water. A suitable surfactant can be included such as, for example, hydroxypropylcellulose. Dispersions can also be prepared in glycerol, liquid polyethylene glycols, and mixtures thereof in oils. Further, a preservative can be included to prevent the detrimental growth of microorganisms.

Pharmaceutical compositions of the present invention suitable for injectable use include sterile aqueous solutions or dispersions. Furthermore, the compositions can be in the form of sterile powders for the extemporaneous preparation of such sterile injectable solutions or dispersions. In all cases, the final injectable form must be sterile and must be effectively fluid for easy syringability. The pharmaceutical compositions must be stable under the conditions of manufacture and storage; thus, preferably should be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), vegetable oils, and suitable mixtures thereof.

Pharmaceutical compositions of the present invention can be in a form suitable for topical use such as, for example, an aerosol, cream, ointment, lotion, dusting powder. Further, the compositions can be in a form suitable for use in transdermal devices. These formulations may be prepared, utilizing a compound represented by Formula I-1 of this invention, or a pharmaceutically acceptable salt thereof, via conventional processing methods. As an example, a cream or ointment is prepared by admixing hydrophilic material and water, together with about 5wt% to about 10wt% of the compound, to produce a cream or ointment having a desired consistency.

Pharmaceutical compositions of this invention can be in a form suitable for rectal administration and the carrier is a solid. It is preferable that the mixture forms unit dose suppositories. Suitable carriers include cocoa butter and other materials commonly used in the art. The suppositories may be conveniently formed by first admixing the composition with the softened or melted carrier(s) followed by chilling and shaping in molds.

In addition to the aforementioned carrier ingredients, the pharmaceutical formulations described above may include, as appropriate, one or more additional carrier ingredients such as diluents, buffers, flavoring agents, binders, surface-active agents, thickeners, lubricants, preservatives (including antioxidants). Furthermore, other adjuvants can be included to render the formulation isotonic with the blood of the intended recipient. Compositions containing a compound described by Formula 1-1, or pharmaceutically acceptable salts thereof, may also be prepared in powder or liquid concentrate form.

Generally, dosage levels on the order of from about 0.01mg/kg to about 150mg/kg of body weight per day are useful in the treatment of the above-indicated conditions, or alternatively about 0.5mg to about 7g per patient per day. For example, inflammation, cancer, psoriasis, allergy/asthma, disease and conditions of the immune system, disease and conditions of the central nervous system (CNS), may be effectively treated by the administration of from about 0.01 to 50 mg of the compound per kilogram of body weight per day, or alternatively about 0.5mg to about 3.5g per patient per day.

It is understood, however, that the specific dose level for any particular patient will depend upon a variety of factors including the age, body weight, general health, sex, diet, time of administration, route of administration, rate of excretion, drug combination and the severity of the particular disease undergoing therapy.

These and other aspects will become apparent from the following written description of the invention.

## Examples

The following Examples are provided to better illustrate the present invention. All parts and percentages are by weight and all temperatures are degrees Celsius, unless explicitly stated otherwise.

The compounds described herein can be prepared in a number of ways based on the teachings contained herein and synthetic procedures known in the art. In the description of the synthetic methods described below, it is to be understood that all proposed reaction conditions, including choice of solvent, reaction atmosphere, reaction temperature, duration of the experiment and workup procedures, can be chosen to be the conditions standard for that reaction, unless otherwise indicated. It is understood by one skilled in the art of organic synthesis that the functionality present on various portions of the molecule should be compatible with the reagents and reactions proposed. Substituents not compatible with the reaction conditions will be apparent to one skilled in the art, and alternate methods are therefore indicated. The starting materials for the examples are either commercially available or are readily prepared by standard methods from known materials.

Examples are provided herein to facilitate a more complete understanding of the disclosure. The following examples serve to illustrate the exemplary modes of making and practicing the subject matter of the disclosure.

The following abbreviations are used in the reaction schemes and synthetic examples, which follow. This list is not meant to be an all-inclusive list of abbreviations used in the application as additional standard abbreviations, which are readily understood by those skilled in the art of organic synthesis, can also be used in the synthetic schemes and examples.

| | |
|---|---|
| NBS | *N*-Bromosuccinimide |
| EA | Ethyl acetate |
| ACN | Acetonitrile |
| THF | Tetrahydrofuran |
| DCM | Dichloromethane |
| DMF | Dimethylformamide |
| TFA | Trifluoroacetic acid |
| TEA | Triethylamine |
| LDA | Lithium diisopropylamide |
| OXONE | PotassiumPeroxomonosulphate |
| FA | Formic acid |
| DIEA | *N*,*N*-Diisopropylethylamine |
| Pd(dppf)$Cl_2$ | [1,1'-Bis(diphenylphosphino)ferrocene]dichloropalladium(II) |
| CDI | *N*,*N*-Carbonyldiimidazole |
| RockPhos Palladacycle Gen.3 | Methanesulfonato(2-(di-t-butylphosphino)-3-methoxy-6-methyl-2',4',6' -tri-i-propyl-1,1'-biphenyl)(2'-amino-1,1'-biphenyl-2-yl)palladium(II) |
| MeOH | Methanol |
| EtOH | Ethanol |
| INT | Intermediate |
| h | hour(s) |
| aq | aqueous |
| sat | saturated |
| TLC | Thin layer chromatography |
| Pre-TLC | Preparative thin layer chromatography |
| Prep - HPLC | Preparation high performance liquid chromatography |

**INTERMEDIATE**

**INT A1: ethyl 4-(5-hydroxy-6-methoxy-benzothiophen-2-yl)-4-oxo-butanoate**

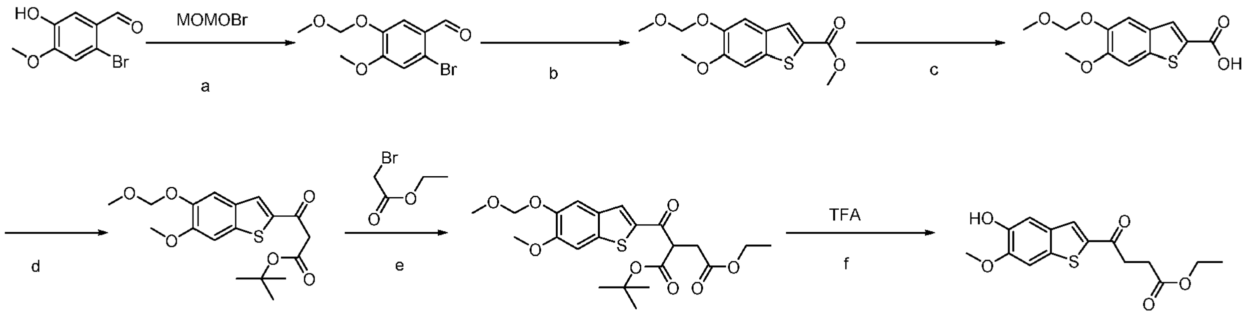


***Step a:*** Bromomethyl methyl ether, (3.15 g, 25.2073 mmol) was added to a solution of DIEA (4.10 g, 46.5111 mmol) and 2-Bromoisovanillin (4.98 g, 21.5544 mmol) in Dichloromethane (200 mL) at 0 °C. The reaction mixture was stirred at 20 °C for 2 h. The resulting solution was quenched by water (10 mL) and washed with water (100 mL) and brine (100 mL). The organics was dried over $Na_2SO_4$, filtered and evaporated under reduced pressure. There was 2-bromo-4-methoxy-5-(methoxymethoxy)benzaldehyde (5.83 g, 21.1926 mmol, 98.3216% yield) obtained as a white solid. $^1$H NMR (400 MHz, DMSO-*d*$_6$) δ 10.06 (s, 1H), 7.51 (s, 1H), 7.39 (s, 1H), 5.25 (s, 2H), 3.93 (s, 3H), 3.38 (d, *J* = 9.3 Hz, 3H).

***Step b:*** Methyl thioglycolate (2.30 *g,* 21.6688 mmol) was added to a solution of 2-bromo-4-methoxy-5-(methoxymethoxy)benzaldehyde (5.83 g, 21.1926 mmol) and $Cs_2CO_3$ (13.99 g, 42.9380 mmol) in *N*,*N*-Dimethylformamide (100 mL) at 20 °C. The reaction mixture was heated to 50 °C and stirred for 16 h. The reaction mixture was cooled to 20 °C, and Methyl Iodide (1.81 g, 12.7520 mmol) was added to the reaction solution, and stirred for 2 h at 20 °C. The reaction mixture was diluted with EA (500 mL), washed with water (2 x 100 mL) and brine (100 mL). The organics was evaporated under reduced pressure. The residue was purified on silica gel column EA/hept (0-50%). The pure fractions was concentrated and dried under vacuo. There was methyl 6-methoxy-5-(methoxymethoxy)benzothiophene-2-carboxylate (2.98 g, 10.5557 mmol, 49.8084% yield) obtained as a yellow solid. $^1$H NMR (400 MHz, DMSO-*d*$_6$) δ 8.05 (s, 1H), 7.66 (s, 1H), 7.63 (s, 1H), 5.23 (s, 2H), 3.87 (d, *J* = 9.2 Hz, 6H), 3.42 (s, 3H).

***Step c:*** LiOH (0.37 g, 15.4499 mmol) was added to a solution of methyl 6-methoxy-5-(methoxymethoxy) benzothiophene-2-carboxylate (2.98 g, 10.5557 mmol) in Tetrahydrofuran (100 mL) and Water (25 mL) at 20 °C. The reaction mixture was heated to 50 °C and stirred for 2 h. The reaction mixture was evaporated under reduced pressure and diluted with water and adjusted pH = 3 with HCl (1 M). The precipitate was filtered. Filter cake was washed with water (20 mL). There was 6-methoxy-5-(methoxymethoxy) benzothiophene-2-carboxylic acid (2.41 g, 8.9830 mmol, 85.1008% yield) obtained as a white solid. $^1$H NMR (400 MHz, DMSO-*d*$_6$) δ 13.22 (s, 1H), 7.93 (s, 1H), 7.62 (d, *J* = 10.5 Hz, 2H), 5.24 (d, *J* = 14.6 Hz, 2H), 3.87 (s, 3H), 3.52 - 3.35 (m, 3H).

***Step d:*** To a stirred solution of 6-methoxy-5-(methoxymethoxy)benzothiophene-2-carboxylic acid (2.41 g, 8.9830 mmol) in N,N-Dimethylformamide (20 mL) was added 1,1'-Carbonyldiimidazole (2.84 g, 17.5148 mmol) at 20 °C. The resulting mixture was stirred for 1 h at 20 °C under nitrogen atmosphere. To the mixture above was added 3-tert-Butoxy-3-oxopropanoic acid and Magnesium chloride (1.31 g, 13.7589 mmol). The reaction mixture was stirred at 20 °C for 16 h. The reaction mixture was concentrated and diluted with EA (500 mL), washed with $NaHCO_3$ aq. (2 x 300 mL) and brine (200 mL). The organics was dried over $Na_2SO_4$, filtered and evaporated under reduced pressure. The residue was purified on silica gel column EA/PE (0-80%). The pure fractions was concentrated and dried under vacuo. There was tert-butyl 3-[6-methoxy-5-(methoxymethoxy) benzothiophen-2-yl]-3-oxo-propanoate (2.63 g, 7.1774 mmol, 79.9000% yield) obtained as a yellow oil. $^1$H NMR (400 MHz, DMSO-*d*$_6$) δ 8.20 (s, 1H), 7.67 (s, 1H), 7.62 (s, 1H), 5.24 (s, 2H), 4.03 (d, *J* = 9.8 Hz, 2H), 3.89 (s, 3H), 3.42 (s, 3H), 1.41 (s, 9H).

***Step e:*** To a stirred solution of tert-butyl 3-[6-methoxy-5-(methoxymethoxy)benzothiophen-2-yl]-3-oxo-propanoate (2.62 g, 7.1501 mmol) in *N*,*N*-Dimethylformamide (20 mL) was added Potassium carbonate (2.0621 g, 14.9207 mmol) and Ethyl bromoacetate (1.3648 g, 8.1724 mmol) at 20 °C . The resulting mixture was stirred for 3 h at room temperature under nitrogen atmosphere. The reaction mixture was concentrated and diluted with EA (200 mL), washed water with (100 mL) and brine (50 mL). The organics was dried over $Na_2SO_4$, filtered and evaporated under reduced pressure. The residue was purified on silica gel column EA/PE (0-80%). The pure fractions was concentrated and dried under vacuo. There was O1-tert-butyl O4-ethyl 2-[6-methoxy-5-(methoxymethoxy) benzothiophene-2-carbonyl]butanedioate (2.82 g, 6.2318 mmol, 87.1569% yield) obtained as a yellow oil. $^1$H NMR (400 MHz, DMSO-*d*$_6$) δ 8.35 (s, 1H), 7.67 (s, 2H), 5.31 - 5.20 (m, 2H), 4.79 (t, *J* = 7.3 Hz, 1H), 4.15 - 3.96 (m, 2H), 3.89 (d, *J* = 6.7 Hz, 3H), 3.42 (d, *J* = 4.2 Hz, 3H), 3.32 (s, 3H), 2.98 - 2.81 (m, 2H), 1.31 (s, 9H).

***Step f:*** TFA (5 mL) was added to O1-tert-butyl O4-ethyl 2-[6-methoxy-5-(methoxymethoxy)benzothiophene-2-carbonyl]butanedioate (2.82 g, 6.2318 mmol) in Toluene (25 mL) at 20 °C. The reaction mixture was heated to 50 °C and stirred for 30 min. The reaction mixture was evaporated under reduced pressure. The reaction mixture was concentrated and diluted with EA (200 mL), washed with water (100 mL) and brine (50 mL). The organics was dried over $Na_2SO_4$, filtered and evaporated under reduced pressure. The residue was purified on silica gel column EA/Heptane (0-100%). The pure

fractions was concentrated and dried under vacuo to afford crude product. The residue was purified on silica gel column MeCN/water (0-100%). The pure fractions was concentrated and dried under vacuo. There was ethyl 4-(5-hydroxy-6-methoxy-benzothiophen-2-yl)-4-oxo-butanoate (0.79 g, 2.5620 mmol, 41.1122% yield) obtained as a white solid. $^1H$ NMR (400 MHz, DMSO-$d_6$) δ 9.39 (s, 1H), 8.17 (s, 1H), 7.54 (s, 1H), 7.32 (s, 1H), 4.06 (q, *J* = 7.1 Hz, 2H), 3.87 (s, 3H), 3.31 - 3.26 (m, 2H), 2.65 (t, *J* = 6.3 Hz, 2H), 1.27 - 1.05 (m, 3H).

**INT A2: methyl 4-(5-chloro-6-methoxythieno[3,2-b]pyridin-2-yl)-4-oxobutanoate**

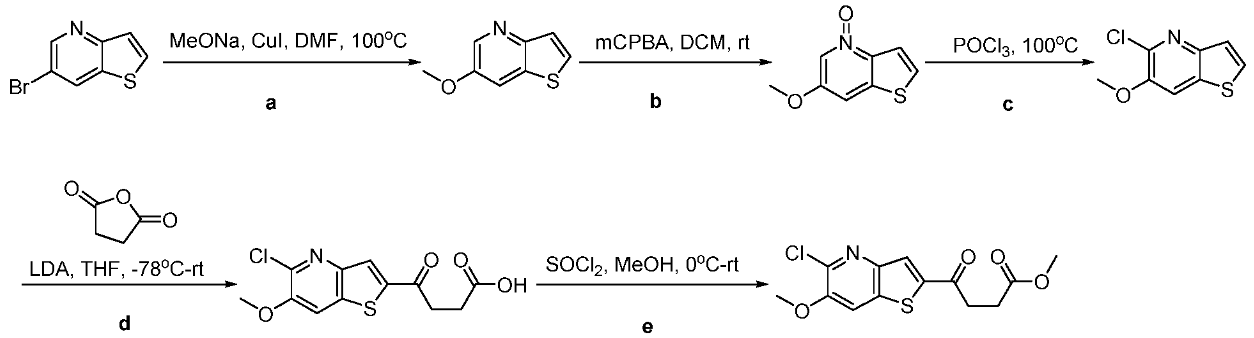


***Step a*:** To a solution of 6-bromothieno[3,2-b]pyridine (30.0 g, 140.1 mmol) in DMF (300 mL) was added a solution of 25% MeONa in MeOH (605.6 g, 2802.6 mmol) and CuI (26.7 g, 140.1 mmol). The reaction mixture was heated to 100 ° C and stirred for 2 h. Then the reaction mixture was cooled to room temperature, poured into ice water (900 mL) and filtered. The filtrate was diluted with $NH_3.H_2O$ (300 mL), extracted with EtOAc (1.0 L x 2) and washed with brine (1.0 L). The organic layer was dried over $Na_2SO_4$, filtered and the filtrate was concentrated to afford 6-methoxythieno[3,2-b]pyridine (19.0 g, 82.3%) as a yellow solid which was used for next step without further purification. $^1H$ NMR (300 MHz, $CDCl_3$): δ 8.43 (d, *J* = 2.7 Hz, 1H), 7.62 (d, *J* = 2.4 Hz, 1H), 7.53-7.51 (m, 1H), 7.47-7.45 (m, 1H), 3.91 (s, 3H). LCMS: (ESI, m/z): $[M+H]^+$ = 166.2.

***Step b:*** To a solution of 6-methoxythieno[3,2-b]pyridine (19.0 g, 115.0 mmol) in DCM (200 mL) was added m-CPBA (19.8 g, 115.0 mmol). The reaction mixture was stirred at room temperature for 16 h. Then the reaction mixture was diluted with DCM/MeOH = 10: 1 (300 mL) and washed with saturated sodium bicarbonate solution (300 mL x 2) and brine (300 mL). The organic layer was dried over $Na_2SO_4$, filtered and the filtrate was concentrated to afford 6-methoxythieno [3,2-b]pyridine 4-oxide (13.0 g, 57.7%) as a yellow solid which was used for next step without further purification. $^1H$ NMR (300 MHz, $CDCl_3$): δ 8.14 (d, *J* = 2.1 Hz, 1H), 7.74 (dd, *J* = 5.7 Hz, 0.6 Hz, 1H), 7.48-7.46 (m, 1H), 7.31-7.30 (m, 1H), 3.91 (s, 3H). LCMS: (ESI, m/z): $[M+H]^+$ = 182.0.

***Step c:*** Add 6-methoxythieno[3,2-b]pyridine 4-oxide (15.5 g, 85.5 mmol) portion wise to $POCl_3$ (200 mL) at room temperature. The reaction mixture was heated to 100 °C and stirred for 16 h. Then the reaction mixture was cooled to room temperature and concentrated. The residue was diluted with water (200 mL), adjusted to pH = 8 with saturated sodium bicarbonate solution and extracted with DCM (300 mL x 2). The organic layers were washed with brine (200 mL), dried over $Na_2SO_4$, filtered and the filtrate was concentrated to give the residue which was purified by column chromatography on silica gel (eluted with petroleum ether/EtOAc = 10: 1) to afford 5-chloro-6-methoxythieno[3,2-b]pyridine (9.0 g, 52.6%) as a white solid. $^1H$ NMR (300 MHz, $CDCl_3$): $\delta$ 7.65 (s, 1H), 7.58-7.56 (m, 1H), 7.42 (dd, *J* = 5.7 Hz, 0.6 Hz, 1H), 3.99 (s, 3H). LCMS: (ESI, m/z): $[M+H]^+$ = 200.0.

***Step d:*** To a solution of 5-chloro-6-methoxythieno[3,2-b]pyridine (8.0 g, 40.1 mmol) in THF (80 mL) was added LDA (2.0 M, 80 mL) dropwise at -78 °C under nitrogen. After 15 min, a solution of dihydrofuran-2,5-dione (22.0 g, 220.4 mmol) in THF (240 mL) was added to the mixture dropwise at -40 °C under nitrogen. Then the reaction mixture was warmed to room temperature and stirred for 2 h. After that the reaction was quenched with 2N HCl (100 mL) and extracted with EtOAc (300 mL x 2). The organic layers were washed with brine (200 mL), dried over $Na_2SO_4$, filtered and the filtrate was concentrated. The residue was triturated with petroleum ether/EtOAc = 1: 1 and filtered to afford 4-(5-chloro-6-methoxythieno[3,2-b]pyridin-2-yl)-4-oxobutanoic acid (6.0 g, crude) as a white solid which was used for next step without further purification. LCMS: (ESI, m/z): $[M-H]^-$ = 298.8.

***Step e:*** To a solution of 4-(5-chloro-6-methoxythieno[3,2-b]pyridin-2-yl)-4-oxobutanoic acid (6.0 g crude, 20.0 mmol) in MeOH (120 mL) was added $SOCl_2$ (12.0 g, 100.1 mmol) dropwise at 0 °C. Then the reaction mixture was warmed to room temperature and stirred for 2 h. After that the reaction was concentrated, diluted with water (100 mL), adjusted to pH = 8 with saturated sodium bicarbonate solution and extracted with DCM/MeOH (200 mL x 2). The organic layers were washed with brine (100 mL), dried over $Na_2SO_4$, filtered.

The filtrate was concentrated, white solid precipitated and filtered to afford methyl 4-(5-chloro-6-methoxythieno[3,2-b]pyridin-2-yl)-4-oxobutanoate (1.1 g, 8.5% in total of *d* and e two steps) as a white solid $^1$H NMR (400 MHz, $CDCl_3$): $\delta$ 8.01 (s, 1H), 7.59 (s, 1H), 4.02 (s, 3H), 3.71 (s, 3H), 3.35 (t, *J* = 6.8 Hz, 2H), 2.80 (t, *J* = 6.8 Hz, 2H), LCMS: (ESI, m/z): [M+H] $^+$ = 569.20.

**INT A3: methyl**

**trans-2-(5-bromo-6-methoxybenzo[b]thiophene-2-carbonyl)cyclopropane-1-carboxylate**

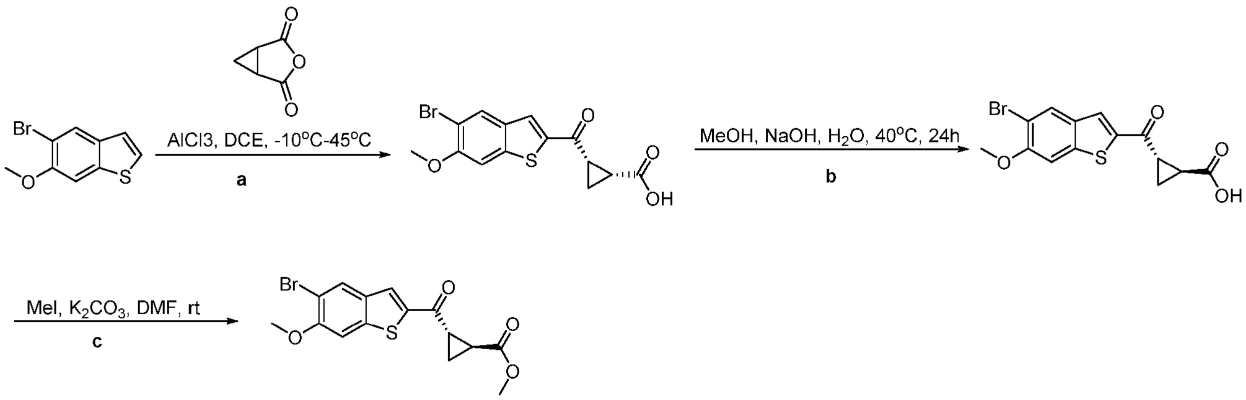


***Step a:*** To a solution of 3-oxabicyclo [3.1.0]hexane-2,4-dione (6.3 g, 56.1 mmol) and $AlCl_3$ (12.3 g, 92.6 mmol) in DCE (150 mL) was added a solution of 5-bromo-6-methoxybenzo[b]thiophene (15.0 g, 61.7 mmol) in DCE (50 mL) at -10°C. After that the reaction mixture was heated to 45 °C and stirred for 16 h. Then the reaction mixture was poured into ice-water (200 mL) and extracted with DCM/MeOH=10:1 (100 mL x 2). The organic layer washed with brine (100 mL), dried over $Na_2SO_4$, filtered and the filtrate was concentrated to dryness. The residue was triturated with Petroleum ether/EtOAc = 1: 1 (50 mL) and filtered to afford cis-2-(5-bromo-6-methoxybenzo[b]thiophene-2-carbonyl)cyclopropane-1-carboxylic acid (3.0 g, yield 15.1%) as a yellow solid. $^1$H NMR (400 MHz, DMSO-$d_6$): $\delta$ 8.32 (s, 1H), 8.26 (s, 1H), 7.80 (s, 1H), 4.05 (s, 3H), 3.01-3.09 (m, 1H), 2.25-2.31 (m, 1H), 1.65-1.68 (m, 1H), 1.58-1.52 (m, 1H).

***Step b:*** To a solution of *cis* -2-(5-bromo-6-methoxybenzo[b]thiophene-2-carbonyl)cyclopropane-1-carboxylic acid (3.0 g, 8.4 mmol) in MeOH (30 mL) was added 50% aq. NaOH (45 mL) at room temperature. Then the reaction mixture was heated to 40 °C and stirred for 24 h. After the reaction completed analysis by HPLC, the solvent was removed by concentrated. The residue was diluted with water (30 mL) and was adjustd pH~2 with 6 N HCl. The white solid was collected by filtration. The solid was dryness under vacuum at 50°C to afford *trans*-2-(5-bromo-6-methoxybenzo[b]thiophene-2-carbonyl)cyclopropane-1-carboxylic acid (2.5 g, yield 83.3%) as a yellow solid. $^1$H NMR (400 MHz, DMSO-$d_6$): $\delta$ 8.52 (s, 1H), 8.27 (s, 1H), 7.80 (s, 1H), 3.96 (s, 3H), 3.27-3.23 (m, 1H), 2.16-2.11 (m, 1H), 1.54-1.49 (m, 2H).

***Step c:*** To a solution of

*trans*-2-(5-bromo-6-methoxybenzo[b]thiophene-2-carbonyl)cyclopropane-1-carboxylic acid (2.5 g, 7.0 mmol) in DMF (25 mL) was added $K_2CO_3$ (2.4 g, 17.5 mmol) and MeI (2.0 g, 14.1 mmol) at room temperature. The reaction mixture was stirred for 16 h. Then the mixture was poured into water (50 mL) and extracted with EtOAc (50 mL x 2). The organic layer was washed with water (50 mL x 2) and brine (50 mL x 2). The organic layer was dried with anhydrous $Na_2SO_4$, filtered and the filtrate was concentrated to dryness. The residue was triturated with Petroleum ether/EtOAc = 5: 1 (50 mL) and filtered to afford methyl *trans*-2-(5-bromo-6-methoxybenzo[b]thiophene-2-carbonyl)cyclopropane-1-carboxylate (2 g, yield 77.5%) as a yellow solid. LCMS: (ESI, m/z): [M+H] $^+$ =368.8, 370.8. $^1$H NMR (400 MHz, $CDCl_3$): $\delta$ 8.07 (s, 1H), 7.96 (s, 1H), 7.30 (s, 1H), 3.99 (s, 3H), 3.75 (s, 3H), 3.15-3.11 (m, 1H), 2.45-2.40 (m, 1H), 1.70-1.64 (m, 2H).

**INT A4: ethyl 4-(4-chloro-5-hydroxy-6-methoxybenzo[b]thiophen-2-yl)-4-oxobutanoate**

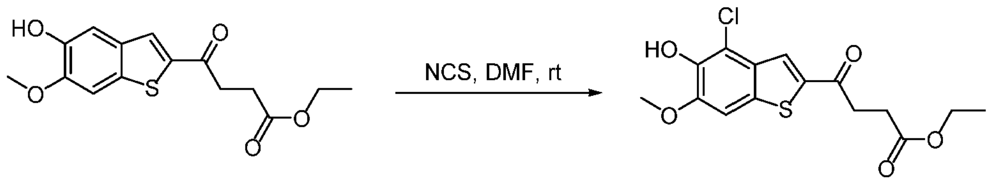


To a solution of ethyl 4-(5-hydroxy-6-methoxybenzo[b]thiophen-2-yl)-4-oxobutanoate (300 mg, 0.97 mmol) in DMF (6 mL) was added NCS (156 mg, 1.14 mmol) portion wise at room temperature. After that the reaction mixture was stirred for 5 h. Then the reaction mixture was poured into ice-water (18 mL). The mixture was filtered and the filter cake was dissolved with EtOAc (20 mL). The organic layer was washed with sat. aq. $NaHCO_3$ (15 mL) and brine (10 mL), dried over $Na_2SO_4$ and concentrated to afford a crude product which was purified by HPLC to give ethyl 4-(4-chloro-5-hydroxy-6-methoxybenzo[b]thiophen-2-yl)-4-oxobutanoate (240 mg, yield 72%) as a white solid. $^1$H NMR (300 MHz, $CDCl_3$): δ 8.02 (s, 1H), 7.17 (s, 1H), 6.04 (brs, 1H), 4.20-4.12 (m, 2H), 4.02 (s, 3H), 3.36 (t, *J* = 8.8 Hz, 2H), 2.79 (t, *J* = 8.8 Hz, 2H), 1.30-1.20 (m, 3H). LCMS: (ESI, m/z): [M+H] $^+$ = 342.9.

**INT A5: ethyl 4-(5-methoxyisoindolin-2-yl)-4-oxo-butanoate**
**INT A6: ethyl 4-(5-bromo-6-methoxy-isoindolin-2-yl)-4-oxo-butanoate**
**INT A7: ethyl 4-(5-hydroxy-6-methoxyisoindolin-2-yl)-4-oxobutanoate**
**AND INT A8: ethyl 4-(5-(3-bromopropoxy)-6-methoxyisoindolin-2-yl)-4-oxobutanoate**

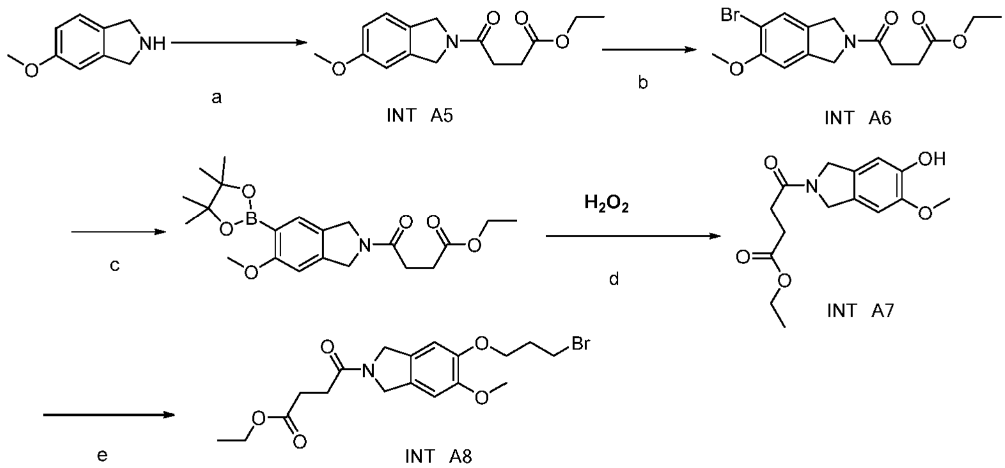

INT A5

INT A6

INT A7

INT A8

***Step a:*** Succinic anhydride (4.40 g, 43.9681 mmol) was added to a solution of 5-Methoxyisoindoline hydrochloride (4.89 g, 26.3399 mmol) and Triethylamine (4.73 g, 46.7440 mmol) in Ethanol (200 mL) at 20 °C. The reaction mixture was stirred for 1 h at 20 °C. $SOCl_2$ (20 mL) was added to the solution above at 0 °C. The reaction mixture was stirred for 3 h at 20 °C. The reaction mixture was evaporated under reduced pressure. The reaction mixture was diluted with EA (200 mL), washed with water (100 mL) and brine (50 mL). The organics was dried over $Na_2SO_4$, filtered and evaporated under reduced pressure. There was ethyl 4-(5-methoxyisoindolin-2-yl)-4-oxo-butanoate (17.5 g, 31.5526 mmol, 119.7901% yield) obtained as a yellow solid.

$^1$H NMR (400 MHz, DMSO-$d_6$) δ 7.28 - 7.21 (m, 1H), 6.94 (s, 0.5H), 6.92 (s, 0.5H), 6.88 (s, 0.5H), 6.86 (s, 0.5H), 4.81 (s, 1H), 4.76 (s, 1H), 4.58 (s, 1H), 4.54 (s, 1H), 4.02 - 3.95 (m, 2H), 3.75 (s, 3H), 2.65 - 2.58 (m, 2H), 2.58 - 2.54 (m, 2H), 1.17 (t, *J* = 3.5 Hz, 3H).

***Step b:*** NBS (10.51 g, 59.0503 mmol) was added to ethyl 4-(5-methoxyisoindolin-2-yl)-4-oxo-butanoate in Tetrahydrofuran (100 mL) and Acetonitrile (100 mL) at 20 °C. The reaction mixture was stirred overnight at 20 °C. The reaction mixture was evaporated under reduced pressure. The reaction mixture was concentrated and diluted with DCM (500 mL), washed with $NaHCO_3$ aq. (2 x 300 mL) and brine (200 mL). The organics was dried over $Na_2SO_4$, filtered and evaporated under reduced pressure. The residue was purified on silica gel column MeOH/DCM (0-10%). The pure fractions was concentrated and dried under vacuo. There was ethyl 4-(5-bromo-6-methoxy-isoindolin-2-yl)-4-oxo-butanoate (7.26 g, 20.3812 mmol, 64.5943% yield) obtained as a yellow solid.

$^1$H NMR (400 MHz, DMSO-$d_6$) δ 7.58 (s, 0.5H), 7.57 (s, 0.5H), 7.15 (s, 0.5H), 7.12 (s, 0.5H), 4.80 (s, 1H), 4.77 (s, 1H), 4.57 (s, 1H), 4.54 (s, 1H), 4.09 - 4.01 (m, 2H), 3.84 (s, 3H), 2.65 - 2.59 (m, 2H), 2.56 - 2.53 (m, 2H), 1.18 (t, *J* = 7.1 Hz, 3H).

***Step c:*** Potassium Acetate (3.74 g, 38.1080 mmol) was added to Pd(dppf)$Cl_2$ (0.72 g, 984.0050 μmol), ethyl

4-(5-bromo-6-methoxy-isoindolin-2-yl)-4-oxo-butanoate (3.64 g, 10.2187 mmol) and 4,4,4',4',5,5,5',5'-Octamethyl-2,2'-bi(1,3,2-dioxaborolane) (3.84 g, 15.1218 mmol) in 1,4-Dioxane (100 mL) at 20 °C. The reaction mixture was stirred at 100 °C for 16 h. The reaction mixture was evaporated under reduced pressure. The reaction mixture was concentrated and diluted with DCM (500 mL), washed with $NaHCO_3$ aq. (2 x 300 mL) and brine (200 mL). The organics was dried over $Na_2SO_4$, filtered and evaporated under reduced pressure. The residue was purified on silica gel column EA/Hept (0-100%). The pure fractions was concentrated and dried under vacuo. There was ethyl 4-(5-methoxy-6-(4,4,5,5-tetramethyl-1,3,2-dioxaborolan-2-yl)isoindolin-2-yl)-4-oxobutanoate (2.45 g, 6.0752 mmol, 59.7809% yield) obtained as a yellow solid.

$^1$H NMR (400 MHz, DMSO-$d_6$) δ 7.49 (s, 0.5H), 7.48 (s, 0.5H), 6.98 (s, 0.5H), 6.95 (s, 0.5H), 4.83 (s, 1H), 4.74 (s, 1H), 4.60 (s, 1H), 4.53 (s, 1H), 4.09 - 4.01 (m, 2H), 3.74 (t, *J* = 6.2 Hz, 3H), 2.65 - 2.58 (m, 2H), 2.58 - 2.52 (m, 2H), 1.27 (s, 12H), 1.21 - 1.13 (m, 3H).

***Step d:*** Sodium perborate tetrahydrate (1 g, 6.4994 mmol) was added to ethyl 4-(5-methoxy-6-(4,4,5,5-tetramethyl-1,3,2-dioxaborolan-2-yl)isoindolin-2-yl)-4-oxobutanoate (2.45 g, 6.0752 mmol) in Tetrahydrofuran (20 mL) and Water (20 mL) at 20 °C. The reaction mixture was stirred for 1 h at 20 °C. The reaction mixture was evaporated under reduced pressure. The reaction mixture was concentrated and diluted with DCM (500 mL), washed with $NaHCO_3$ aq. (2 x 300 mL) and brine (200 mL). The organics was dried over $Na_2SO_4$, filtered and evaporated under reduced pressure. The residue was purified on silica gel column MeOH/DCM (0-10%). The pure fractions was concentrated and dried under vacuo. There was ethyl 4-(5-hydroxy-6-methoxyisoindolin-2-yl)-4-oxobutanoate (1.37 g, 4.6708 mmol, 76.8818% yield) obtained as a yellow solid.

$^1$H NMR (400 MHz, DMSO-$d_6$) δ 8.97 (s, 0.5H), 8.96 (s, 0.5H), 6.91 (s, 0.5H), 6.89 (s, 0.5H), 6.73 (s, 0.5H), 6.73 (s, 0.5H), 4.71 (s, 1H), 4.69 (s, 1H), 4.50 (s, 1H), 4.47 (s, 1H), 4.08 - 4.00 (m, 2H), 3.75 (s, 3H), 2.64 - 2.58 (m, 2H), 2.56 - 2.53 (m, 2H), 1.18 (t, *J* = 7.1 Hz, 3H).

***Step e:*** To a solution of ethyl 4-(5-hydroxy-6-methoxy-isoindolin-2-yl)-4-oxo-butanoate (0.72 g, 2.4547 mmol) in *N*,*N*-Dimethylformamide (10 mL) was added 1,3-Dibromopropane (0.73 g, 3.6159 mmol) and potassium carbonate (0.86 g, 6.2226 mmol)2. This mixture was stirred for 16 hours at 50 °C. The reaction mixture was diluted with Ethyl acetate (100 mL), and washed sequentially with water (2 x 100 mL) and saturated brine (1 x 100 mL). The organic layer was dried over $Na_2SO_4$, filtered and evaporated to afford crude product. The crude product was purified by flash silica chromatography, elution gradient 0 to 100% Ethyl acetate in heptane. There was ethyl 4-(5-(3-bromopropoxy)-6-methoxyisoindolin-2-yl)-4-oxobutanoate (0.43 g, 1.0379 mmol, 42.2829% yield) obtained as a white solid.

$^1$H NMR (400 MHz, DMSO-$d_6$) δ 7.01 - 6.93 (m, 2H), 4.75 (s, 2H), 4.53 (s, 2H), 4.10 - 4.00 (m, 4H), 3.76 (s, 3H), 3.71 - 3.62 (m, 2H), 2.65 - 2.58 (m, 2H), 2.58 - 2.53 (m, 2H), 2.28 - 2.18 (m, 2H), 1.18 (t, *J* = 7.1 Hz, 3H).

**INT A9: tert-butyl 3-(5-bromo-4-fluoro-6-methoxy-benzothiophen-2-yl)-3-oxo-propanoate**

***Step a: 3-bromo-2-fluoro-4-methoxy-benzaldehyde***

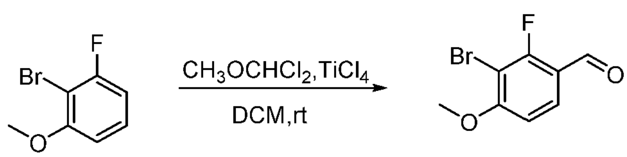


Titanium tetrachloride (38.06 g, 200.6548 mmol) was added dropwise to a solution of 2-Bromo-3-fluoroanisole (10.17 g, 49.6039 mmol) in Dichloromethane (250 mL) at 0°C for 1 h. The reaction mixture was stirred for 3 h at 20°C. The reaction mixture was quenched with adding of ice-water (200 mL) at 0°C. The reaction mixture was extracted with DCM (2 x 100 mL), washed with $NaHCO_3$ (1 x 200 mL) and brine (100 mL). The organics dried over $Na_2SO_4$, filtered and evaporated under reduced pressure. The residue was dried under vacuo at 60°C. There was 3-bromo-2-fluoro-4-methoxy-benzaldehyde (11.42 g, 49.0057 mmol, 98.7940% yield) obtained as a white solid. LCMS: (ESI, m/z): $[M+H]^+$ = 232.954. $^1$H NMR (400 MHz, $CDCl_3$-*d*) δ 10.24 (s, 1H), 7.87 (t, J = 8.3 Hz, 1H), 6.84 (d, J = 8.8 Hz, 1H), 4.05 - 3.99 (m, 3H).

***Step b: (5E)-5-[(3-bromo-2-fluoro-4-methoxy-phenyl)methylene]-2-thioxo-thiazolidin-4-one***

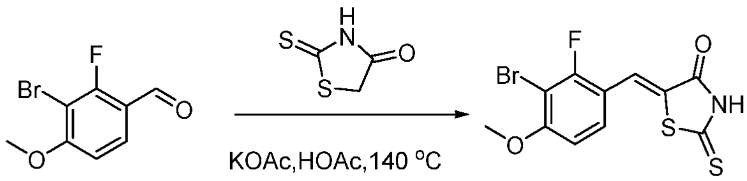


Potassium Acetate (12.67 g, 129.0984 mmol) was added to a mixture of 3-bromo-2-fluoro-4-methoxy-benzaldehyde (10.01 g, 42.9551 mmol) and Rhodanine (5.72 g, 42.9456 mmol) in Acetic acid (100 mL) at 20°C. The reaction mixture was heated to 140°C and stirred overnight. The reaction mixture was added to water (1000 mL) and stirred for 10 min. The precipitate was collected by filtration, washed with water (200 mL). The filtrate cake was dried under vacuo at 60°C. There was (5E)-5-[(3-bromo-2-fluoro-4-methoxy-phenyl)methylene]-2-thioxo-thiazolidin-4-one (9.21 g, 26.4495 mmol, 61.5748% yield) obtained as a yellow solid. LCMS: (ESI, m/z): $[M+H]^+$ = 347.909. $^1$H NMR (400 MHz, DMSO-$d_6$) $\delta$ 13.87 (s, 1H), 7.68 - 7.32 (m, 2H), 7.16 (d, *J* = 8.7 Hz, 1H), 3.97 (s, 3H).

***Step c: (Z)-3-(3-bromo-2-fluoro-4-methoxy-phenyl)-2-sulfanyl-prop-2-enoic acid***

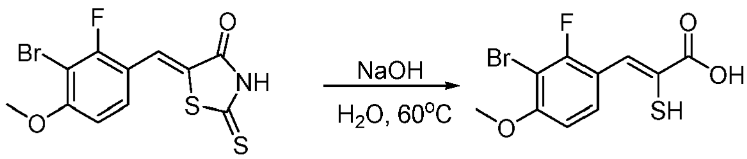


To a stirred mixture of (5E)-5-[(3-bromo-2-fluoro-4-methoxy-phenyl)methylene]-2-thioxo-thiazolidin-4 -one (8.03 g, 23.0607 mmol) in Water (200 mL) was added NaOH (9.15 g, 228.7666 mmol) in water (80 mL) at 20°C. The reaction mixture was stirred at 60°C for 1 h. After the reaction completed, adjusted to pH=3 with HCl. The precipitate was collected by filtration, washed with water (1 x 100 mL). The filtrate cake was dried under vacuo at 60°C. There was (Z)-3-(3-bromo-2-fluoro-4-methoxy-phenyl)-2-sulfanyl-prop-2-enoic acid (7.37 g, 23.9959 mmol, 104.0552% yield) obtained as a yellow solid. LCMS: (ESI, m/z): $[M+H]^+$ = 306.936.

***Step d: 5-bromo-4-fluoro-6-methoxy-benzothiophene-2-carboxylic acid***

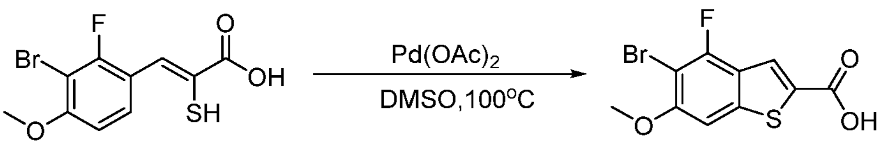


Palladium (II) acetate (4.0934 g, 18.2330 mmol) was added to a solution of (Z)-3-(3-bromo-2-fluoro-4-methoxy-phenyl)-2-sulfanyl-prop-2-enoic acid (7 g, 22.7912 mmol) in Methyl sulfoxide (50 mL) at 20°C under $N_2$ atmosphere. The reaction mixture was heated to 100°C and stirred for 1 h. The reaction mixture was purified on C-18 column MeCN/water (0-100%). The pure fractions was concentrated and dried under vacuo. There was 5-bromo-4-fluoro-6-methoxy-benzothiophene-2-carboxylic acid (3.9 g, 12.7819 mmol, 56.0824% yield) obtained as a yellow solid. LCMS: (ESI, m/z): $[M+H]^+$ = 304.921. $^1$H NMR (400 MHz, DMSO-$d_6$) $\delta$ 7.96 (s, 1H), 7.70 (s, 1H), 3.97 (s, 3H).

***Step e: tert-butyl 3-(5-bromo-4-fluoro-6-methoxy-benzothiophen-2-yl)-3-oxo-propanoate***

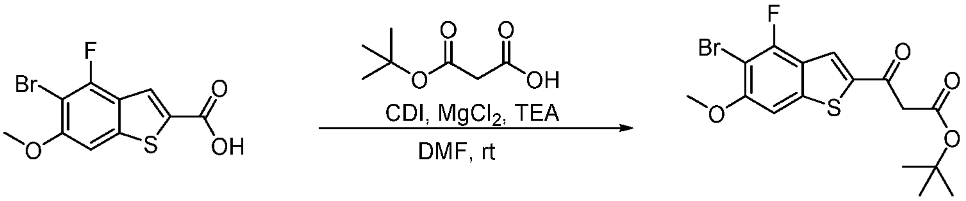


To a stirred solution of 5-bromo-4-fluoro-6-methoxy-benzothiophene-2-carboxylic acid (3.88 g, 12.7163 mmol) in DMF (50 mL) was added Carbonyl diimidazole (4.12 g, 25.4088 mmol) at 20°C. The resulting mixture was stirred for 1 h at 20°C under nitrogen atmosphere. To the mixture above was added Magnesium chloride (1.81 g, 19.0104 mmol), 3-tert-Butoxy-3-oxopropanoic acid (3.13 g, 19.5421 mmol) and Triethylamine (3.91 g, 38.6404 mmol). The reaction mixture was stirred overnight at 20°C. The reaction mixture was concentrated and diluted with EA (500 mL), washed with $NaHCO_3$ aq (2 x 500 mL) and brine (100 mL). The organics dried over $Na_2SO_4$, filtered and evaporated under reduced pressure. The residue was purified by flash silica chromatography, elution gradient 0 to 100% Ethyl acetate in heptane. The pure fractions was concentrated and dried under vacuo. There was tert-butyl 3-(5-bromo-4-fluoro-6-methoxy-benzothiophen-2-yl)-3-oxo-propanoate (3.29 g, 8.1585 mmol, 64.1574% yield) obtained as a yellow solid. LCMS: (ESI, m/z): $[M+H]^+$ = 402.994. $^1H$ NMR (400 MHz, DMSO-$d_6$) δ 8.38 (d, *J* = 2.2 Hz, 1H), 7.74 (d, *J* = 8.5 Hz, 1H), 4.16 (s, 2H), 3.97 (d, *J* = 8.2 Hz, 3H), 1.41 (d, *J* = 1.6 Hz, 10H).

**INT A10: tert-butyl 4-fluoro-5-hydroxy-6-methoxy-isoindoline-2-carboxylate**

***Step a: 4-fluoro-5-methoxy-isoindoline***

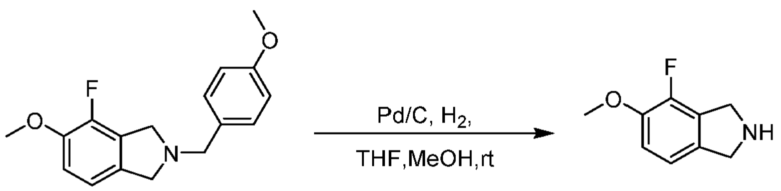


10% Pd/C (7.68 g, contain 55% $H_2O$) was added to a solution of 4-fluoro-5-methoxy-2-[(4-methoxyphenyl)methyl]isoindoline (7.18 g, 24.9889 mmol) in THF (70 mL) and MeOH (70 mL) at 20°C. The reaction mixture was stirred overnight at 20°C. The reaction mixure was filtered and the filter cake was washed with MeOH (70 mL). The filtrate was evaporated under reduced pressure. There was 4-fluoro-5-methoxy-isoindoline (4.88 g, 29.1901 mmol, 100% yield) obtained as a brown oil, which was used directly in the next step. LCMS: (ESI, m/z): $[M+H]^+$ = 168.200.

***Step b: 4-fluoroisoindolin-5-ol hydrobromate***

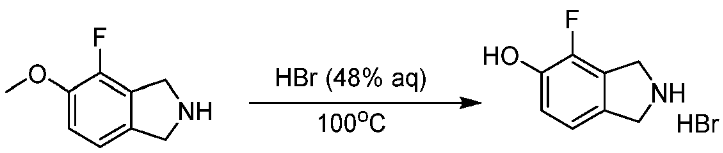


4-fluoro-5-methoxy-isoindoline (4.5 g, 26.9171 mmol) was dissolved in Hydrobromic acid (120 mL, 48% aq) at 20°C. The reaction mixture was heated to 100°C and stirred for 6 h. The resulting reaction mixture was evaporated under reduced pressure. The residue was treated with n-hexane/EA (1:2). The solid was collected by filtration, and dried under reduced pressure. There was 4-fluoroisoindolin-5-ol hydrobromate (4.70 g, 20.0799 mmol, 74.60% yield) obtained as a brown solid. LCMS: (ESI, m/z): $[M+H]^+$ = 154.200. $^1H$ NMR (400 MHz, DMSO-$d_6$) δ 10.09 (s, 1H), 9.58 (s, 2H), 7.10 - 6.93 (m, 2H), 4.57 (s, 2H), 4.44 (s, 2H).

***Step c: tert-butyl 4-fluoro-5-hydroxy-isoindoline-2-carboxylate***

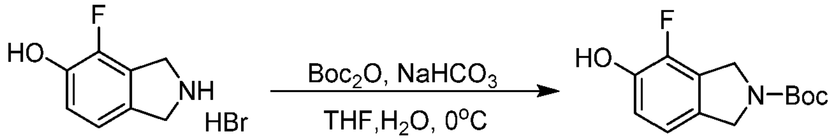


$NaHCO_3$ (3.43 g, 40.8301 mmol) was added to a solution of 4-fluoroisoindolin-5-olhydrobromate (4.70 g, 20.0799 mmol) in Water (80 mL) at 0°C. After the reaction mixure was stirred for 20 min, THF (60 mL) was added to the reaction mixture, and Di-tert-butyl dicarbonate (4.45 g, 20.3898 mmol) in THF (20 mL) was added dropwise to the mixure at 0°C. After stirring for 20 min, the reaction mixure was warmed to 20°C and stirred for 2 h. The resulting reaction mixture was diluted with EA (150 mL) and washed with brine (150 mL). The organic layer was dried over $Na_2SO_4$, filtered and evaporated under reduced pressure. The residue was purified by flash chromatography, eluting with ethyl acetate in n-hexane (0-40%). The pure fractions was concentrated and dried under reduced pressure. There was tert-butyl 4-fluoro-5-hydroxy-isoindoline-2-carboxylate (3.14 g, 12.3979 mmol, 60.78% yield) obtained as a reddish brown solid. LCMS: (ESI, m/z): $[M-H]^-$ = 252.000. $^1H$ NMR (400 MHz, $CDCl_3$-*d*) $\delta$ 7.00 - 6.93 (m, 1H), 6.93 - 6.83 (m, 1H), 4.72 (s, 2H), 4.64 (s, 2H), 1.54 (s, 9H).

***Step d: tert-butyl 6-bromo-4-fluoro-5-hydroxy-isoindoline-2-carboxylate***

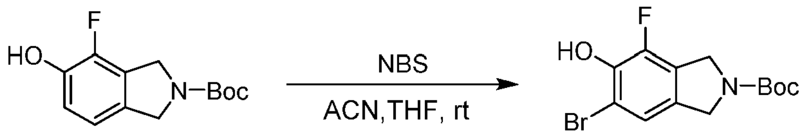


NBS (2.20 g, 12.3607 mmol) was added to a solution of tert-butyl 4-fluoro-5-hydroxy-isoindoline-2-carboxylate (3.10 g, 12.2400 mmol) in ACN (40 mL) and THF (20 mL) at 0°C. The reaction mixture was stirred for 2.5 h at 20°C. The reaction mixture was concentrated under reduced pressure. The residue was dissloved with EA/MeOH (200 mL/10 mL), and washed with $H_2O$ (200 mL). The organic layer was dried over $Na_2SO_4$, filtered and evaporated in vacuo. The residue was purified by flash chromatography, eluting with MeOH in DCM (0-5%). The pure fractions was concentrated and dried under reduced pressure. There was tert-butyl 6-bromo-4-fluoro-5-hydroxy-isoindoline-2-carboxylate (2.70 g, 8.1285 mmol, 66.34% yield) obtained as a white solid. LCMS: (ESI, m/z): $[M-H]^-$ = 331.950. $^1H$ NMR (400 MHz, DMSO-$d_6$) $\delta$ 10.36 (s, 1H), 7.33 (s, 0.5H), 7.32 (s, 0.5H), 4.58 (s, 1H), 4.55 (s, 1H), 4.52 (s, 1H), 4.50 (s, 1H), 1.45 (s, 9H).

***Step e: tert-butyl 5-benzyloxy-6-bromo-4-fluoro-isoindoline-2-carboxylate***

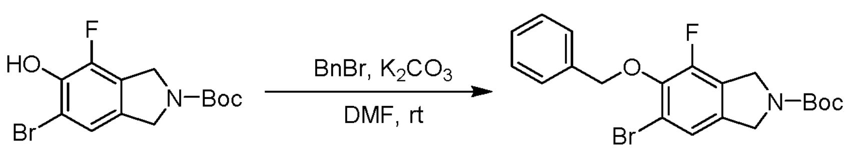


$K_2CO_3$ (2.20 g, 15.9183 mmol) was added to a solution of tert-butyl 6-bromo-4-fluoro-5-hydroxy-isoindoline-2-carboxylate (2.70 g, 8.1285 mmol) in DMF (50 mL) at 0°C. After the reaction mixture was stirred for 0.5 h at 0°C, BnBr (1.90 g, 11.1089 mmol) was added to the reaction mixture. The reaction mixture was stirred for 9 h at 20°C. The reaction mixture was diluted with EA (150 mL) and washed with water (150 mL) and brine (2 x 150 mL). The organics dried over $Na_2SO_4$, filtered and evaporated under reduced pressure. The residue was purified by flash chromatography, eluting with ethyl acetate in n-hexane (0-10%). The pure fractions was concentrated and dried under reduced pressure. There was tert-butyl 5-benzyloxy-6-bromo-4-fluoro-isoindoline-2-carboxylate (3.43 g, 8.1224 mmol, 99.93% yield) obtained as a white semi-solid. LCMS: (ESI, m/z): $[M-tBu+ACN]^+$ = 407.050. $^1H$ NMR (400 MHz, $CDCl_3$-*d*) $\delta$ 7.54 (d, *J* = 7.3 Hz, 2H), 7.45 - 7.35 (m, 3H), 7.27 (s, 0.5H), 7.20 (s, 0.5H), 5.13 (s, 2H), 4.70 (s, 1H), 4.65 (s, 2H), 4.62 (s, 1H), 1.54 (s, 9H) .

***Step f: tert-butyl 5-benzyloxy-4-fluoro-6-hydroxy-isoindoline-2-carboxylate***

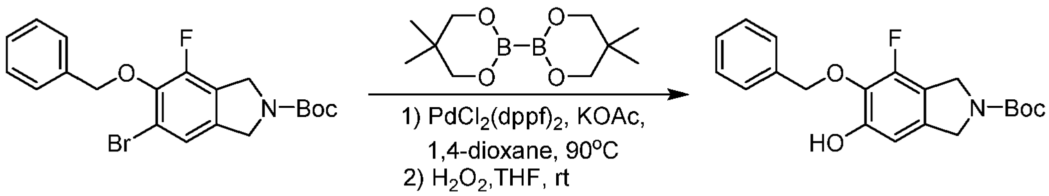


Pd(dppf)$Cl_2$ (1.12 g, 1.5307 mmol) was added to a solution of tert-butyl 5-benzyloxy-6-bromo-4-fluoro-isoindoline-2-carboxylate (3.30 g, 7.8146 mmol), bis(neopentylglycolato)diboron (5.46 g, 24.1716 mmol) and KOAc (2.32 g, 23.6392 mmol) in 1,4-Dioxane (80 mL) at 20°C. The reaction mixture was heated to 90°C and stirred overnight under nitrogen atmosphere.

The reaction mixture was diluted with EA (100 mL) and filtered through a celite. The filtrate was evaporated under reduced pressure. $H_2O_2$ (88.1974 mmol, 10 mL, 30% aqueous solution) was added to a mixture of the residue and $NaHCO_3$ ( 11.9038 mmol, 20 mL, 5% aqueous solution) in THF (80 mL) at 0°C. The reaction mixture was stirred for 2 h at 20°C. The resulting reaction mixture was diluted with brine (150 mL) and saturated $NaHSO_3$ (100 mL), and extracted with EA (200 mL). The organic layer was collected and dried over $Na_2SO_4$, filtered and evaporated under reduced pressure. The residue was purified on silica gel column chromatography, eluting with ethyl acetate/n-hexane (0-10%). There was tert-butyl 5-benzyloxy-4-fluoro-6-hydroxy-isoindoline-2-carboxylate (1.93 g, 5.3702 mmol, 68.93% yield) obtained as an off-white solid. LCMS: (ESI, m/z): $[M-H]^-$ = 358.100. $^1H$ NMR (400 MHz, $CDCl_3$-*d*) $\delta$ 7.46 - 7.36 (m, 5H), 6.62 (s, 0.5H), 6.58 (s, 0.5H), 5.15 (s, 2H), 4.68 (s, 1H), 4.65 (s, 1H), 4.61 (s, 1H), 4.58 (s, 1H), 1.54 (s, 9H).

***Step g: tert-butyl 5-benzyloxy-4-fluoro-6-methoxy-isoindoline-2-carboxylate***

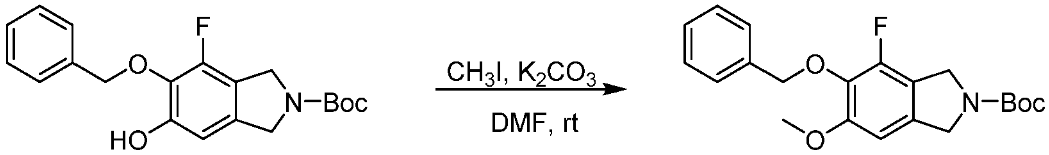


$K_2CO_3$ (1.18 g, 8.5380 mmol) was added to a solution of tert-butyl 5-benzyloxy-4-fluoro-6-hydroxy-isoindoline-2-carboxylate (1.9 g, 5.2867 mmol) in DMF (25 mL) at 0°C. After the mixture was stirred for 30 min at 0°C, $CH_3I$ (1.12 g, 7.8908 mmol) was added. The reaction mixture was stirred for 3 h at 20°C. The resulting reaction mixture was diluted with water (200 mL) and extracted with EA (200 mL). The organic layer was separated and washed with brine (2 x 150 mL). The organic layer was collected and dried over $Na_2SO_4$, filtered and evaporated under reduced pressure. There was tert-butyl 5-benzyloxy-4-fluoro-6-methoxy-isoindoline-2-carboxylate (1.98 g, 5.3024 mmol, 100% yield) obtained as a colorless oil, which was directly used in the next step without purification. LCMS: (ESI, m/z): $[M-tBu+ACN]^+$ = 359.150. $^1H$ NMR (400 MHz, $CDCl_3$-*d*) $\delta$ 7.39 (d, *J* = 7.4 Hz, 2H), 7.31 - 7.21 (m, 3H), 6.51 (s, 1H), 4.99 (s, 2H), 4.55 (s, 4H), 3.77 (s, 3H), 1.44 (s, 9H).

***Step h: tert-butyl 4-fluoro-5-hydroxy-6-methoxy-isoindoline-2-carboxylate***

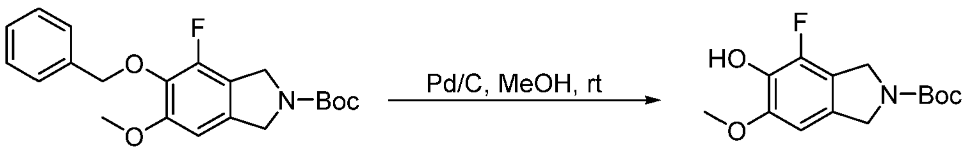


10% Pd/C (1.98 g, contain 55% $H_2O$) was added to a solution of tert-butyl 5-benzyloxy-4-fluoro-6-methoxy-isoindoline-2-carboxylate (1.96 g, 5.2488 mmol) in MeOH (40 mL) and THF (10 mL) at 20°C. The reaction mixture was stirred for 5 h at 20°C under $H_2$ atmosphere. The reaction mixture was diluted with EA (20 mL) and filtered through a celite.

The filtrate was evaporated under reduced pressure. The residue was purified on silica gel column chromatography, eluting with ethyl acetate/n-hexane (0-40%). The pure fractions was concentrated and dried under reduced pressure. There was tert-butyl 4-fluoro-5-hydroxy-6-methoxy-isoindoline-2-carboxylate (1.21 g, 4.2712 mmol, 80.67% yield) obtained as a brown semi-solid. LCMS: (ESI, m/z): $[M-H]^-$ = 282.100. $^1$H NMR (400 MHz, $CDCl_3$-*d*) δ 6.58 (s, 1H), 4.67 (s, 2H), 4.63 (s, 2H), 3.92 (s, 3H), 1.53 (s, 9H).

**INT A11: ethyl 4-(4-fluoro-5-hydroxy-6-methoxy-isoindolin-2-yl)-4-oxo-butanoate**

***Step a: 7-fluoro-6-methoxy-2-[(4methoxyphenyl) methyl] isoindolin-1-one***

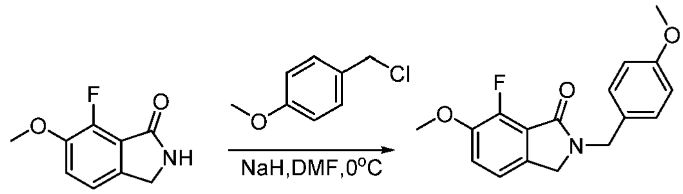


4-Methoxybenzylchloride (4.141g, 26.4416 mmol) was added to a solution of NaH (1.000 g, 25.0024 mmol) and 7-fluoro-6-methoxyisoindolin-1-one (4.336 g, 23.9342 mmol) in DMF (100 mL) at 0°C under $N_2$ atmosphere. The reaction mixture was stirred for 3 h and warmed up to 20°C naturally. The reaction mixture was quenched with adding to water (300 mL) at 20°C, extracted with EA (3 x 200 mL) and washed with brine (150 mL). The organics dried over $Na_2SO_4$, filtered and evaporated under reduced pressure. The residue was purified on silica gel column EA/n-Hex (0-100%). The pure fractions was concentrated and dried under vacuo. There was 7-fluoro-6-methoxy-2-[(4methoxyphenyl) methyl] isoindolin-1-one (6.51 g, 21.6055 mmol, 90.2706% yield) obtained as a yellow solid. LCMS: (ESI, m/z): $[M+H]^+$ = 302.110. $^1$H NMR (400 MHz, DMSO-$d_6$) δ 7.39 (t, *J* = 7.9 Hz, 1H), 7.27 (d, *J* = 8.2 Hz, 1H), 7.22 (d, *J* = 8.2 Hz, 2H), 6.91 (d, *J* = 8.3 Hz, 2H), 4.60 (s, 2H), 4.25 (s, 2H), 3.87 (s, 3H), 3.73 (s, 3H).

***Step b: 4-fluoro-5-methoxy-2[(4methoxyphenyl)methyl]isoindoline***

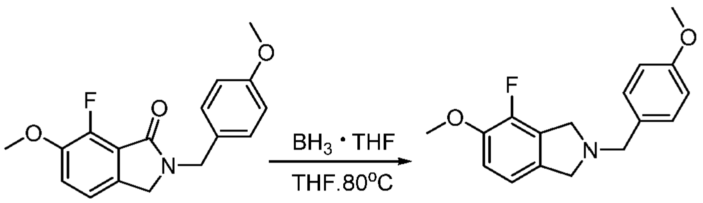


Borane-tetrahydrofuran complex (120 mL,120 mmol) was added to a solution of 7-fluoro-6-methoxy-2-(4-methoxybenzyl)isoindolin-1-one (6.17 g, 20.4771 mmol) in THF (60 mL) at 20°C under $N_2$ atmosphere. The reaction mixture was heated to 80°C and stirred for 5 h. The reaction mixture was quenched with pouring into MeOH (300 mL) at 20°C and evaporated under reduced pressure. The residue was diluted with MeOH (100 mL). The precipitate was collected by filtration, washed with MeOH (50 mL). The filter cake was dried under vacuo. There was 4-fluoro-5-methoxy-2 [(4methoxyphenyl)methyl]isoindoline (3.854 g, 13.4132 mmol, 65.5036% yield) obtained as a white solid. LCMS: (ESI, m/z): $[M+H]^+$ = 288.130.$^1$H NMR (400 MHz, DMSO-$d_6$) δ 7.41 (d, *J* = 8.0 Hz, 2H), 7.06 - 6.93 (m, 2H), 6.88 (d, *J* = 8.0 Hz, 2H), 4.53 - 4.37 (m, 2H), 4.22 - 4.08 (m, 4H), 3.79 (s, 3H),3.74 (s, 3H).

***Step c: ethyl 4-(4-fluoro-5-methoxy-isoindolin-2-yl)-4-oxo-butanoate***

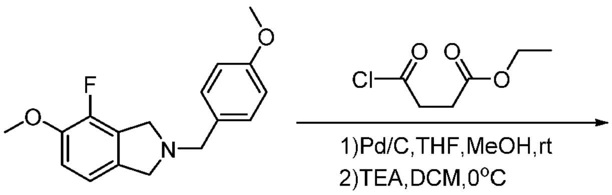


Pd/C (2.17 g, 2.0391 mmol) was added to a solution of 4-fluoro-5-methoxy-2-(4-methoxybenzyl)isoindoline (3.36 g, 11.6940 mmol) in THF (20 mL) and MeOH (20 mL) at 20°C. The reaction mixture was stirred overnight at 20°C under $H_2$ atmosphere. The precipitate was collected by filtration, washed with MeOH (50 mL). The filtrate was evaporated under reduced pressure. Ethyl Succinyl Chloride (5.05 g, 30.6830 mmol) was added to a solution of the residue and TEA (3.91 g, 38.6404 mmol) in DCM (50 mL) at 0°C under $N_2$ atmosphere. The reaction mixture was stirred for 1 h at 20°C. The reaction mixture was quenched with adding of water (50 mL) at 20°C, extracted with DCM (3 x 50 mL) and washed with brine (50 mL). The organics dried over $Na_2SO_4$, filtered and evaporated under reduced pressure. The residue was purified on silica gel column EA/n-Hex (0-45%). The pure fractions was concentrated and dried under vacuo. There was ethyl 4-(4-fluoro-5-methoxy-isoindolin-2-yl)-4-oxo-butanoate (2.619 g, 8.8688 mmol, 75.8406% yield) obtained as a yellow solid. LCMS: (ESI, m/z): $[M+H]^+$ = 296.120. $^1H$ NMR (400 MHz, DMSO-$d_6$) $\delta$ 7.16 - 7.09 (m, 2H), 4.91 (s, 1H), 4.81 (s, 1H), 4.64 (s, 1H), 4.58 (s, 1H), 4.05 (q, $J$ = 7.2 Hz, 2H), 3.84 (s, 3H), 2.73 - 2.53 (m, 4H), 1.18 (t, $J$ = 7.2 Hz, 3H).

***Step d: ethyl 4-(4-fluoro-5-hydroxy-isoindolin-2-yl)-4-oxo-butanoate***

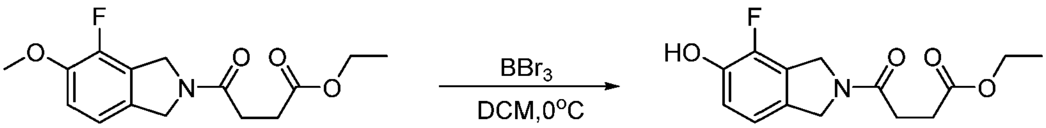


Tribromoboron (20 mL, 20 mmol) was added to a solution of ethyl 4-(4-fluoro-5-methoxyisoindolin-2-yl)-4-oxobutanoate (2.597 g, 8.7943 mmol) in DCM (40 mL) at 0°C under $N_2$ atmosphere. The reaction mixture was stirred for 2 h at 20°C. The reaction mixture was quenched with adding to EtOH (100 mL) at 20°C, The reaction mixture was evaporated under reduced pressure and diluted with $H_2O$ (200 mL), extracted with EA (2 x 100 mL) and washed with brine (100 mL). The organics dried over $Na_2SO_4$, filtered and evaporated under reduced pressure. There was ethyl 4-(4-fluoro-5-hydroxy-isoindolin-2-yl)-4-oxo-butanoate (2.408 g, 8.5609 mmol, 97.3461% yield) obtained as a yellow solid. LCMS: (ESI, m/z): $[M+H]^+$ = 282.110. $^1H$ NMR (400 MHz, DMSO-$d_6$) $\delta$ 9.83 (s, 1H), 6.99 - 6.85 (m, 2H), 4.88 (s, 1H), 4.77 (s, 1H), 4.62 (s, 1H), 4.54 (s, 1H), 4.05 (q, $J$ = 7.2 Hz, 2H), 2.66 - 2.53 (m, 4H), 1.18 (t, $J$ = 7.2 Hz, 3H).

***Step e: ethyl 4-(6-bromo-4-fluoro-5-hydroxy-isoindolin-2-yl)-4-oxo-butanoate***

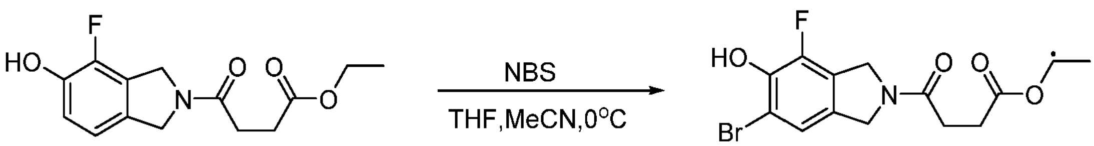


NBS (1.194 g, 6.7085 mmol) was added to a solution of ethyl 4-(4-fluoro-5-hydroxy-isoindolin-2-yl)-4-oxo-butanoate (2.106 g, 7.4872 mmol) in MeCN (20 mL) and THF (20 mL) at 0°C under $N_2$ atmosphere. The reaction mixture was stirred for 2 h and warmed up to 20°C naturally. The precipitate was collected by filtration. The filter cake was dried under vacuo. There was ethyl 4-(6-bromo-4-fluoro-5-hydroxy-isoindolin-2-yl)-4-oxo-butanoate (2.84 g, 7.8851 mmol, 105.3134% yield) obtained as a white solid. LCMS: (ESI, m/z): $[M+H]^+$ = 359.010. $^1H$ NMR (400 MHz, DMSO-$d_6$) $\delta$ 10.41 (s, 1H), 7.36 (s, 0.5H), 7.35 (s, 0.5H), 4.87 (s, 1H), 4.78 (s, 1H), 4.60 (s, 1H), 4.55 (s, 1H), 4.14 - 3.97 (q, $J$ = 7.2 Hz, 2H), 2.70 - 2.52 (m, 4H), 1.18 (t, $J$ = 7.2 Hz, 3H).

***Step f: ethyl 4-(6-bromo-4-fluoro-5-(methoxymethoxy)isoindolin-2-yl)-4-oxobutanoate***

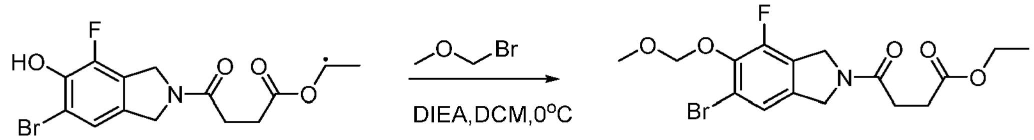


Bromomethyl methyl ether (1.20 g, 9.6028 mmol) was added to a solution of ethyl 4-(6-bromo-4-fluoro-5-hydroxy-isoindolin-2-yl)-4-oxo-butanoate (2.23 g, 6.1914 mmol) and DIEA (2.55 g, 19.7304 mmol) in DCM (50 mL) at 0°C under $N_2$ atmosphere. The reaction mixture was stirred for 2 h and warmed up to 20°C naturally. The reaction mixture was quenched with adding of water (50 mL) at 20°C, extracted with DCM (3 x 50 mL) and washed with brine (50 mL). The organics dried over $Na_2SO_4$, filtered and evaporated under reduced pressure. The residue was purified on C18 column ACN/$H_2O$ (0.1%FA)(0-50%). The pure fractions was concentrated and dried under vacuo. There was ethyl 4-(6-bromo-4-fluoro-5-(methoxymethoxy)isoindolin-2-yl)-4-oxobutanoate (1.809 g, 4.4752 mmol, 72.2806% yield) obtained as a yellow solid. LCMS: (ESI, m/z): $[M+H]^+$ = 403.140. $^1H$ NMR (400 MHz, DMSO-*d*$_6$) δ 7.52 (s, 0.5H), 7.51 (s, 0.5H), 5.16 (s, 2H), 4.90 (s, 1H), 4.85 (s, 1H), 4.63 (s, 1H), 4.61 (s, 1H), 4.05 (q, *J* = 7.2 Hz, 2H), 3.54 (s, 3H), 2.72 - 2.53 (m, 4H), 1.24 - 1.12 (t, *J* = 7.2 Hz, 3H).

***Step g: ethyl 4-(4-fluoro-6-hydroxy-5-(methoxymethoxy)isoindolin-2-yl)-4-oxobutanoate***

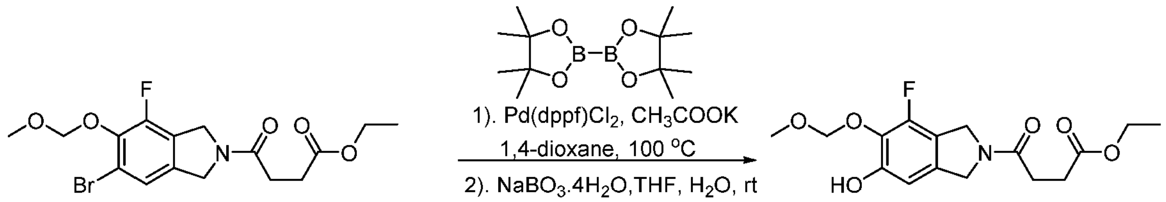


Pd(dppf)$Cl_2$ (0.228 g, 311.6016 μmol), 4, 4,4',4',5,5,5',5'-Octamethyl-2,2'-bi(1,3,2-dioxaborolane) (1.170 g, 4.6074 mmol) and Potassium Acetate (0.743 g, 7.5706 mmol) was added to a solution of ethyl 4-(6-bromo-4-fluoro-5-(methoxymethoxy)isoindolin-2-yl)-4-oxobutanoate (0.735 g, 1.8183 mmol) in 1,4-Dioxane (20 mL) at 20°C under $N_2$ atmosphere. The reaction mixture was heated to 100°C and stirred for 12 h. The reaction mixture was quenched with adding of water (150 mL) at 20°C, extracted with EA (3 x 150 mL) and washed with brine (50 mL). The organics dried over $Na_2SO_4$, filtered and evaporated under reduced pressure. Sodium perborate (0.91g, 5.9145 mmol) was added to a solution of the residue in THF (10 mL) and $H_2O$ (10 mL) at 20°C under $N_2$ atmosphere. The reaction mixture was stirred for 1 h at 20°C. The reaction mixture was evaporated under reduced pressure. The residue was purified on C18 column ACN/$H_2O$ (0.1%FA)(0-38%). The pure fractions was concentrated and dried under vacuo. There was ethyl 4-(4-fluoro-6-hydroxy-5-(methoxymethoxy)isoindolin-2-yl)-4-oxobutanoate (0.365 g, 1.0693 mmol, 58.8% yield) obtained as a white solid. LCMS: (ESI, m/z): $[M+H]^+$ =342.130. $^1H$ NMR (400 MHz, DMSO-*d*$_6$) δ 9.97 (s, 1H), 6.68 (s, 1H), 5.04 (s, 2H), 4.80 (s, 1H), 4.76 (s, 1H), 4.55 (s, 1H), 4.53 (s, 1H), 4.05 (q, *J* = 7.2 Hz, 2H), 3.47 (s, 3H), 2.67 - 2.51 (m, 4H), 1.18 (t, *J* = 7.2 Hz, 3H).

***Step h: ethyl 4-(4-fluoro-5-hydroxy-6-methoxy-isoindolin-2-yl)-4-oxo-butanoate***

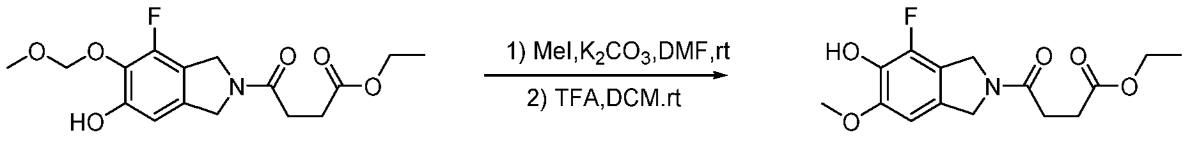


Methyl Iodidle (0.345 g, 2.4306 mmol) was added to a mixture of Potassium carbonate (0.448 g, 3.2415 mmol) and ethyl 4-(4-fluoro-6-hydroxy-5-(methoxymethoxy)isoindolin-2-yl)-4-oxobutanoate (0.328 g, 960.9441 μmol) in DMF (10 mL) at 20°C under $N_2$ atmosphere. The reaction mixture was stirred for 1 h at 20°C. The reaction mixture was quenched with adding of water (50 mL) at 20°C, extracted with DCM (3 x 50 mL) and washed with brine (50 mL). The organics dried over $Na_2SO_4$, filtered and evaporated under reduced pressure. Trifluoroacetic acid (2 mL) was added to a solution of the residue (0.446 g, 1.2551 mmol) in DCM (5 mL) at 20°C under $N_2$ atmosphere. The reaction mixture was stirred for 2 h at 20°C. The reaction mixture was evaporated under reduced pressure. The residue was purified on C18 column ACN/ $H_2O$

(0-80%). The pure fractions was concentrated and dried under vacuo. There was ethyl 4-(4-fluoro-5-hydroxy-6-methoxy-isoindolin-2-yl)-4-oxo-butanoate (0.274 g, 880.1663 μmol, 91.6% yield) obtained as a white solid. LCMS: (ESI, m/z): $[M+H]^+$ = 312.12. $^1H$ NMR (400 MHz, DMSO-$d_6$) δ 9.17 (s, 1H), 6.83 (s, 0.5H), 6.80 (s, 0.5H), 4.82 (s, 1H), 4.77 (s, 1H), 4.57 (s, 1H), 4.54 (s, 1H), 4.05 (q, $J$ = 7.2 Hz, 2H), 3.80 (d, $J$ = 2.0 Hz, 3H), 2.68 - 2.51 (m, 4H), 1.18 (t, $J$ = 7.2 Hz, 3H).

**INT A12: ethyl 4-(6-hydroxy-5-methoxythieno[3,2-b]pyridin-2-yl)-4-oxobutanoate**

***Step a: 6-bromo-5-methoxythieno[3,2-b]pyridine***

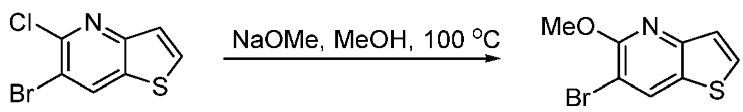


A solution of 6-bromo-5-chlorothieno[3,2-b]pyridine (1.0 g, 4.0 mmol) and NaOMe (9.3 ml, 40 mmol) in MeOH (6 mL) was stirred at 100 °C for 1 h under $N_2$ atmosphere in microwave irradiation reactor. 1 N citric acid (80 mL) was added, then extracted with EtOAc (70 mL x 3). The combined organic solution was dried over $Na_2SO_4$, concentrated to give 6-bromo-5-methoxythieno[3,2-b]pyridine (1.0 g, 4.0 mmol, 99% yield) as a brown solid. $^1H$ NMR (300 MHz, DMSO-$d_6$): δ 8.75 (d, $J$ = 0.5 Hz, 1H), 8.08 (d, $J$ = 5.5 Hz, 1H), 7.42 (dd, $J$ = 5.5, 0.5 Hz, 1H), 3.98 (s, 3H). LCMS: (ESI, m/z): $[M+H]^+$ = 244.0.

***Step b: 6-bromo-5-methoxythieno[3,2-b]pyridine-2-carboxylic acid***

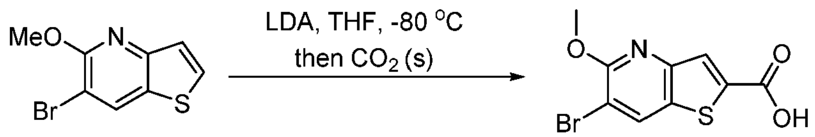


To a solution of 6-bromo-5-methoxythieno[3,2-b]pyridine (5.0 g, 20.0 mmol) in THF (200 mL) was added LDA (20 ml, 40 mmol) at -80 °C. The reaction mixture was stirred at -80 °C for 30 min under $N_2$ atmosphere. Then the solution was poured onto $CO_2$ solid. The reaction mixture was stirred for about 1 h. 1 N HCl (50 mL) and water (200 ml) was added, then extracted with EtOAc (70 mL x 3). The combined organic solution was dried over $Na_2SO_4$, concentrated to give 6-bromo-5-methoxythieno[3,2-b]pyridine-2-carboxylic acid (4.9 g, 17.0 mmol, 85% yield) as a brown solid. $^1H$ NMR (300 MHz, DMSO-$d_6$): δ 8.84 (s, 1H), 7.92 (s, 1H), 4.00 (s, 3H). LCMS: (ESI, m/z): $[M+H]^+$ = 287.9.

***Step c: tert-butyl 3-(6-bromo-5-methoxythieno[3,2-b]pyridin-2-yl)-3-oxopropanoate***

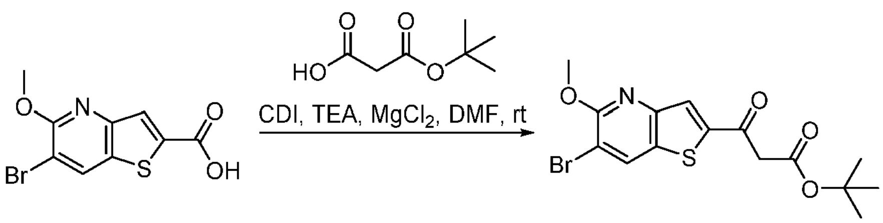


To a solution of 6-bromo-5-methoxythieno[3,2-b]pyridine-2-carboxylic acid (4.9 g, 17.0 mmol) in DMF (147 mL) was added CDI (5.5 g, 34.0 mmol). After stirring at RT for 2 h under $N_2$ atmosphere, 3-tert-Butoxy-3-oxopropanoic acid (5.5 g, 34.0 mmol), TEA (5.2 g, 51.0 mmol, 3.0 eq) and $MgCl_2$ (3.3 g, 34.0 mmol) was added, then stirred at RT for overnight. Upon completion, sat. $NaHCO_3$ solution (200 mL) was added, then extracted with EtOAc (200 mL x 3). The combined organic solution was dried over $Na_2SO_4$, concentrated to give a residue, purified by column chromatography (Petroleum ether/Ethyl acetate = 20: 1) to give tert-butyl 3-(6-bromo-5-methoxythieno[3,2-b]pyridin-2-yl)-3-oxopropanoate (5.0 g, 12.9 mmol, 62% yield) as a yellow solid.

$^1H$ NMR (300 MHz, DMSO-$d_6$): δ 8.87 (s, 1H), 8.33 (s, 1H), 4.17 (s, 2H), 4.01 (s, 3H), 1.40 (s, 9H). LCMS: (ESI, m/z): [M+H]$^+$ = 386.0.

***Step d: 1-(tert-butyl) 4-ethyl 2-(6-bromo-5-methoxythieno[3,2-b]pyridine-2-carbonyl)succinate***

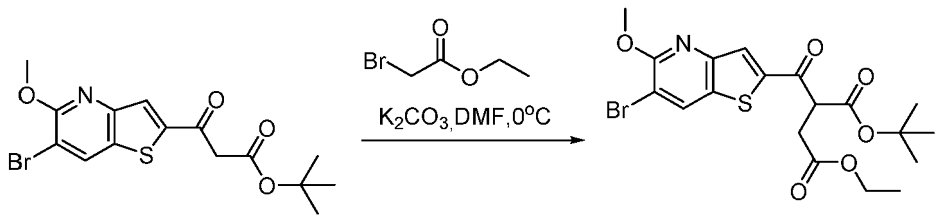


To a solution of tert-butyl 3-(6-bromo-5-methoxythieno[3,2-b]pyridin-2-yl)-3-oxopropanoate (3.0 g, 7.8 mmol) in DMF (60 mL) was added $K_2CO_3$ (1.8 g, 12.5 mmol) and Ethyl bromoacetate (1.3 g, 78mmol) at 0 °C. The reaction mixture was stirred at 0°C for 5 h under $N_2$ atmosphere. $NaHCO_3$ solution (100 mL) was added, then extracted with EtOAc (100 mL x 3). The combined organic solution was dried over $Na_2SO_4$, concentrated to give a residue, purified by column chromatography (Petroleum ether/Ethyl acetate = 20: 1) to give 1-(tert-butyl) 4-ethyl 2-(6-bromo-5-methoxythieno[3,2-b]pyridine-2-carbonyl)succinate (3.3 g, 7.0 mmol, 89% yield) as a white solid.

$^1H$ NMR (300 MHz, DMSO-$d_6$): δ 8.89 (s, 1H), 8.47 (s, 1H), 5.00 (*t, J* = 7.3 Hz, 1H), 4.12 - 3.93 (m, 5H), 2.94 (t, *J* = 7.3 Hz, 2H), 1.31 (d, *J* = 4.5 Hz, 9H), 1.13 (t, *J* = 7.1 Hz, 3H).
LCMS: (ESI, m/z): [M+H]$^+$ = 472.0.

***Step e: ethyl 4-(6-bromo-5-methoxythieno[3,2-b]pyridin-2-yl)-4-oxobutanoate***

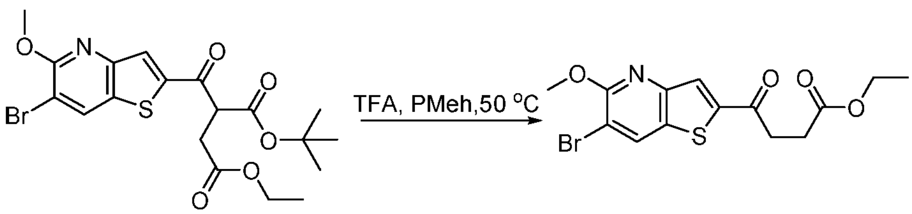


To a solution of 1-(tert-butyl) 4-ethyl 2-(6-bromo-5-methoxythieno[3,2-b]pyridine-2-carbonyl)succinate (3.3 g, 7.0 mmol) in PhMe (66 mL) was added TFA (33 ml). After stirring at 50 °C for 1.5 h under $N_2$ atmosphere, the solution was concentrated to give a residue, and the obtained solid was triturated with Petroleum ether / ethyl acetate (10/1) to obtain crude compound ethyl 4-(6-bromo-5-methoxythieno[3,2-b]pyridin-2-yl)-4-oxobutanoate (2.2 g, 5.9 mmol, 84% yield) as a brown solid. $^1H$ NMR (300 MHz, DMSO-$d_6$): δ 8.87 (s, 1H), 8.37 (s, 1H), 4.11 - 3.98 (m, 5H), 3.45 - 3.36 (m, 2H), 2.71 - 2.63 (m, 2H), 1.17 (t, *J* = 7.1 Hz, 3H).
LCMS: (ESI, m/z): [M+H]$^+$ = 372.0.

*Step f: (2-(4-ethoxy-4-oxobutanoyl)-5-methoxythieno[3,2-b]pyridin-6-yl)boronic acid*

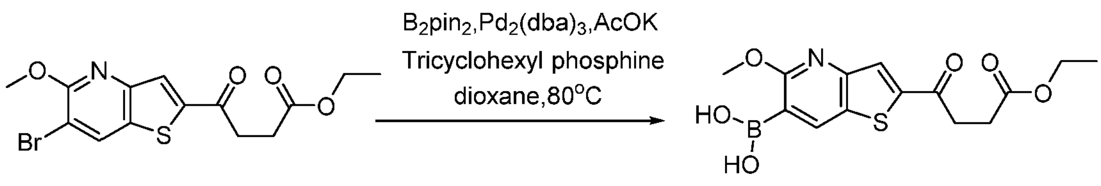


Ethyl 4-(6-bromo-5-methoxythieno[3,2-b]pyridin-2-yl)-4-oxobutanoate (1.6 g, 4.3 mmol), $B_2Pin_2$ (1.4 g, 5.3

mmol), AcOK (688 mg, 6.9 mmol), Pd2(dba)3 (112 mg, 0.1 mmol) and Tricyclohexyl phosphine (128 mg, 0.43 mmol) were placed in the reaction bottle. The bottle was evacuated and filled with argon for three times. Then dioxane (32 mL) was added at room temperature. The mixture was stirred at 80 °C for 1 h. Water (50 mL) was added to the reaction mixture, extracted with EtOAc (3 x 50 mL), and the combined organic layers were washed with saturated brine and dried over anhydrous sodium sulfate. The solution was concentrated to give (2-(4-ethoxy-4-oxobutanoyl)-5-methoxythieno[3,2-b]pyridin-6-yl)boronic acid (3.2 g) a crude residue. It was used for next step without any further purification. LCMS: (ESI, m/z): [M+H] + = 338.1.

***Step g: ethyl 4-(6-hydroxy-5-methoxythieno[3,2-b]pyridin-2-yl)-4-oxobutanoate***

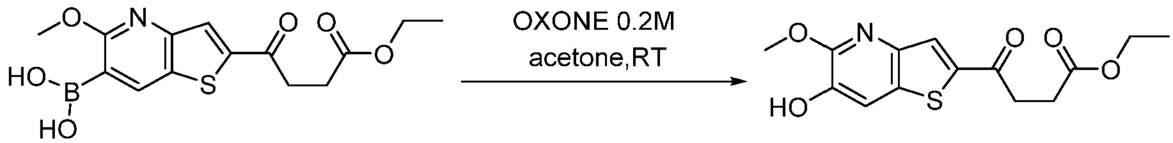


To a solution of (2-(4-ethoxy-4-oxobutanoyl)-5-methoxythieno[3,2-b]pyridin-6-yl)boronic acid (3.2 g, 4.3 mmol, crude) in acetone (48 mL) was added OXONE (36 ml, 6.5 mmol). After stirring at RT for 2 h. Sat. $NaHSO_3$ solution (120 mL) was added, after stirring at RT for 1 h, the mixture was extracted with EtOAc (3 x 30 mL), and the combined organic layers were washed with saturated brine and dried over anhydrous sodium sulfate. The solution was concentrated to give a crude residue, purified by column chromatography (Petroleum ether/Ethyl acetate = 10: 1 to 5:1) to give ethyl 4-(6-hydroxy-5-methoxythieno[3,2-b]pyridin-2-yl)-4-oxobutanoate (340 mg, 1.1 mmol, 25% yield) as a yellow solid. $^1$H NMR (400 MHz, *DMSO-$d_6$):* 2H), 3.97 (s, 3H), ~ 3.30-3.31 (br, 2 h), 2.65 (t, *J* = 6.1 Hz, 2H), 1.17 (t, *J* = 7.1 Hz, 3H).
LCMS: (ESI, m/z): [M+H] + = 310.1.

The following intermediates were synthesized with the above steps or improved procedure with the corresponding starting materials and intermediates.

| | | |
|---|---|---|
| 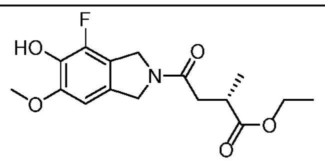 | 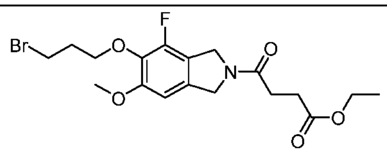 | 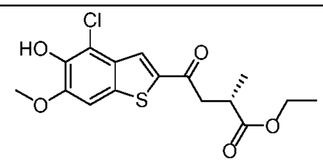 |

| | | |
|---|---|---|
| 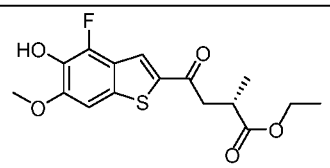 | 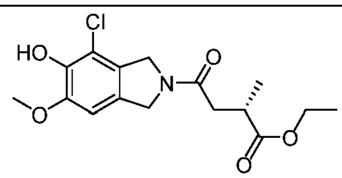 | 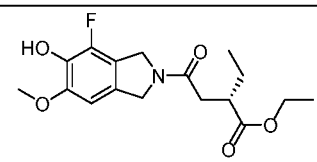 |
| 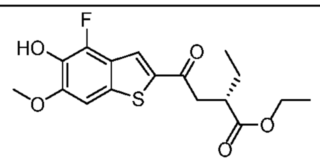 | 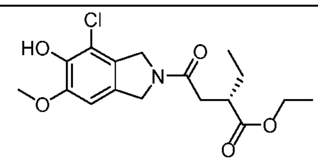 | 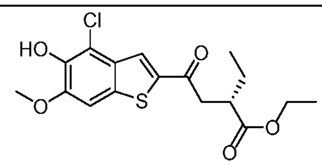 |
| 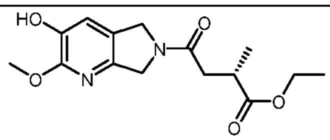 | 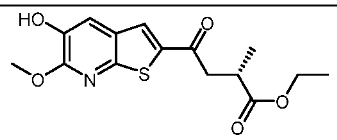 | |

**EXAMPLE 1**

**4-(5-(3-((2-(3-carboxypropanoyl)-6-methoxybenzo[b]thiophen-5-yl)oxy)propoxy)-6-methoxyisoindolin-2-yl)-4-oxobutanoic acid**

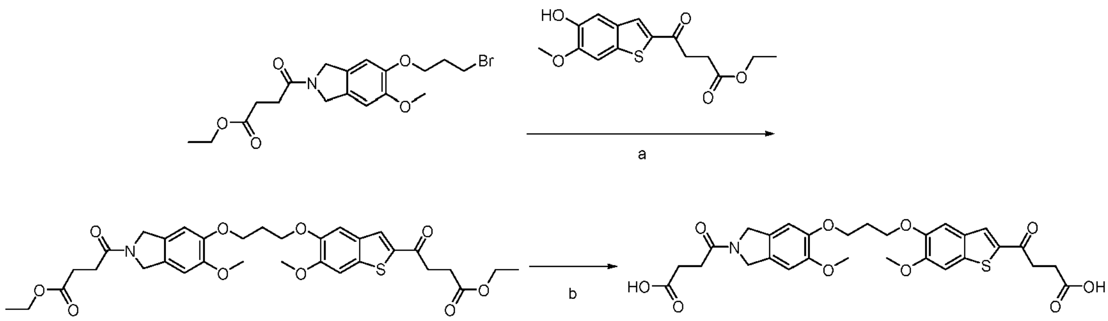


***Step a:*** Potassium carbonate (0.240 g, 1.7365 mmol) was added to a solution of ethyl 4-(5-(3-bromopropoxy)-6-methoxyisoindolin-2-yl)-4-oxobutanoate (0.240 g, 579.3045 μmol) and ethyl 4-(5-hydroxy-6-methoxybenzo[b]thiophen-2-yl)-4-oxobutanoate (0.169 g, 576.1737 μmol) in *N,N*-Dimethylformamide (10 mL) at 20 °C. The reaction mixture was heated to 50 °C and stirred for 3 h. The reaction mixture was evaporated under reduced pressure. The reaction mixture was concentrated and diluted with EA (500 mL), washed with $NaHCO_3$ aq. (2 x 300 mL) and brine (200 mL). The organics was dried over $Na_2SO_4$, filtered and evaporated under reduced pressure. The residue was purified on silica gel column EA/Hept (0-100%). The pure fractions was concentrated and dried under vacuo. There was ethyl 4-(5-(3-((2-(4-ethoxy-4-oxobutanoyl)-6-methoxybenzo[b]thiophen-5-yl)oxy)propoxy)-6-methoxyisoindolin-2-yl)-4-oxobutanoate (0.147 g, 229.0694 μmol, 62.8484% yield) obtained as a yellow solid.

$^1H$ NMR (400 MHz, DMSO-*d6*) δ 8.18 (s, 1H), 7.61 (d, *J* = 1.9 Hz, 1H), 7.53 (s, 1H), 6.98 (dd, *J* = 15.7, 11.9 Hz, 2H), 4.73 (d, *J* = 9.4 Hz, 2H), 4.52 (s, 2H), 4.17 (dd, *J* = 30.4, 7.5 Hz, 4H), 4.05 (qd, *J* = 7.1, 3.4 Hz, 4H), 3.86 (d, *J* = 1.6 Hz, 3H), 3.75 (d, *J* = 2.0 Hz, 3H), 2.70 - 2.53 (m, 6H), 2.30 - 2.15 (m, 2H), 1.24 - 1.11 (m, 6H).

***Step b:*** LiOH (0.022 g, 918.6456 μmol) was added to a solution of ethyl 4-(5-(3-((2-(4-ethoxy-4-oxobutanoyl)-6-methoxybenzo[b]thiophen-5-yl)oxy)propoxy)-6-methoxyisoindolin-2-yl)-4-oxobutanoate in Tetrahydrofuran (10 mL) and Water (2 mL) at 20 °C. The reaction mixture was stirred for 2 h. The reaction mixture was evaporated under reduced pressure and diluted with water and adjusted pH = 3 with HCl (1 M). The precipitate was filtered. Filter cake was washed with water (20 mL). The filtrate cake was dried under vacuo at 60 °C. There was 4-(5-(3-((2-(3-carboxypropanoyl)-6-methoxybenzo[b]thiophen-5-yl)oxy)propoxy)-6-methoxyisoindolin-2-yl)-4-oxobutanoic acid (0.119 g, 203.2031 μmol, 90.5562% yield) obtained as a yellow solid.

$^1H$ NMR (400 MHz, DMSO-*d6*) δ 12.12 (s, 2H), 8.17 (s, 1H), 7.67 - 7.55 (m, 1H), 7.53 (s, 1H), 6.98 (dd, *J* = 16.6, 12.5 Hz, 2H), 4.73 (d, *J* = 8.2 Hz, 2H), 4.53 (s, 2H), 4.18 (ddd, *J* = 23.1, 13.6, 6.3 Hz, 4H), 3.86 (d, *J* = 1.0 Hz, 3H), 3.75 (d, *J* = 1.9 Hz, 3H), 3.25 (d, *J* = 6.6 Hz, 2H), 2.67 - 2.53 (m, 4H), 2.30 - 2.11 (m, 2H).

**EXAMPLE 2**

**4-(5-(3-((2-(3-carboxypropanoyl)-4-chloro-6-methoxyisoindolin-5-yl)oxy)propoxy)-4-fluoro-6-methoxy benzo [b]thiophen-2-yl)-4-oxobutanoic acid**

***Step a: O1-tert-butyl O4-ethyl 2-(5-bromo-4-fluoro-6-methoxy-benzothiophene-2-carbonyl)butanedioate***

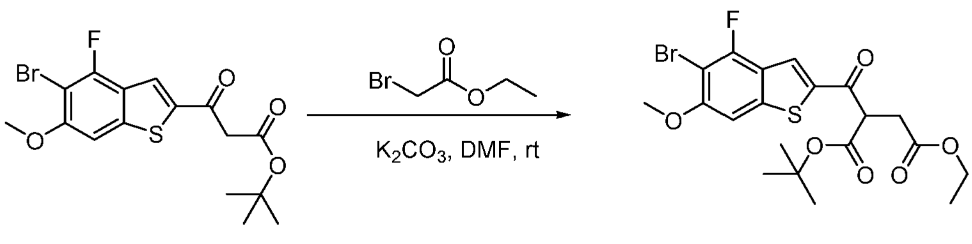


To a stirred solution of tert-butyl 3-(5-bromo-4-fluoro-6-methoxy-benzothiophen-2-yl)-3-oxo-propanoate (1.58 g, 3.9180 mmol) in *N,N*-Dimethylformamide (10 mL) was added $K_2CO_3$ (1.10 g, 7.9592 mmol) and ethyl bromoacetate (0.72

g, 4.3114 mmol) at 20°C. The resulting mixture was stirred for 2 h at 20°C under nitrogen atmosphere. The reaction mixture was concentrated and diluted with EA (200 mL), washed with water (100 mL) and brine (50 mL). The organics dried over $Na_2SO_4$, filtered and evaporated under reduced pressure. The residue was purified by flash silica chromatography, elution gradient 0 to 100% Ethyl acetate in heptane. The pure fractions was concentrated and dried under vacuo. There was O1-tert-butyl O4-ethyl 2-(5-bromo-4-fluoro-6-methoxy-benzothiophene-2-carbonyl)butanedioate (1.925 g, 3.9338 mmol, 100.4016% yield) obtained as a yellow oil. LCMS: (ESI, m/z): $[M+H]^+$ = 489.030.

***Step b: ethyl 4-(5-bromo-4-fluoro-6-methoxy-benzothiophen-2-yl)-4-oxo-butanoate***

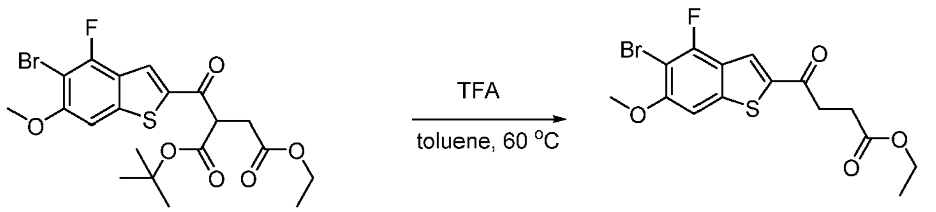


Trifluoroacetic acid (2 mL) was added to a solution of O1-tert-butyl O4-ethyl 2-(5-bromo-4-fluoro-6-methoxy-benzothiophene-2-carbonyl)butanedioate (1.85 g, 3.7805 mmol) in Toluene (10 mL) at 20°C. The reaction mixture was stirred at 60°C for 2 h. The reaction mixture was evaporated under reduced pressure. The residue was concentrated and diluted with DCM (200 mL), washed with $NaHCO_3$ aq (2 x 100 mL) and brine (100 mL). The organics dried over $Na_2SO_4$, filtered and evaporated under reduced pressure. The residue was purified by flash silica chromatography, elution gradient 0 to 100% Ethyl acetate in heptane. The pure fractions was concentrated and dried under vacuo. There was ethyl 4-(5-bromo-4-fluoro-6-methoxy-benzothiophen-2-yl)-4-oxo-butanoate (0.727 g, 1.8678 mmol, 49.4050% yield) obtained as a yellow solid. LCMS: (ESI, m/z): $[M+H]^+$ = 388.978. $^1H$ NMR (400 MHz, DMSO-$d_6$) $\delta$ 8.39 (s, 1H), 7.71 (s, 1H), 4.06 (q, $J$ = 6.9 Hz, 2H), 3.98 (s, 3H), 3.39 (d, $J$ = 6.2 Hz, 2H), 2.73 - 2.62 (m, 2H), 1.18 (t, $J$ = 7.1 Hz, 3H).

***Step c: ethyl***

***4-(4-fluoro-6-methoxy-5-(4,4,5,5-tetramethyl-1,3,2-dioxaborolan-2-yl)benzo[b]thiophen-2-yl)-4-oxobutan oate***

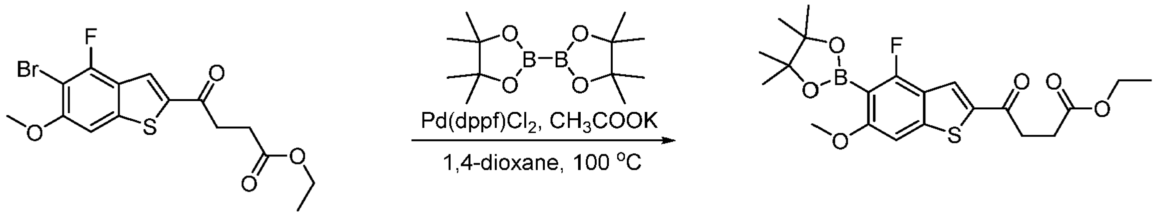


Pd(dppf)$Cl_2$ (0.15 g, 205.0010 μmol) was added to a mixture of Potassium Acetate (0.60 g, 6.1136 mmol), 4,4,4',4',5,5,5',5'-Octamethyl-2,2'-bi(1,3,2-dioxaborolane) (0.97 g, 3.8198 mmol) and ethyl 4-(5-bromo-4-fluoro-6-methoxy-benzothiophen-2-yl)-4-oxo-butanoate (0.72 g, 1.8498 mmol) in 1,4-Dioxane (30 mL) at 20°C. The reaction mixture was stirred at 100°C for 20 h under $N_2$ atmosphere. The reaction mixture was evaporated under reduced pressure. The residue was concentrated and diluted with EA (200 mL), washed with $NaHCO_3$ aq (2 x 100 mL) and brine (100 mL). The organics dried over $Na_2SO_4$, filtered and evaporated under reduced pressure. The residue was purified by flash silica chromatography, elution gradient 0 to 100% Ethyl acetate in heptane. The pure fractions was concentrated and dried under vacuo. There was ethyl 4-(4-fluoro-6-methoxy-5-(4,4,5,5-tetramethyl-1,3,2-dioxaborolan-2-yl)benzo[b]thiophen-2-yl)-4-oxobutanoa te (0.38 g, 870.9586 μmol, 47.0845% yield) obtained as a white solid. LCMS: (ESI, m/z): $[M+H]^+$ = 437.153.

***Step d: ethyl 4-(4-fluoro-5-hydroxy-6-methoxy-benzothiophen-2-yl)-4-oxo-butanoate***

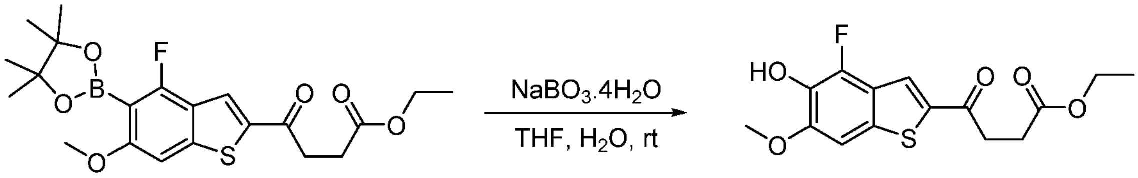


Sodium perborate tetrahydrate (0.21 g, 1.3649 mmol) was added to a solution of ethyl 4-(4-fluoro-6-methoxy-5-(4,4,5,5-tetramethyl-1,3,2-dioxaborolan-2-yl)benzo[b]thiophen-2-yl)-4-oxobutanoa te in Tetrahydrofuran (10 mL) and water (10 mL) at 20°C. The reaction mixture was stirred at 20°C for 1 h. The reaction mixture was concentrated and diluted with DCM (100 mL), washed with $NaHCO_3$ aq (2 x 500 mL) and brine (50 mL). The organics dried over $Na_2SO_4$, filtered and evaporated under reduced pressure. The residue was purified on silica gel column MeOH/DCM (0-5%). The pure fractions was concentrated and dried under vacuo. There was ethyl 4-(4-fluoro-5-hydroxy-6-methoxy-benzothiophen-2-yl)-4-oxo-butanoate (0.263 g, 805.9094 μmol, 92.5313% yield) obtained as a yellow solid. LCMS: (ESI, m/z): $[M+H]^+$ = 327.062. $^1H$ NMR (400 MHz, DMSO-$d_6$) δ 9.53 (s, 1H), 8.25 (s, 1H), 7.47 (s, 1H), 4.06 (q, $J$ = 7.1 Hz, 2H), 3.88 (s, 3H), 3.39 - 3.34 (m, 2H), 2.69 - 2.60 (m, 2H), 1.17 (t, $J$ = 7.1 Hz, 3H).

***Step e: ethyl 4-(4-chloro-5-hydroxy-6-methoxy-isoindolin-2-yl)-4-oxo-butanoate***

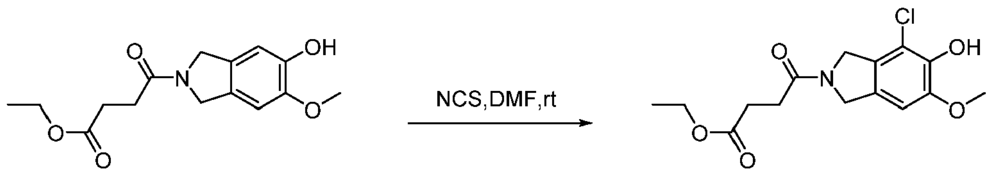


NCS (0.105 g, 786.3231 μmol) was added to a solution of ethyl 4-(5-hydroxy-6-methoxyisoindolin-2-yl)-4-oxobutanoate (0.205 g, 698.9090 μmol) in DMF (10 mL) at 0°C under $N_2$ atmosphere. The reaction mixture was stirred for 12 h and warmed up to 20°C naturally. The reaction mixture was purified on C18 column ACN/$H_2O$ (0-25%). The pure fractions was concentrated and dried under vacuo. There was ethyl 4-(4-chloro-5-hydroxy-6-methoxy-isoindolin-2-yl)-4-oxo-butanoate (0.148 g, 451.5507 μmol, 64.6079% yield) obtained as a white solid. LCMS: (ESI, m/z): $[M+H]^+$ = 328.100. $^1H$ NMR (400 MHz, DMSO-$d_6$) δ 9.41 (s, 1H), 6.97 (s, 0.5H), 6.95 (s, 0.5H), 4.81 (s, 1H), 4.76 (s, 1H), 4.59 (s, 1H), 4.51 (s, 1H), 4.05 (q, $J$ = 7.2 Hz, 2H), 3.82 (s, 3H), 2.69 - 2.53 (m, 4H), 1.18 (t, $J$ = 7.2 Hz, 3H).

***Step f: ethyl 4-[5-(3-bromopropoxy)-4-chloro-6-methoxy-isoindolin-2-yl]-4-oxo-butanoate***

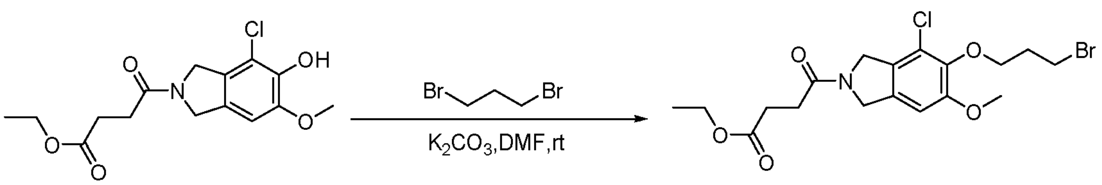


1,3-Dibromopropane (0.495 g, 2.4519 mmol) was added to a mixture of $K_2CO_3$ (0.199 g, 1.4399 mmol), ethyl 4-(4-chloro-5-hydroxy-6-methoxyisoindolin-2-yl)-4-oxobutanoate (0.149 g, 454.6023 μmol) in DMF (5 mL) at 20°C under $N_2$ atmosphere. The reaction mixture was stirred for 4 h at 20°C. The reaction mixture was purified on C18 column ACN/$H_2O$ (0.1%FA)(0-50%). The pure fractions was concentrated and dried under vacuo. There was ethyl 4-(5-(3-bromopropoxy)-4-chloro-6-methoxyisoindolin-2-yl)-4-oxobutanoate (0.163 g, 363.2433 μmol, 79.9035% yield) obtained as a white solid. LCMS: (ESI, m/z): $[M+H]^+$ = 448.040. $^1H$ NMR (400 MHz, DMSO-$d_6$) δ 7.10 (s, 0.5H), 7.08 (s, 0.5H), 4.87 (s, 1H), 4.79 (s, 1H), 4.64 (s, 1H), 4.53 (s, 1H), 4.06 - 4.02 (m, 4H), 3.83 (d, $J$ = 2.4 Hz, 3H), 3.78 - 3.70 (m, 2H), 2.71 - 2.53 (m, 4H), 2.23 - 2.20 (m, 2H), 1.18 (t, $J$ = 7.2 Hz, 3H).

***Step g: ethyl 4-(5-(3-((4-chloro-2-(4-ethoxy-4-oxobutanoyl)-6-methoxyisoindolin-5-yl)oxy)propoxy)-4-fluoro-6-methox ybenzo[b]thiophen-2-yl)-4-oxobutanoate***

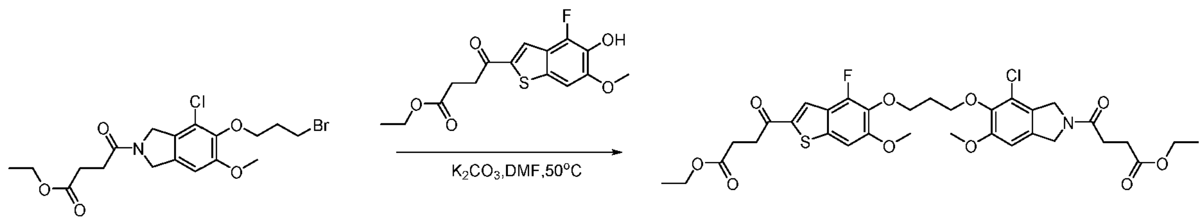


$K_2CO_3$ (0.115 g, 832.0942 μmol) was added to a solution of ethyl 4-(5-(3-bromopropoxy)-4-chloro-6-methoxyisoindolin-2-yl)-4-oxobutanoate (0.068 g, 151.5371 μmol), ethyl 4-(4-fluoro-5-hydroxy-6-methoxybenzo[b]thiophen-2-yl)-4-oxobutanoate (0.049 g, 150.1504 μmol) in DMF (3 mL) at 20°C under $N_2$ atmosphere. The reaction mixture was stirred for 4 h at 50°C. The reaction mixture was purified on C18 column ACN/$H_2O$ (0.1%FA) (0-60%). The pure fractions was concentrated and dried by lyophilization. There was ethyl 4-(5-(3-((4-chloro-2-(4-ethoxy-4-oxobutanoyl)-6-methoxyisoindolin-5-yl)oxy)propoxy)-4-fluoro-6-methoxy benzo[b]thiophen-2-yl)-4-oxobutanoate (0.040 g, 57.6234 μmol, 38.0259% yield) obtained as a white solid. LCMS: (ESI, m/z): $[M+H]^+$ = 694.180. $^1H$ NMR (400 MHz, DMSO-$d_6$) $\delta$ 8.31 (s, 1H), 7.57 (s, 1H), 7.07 (s, 0.5H), 7.04 (s, 0.5H), 4.84 (s, 1H), 4.73 (s, 1H), 4.62 (s, 1H), 4.49 (s, 1H), 4.29 (t, *J* = 6.0 Hz, 2H), 4.17 (t, *J* = 6.0 Hz, 2H), 4.10 - 4.01 (m, 4H), 3.89 (s, 3H), 3.80 (d, *J* = 2.0 Hz, 3H), 3.40 - 3.34 (m, 2H), 2.72 - 2.53 (m, 6H), 2.09 - 2.06 (m, 2H), 1.22 - 1.15 (m, 6H).

***Step h: 4-(5-(3-((2-(3-carboxypropanoyl)-4-chloro-6-methoxyisoindolin-5-yl)oxy)propoxy)-4-fluoro-6-methoxyben zo[b]thiophen-2-yl)-4-oxobutanoic acid***

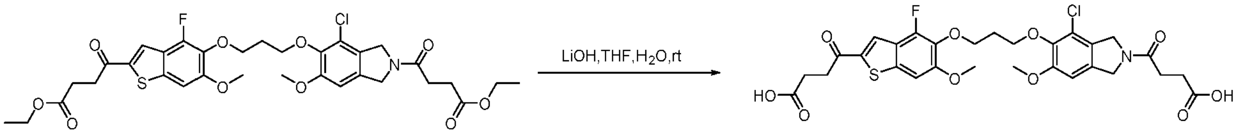


LiOH (0.048 g, 2.0043 mmol) was added to a solution of ethyl 4-(5-(3-((4-chloro-2-(4-ethoxy-4-oxobutanoyl)-6-methoxyisoindolin-5-yl)oxy)propoxy)-4-fluoro-6-methoxy benzo[b]thiophen-2-yl)-4-oxobutanoate (0.027 g, 38.8958 μmol) in THF (1 mL) and Water (1 mL) at 20°C. The reaction mixture was stirred for 1 h at 20°C. The reaction mixture was adjusted to pH=3 with HCl (1 mol/L), The reaction mixture was purified on C18 column ACN/$H_2O$ (0.1%FA)(0-52%). The pure fractions was concentrated and dried by lyophilization. There was 4-(5-(3-((2-(3-carboxypropanoyl)-4-chloro-6-methoxyisoindolin-5-yl)oxy)propoxy)-4-fluoro-6-methoxybenz o[b]thiophen-2-yl)-4-oxobutanoic acid (0.017 g, 26.6434 μmol, 69.6846% yield) obtained as a white solid. LCMS: (ESI, m/z): $[M+H]^+$ = 638.150. $^1H$ NMR (400 MHz, DMSO-$d_6$) $\delta$ 12.2(s, 2H), 8.29 (s, 1H), 7.56 (s, 1H), 7.07 (s, 0.5H), 7.03 (s, 0.5H), 4.83 (s, 1H), 4.72 (s, 1H), 4.62 (s, 1H), 4.49 (s, 1H), 4.32 - 4.25 (m, 2H), 4.17 (t, *J* = 6.1 Hz, 2H), 3.89 (s, 3H), 3.80 (d, *J* = 1.7 Hz, 3H), 3.30 - 3.27 (m, 2H), 2.61 - 2.55 (m, 4H), 2.48 - 2.46 (m, 2H), 2.09 - 2.06 (m, 2H).

**EXAMPLE 3 4-(5-(3-((2-(3-carboxypropanoyl)-4-chloro-6-methoxybenzo[b]thiophen-5-yl)oxy)propoxy)-4-fluoro-6-methoxyisoindolin-2-yl)-4-oxobutanoic acid**

***Step a: tert-butyl 5-(3-bromopropoxy)-4-fluoro-6-methoxy-isoindoline-2-carboxylate***

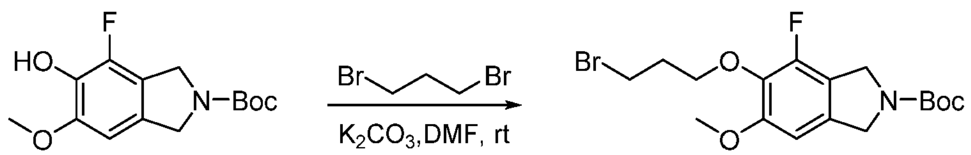

To a solution of tert-butyl 4-fluoro-5-hydroxy-6-methoxy-isoindoline-2-carboxylate (0.50 g, 1.7649 mmol) in DMF (10 mL), was added 1,3-Dibromopropane (2.48 g, 12.2841 mmol) and $K_2CO_3$ (0.71 g, 5.1373 mmol) . The reaction mixture was stirred for 3 h at 20°C. The reaction mixture was diluted with Ethyl acetate (200 mL), and washed sequentially with water (2 x 100 mL) and saturated brine (100 mL). The organic layer was dried over $Na_2SO_4$, filtered and evaporated to afford crude product. The crude product was purified on flash silica chromatography, elution gradient 0% to 100% Ethyl acetate in heptane. There was tert-butyl 5-(3-bromopropoxy)-4-fluoro-6-methoxy-isoindoline-2-carboxylate (0.637 g, 1.5757 mmol, 89.2763% yield) obtained as a white solid. LCMS: (ESI, m/z): $[M+H]^+$ = 331.100.

***Step b: tert-butyl 5-(3-((4-chloro-2-(4-ethoxy-4-oxobutanoyl)-6-methoxybenzo[b]thiophen-5-yl)oxy)propoxy)-4-fluoro-6-met hoxyisoindoline-2-carboxylate***

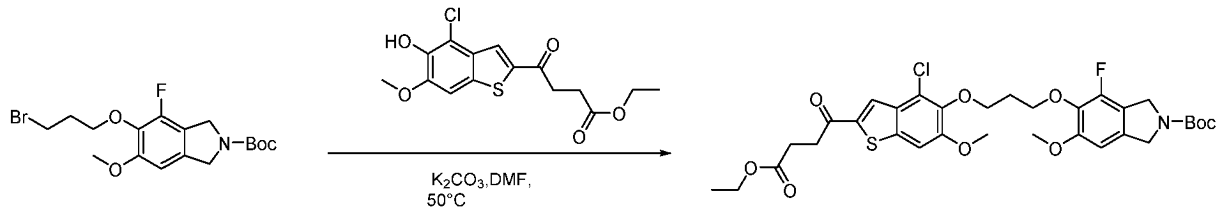


$K_2CO_3$ (0.166 g, 1.2011 mmol) was added to a solution of ethyl 4-(4-chloro-5-hydroxy-6-methoxybenzo[b] thiophen-2-yl)-4-oxobutanoate (0.147 g, 428.8290 μmol) and tert-butyl 5-(3-bromopropoxy)-4-fluoro-6-methoxy-isoindoline-2-carboxylate (0.173 g, 427.9318 μmol) in DMF (5 mL) at 20°C under $N_2$ atmosphere. The reaction mixture was stirred for 3 h at 50°C. The reaction mixture was purified on C18 column ACN/$H_2O$ (0.1% FA)(0-75%). The pure fractions was concentrated and dried under vacuo. There was tert-butyl 5-(3-((4-chloro-2-(4-ethoxy-4-oxobutanoyl)-6-methoxybenzo [b]thiophen-5-yl)oxy)propoxy)-4-fluoro-6-met hoxyisoindoline-2-carboxylate (0.138 g, 207.1598 μmol, 48.3083% yield) obtained as a yellow solid. LCMS: (ESI, m/z): $[M+H]^+$ = 666.190. $^1H$ NMR (400 MHz, DMSO-$d_6$) $\delta$ 8.21 (s, 1H), 7.74 (s, 1H), 6.89 (s, 0.5H), 6.87 (s, 0.5H), 4.53 (s, 4H), 4.21 (d, $J$ = 6.2, 4H), 4.06 (q, $J$ = 7.1 Hz, 2H), 3.90 (s, 3H), 3.77 (d, $J$ = 3.7 Hz, 3H), 3.39 (t, $J$ = 6.4 Hz, 2H), 2.66 (t, $J$ = 6.3 Hz, 2H), 2.11 - 2.09 (m, 2H), 1.45 (s, 9H), 1.18 (t, $J$ = 7.1 Hz, 3H).

***Step c: ethyl 4-(4-chloro-5-(3-((4-fluoro-6-methoxyisoindolin-5-yl)oxy)propoxy)-6-methoxybenzo[b]thiophen-2-yl)-4-ox obutanoate***

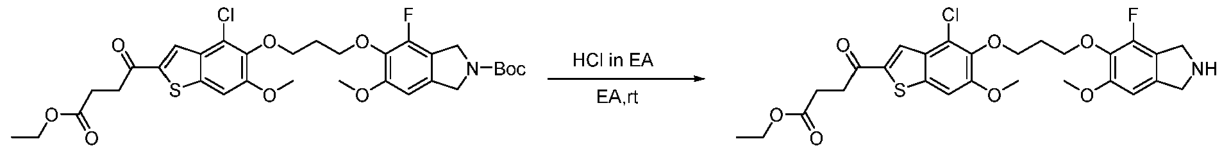


HCl (5 mL, 20 mmol, 4 M in EA) was added to a solution of tert-butyl 5-(3-((4-chloro-2-(4-ethoxy-4-oxobutanoyl)-6-methoxybenzo[b]thiophen-5-yl)oxy)propoxy)-4-fluoro-6-met hoxyisoindoline-2-carboxylate (0.117 g, 175.6356 μmol) in EA (5 mL) at 20°C. The reaction mixture was stirred for 2 h at 20°C. The reaction mixture was evaporated under reduced pressure. There was ethyl 4-(4-chloro-5-(3-((4-fluoro-6-methoxyisoindolin-5-yl)oxy)propoxy)-6-methoxybenzo [b]thiophen-2-yl)-4-ox obutanoate (0.182 g, 321.5338 μmol, 183.0687% yield) obtained as a brown solid. LCMS: (ESI, m/z): $[M+H]^+$ = 566.130. $^1H$ NMR (400 MHz, DMSO-$d_6$) $\delta$ 10.34 (s, 2H), 8.22 (s, 1H), 7.75 (s, 1H), 6.97 (s, 1H), 4.49 (s, 2H), 4.46 (s, 2H), 4.23 - 4.20 (m, 4H), 4.06 (q, $J$ = 7.1 Hz, 2H), 3.90 (s, 3H), 3.79 (s, 3H), 2.67 (t, $J$ = 6.3 Hz, 2H), 2.09 - 2.11 (m, 2H), 1.18 (t, $J$ = 7.1 Hz, 3H).

***Step d: ethyl 4-(5-(3-((4-chloro-2-(4-ethoxy-4-oxobutanoyl)-6-methoxybenzo[b]thiophen-5-yl)oxy)propoxy)-4-fluoro-6-methoxyisoindolin-2-yl)-4-oxobutanoate***

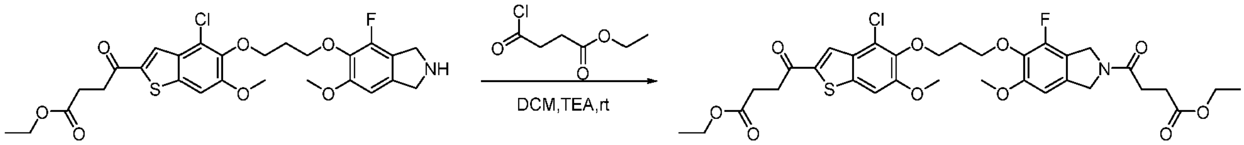


Ethyl Succinyl Chloride (0.097 g, 589.3557 μmol) was added to a solution of ethyl 4-(4-chloro-5-(3-((4-fluoro-6-methoxyisoindolin-5-yl)oxy)propoxy)-6-methoxybenzo[b]thiophen-2-yl)-4-ox obutanoate (0.167 g, 295.0337 μmol) and TEA (0.164 g, 1.6207 mmol) in DCM (5 mL) at 20°C under $N_2$ atmosphere. The reaction mixture was stirred for 1 h at 20°C. The reaction mixture was evaporated under reduced pressure. The residue was purified on C18 column ACN/$H_2O$ (0.1% FA)(0-60%). The pure fractions was concentrated and dried by lyophilization. There was ethyl 4-(5-(3-((4-chloro-2-(4-ethoxy-4-oxobutanoyl)-6-methoxybenzo[b]thiophen-5-yl)oxy)propoxy)-4-fluoro-6-methoxyisoindolin-2-yl)-4-oxobutanoate (0.081 g, 116.6874 μmol, 39.5505% yield) obtained as a white solid. LCMS: (ESI, m/z): $[M+H]^+$ = 694.180. $^1H$ NMR (400 MHz, DMSO-$d_6$) δ 8.22 (s, 1H), 7.74 (d, $J$ = 2.2 Hz, 1H), 6.91 (s, 0.5H), 6.89 (s, 0.5H), 4.81 (s, 1H), 4.80 (s, 1H), 4.57 (s, 2H), 4.22 (q, $J$ = 5.9 Hz, 4H), 4.09 - 4.03 (m, 4H), 3.90 (d, $J$ = 1.1 Hz, 3H), 3.78 (d, $J$ = 2.5 Hz, 3H), 3.40 (s, 2H), 2.70 - 2.58 (m, 4H), 2.57 - 2.53 (m, 2H), 2.15 - 2.04 (m, 2H), 1.26 - 1.14 (m, 6H).

***Step e: 4-(5-(3-((2-(3-carboxypropanoyl)-4-chloro-6-methoxybenzo[b]thiophen-5-yl)oxy)propoxy)-4-fluoro-6-met hoxyisoindolin-2-yl)-4-oxobutanoic acid***

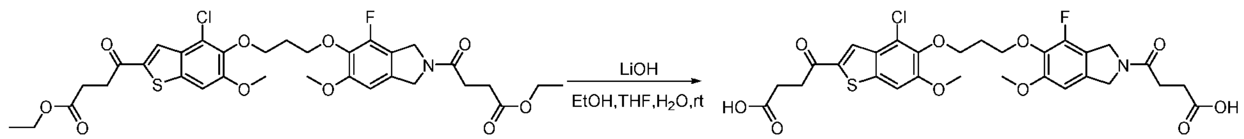


LiOH (0.045 g, 1.8790 mmol) was added to a solution of ethyl 4-(5-(3-((4-chloro-2-(4-ethoxy-4-oxobutanoyl)-6-methoxybenzo[b]thiophen-5-yl)oxy)propoxy)-4-fluoro-6-methoxyisoindolin-2-yl)-4-oxobutanoate (0.044 g, 63.3858 μmol) in THF (2 mL), $H_2O$ (2 mL) and Ethanol (1 mL) at 20°C. The reaction mixture was stirred for 1 h at 20°C. The reaction mixture was adjusted pH=3 with HCl (1 mol/L). The reaction mixture was evaporated under reduced pressure. The residue was purified on C18 column ACN/ $H_2O$ (0.1% FA)(0-52%).The pure fractions was concentrated and dried by lyophilization. There was 4-(5-(3-((2-(3-carboxypropanoyl)-4-chloro-6-methoxybenzo[b]thiophen-5-yl)oxy)propoxy)-4-fluoro-6-meth oxyisoindolin-2-yl)-4-oxobutanoic acid (0.032 g, 50.1523 μmol, 79.1223% yield) obtained as a white solid. LCMS: (ESI, m/z): $[M+H]^+$ = 638.120. $^1H$ NMR (400 MHz, DMSO-$d_6$) δ 8.19 (s, 1H), 7.73 (s, 1H), 6.91 (s, 0.5H), 6.88 (s, 0.5H), 4.79 (s, 2H), 4.57 (s, 2H), 4.25 - 4.19 (m, 4H), 3.90 (s, 3H), 3.78 (d, $J$ = 1.7 Hz, 3H), 3.33 (t, $J$ = 12.0 Hz, 2H), 2.63 - 2.54 (m, 4H), 2.49 - 2.45 (m, 2H), 2.15 - 2.04 (m, 2H).

**EXAMPLE 4 4-(5-(3-((2-(3-carboxypropanoyl)-4-fluoro-6-methoxybenzo[b]thiophen-5-yl)oxy)propoxy)-4-fluoro-6-m ethoxyisoindolin-2-yl)-4-oxobutanoic acid**

***Step a: ethyl 4-(5-(3-bromopropoxy)-4-fluoro-6-methoxyisoindolin-2-yl)-4-oxobutanoate***

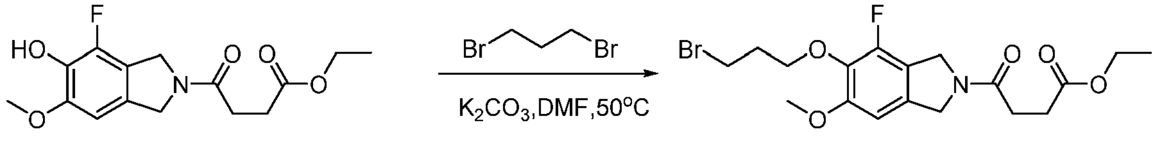


1,3-Dibromopropane (0.708 g, 3.5069 mmol) was added to a mixture of ethyl 4-(4-fluoro-5-hydroxy-6-methoxyisoindolin-2-yl)-4-oxo-butanoate (0.217 g, 697.0656 μmol) and Potassium carbonate (0.346 g, 2.5035 mmol) in DMF (5 mL) at 20°C. The reaction mixture was stirred for 3 h at 20°C. The reaction mixture was purified on C18 column ACN/ $H_2O$ (0.1%FA)(0-45%). The pure fractions was concentrated and dried under vacuo. There was ethyl 4-(5-(3-bromopropoxy)-4-fluoro-6-methoxyisoindolin-2-yl)-4-oxobutanoate (0.211 g, 488.1091 μmol, 70.0234% yield) obtained as a white solid. LCMS: (ESI, m/z): $[M+H]^+$ = 432.070.

***Step b: ethyl 4-(5-(3-((2-(4-ethoxy-4-oxobutanoyl)-4-fluoro-6-methoxybenzo[b]thiophen-5-yl)oxy)propoxy)-4-fluoro-6-methoxyisoindolin-2-yl)-4-oxobutanoate***

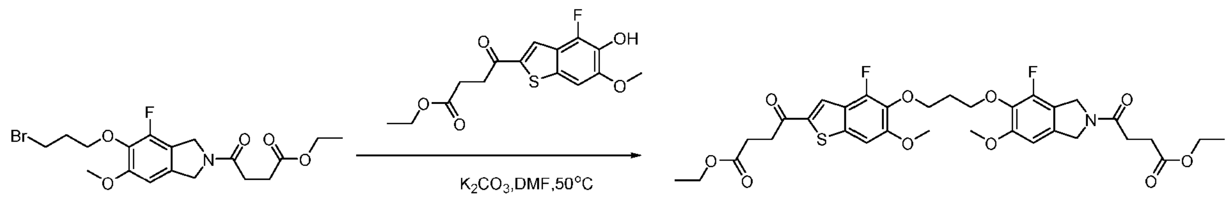


Potassium carbonate (0.079 g, 571.6126 µmol) was added to a solution of ethyl 4-(5-(3-bromopropoxy)-4-fluoro-6-methoxyisoindolin-2-yl)-4-oxobutanoate (0.070 g, 161.9319 µmol) and ethyl 4-(4-fluoro-5-hydroxy-6-methoxybenzo[b]thiophen-2-yl)-4-oxobutanoate (0.065 g, 199.1791 µmol) in N,N-Dimethylformamide (3 mL) at 20°C under $N_2$ atmosphere. The reaction mixture was stirred overnight at 50°C. The reaction mixture was purified on C18 column ACN/$H_2O$ (0.1%FA)(0-70%). The pure fractions was concentrated and dried by lyophilization. There was ethyl 4-(5-(3-((2-(4-ethoxy-4-oxobutanoyl)-4-fluoro-6-methoxybenzo[b]thiophen-5-yl)oxy)propoxy)-4-fluoro-6-m ethoxyisoindolin-2-yl)-4-oxobutanoate (0.056 g, 82.6315 µmol, 51.0285% yield) obtained as a yellow solid. LCMS: (ESI, m/z): $[M+H]^+$ = 678.210. $^1$H NMR (400 MHz, DMSO-$d_6$) δ 8.31 (s, 1H), 7.56 (s, 1H), 6.89 (s, 0.5H), 6.86 (s, 0.5H), 4.78 (s, 2H), 4.56 (s, 1H), 4.54 (s, 1H), 4.25 (t, $J$ = 6.0 Hz, 2H), 4.19 (t, $J$ = 6.0 Hz, 2H), 4.09 - 4.02 (m, 4H), 3.88 (s, 3H), 3.77 (d, $J$ = 2.0 Hz, 3H), 3.36 (t, $J$ = 6.4 Hz, 2H), 2.70 - 2.51 (m, 6H), 2.10 - 1.99 (m, 2H), 1.22 - 1.14 (m, 6H).

***Step c: 4-(5-(3-((2-(3-carboxypropanoyl)-4-fluoro-6-methoxybenzo[b]thiophen-5-yl)oxy)propoxy)-4-fluoro-6-met hoxyisoindolin-2-yl)-4-oxobutanoic acid***

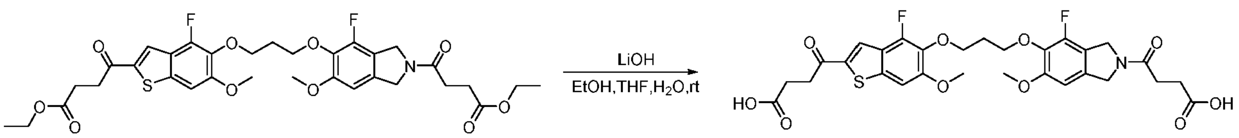


LiOH (0.043 g, 1.7955 mmol) was added to a solution of ethyl 4-(5-(3-((2-(4-ethoxy-4-oxobutanoyl)-4-fluoro-6-methoxybenzo[b]thiophen-5-yl)oxy)propoxy)-4-fluoro-6-m ethoxyisoindolin-2-yl)-4-oxobutanoate (0.039 g, 57.5469 µmol) in THF (2 mL), $H_2O$ (2 mL) and Ethanol (1 mL) at 20°C. The reaction mixture was stirred overnight at 20°C. The reaction mixture was adjusted to pH=3 with HCl (aq.1 M), The reaction mixture was evaporated under reduced pressure. The residue was purified on C18 column ACN/$H_2O$ (0.1%FA)(0-50%). The pure fractions was concentrated and dried under vacuo. There was 4-(5-(3-((2-(3-carboxypropanoyl)-4-fluoro-6-methoxybenzo[b]thiophen-5-yl)oxy)propoxy)-4-fluoro-6-meth oxyisoindolin-2-yl)-4-oxobutanoic acid (0.012 g, 19.3050 µmol, 33.5465% yield) obtained as a white solid. LCMS: (ESI, m/z): $[M+H]^+$ = 622.150. $^1$H NMR (400 MHz, DMSO-$d_6$) δ 12.16 (s, 2H), 8.29 (s, 1H), 7.56 (s, 1H), 6.90 (s, 0.5H), 6.86 (s, 0.5H), 4.77 (s, 2H), 4.56 (s, 1H), 4.55 (s, 1H), 4.29 - 4.15 (m, 4H), 3.88 (s, 3H), 3.77 (s, 3H), 3.31 - 3.29 (m, 2H), 2.63 - 2.53 (m, 4H), 2.50 - 2.46 (m, 2H), 2.10 - 1.99 (m, 2H).

**EXAMPLE 5**

**sodium (S)-4-(5-(3-((2-((S)-3-carboxylatobutanoyl)-6-methoxybenzo[b]thiophen-5-yl)oxy)propoxy)-6-methoxyi soindolin-2-yl)-2-methyl-4-oxobutanoate**

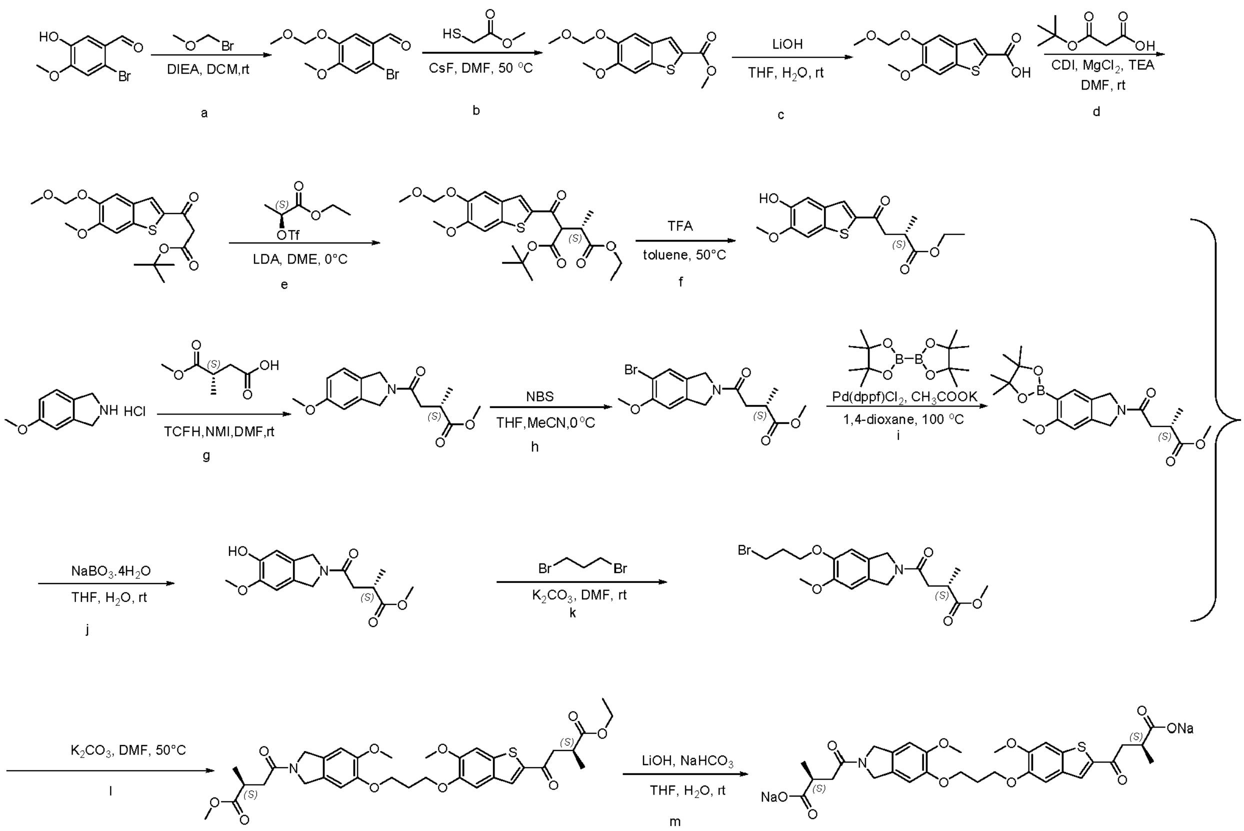


***Step a:*** Bromomethyl methyl ether (60.56 g, 484.6196 mmol) was added to a solution of N,N-Diisopropylethylamine (97.64 g, 755.4796 mmol) and 2-bromo-5-hydroxy-4-methoxybenzaldehyde (102.80 g, 444.9386 mmol) in DCM (1500 mL) at 0°C under $N_2$ atmosphere. The reaction mixture was stirred at 25°C for 2 h. The resulting solution was quenched with adding of water (1000 mL) at 25°C. The resulting mixture was extracted with DCM (2 x 1000 mL), washed with water (500 mL) and brine (500 mL). The organics dried over $Na_2SO_4$, filtered and evaporated under reduced pressure. There was 2-bromo-4-methoxy-5-(methoxymethoxy)benzaldehyde (115.25 g, 418.9454 mmol, 94.1580% yield) obtained as a light yellow soilid. LCMS: (ESI, m/z): $[M+H]^+$ = 274.984. $^1H$ NMR (400 MHz, DMSO-$d_6$) $\delta$ 10.06 (s, 1H), 7.51 (s, 1H), 7.39 (s, 1H), 5.25 (s, 2H), 3.93 (s, 3H), 3.39 (s, 3H).

***Step b:*** Methyl thioglycolate (53.415 g, 503.2348 mmol) was added to a solution of 2-bromo-4-methoxy-5-(methoxymethoxy)benzaldehyde (115.11 g, 418.4365 mmol) and Caesium fluoride (130.32 g, 857.9136 mmol) in N,N-Dimethylformamide (1200 mL) at 25°C under $N_2$ atmosphere. The reaction mixture was heated to 50°C and stirred overnight. The reaction mixture was pour into the water (10 L) and stirred for 1 h. The precipitate was collected by filtration, washed with water (3 x 200 mL). The filter cake was dried under vacuo at 60°C to obtain a crude product. The crude product was diluted with DCM (200 mL). Pour the mixture into n-Hexane (2000 mL) and stirred for 1 h. The precipitate was collected by filtration, washed with n-Hexane (3 x 100 mL). The filter cake was dried under vacuo at 60°C. There was methyl 6-methoxy-5-(methoxymethoxy)benzothiophene-2-carboxylate (65.50 g, 232.0131 mmol, 55.4476% yield) obtained as a yellow solid. LCMS: (ESI, m/z): $[M+H]^+$ = 284.056. $^1H$ NMR (400 MHz, DMSO-$d_6$) $\delta$ 8.07 (s, 1H), 7.69 (s, 1H), 7.65 (s, 1H), 5.25 (s, 2H), 3.90 (s, 3H), 3.88 (s, 3H), 3.44 (s, 3H).

***Step c:*** LiOH (17.58 g, 734.0813 mmol) was added to a solution of methyl 6-methoxy-5-(methoxymethoxy) benzothiophene-2-carboxylate (51.16 g, 181.2181 mmol) in THF (600 mL) and Water (200 mL) at 25°C. The reaction mixture was stirred for 4 h at 25°C. The reaction mixture was evaporated under reduced pressure. The residue was diluded with water and adjusted to pH=4 with HCl (1 M). The precipitate was collected by filtration, washed with water (2 x 50 mL). The filtrate cake was dried under vacuo. There was 6-methoxy-5-(methoxymethoxy)benzothiophene-2-carboxylic acid (44.17 g, 164.6382 mmol, 90.8509% yield) obtained as a yellow solid. LCMS: (ESI, m/z): $[M-H]^-$ = 267.041. $^1H$ NMR (400 MHz, DMSO-$d_6$) $\delta$ 13.17 (s, 1H), 7.95 (s, 1H), 7.64 (s, 1H), 7.62 (s, 1H), 5.23 (s, 2H), 3.88 (s, 3H), 3.43 (s, 3H). ***Step d:*** To a stirred solution of 6-methoxy-5-(methoxymethoxy)benzothiophene-2-carboxylic acid (53.21 g, 198.3337 mmol) in DMF (1000 mL) was added CDI (64.55 g, 398.0919 mmol) at 25°C. The reaction mixture was stirred for 2 h at 25°C. To the mixture above was added 3-(tert-butoxy)-3-oxopropanoic acid (49.03 g, 306.1174 mmol), TEA (62.33 g, 615.9736 mmol) and Magnesium chloride (29.11 g, 305.7420 mmol). The reaction mixture was stirred overnight at 25°C. The reaction mixture was concentrated and diluted with EA (3000 mL), washed with water (2 x 3000 mL) and brine (3000 mL). The organics dried over $Na_2SO_4$, filtered and evaporated under reduced pressure. The residue was purified on silica gel

column EA/n-Hexane (0-50%). The pure fractions was concentrated and dried under vacuo. There was tert-butyl 3-(6-methoxy-5-(methoxymethoxy)benzo[b]thiophen-2-yl)-3-oxopropanoate (36.19 g, 98.7643 mmol, 49.7971% yield) obtained as a yellow oil. LCMS: (ESI, m/z): $[M+H]^+$ = 367.114. $^1$H NMR (400 MHz, DMSO-$d_6$) $\delta$ 8.20 (s, 1H), 7.67 (s, 1H), 7.62 (s, 1H), 5.24 (s, 2H), 4.05 (s, 2H), 3.89 (s, 3H), 3.42 (s, 3H), 1.41 (s, 9H).

***Step e:*** LDA (7.9020 g, 73.7662 mmol) was added to a solution of tert-butyl 3-(6-methoxy-5-(methoxymethoxy)benzo[b]thiophen-2-yl)-3-oxopropanoate (18.02 g, 49.1775 mmol) in 2,5-Dioxahexane (200 mL) at 0°C under $N_2$ atmosphere. The reaction mixture was stirred for 40 min at 0°C under $N_2$ atmosphere. Ethyl (S)-2-(((trifluoromethyl)sulfonyl)oxy)propanoate (18.06 g, 72.1843 mmol) was added to the mixture at 0°C under $N_2$ atmosphere. The reaction mixture was stirred for 3 h at 0°C under $N_2$ atmosphere. The reaction mixture was quenched with adding of $KHCO_3$ (100 mL) at 0°C. The reaction mixture was concentrated and diluted with EA (500 mL), washed with water (200 mL) and brine (100 mL). The organics dried over $Na_2SO_4$, filtered and evaporated under reduced pressure. The residue was purified on silica gel column EA/n-Hexane (0-100%). The pure fractions was concentrated and dried under vacuo. There was 1-(tert-butyl) 4-ethyl(3S)-2-(6-methoxy-5-(methoxymethoxy)benzo[b]thiophene-2-carbonyl)-3-methylsuccinate (21.55 g, 46.1908 mmol, 93.9267% yield) obtained as a yellow oil. LCMS: (ESI, m/z): $[M+H]^+$ = 467.166.

***Step f:*** TFA (101.31 g, 888.5026 mmol) was added to a solution of 1-(tert-butyl) 4-ethyl(3S)-2-(6-methoxy-5-(methoxymethoxy)benzo[b]thiophene-2- carbonyl)-3-methylsuccinate (21.52 g, 46.1265 mmol) in Toluene (198 mL) at 25°C. The reaction mixture was heated to 50°C and stirred for 2 h. The reaction mixture was evaporated under reduced pressure. The residue was purified on silica gel column EA/n-Hexane (0-50%). The pure fractions was concentrated and dried under vacuo. There was ethyl (2S)-4-(5-hydroxy-6-methoxy-benzothiophen-2-yl)-2-methyl-4-oxo-butanoate (6.78 g, 21.0314 mmol, 45.5950% yield) obtained as a yellow solid. LCMS: (ESI, m/z): $[M+H]^+$ = 323.087. $^1$H NMR (400 MHz, DMSO-$d_6$) $\delta$ 9.39 (s, 1H), 8.17 (s, 1H), 7.54 (s, 1H), 7.31 (s, 1H), 4.10 - 3.98 (m, 2H), 3.87 (s, 3H), 3.44 - 3.35 (m, 1H), 3.19 - 3.08 (m, 1H), 2.98 - 2.88 (m, 1H), 1.21 - 1.10 (m, 6H).

***Step g:*** NMI (7.96 g, 96.9507 mmol) was added to a solution of TCFH (8.11 g, 28.9045 mmol) and (S)-4-methoxy-3-methyl-4-oxobutanoic acid (2.81 g, 19.2280 mmol) in DMF (100 mL) at 25°C. The reaction mixture was stirred for 10 min at 20°C. 5-methoxyisoindoline hydrochloride (4.99 g, 26.8785 mmol) was added to the solution at 25°C. The reaction mixture was stirred 2 h at 25°C. The reaction mixture was concentrated and diluted with $H_2O$ (200 mL), extracted with EA (2 x 200 mL) and washed with brine (100 mL). The organics dried over $Na_2SO_4$, filtered and evaporated under reduced pressure. The residue was purified on silica gel column EA/n-Hexane (0-55%). The pure fractions was concentrated and dried under vacuo. There was methyl (2S)-4-(5-methoxyisoindolin-2-yl)-2-methyl-4-oxo-butanoate (3.49 g, 12.5850 mmol, 65.4512% yield) obtained as a yellow solid. LCMS: (ESI, m/z): $[M+H]^+$ = 278.130. $^1$H NMR (400 MHz, DMSO-$d_6$) $\delta$ 7.23 (t, *J= 8.0* Hz, 1H), 6.95 - 6.84 (m, 2H), 4.78 (s, 1H), 4.73 (s, 1H), 4.56 (s, 1H), 4.52 (s, 1H), 3.75 (d, *J* = 1.8 Hz, 3H), 3.60 (s, 3H), 2.91 - 2.81 (m, 1H), 2.76 - 2.66 (m, 1H), 2.54 - 2.51 (m, 0.5H), 2.49 - 2.45 (m, 0.5H), 1.18 - 1.14 (m, 3H).

***Step h:*** NBS (3.717 g, 20.8839 mmol) was added to a solution of methyl (2S)-4-(5-methoxyisoindolin-2-yl)-2-methyl-4-oxo-butanoate (3.426 g, 12.3542 mmol) in THF (30 mL) and MeCN (30 mL) at 0°C. The reaction mixture was stirred for 3 h at 25°C. The reaction mixture was concentrated and diluted with DCM (200 mL), washed with $H_2O$ (100 mL), $KHCO_3$ (aq)(2 x 100 mL) and brine (100 mL). The organics dried over $Na_2SO_4$, filtered and evaporated under reduced pressure. The residue was purified on silica gel column EA/n-Hexane (0-60%). The pure fractions was concentrated and dried under vacuo. There was methyl (2S)-4-(5-bromo-6-methoxy-isoindolin-2-yl)-2-methyl-4-oxo-butanoate (3.02 g, 8.4781 mmol, 68.6255% yield) obtained as a yellow solid. LCMS: (ESI, m/z): $[M+H]^+$ = 356.040. $^1$H NMR (400 MHz, DMSO-$d_6$) $\delta$ 7.57 (s, 0.5H), 7.55 (s, 0.5H), 7.14 (s, 0.5H), 7.09 (s, 0.5H), 4.78 (s, 1H), 4.75 (s, 1H), 4.56 (s, 1H), 4.53 (s, 1H), 3.86 (s, 3H), 3.59 (s, 3H), 2.93 - 2.79 (m, 1H), 2.78 - 2.64 (m, 1H), 2.49 - 2.46 (m, 1H), 1.15 (d, *J* = 7.1 Hz, 3H).

***Step i:*** Potassium Acetate (2.81 g, 28.6319 mmol) was added to a mixture of methyl (2S)-4-(5-bromo-6-methoxy-isoindolin-2-yl)-2-methyl-4-oxo-butanoate (3.00 g, 8.4220 mmol), [1,1'-Bis(diphenylphosphino)ferrocene]dichloropalladium(II) (0.74 g, 1.0113 mmol) and 4,4,4',4',5,5,5',5'-Octamethyl-2,2'-bi(1,3,2-dioxaborolane) (3.18 g, 12.5228 mmol) in 1,4-Dioxane (100 mL) at 25°C. The reaction mixture was heated to 100°C and stirred overnight. The reaction mixture was diluted with EA(200 mL), The precipitate was collected by filtration, washed with EA (1 x 100 mL). The filtrate was evaporated under reduced pressure. The residue was purified on silica gel column EA/n-Hexane (0-50%). The pure fractions was concentrated and dried under vacuo. There was methyl (2S)-4-[5-methoxy-6-(4,4,5,5-tetramethyl-1,3,2-dioxaborolan-2-yl)isoindolin-2-yl]-2-methyl-4-oxo-butanoat e (2.38 g, 5.9017 mmol, 70.0746% yield) obtained as a brown solid. LCMS: (ESI, m/z): $[M+H]^+$ = 404.222. $^1$H NMR (400 MHz, DMSO-$d_6$) $\delta$ 7.49 (s, 0.5H), 7.46 (s, 0.5H), 6.97 (s, 0.5H), 6.93 (s, 0.5H), 4.81 (s, 1H), 4.73 (s, 1H), 4.59 (s, 1H), 4.52 (s, 1H), 3.73 (s, 3H), 3.59 (s, 3H), 2.90 - 2.81 (m, 1H), 2.76 - 2.65 (m, 1H), 2.54 - 2.51 (m, 1H), 1.27 (s, 12H), 1.15 (d, *J* = 6.6 Hz, 3H).

***Step j:*** Sodium perborate tetrahydrate (0.80 g, 5.1995 mmol) was added to a solution of methyl (2S)-4-[5-methoxy-6-(4,4,5,5-tetramethyl-1,3,2-dioxaborolan-2-yl)isoindolin-2-yl]-2-methyl-4-oxo-butanoat e (1.99 g, 4.9346 mmol) in THF (25 mL) and $H_2O$ (25 mL) at 25°C. The reaction mixture was stirred for 1 h at 25°C. The reaction mixture was concentrated and diluted with $H_2O$(200 mL), extracted with DCM (3 x 100 mL) and washed with brine (100 mL). The organics dried over $Na_2SO_4$, filtered and evaporated under reduced pressure. The residue was purified on C18 column

ACN/$H_2O$ (0-40%). The pure fractions was concentrated and dried under vacuo. There was methyl (2S)-4-(5-hydroxy-6-methoxy-isoindolin-2-yl)-2-methyl-4-oxo-butanoate (1.23 g, 4.1935 mmol, 84.9809% yield) obtained as a white solid. LCMS: (ESI, m/z): $[M+H]^+$ = 294.130.

***Step k:*** Potassium carbonate (0.462 g,3.3428 mmol) was added to a solution of methyl (2S)-4-(5-hydroxy-6-methoxy-isoindolin-2-yl)-2-methyl-4-oxo-butanoate (0.311 g, 1.0603 mmol) and 1,3-Dibromopropane (1.158 g, 5.7359 mmol) in DMF (20 mL) at 25°C. The reaction mixture was stirred for 3 h at 25°C. The reaction mixture was purified on C18 column ACN/$H_2O$ (0-45%). The pure fractions was concentrated and dried under vacuo. There was methyl (2R)-4-[5-(3-bromopropoxy)-6-methoxy-isoindolin-2-yl]-2-methyl-4-oxo-butanoate (0.314 g, 757.9234 μmol, 71.4822% yield) obtained as a colorless oil. LCMS: (ESI, m/z): $[M+H]^+$ = 414.080.

***Step l:*** Potassium carbonate (0.214 g, 1.5484 mmol) was added to a solution of methyl (S)-4-(5-(3-bromopropoxy)-6-methoxyisoindolin-2-yl)-2-methyl-4-oxobutanoate (0.206 g, 497.2362 μmol) and ethyl (2S)-4-(5-hydroxy-6-methoxy-benzothiophen-2-yl)-2-methyl-4-oxo-butanoate (0.243 g, 753.7805 μmol) in DMF (5 mL) at 25°C. The reaction mixture was heated to 50°C and stirred overnight. The reaction mixture was concentrated and diluted with EA (200 mL), washed with water (100 mL) and brine (100 mL). The organics dried over $Na_2SO_4$, filtered and evaporated under reduced pressure. The residue was purified on silica gel column EA/n-Hexane (0-100%). The pure fractions was concentrated and dried under vacuo. There was ethyl (S)-4-(6-methoxy-5-(3-((6-methoxy-2-((S)-4-methoxy-3-methyl-4-oxobutanoyl)isoindolin-5-yl)oxy)propoxy )benzo[b]thiophen-2-yl)-2-methyl-4-oxobutanoate (0.130 g, 198.2454 μmol, 39.8695% yield) obtained as a yellow solid. LCMS: (ESI, m/z): $[M+H]^+$ = 656.245.

***Step m:*** LiOH (0.017 g, 709.8625 μmol) was added to a solution of ethyl (S)-4-(6-methoxy-5-(3-((6-methoxy-2-((S)-4-methoxy-3-methyl-4-oxobutanoyl)isoindolin-5-yl)oxy)propoxy )benzo[b]thiophen-2-yl)-2-methyl-4-oxobutanoate (0.120 g, 182.9957 μmol) in THF (4 mL) and Water (1 mL) at 25°C. The reaction mixture was stirred overnight at 25°C. The reaction mixture was adjusted to pH=7 with HCl (1 M). The reaction mixture was evaporated under reduced pressure to obtain a crude product. The crude product was purified by Prep-HPLC with the following conditions: (CXTH LC6000, HPLC-P1): Column, Agela Durashell C18, 30mm*250mm, 10um; mobile phase, Water (0.1% NH3.H2O) and MeCN-(5-20-20%B(2-32-60min)); Detector, uv 210 nm). The solvent was removed by lyophilization. $NaHCO_3$ (0.019 g, 226.1727 μmol) was added to the lyophilized product in water, and stirred for 30 min at 25°C. The solvent was removed by lyophilization. There was sodium (S)-4-(5-(3-((2-((S)-3-carboxylatobutanoyl)-6-methoxybenzo[b]thiophen-5-yl)oxy)propoxy)-6-methoxyisoin dolin-2-yl)-2-methyl-4-oxobutanoate (0.064 g, 97.3180 μmol, 53.1805% yield) obtained as a white solid. LCMS: (ESI, m/z): $[M+H]^+$ = 614.198. $^1H$ NMR (400 MHz, DMSO-$d_6$) $\delta$ 8.08 (s, 0.5H), 8.05 (s, 0.5H), 7.56 (s, 1H), 7.51 (s, 0.5H), 7.50 (s, 0.5H), 7.02 - 6.89 (m, 2H), 4.90 - 4.77 (m, 1H), 4.73 - 4.62 (m, 1H), 4.50 (s, 2H), 4.24 - 4.16 (m, 2H), 4.15 - 4.05 (m, 2H), 3.85 (s, 3H), 3.74 (s, 3H), 3.45 - 3.38 (m, 2H), 2.75 - 2.57 (m, 2H), 2.47 - 2.39 (m, 1H), 2.27 - 2.14 (m, 2H), 2.11 - 1.97 (m, 1H), 1.02 (t, $J$ = 6.5 Hz, 6H).

**EXAMPLE 6**

**sodium (S)-4-(5-(3-((2-((S)-3-carboxylatobutanoyl)-4-fluoro-6-methoxybenzo[b]thiophen-5-yl)oxy)propoxy)-6-methoxyisoindolin-2-yl)-2-methyl-4-oxobutanoate**

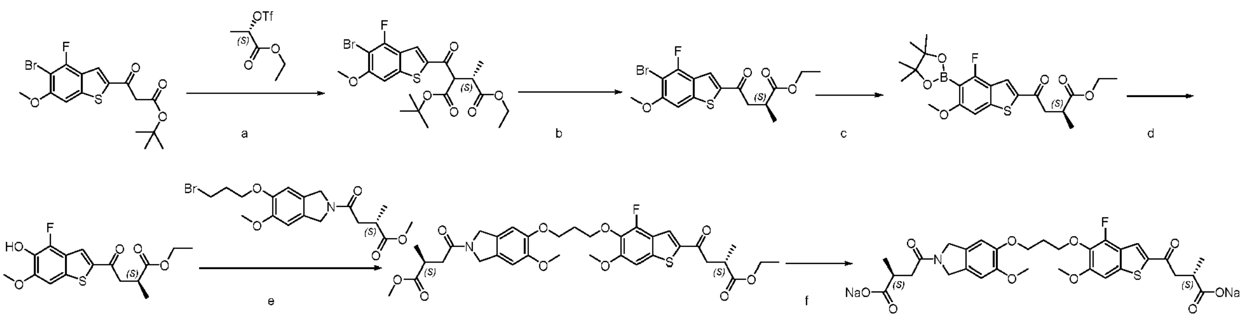


***Step a:*** Lithium diisopropylamide, (552.5297 mg, 5.1579 mmol) was added to a solution of tert-butyl 3-(5-bromo-4-fluoro-6-methoxybenzo[b]thiophen-2-yl)-3-oxopropanoate (1.6 g, 3.9676 mmol) in 2,5-Dioxahexane (50 mL) at 0°C under $N_2$ atmosphere. The reaction mixture was stirred for 40 min at 0°C under $N_2$ atmosphere. Ethyl (S)-2-(((trifluoromethyl)sulfonyl)oxy)propanoate (2.9780 g,11.9029 mmol) was added to the mixture at 0°C under $N_2$ atmosphere. The reaction mixture was stirred overnight at 25°C under $N_2$ atmosphere. The reaction mixture was concentrated and diluted with EA (100 mL), washed with $NaHCO_3$ (aq) (2 x 500 mL) and brine (50 mL). The organics dried over $Na_2SO_4$, filtered and evaporated under reduced pressure. The residue was purified on silica gel column EA/n-Hexane (0-20%). The

pure fractions was concentrated and dried under vacuo. There was 1-(tert-butyl) 4-ethyl (3S)-2-(5-bromo-4-fluoro-6-methoxybenzo[b]thiophene-2-carbonyl)-3-methylsuccinate (1.15 g, 2.2846 mmol, 57.5800% yield) obtained as a colorless oil. LCMS: (ESI, m/z): $[M+H]^+$ = 503.046. $^1H$ NMR (400 MHz, DMSO-$d_6$) δ 8.61 (s, 0.5H), 8.46 (s, 0.5H), 7.73 (s, 1H), 4.93 - 4.77 (m, 1H), 4.13 - 4.02 (m, 2H), 4.00 - 3.94 (m, 3H), 3.24 - 3.11 (m, 1H), 1.31 (d, *J* = 11.5 Hz, 9H), 1.25 - 1.16 (m, 3H), 1.10 (t, *J* = 7.8 Hz, 3H).

***Step b:*** Trifluoroacetic acid (4 mL) was added to a solution of 1-(tert-butyl) 4-ethyl (3S)-2-(5-bromo-4-fluoro-6-methoxybenzo[b]thiophene-2-carbonyl)-3-methylsuccinate (1.02 g, 2.0263 mmol) in Toluene (20 mL) at 25°C. The reaction mixture was stirred at 50°C for 2 h. The reaction mixture was concentrated and purified on C-18 column MeCN /water (0-100%). The pure fractions was concentrated and dried under vacuo. There was ethyl (S)-4-(5-bromo-4-fluoro-6-methoxybenzo[b]thiophen-2-yl)-2-methyl-4-oxobutanoate (0.76 g, 1.8846 mmol, 93.0079% yield) obtained as a yellow solid. LCMS: (ESI, m/z): $[M+H]^+$ = 402.994. $^1H$ NMR (400 MHz, MeOD-$d_4$) δ 8.14 (s, 1H), 7.40 (s, 1H), 4.19 - 4.03 (m, 2H), 3.98 (s, 3H), 3.60 - 3.39 (m, 1H), 3.25 - 2.97 (m, 2H), 1.28 (d, *J* = 7.1 Hz, 3H), 1.23 (t, *J* = 7.1 Hz, 3H).

***Step c:*** [1,1'-Bis(diphenylphosphino)ferrocene]dichloropalladium(II) (0.13 g, 177.6676 μmol) was added to a mixture of Potassium Acetate (0.53 g, 5.4003 mmol) ethyl (2S)-4-(5-bromo-4-fluoro-6-methoxy-benzothiophen-2-yl)-2-methyl-4-oxo-butanoate (0.70 g, 1.7358 mmol) and 4,4,4',4',5,5,5',5'-Octamethyl-2,2'-bi(1,3,2-dioxaborolane) (0.87 g, 3.4260 mmol) in 1,4-Dioxane (30 mL) at 25°C. The reaction mixture was stirred overnight at 100°C under $N_2$ atmosphere. The reaction mixture was evaporated under reduced pressure. The residue was concentrated and diluted with EA (200 mL), washed with $NaHCO_3$ (aq) (2 x 100 mL) and brine (100 mL). The organics dried over $Na_2SO_4$, filtered and evaporated under reduced pressure. The residue was purified on silica gel column EA/Hept (0-50%). The pure fractions was concentrated and dried under vacuo. There was ethyl (S)-4-(4-fluoro-6-methoxy-5-(4,4,5,5-tetramethyl-1,3,2-dioxaborolan-2-yl)benzo[b]thiophen-2-yl)-2-methyl-4-oxobutanoate (452 mg, 1.0037 mmol, 57.8229% yield) obtained as a white solid. LCMS: (ESI, m/z): $[M+H]^+$ = 451.168. $^1H$ NMR (400 MHz, DMSO-$d_6$) δ 8.29 (s, 1H), 7.49 (s, 1H), 4.15 - 3.99 (m, 2H), 3.86 (s, 3H), 3.57 - 3.40 (m, 2H), 3.18 (s, 1H), 1.41 - 1.23 (m, 12H), 1.19 - 1.14 (m, 6H).

***Step d:*** Sodium perborate tetrahydrate (0.23 g, 1.4949 mmol) was added to a solution of ethyl (S)-4-(4-fluoro-6-methoxy-5-(4,4,5,5-tetramethyl-1,3,2-dioxaborolan-2-yl)benzo[b]thiophen-2-yl)-2-methyl-4-oxobutanoate (0.46 g, 1.0215 mmol) in Tetrahydrofuran (20 mL) and Water (20 mL) at 25°C. The reaction mixture was stirred at 25°C for 1 h. The reaction mixture was evaporated under reduced pressure. The residue was concentrated and diluted with DCM (200 mL), washed with water (2 x 100 mL) and brine (200 mL). The organics dried over $Na_2SO_4$, filtered and evaporated under reduced pressure. There was ethyl (2S)-4-(4-fluoro-5-hydroxy-6-methoxy-benzothiophen-2-yl)-2-methyl-4-oxo-butanoate (0.28 g, 822.6441 μmol, 80.5345% yield) obtained as a yellow solid. LCMS: (ESI, m/z): $[M+H]^+$ = 341.078. $^1H$ NMR (400 MHz, DMSO-$d_6$) δ 9.51 (s, 1H), 8.25 (s, 1H), 7.46 (s, 1H), 4.09 - 4.00 (m, 2H), 3.96 - 3.88 (m, 3H), 3.52 - 3.41 (m, 1H), 3.24 - 3.16 (m, 1H), 2.94 (s, 1H), 1.22 - 1.10 (m, 6H).

***Step e:*** Ethyl (2S)-4-(4-fluoro-5-hydroxy-6-methoxy-benzothiophen-2-yl)-2-methyl-4-oxo-butanoate (0.16 g, 470.0822 μmol) was added to a mixture of methyl (S)-4-(5-(3-bromopropoxy)-6-methoxyisoindolin-2-yl)-2-methyl-4-oxobutanoate (0.20 g, 482.7536 μmol) and Potassium carbonate (0.15 g, 1.0853 mmol) in N,N-Dimethylformamide (5 mL) at 25°C. The reaction mixture was stirred overnight at 25°C. The reaction mixtire was purified on C18 column ACN/$H_2O$ (0-45%). The pure fractions was concentrated and dried under vacuo. There was ethyl (S)-4-(4-fluoro-6-methoxy-5-(3-((6-methoxy-2-((S)-4-methoxy-3-methyl-4-oxobutanoyl)isoindolin-5-yl)oxy )propoxy)benzo[b]thiophen-2-yl)-2-methyl-4-oxobutanoate (0.268 g, 397.7772 μmol, 82.3976% yield) obtained as a colorless oil. LCMS: (ESI, m/z): $[M+H]^+$ = 674.236. $^1H$ NMR (400 MHz, DMSO-$d_6$) δ 8.31 (s, 1H), 7.57 (s, 0.5H), 7.54 (s, 0.5H), 7.03 - 6.83 (m, 2H), 4.72 (s, 2H), 4.52 (s, 2H), 4.31 - 4.09 (m, 4H), 4.09 - 3.96 (m, 2H), 3.87 (s, 3H), 3.72 (s, 3H), 3.59 (s, 3H), 3.51 - 3.41 (m, 1H), 3.24 - 3.15 (m, 1H), 3.02 - 2.65 (m, 4H), 2.12 (s, 2H), 1.32 - 0.96 (m, 9H).

***Step f:*** LiOH (0.030 g, 1.2527 mmol) was added to a solution of ethyl (2S)-4-[4-fluoro-6-methoxy-5-[3-[6-methoxy-2-[(3S)-4-methoxy-3-methyl-4-oxo-butanoyl]isoindolin-5-yl]o xypropoxy]benzothiophen-2-yl]-2-methyl-4-oxo-butanoate (0.258 g, 382.9348 μmol) in Tetrahydrofuran (10 mL) and Water (2 mL) at 25°C. The reaction mixture was stirred overnight at 25°C. The reaction mixture was purified by HPLC. The pure fractions was concentrated and dried under vacuo to obtain the free acid. $NaHCO_3$ (0.016 g, 190.4612 μmol) was added to the free acid in Acetonitrile (10 mL) and Water (10 mL) at 25°C. The mixture was stirred at 25°C for 1 h. The solvent was removed by lyophilization. There was sodium (S)-4-(5-(3-((2-((S)-3-carboxylatobutanoyl)-4-fluoro-6-methoxybenzo[b]thiophen-5-yl)oxy)propoxy)-6-meth oxyisoindolin-2-yl)-2-methyl-4-oxobutanoate (0.063 g, 93.2466 μmol, 24.3505% yield) obtained as a white solid. LCMS: (ESI, m/z): $[M+H]^+$ = 632.289. $^1H$ NMR (400 MHz, DMSO-$d_6$) δ 8.17 - 8.05 (m, 1H), 7.51 (s, 1H), 6.96 - 6.80 (m, 2H), 4.92 - 4.72 (m, 2H), 4.74 - 4.58 (m, 2H), 4.48 (s, 2H), 4.29 - 4.08 (m, 5H), 3.87 (s, 3H), 3.74 - 3.65 (m, 3H), 2.80 - 2.57 (m, 4H), 2.10 (s, 3H), 1.03 (d, *J* = 6.9 Hz, 6H).

## EXAMPLE 7

**sodium 4-(5-(3-((2-(3-carboxylatopropanoyl)-6-hydroxybenzo[b]thiophen-5-yl)oxy)propoxy)-6-methoxyisoindo lin-2-yl)-4-oxobutanoate**

***Step a: 5-bromo-2-fluoro-4-(methoxymethoxy)benzaldehyde***

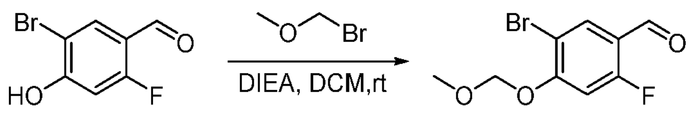


Bromomethyl methyl ether (0.71 g, 5.6816 mmol) was added to a solution of DIEA (0.83 g, 6.4220 mmol) and 5-bromo-2-fluoro-4-hydroxybenzaldehyde (0.94 g, 4.2921 mmol) in DCM (20 mL) at 0°C. The reaction mixture was stirred for 1 h at 25°C. The reaction mixture was quenched with adding of water (10 mL) at 20°C. The resulted mixture was washed with water (20 mL) and brine (20 mL). The organics dried over $Na_2SO_4$, filtered and evaporated under reduced pressure. There was a crude 5-bromo-2-fluoro-4-(methoxymethoxy)benzaldehyde (1.16 g, 4.4096 mmol, 102.7388% yield) obtained as a white solid. LCMS: (ESI, m/z): $[M+H]^+$ = 262.950. $^1H$ NMR (400 MHz, DMSO-$d_6$) $\delta$ 10.03 (s, 1H), 8.02 (d, $J$ = 7.5 Hz, 1H), 7.28 (d, $J$ = 12.6 Hz, 1H), 5.45 (s, 2H), 3.43 (s, 3H).

***Step b: methyl 5-bromo-6-(methoxymethox y)benzothiophene-2-carboxylate***

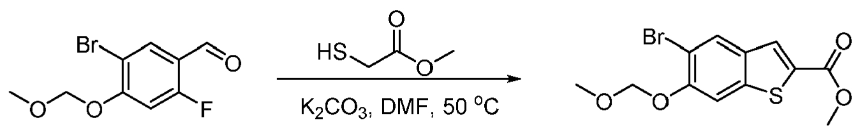


Methyl thioglycolate (0.4 mL, 4.4732 mmol) was added to a solution of 5-bromo-2-fluoro-4-(methoxymethoxy) benzaldehyde (1.06 g, 4.0295 mmol) and Potassium carbonate (0.15 g, 1.0853 mmol) in DMF (10 mL) at 25°C. The reaction mixture was heated to 50°C and stirred for 2 h. The reaction mixture was quenched with adding of water (100 mL) at 20°C. The precipitate was collected by filtration, washed with water (3 x 20 mL). The filtrate cake was dried under vacuo at 60°C. There was methyl 5-bromo-6-(methoxymethox y)benzothiophene-2-carboxylate (1.12 g, 3.3818 mmol, 83.9267% yield) obtained as a white solid. LCMS: (ESI, m/z): $[M+H]^+$ = 331.100.

***Step c: 5-bromo-6-(methoxymethoxy) benzothiophene-2-carboxylic acid***

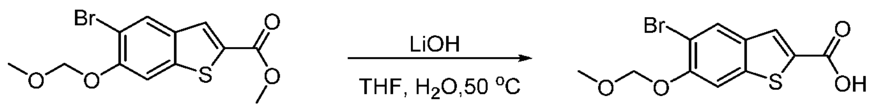


LiOH (0.18 g, 7.5162 mmol) was added to a solution of methyl 5-bromo-6-(methoxymethoxy) benzothiophene-2-carboxylate (1.10 g, 3.3214 mmol) in THF (40 mL) and Water (10 mL) at 25°C. The reaction mixture was stirred for 2 h at 50°C. The reaction mixture was evaporated under reduced pressure. The residue was diluted with water and acidified pH=3 with HCl (aq., 1 M). The precipitate was filtered and filtrate cake was washed with water (20 mL). The filtrate cake was dried under vacuo at 60°C. There was 5-bromo-6-(methoxymethoxy) benzothiophene-2-carboxylic acid (1.04 g, 3.2791 mmol, 98.7268% yield) obtained as a white solid. LCMS: (ESI, m/z): $[M-H]^-$ = 314.900. $^1H$ NMR (400 MHz, DMSO-$d_6$) $\delta$ 13.49 (s, 1H), 8.30 (s, 1H), 7.99 (s, 1H), 7.86 (s, 1H), 5.39 (s, 2H), 3.44 (s, 3H).

***Step d: tert-butyl 3-(5-bromo-6-(methoxymethoxy)benzo[b]thiophen-2-yl)-3-oxopropanoate***

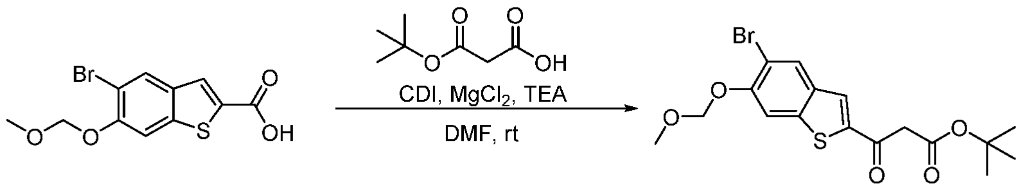


CDI (1.13 g, 6.9689 mmol) was added to a solution of 5-bromo-6-(methoxymethoxy) benzothiophene-2-carboxylic acid (1.01 g, 3.1846 mmol) in DMF (20 mL) at 25°C. The reaction mixture was stirred for 2 h at 25°C under nitrogen atmosphere. Magnesium chloride (0.64 g, 6.7219 mmol), 3-tert-Butoxy-3-oxopropanoic acid (0.81 g, 5.0572 mmol) and TEA (1.06 g, 10.4754 mmol) were added to the reaction mixture. The reaction mixture was stirred overnight at 25°C. The reaction mixture was concentrated and diluted with EA (100 mL), washed with saturated aqueous $NaHCO_3$ solution (2 x 50 mL) and brine (50 mL). The organics dried over $Na_2SO_4$, filtered and evaporated under reduced pressure. The residue was purified on silica gel column EA/n-Hexane (0-20%). The pure fractions was concentrated and dried under vacuo. There was tert-butyl 3-(5-bromo-6-(methoxymethoxy)benzo[b]thiophen-2-yl)-3-oxopropanoate (1.12 g, 2.6969 mmol, 84.6855% yield) obtained as a yellow solid. LCMS: (ESI, m/z): $[M-H]^-$ = 412.950. $^1H$ NMR (400 MHz, DMSO-$d_6$) $\delta$ 8.33 (s, 1H), 8.22 (s, 1H), 7.87 (s, 1H), 5.39 (d, *J* = 10.0 Hz, 2H), 4.06 (d, *J* = 8.4 Hz, 2H), 3.45 (s, 3H), 1.40 (s, 9H).

***Step e: 1-(tert-butyl) 4-ethyl 2-(5-bromo-6-(methoxymethoxy)benzo[b]thiophene-2-carbonyl)succinate***

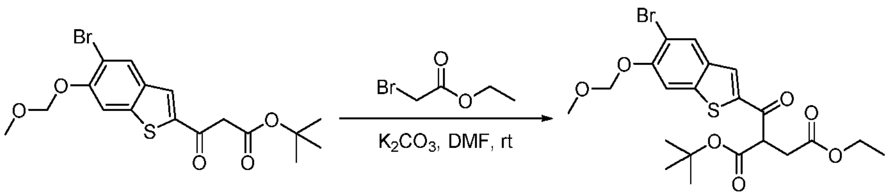


Ethyl 2-bromoacetate (0.356 g, 2.1317 mmol) was added to a solution of tert-butyl 3-(5-bromo-6-(methoxymethoxy)benzo[b]thiophen-2-yl)-3-oxopropanoate (0.891 g, 2.1454 mmol) and Potassium carbonate (0.587 g, 4.2473 mmol) in DMF (10 mL) at 25°C. The reaction mixture was stirred overnight at 25°C under nitrogen atmosphere. The reaction mixture was concentrated and diluted with EA (200 mL), washed with water (100 mL) and brine (100 mL). The organics dried over $Na_2SO_4$, filtered and evaporated under reduced pressure. The residue was purified on silica gel column EA/n-Hexane (0-50%). The pure fractions was concentrated and dried under vacuo. There was 1-(tert-butyl) 4-ethyl 2-(5-bromo-6-(methoxymethoxy)benzo[b]thiophene-2-carbonyl)succinate (0.771 g, 1.5377 mmol, 71.6743% yield) obtained as a yellow oil. LCMS: (ESI, m/z): $[M+H]^+$ = 501.050.

*Step **f: ethyl 4-(5-bromo-6-hydroxy-benzothiophen-2-yl)-4-oxo-butanoate***

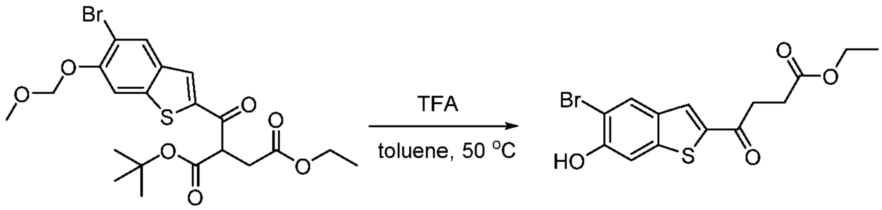


Trifluoroacetic acid (3 mL) was added to a solution of 1-(tert-butyl) 4-ethyl 2-(5-bromo-6-(methoxymethoxy) benzo[b]thiophene-2-carbonyl)succinate (0.750 g, 1.4959 mmol) in toluene (9 mL) at 25°C. The reaction mixture was heated to 50°C and stirred for 2 h. The reaction mixture was evaporated under reduced pressure. The residue was purified on C18 column MeCN/water (0-50%). The pure fractions was concentrated and dried under vacuo. There was ethyl 4-(5-bromo-6-hydroxy-benzothiophen-2-yl)-4-oxo-butanoate (0.431 g, 1.2065 mmol, 80.6593% yield) obtained as a white solid. LCMS: (ESI, m/z): $[M+H]^+$ = 357.000.

***Step g: ethyl 4-(5-bromo-6-(methoxymethoxy)benzo[b]thiophen-2-yl)-4-oxobutanoate***

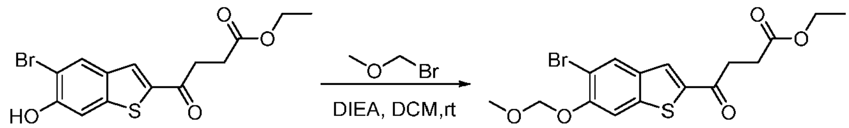


Bromomethyl methyl ether (0.198 g, 1.5845 mmol) was added to a solution of ethyl 4-(5-bromo-6-hydroxybenzothiophen-2-yl)-4-oxo-butanoate (0.427 g, 1.1953 mmol) and DIEA (0.235 g, 1.8183 mmol) in DCM (10 mL) at 25°C. The reaction mixture was stirred for 1 h at 25°C. The reaction mixture was concentrated and diluted with DCM (100 mL), washed with water (50 mL) and brine (50 mL). The organics dried over $Na_2SO_4$, filtered and evaporated under reduced pressure. There was ethyl 4-(5-bromo-6-(methoxymethoxy)benzo[b]thiophen-2-yl)-4-oxobutanoate (0.503 g, 1.1282 mmol, 94.3798% yield) obtained as a yellow solid. LCMS: (ESI, m/z): $[M+H]^+$ = 401.100.

***Step h: ethyl***

***4-(6-(methoxymethoxy)-5-(4,4,5,5-tetramethyl-1,3,2-dioxaborolan-2-yl)benzo[b]thiophen-2-yl)-4-oxobutan oate***

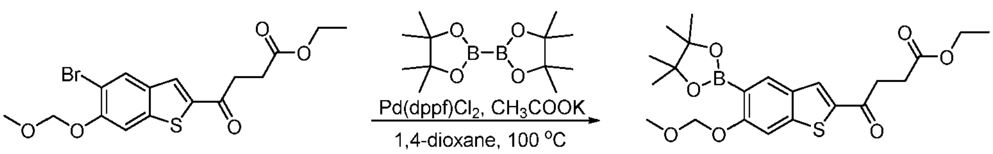


A mixture of [1,1'-Bis(diphenylphosphino)ferrocene]dichloropalladium(II) (0.110 g, 150.3341 μmol), ethyl 4-(5-bromo-6-(methoxymethoxy)benzo[b]thiophen-2-yl)-4-oxobutanoate (0.478 g, 1.1912 mmol), 4,4,4',4',5,5,5',5'-Octamethyl-2,2'-bi(1,3,2-dioxaborolane) (0.491 g, 1.9335 mmol) and Potassium Acetate (0.389 g, 3.9636 mmol) dissolve in 1,4-Dioxane (10 mL) at 25°C. The reaction mixture was heated to 100°C and stirred for 4.5 h under $N_2$ atmosphere. The reaction mixture was concentrated and diluted with EA (200 mL), washed with water (100 mL) and brine (100 mL). The organics dried over $Na_2SO_4$, filtered and evaporated under reduced pressure. The residue was purified on silica gel column EA/n-Hexane (0-50%). The pure fractions was concentrated and dried under vacuo. There was ethyl 4-(6-(methoxymethoxy)-5-(4,4,5,5-tetramethyl-1,3,2-dioxaborolan-2-yl)benzo[b]thiophen-2-yl)-4-oxobutano ate (0.420 g, 936.7968 μmol, 78.6423% yield) obtained as a yellow oil. LCMS: (ESI, m/z): $[M+H]^+$ = 449.200.

***Step i: ethyl 4-(5-hydroxy-6-(methoxymethoxy)benzo[b]thiophen-2-yl)-4-oxobutanoate***

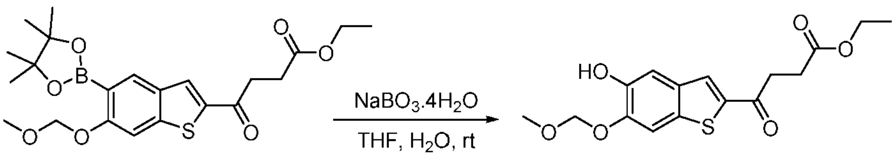


Sodium perborate tetrahydrate (0.200 g, 1.2999 mmol) was added to a solution of ethyl 4-(6-(methoxymethoxy)-5-(4,4,5,5-tetramethyl-1,3,2-dioxaborolan-2-yl)benzo[b]thiophen-2-yl)-4-oxobutano ate (0.407 g, 907.8013 μmol) in THF (5 mL) and Water (5 mL) at 25°C. The reaction mixture was stirred for 2 h at 25°C. The reaction mixture was evaporated under reduced pressure. The residue was purified on C18 column MeCN/water (0-50%). The pure fractions was concentrated and dried under vacuo. There was ethyl 4-(5-hydroxy-6-(methoxymethoxy)benzo[b]thiophen-2-yl)-4-oxobutanoate (0.250 g, 738.8257 μmol, 81.3863% yield) obtained as a yellow solid. LCMS: (ESI, m/z): $[M+H]^+$ = 339.100.

***Step j: ethyl 4-(5-methoxyisoindolin-2-yl)-4-oxo-butanoate***

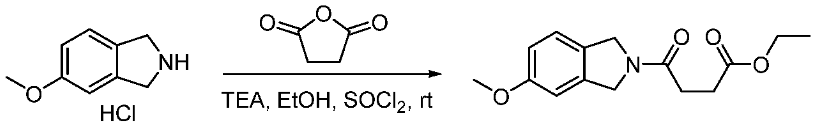


Succinic anhydride (4.40 g, 43.9681 mmol) was added to a solution of 5-Methoxyisoindoline hydrochloride (4.89 g, 26.3399 mmol) and TEA (4.73 g, 46.7440 mmol) in Ethanol (200 mL) at 25°C. The reaction mixture was stirred for 1 h at 25°C. Thionyl chloride (20 mL) was added to the solution aboved at 0°C. The reaction mixture was stirred for 3 h at 25°C. The reaction mixture was evaporated under reduced pressure. The residue was diluted with EA (200 mL), washed with water (100 mL) and brine (50 mL). The organics dried over $Na_2SO_4$, filtered and evaporated under reduced pressure. There was ethyl 4-(5-methoxyisoindolin-2-yl)-4-oxo-butanoate (17.50 g, 31.5526 mmol, 119.7901% yield) obtained as a yellow solid. LCMS: (ESI, m/z): $[M+H]^+$ = 278.150. $^1H$ NMR (400 MHz, DMSO-$d_6$) $\delta$ 7.28 - 7.21 (m, 1H), 6.94 (s, 0.5H), 6.92 (s, 0.5H), 6.88 (s, 0.5H), 6.86 (s, 0.5H), 4.81 (s, 1H), 4.76 (s, 1H), 4.58 (s, 1H), 4.54 (s, 1H), 4.02 - 3.95 (m, 2H), 3.75 (s, 3H), 2.65 - 2.58 (m, 2H), 2.58 - 2.54 (m, 2H), 1.17 (t, $J$ = 3.5 Hz, 3H).

***Step k: ethyl 4-(5-bromo-6-methoxy-isoindolin-2-yl)-4-oxo-butanoate***

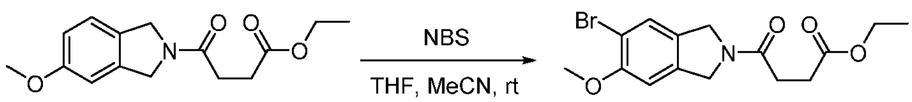


NBS (10.51 g, 59.0503 mmol) was added to a solution of ethyl 4-(5-methoxyisoindolin-2-yl)-4-oxo-butanoate (17.50 g, 31.5526 mmol) in THF (100 mL) and MeCN (100 mL) at 25°C. The reaction mixture was stirred overnight at 25°C. The reaction mixture was evaporated under reduced pressure. The residue was diluted with DCM (500 mL), washed with saturated aqueous $NaHCO_3$ solution (2 x 300 mL) and brine (200 mL). The organics dried over $Na_2SO_4$, filtered and evaporated under reduced pressure. The residue was purified on silica gel column MeOH/DCM (0-10%). The pure fractions was concentrated and dried under vacuo. There was ethyl 4-(5-bromo-6-methoxy-isoindolin-2-yl)-4-oxo-butanoate (7.26 g, 20.3812 mmol, 64.5943% yield) obtained as a yellow solid. LCMS: (ESI, m/z): $[M+H]^+$ = 356.000. $^1H$ NMR (400 MHz, DMSO-$d_6$) $\delta$ 7.58 (s, 0.5H), 7.57 (s, 0.5H), 7.15 (s, 0.5H), 7.12 (s, 0.5H), 4.80 (s, 1H), 4.77 (s, 1H), 4.57 (s, 1H), 4.54 (s, 1H), 4.09 - 4.01 (m, 2H), 3.84 (s, 3H), 2.65 - 2.59 (m, 2H), 2.56 - 2.53 (m, 2H), 1.18 (t, $J$ = 7.1 Hz, 3H).

***Step l: ethyl 4-(5-methoxy-6-(4,4,5,5-tetramethyl-1,3,2-dioxaborolan-2-yl)isoindolin-2-yl)-4-oxobutanoate***

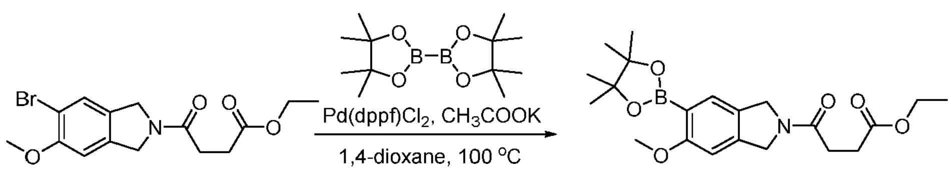


A mixture of Potassium Acetate (3.74 g, 38.1080 mmol) , [1,1'-Bis(diphenylphosphino)ferrocene] dichloropalladium(II) (0.72 g, 984.0050 µmol), ethyl 4-(5-bromo-6-methoxy-isoindolin-2-yl)-4-oxo-butanoate (3.64 g, 10.2187 mmol) and 4,4,4',4',5,5,5',5'-Octamethyl-2,2'-bi(1,3,2-dioxaborolane) (3.84 g, 15.1218 mmol) dissolve in 1,4-Dioxane (100 mL) at 25°C. The reaction mixture was heated to 100°C and stirred overnight under $N_2$ atmosphere. The reaction mixture was diluted with EA (400 mL). The precipitate was filtrated by celite, the filtrate cake was washed with EA (1 x 100 mL). The combined filtrate was evaporated under reduced pressure. The residue was purified on silica gel column EA/n-Hexane (0-100%). The pure fractions was concentrated and dried under vacuo. There was ethyl 4-(5-methoxy-6-(4,4,5,5-tetramethyl-1,3,2-dioxaborolan-2-yl)isoindolin-2-yl)-4-oxobutanoate (2.70 g, 6.6952 mmol, 65.5190% yield) obtained as a yellow solid. LCMS: (ESI, m/z): $[M+H]^+$ = 404.200. $^1H$ NMR (400 MHz, DMSO-$d_6$) $\delta$ 7.49 (s, 0.5H), 7.48 (s, 0.5H), 6.98 (s, 0.5H), 6.95 (s, 0.5H), 4.83 (s, 1H), 4.74 (s, 1H), 4.60 (s, 1H), 4.53 (s, 1H), 4.09 - 4.01 (m, 2H), 3.74 (t, $J$ = 6.2 Hz, 3H), 2.65

- 2.58 (m, 2H), 2.58 - 2.52 (m, 2H), 1.27 (s, 12H), 1.21 - 1.13 (m, 3H).

***Step m: ethyl 4-(5-hydroxy-6-methoxy-isoindolin-2-yl)-4-oxo-butanoate***

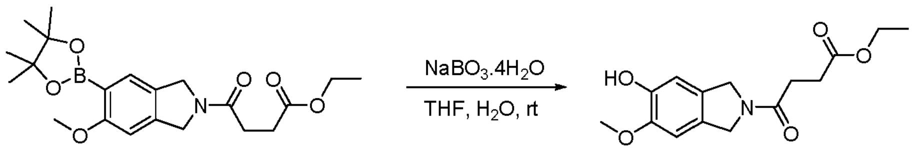


Sodium perborate tetrahydrate (1.00 g, 6.4994 mmol) was added to a solution of ethyl 4-(5-methoxy-6-(4,4,5,5-tetramethyl-1,3,2-dioxaborolan-2-yl)isoindolin-2-yl)-4-oxobutanoate (2.45 g, 6.0752 mmol) in THF (20 mL) and Water (20 mL) at 25°C. The reaction mixture was stirred for 1 h at 20°C. The reaction mixture was evaporated under reduced pressure. The reaction mixture was concentrated and diluted with DCM (500 mL), washed with saturated aqueous $NaHCO_3$ solution (2 x 300 mL) and brine (200 mL). The organics dried over $Na_2SO_4$, filtered and evaporated under reduced pressure. The residue was purified on silica gel column MeOH/DCM (0-10%). The pure fractions was concentrated and dried under vacuo. There was ethyl 4-(5-hydroxy-6-methoxy-isoindolin-2-yl)-4-oxo-butanoate (1.37 g, 4.6708 mmol, 76.8818% yield) obtained as a yellow solid. LCMS: (ESI, m/z): $[M+H]^+$ = 294.100. $^1H$ NMR (400 MHz, DMSO-$d_6$) $\delta$ 8.97 (s, 0.5H), 8.96 (s, 0.5H), 6.91 (s, 0.5H), 6.89 (s, 0.5H), 6.73 (s, 0.5H), 6.73 (s, 0.5H), 4.71 (s, 1H), 4.69 (s, 1H), 4.50 (s, 1H), 4.47 (s, 1H), 4.08 - 4.00 (m, 2H), 3.75 (s, 3H), 2.64 - 2.58 (m, 2H), 2.56 - 2.53 (m, 2H), 1.18 (t, $J$ = 7.1 Hz, 3H).

***Step n: ethyl 4-(5-(3-bromopropoxy)-6-methoxyisoindolin-2-yl)-4-oxobutanoate***

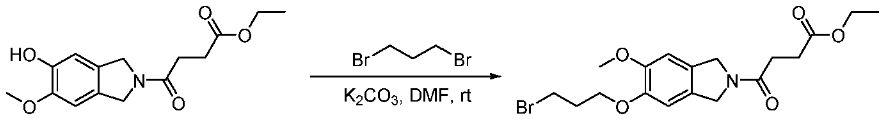


1,3-Dibromopropane (0.73 g, 3.6159 mmol) was added to a solution of ethyl 4-(5-hydroxy-6-methoxy-isoindolin-2-yl)-4-oxo-butanoate (0.72 g, 2.4547 mmol) and Potassium carbonate (0.86 g, 6.2226 mmol) in DMF (10 mL). The reaction mixture was stirred overnight at 50°C. The reaction mixture was diluted with EA (100 mL), and washed sequentially with water (2 x 100 mL) and saturated brine (100 mL). The organic layer was dried over $Na_2SO_4$, filtered and evaporated to afford crude product. The crude product was purified by flash silica chromatography, elution gradient 0 to 100% Ethyl acetate in heptane. There was ethyl 4-(5-(3-bromopropoxy)-6-methoxyisoindolin-2-yl)-4-oxobutanoate (0.43 g, 1.0379 mmol, 42.2829% yield) obtained as a white solid. LCMS: (ESI, m/z): $[M+H]^+$ = 414.050. $^1H$ NMR (400 MHz, DMSO-$d_6$) $\delta$ 7.01 - 6.93 (m, 2H), 4.75 (s, 2H), 4.53 (s, 2H), 4.10 - 4.00 (m, 4H), 3.76 (s, 3H), 3.71 - 3.62 (m, 2H), 2.65 - 2.58 (m, 2H), 2.58 - 2.53 (m, 2H), 2.28 - 2.18 (m, 2H), 1.18 (t, $J$ = 7.1 Hz, 3H).

***Step o: ethyl 4-(5-(3-((2-(4-ethoxy-4-oxobutanoyl)-6-(methoxymethoxy)benzo[b]thiophen-5-yl)oxy)propoxy)-6-methoxy isoindolin-2-yl)-4-oxobutanoate***

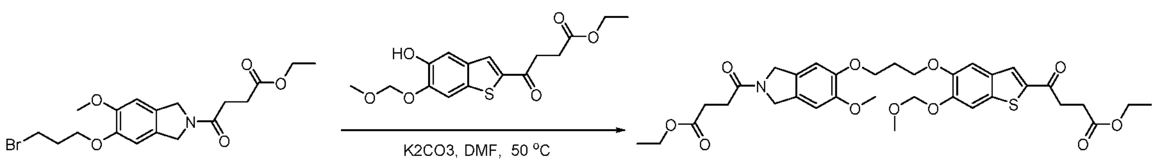


Ethyl 4-[5-hydroxy-6-(methoxymethoxy) benzothiophen-2-yl]-4-oxo-butanoate (0.101 g, 298.4856 μmol) was added to a solution of ethyl 4-(5-(3-bromopropoxy)-6-methoxyisoindolin-2-yl)-4-oxobutanoate (0.127 g, 306.5486 μmol) and Potassium carbonate (0.142 g, 1.0275 mmol) in DMF (5 mL) at 25°C. The reaction mixture was heated to 50°C and stirred overnight. The reaction mixture was concentrated and diluted with EA (200 mL), washed with water (100 mL) and

brine (100 mL). The organics dried over $Na_2SO_4$, filtered and evaporated under reduced pressure. The residue was purified on silica gel column EA/n-Hexane (0-100%). The pure fractions was concentrated and dried under vacuo. There was ethyl 4-(5-(3-((2-(4-ethoxy-4-oxobutanoyl)-6-(methoxymethoxy)benzo[b]thiophen-5-yl)oxy)propoxy)-6-methoxyi soindolin-2-yl)-4-oxobutanoate (0.055 g, 81.8754 μmol, 27.4303% yield) obtained as a yellow solid. LCMS: (ESI, m/z): [M+H] + = 672.250.

***Step p: ethyl 4-(5-(3-((2-(4-ethoxy-4-oxobutanoyl)-6-hydroxybenzo[b]thiophen-5-yl)oxy)propoxy)-6-methoxyisoindolin-2-yl)-4-oxobutanoate***

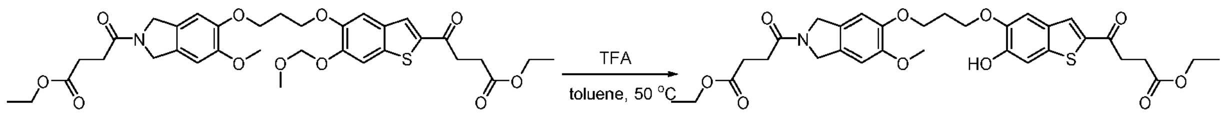


Trifluoroacetic acid (1.0 mL, 13.4622 mmol) was added to a solution of ethyl 4-(5-(3-((2-(4-ethoxy-4-oxobutanoyl)-6-(methoxymethoxy)benzo[b]thiophen-5-yl)oxy)propoxy)-6-methoxyi soindolin-2-yl)-4-oxobutanoate (0.054 g, 80.3867 μmol) in toluene (2 mL) at 25°C. The reaction mixture was heated to 50°C and stirred for 30 min. The reaction mixture was evaporated under reduced pressure. The residue was diluted with water and neutralized pH=8 with saturated aqueous $NaHCO_3$ solution. The resulting mixture was extracted with EA (100 mL), washed with water (50 mL) and brine (50 mL). The organics dried over $Na_2SO_4$, filtered and evaporated under reduced pressure. There was ethyl 4-(5-(3-((2-(4-ethoxy-4-oxobutanoyl)-6-hydroxybenzo[b]thiophen-5-yl)oxy)propoxy)-6-methoxyisoindolin-2-yl)-4-oxobutanoate (0.057 g, 77.1865 μmol, 96.0190% yield) obtained as a yellow solid. LCMS: (ESI, m/z): [M+H] + = 628.200.

***Step q: sodium 4-(5-(3-((2-(3-carboxylatopropanoyl)-6-hydroxybenzo[b]thiophen-5-yl)oxy)propoxy)-6-methoxyisoindolin-2-yl)-4-oxobutanoate***

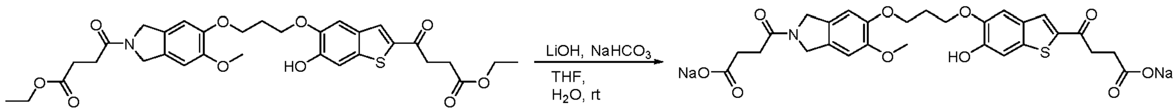


LiOH (0.023 g, 960.4022 μmol) was added to a solution of ethyl 4-(5-(3-((2-(4-ethoxy-4-oxobutanoyl)-6-hydroxybenzo[b]thiophen-5-yl)oxy)propoxy)-6-methoxyisoindolin-2-yl)-4-oxobutanoate (0.055 g, 87.6215 μmol) in THF (4 mL) and Water (1 mL) at 25°C. The reaction mixture was stirred overnight at 25°C. The reaction mixture was evaporated under reduced pressure. The residue was acidified pH=4 with HCl (1 M). The reaction mixture was evaporated under reduced pressure to obtain a crude product. The crude product was purified by Prep-HPLC with the following conditions: (CXTH LC6000, HPLC-P1): Column, Daisogel-C18, 50mm*250mm, 10um; mobile phase, Water (0.1%TFA) and MeCN-(15-40-60%B(2-30-60min); Detector, uv 216 nm). The solvent was removed by lyophilization. $NaHCO_3$ (0.004 g, 47.6153μmol) was added to the lyophilized product in water, and stirred for 30 min at 25°C. The solvent was removed by lyophilization. There was sodium 4-(5-(3-((2-(3-carboxylatopropanoyl)-6-hydroxybenzo[b]thiophen-5-yl)oxy)propoxy)-6-methoxyisoindolin-2 -yl)-4-oxobutanoate (0.012 g, 19.4945 μmol, 22.2485% yield) obtained as a light yellow solid. LCMS: (ESI, m/z): [M+H] + = 572.150. $^1$H NMR (400 MHz, DMSO-$d_6$) δ 8.00 (s, 0.5H), 7.97 (s, 0.5H), 7.39 (s, 0.5H), 7.37 (s, 0.5H), 7.30 (s, 0.5H), 7.28 (s, 0.5H), 6.99 - 6.87 (m, 2H), 4.74 (s, 1H), 4.70 (s, 1H), 4.49 (s, 1H), 4.47 (s, 1H), 4.20 - 4.08 (m, 4H), 3.73 (s, 3H), 3.07 (t, *J* = 6.8 Hz, 2H), 2.47 - 2.40 (m, 2H), 2.36 - 2.16 (m, 6H).

**EXAMPLE 8 (not part of the invention)**

**sodium 4-(5-(3-((2-(3-carboxylatopropanoyl)-6-methoxyisoindolin-5-yl)oxy)propoxy)-6-methoxythieno[3,2-b]p yridin-2-yl)-4-oxobutanoate**

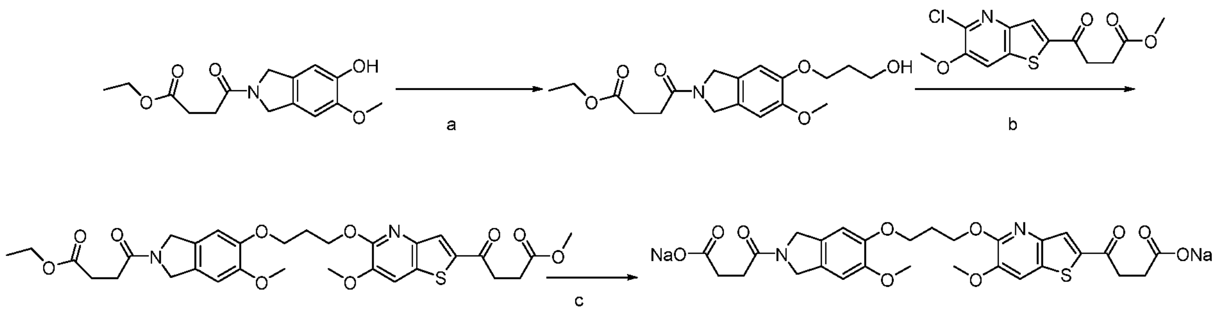


***Step a:*** (3-Bromopropoxy)-tert-butyldimethylsilane (518 mg, 2.0456 mmol) and $K_2CO_3$ (320 mg, 2.3154 mmol) was added to a solution of ethyl 4-(5-hydroxy-6-methoxyisoindolin-2-yl)-4-oxobutanoate (200 mg, 681.8624 μmol) in N,N-Dimethylformamide (8 mL) at 25°C. The reaction mixture was stirred for 2 h at 25°C. Tetrabutylammonium fluoride (178 mg, 681.8624 μmol) was added to the mixture. The reaction mixture was stirred for 2 h at 25°C. The reaction mixture was concentrated and diluted with EA (100 mL), washed with $NaHCO_3$ (aq)(100 mL) and brine (50 mL). The organics dried over $Na_2SO_4$, filtered and evaporated under reduced pressure. The residue was purified on silica gel column MeOH/DCM (0-10%). The pure fractions was concentrated and dried under vacuo. There was ethyl 4-(5-(3-hydroxypropoxy)-6-methoxyisoindolin-2-yl)-4-oxobutanoate (106 mg, 301.6564 μmol, 44.2401% yield) obtained as a white solid. LCMS: (ESI, m/z): $[M+H]^+$ = 352.168. $^1H$ NMR (400 MHz, DMSO-$d_6$) $\delta$ 8.31 (s, 1H), 6.95 (d, J = 9.4 Hz, 2H), 4.74 (s, 2H), 4.53 (s, 2H), 4.00 (t, J = 6.1 Hz, 2H), 3.75 (s, 3H), 3.20 - 3.13 (m, 2H), 2.63 - 2.51 (m, 4H), 1.57 (s, 2H), 1.38 - 1.21 (m, 2H), 0.94 (t, J = 7.1 Hz, 3H).

***Step b:*** $K_3PO_4$ (151 mg, 711.3706 μmol) and RockPhos Palladacycle Gen.3 (11 mg, 13.1041 μmol) was added to a solution of ethyl 4-(5-(3-hydroxypropoxy)-6-methoxyisoindolin-2-yl)-4-oxobutanoate (50 mg, 142.2907 μmol) and methyl 4-(5-chloro-6-methoxy-thieno[3,2-b]pyridin-2-yl)-4-oxo-butanoate (66 mg, 210.3544 μmol) in Toluene (10 mL) at 25°C. The reaction mixture was stirred at 130°C for 16 h under $N_2$ atmosphere. The reaction mixture was purified on C-18 column MeCN/water (0-100%). The pure fractions was concentrated and dried under vacuo. There was ethyl 4-(5-methoxy-6-(3-((6-methoxy-2-(4-methoxy-4-oxobutanoyl)thieno[3,2-b]pyridin-5-yl)oxy)propoxy)isoind olin-2-yl)-4-oxo-butanoate (4.1 mg, 6.5215 μmol, 4.5832% yield) obtained as a yellow oil. LCMS: (ESI, m/z): $[M+H]^+$ = 629.209.

***Step c:*** LiOH (0.006 g, 250.5397 μmol) was added to a solution of ethyl 4-(5-methoxy-6-(3-((6-methoxy-2-(4-methoxy-4-oxobutanoyl)thieno[3,2-b]pyridin-5-yl)oxy)propoxy)isoind olin-2-yl)-4-oxobutanoate (0.013 g, 20.6780 μmol) in Tetrahydrofuran (4 mL) and Water (1 mL) at 25°C. The reaction mixture was stirred for 2 h at 25°C. The reaction mixture was evaporated under reduced pressure. The residue was concentrated and diluted with water (2 mL).The resulting mixture was adjusted to pH=7 with HCl (1 M) at 25°C and evaporated under reduced pressure. The residue was purified by HPLC. The pure fractions was concentrated and dried by lyophilization to obtain the free acid. $NaHCO_3$ (0.001 g, 11.9038 μmol) was added to a mixture of the free acid in Acetonitrile (2 mL) and Water (1 mL) at 25°C. The mixture was stirred at 25°C for 1 h and dried by lyophilization. There was sodium 4-(5-(3-((2-(3-carboxylatopropanoyl)-6-methoxyisoindolin-5-yl)oxy)propoxy)-6-methoxythieno[3,2-b]pyridi n-2-yl)-4-oxobutanoate (0.0043 g, 6.8192 μmol, 32.9781% yield) obtained as a white solid. LCMS: (ESI, m/z): $[M+H]^+$ = 587.162. $^1H$ NMR (400 MHz, DMSO-$d_6$) $\delta$ 8.07 (s, 0.5H), 8.03 (s, 0.5H), 7.96 (s, 1H), 7.02 - 6.93 (m, 2H), 4.80 - 4.73 (m, 2H), 4.50 (s, 4H), 4.13 (s, 2H), 3.88 (s, 3H), 3.75 - 3.73 (m, 3H), 3.08 (s, 2H), 2.43 (s, 2H), 2.24 (s, 4H), 2.15 (s, 2H).

**EXAMPLE 9**

**4-(5-(3-((6-bromo-2-(3-carboxypropanoyl)benzo[b]thiophen-5-yl)oxy)propoxy)-6-methoxyisoindolin-2-yl)-4-oxobutanoic acid**

***Step a: ethyl 4-(5-(3-((6-bromo-2-(4-ethoxy-4-oxobutanoyl)benzo[b]thiophen-5-yl)oxy)propoxy)-6-methoxyisoindolin-2 -yl)-4-oxobutanoate***

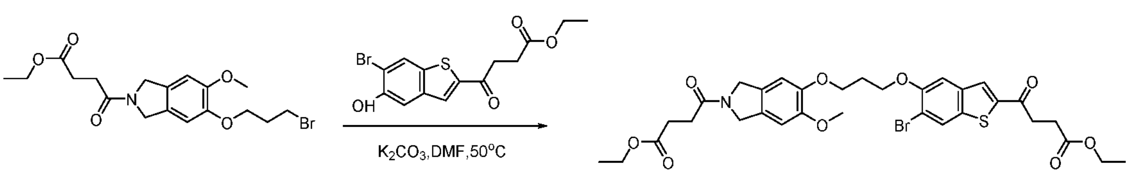

Potassium carbonate (0.080 g, 578.8482 μmol) was added to a solution of ethyl 4-(5-(3-bromopropoxy)-6-methoxyisoindolin-2-yl)-4-oxobutanoate (0.075 g, 181.0327 μmol) and ethyl 4-(6-bromo-5-hydroxybenzo[b]thiophen-2-yl)-4-oxobutanoate (0.071 g, 198.7576 μmol) in DMF (2 mL) at 25°C under $N_2$ atmosphere. The reaction mixture was heated to 50°C and stirred overnight. The reaction mixture was purified on C18 column ACN/$H_2O$ (0.1%FA)(0-60%). The pure fractions was concentrated and dried by lyophilization. There was ethyl 4-(5-(3-((6-bromo-2-(4-ethoxy-4-oxobutanoyl)benzo[b]thiophen-5-yl)oxy)propoxy)-6-methoxyisoindolin-2-yl)-4-oxobutanoate (0.080 g, 115.8418 μmol, 63.9894% yield) obtained as a white solid. LCMS: (ESI, m/z): $[M+H]^+$ = 690.13. $^1H$ NMR (400 MHz, DMSO-$d_6$) δ 8.39 (s, 1H), 8.26 (s, 1H), 7.69 (s, 1H), 7.05 - 6.92 (m, 2H), 4.73 (s, 1H), 4.72 (s, 1H), 4.51 (d, *J* = 3.6 Hz, 2H), 4.29 (s, 2H), 4.19 (t, *J* = 6.4 Hz, 2H), 4.11 - 4.00 (m, 4H), 3.73 (d, *J* = 2.0 Hz, 3H), 3.40 - 3.34 (m, 2H), 2.68 (t, *J* = 6.3 Hz, 2H), 2.63 - 2.53 (m, 4H), 2.26 (t, *J* = 6.3 Hz, 2H), 1.18 (t, *J* = 7.1 Hz, 6H).

***Step b: 4-(5-(3-((6-bromo-2-(3-carboxypropanoyl)benzo[b]thiophen-5-yl)oxy)propoxy)-6-methoxyisoindolin-2-yl)-4-oxobutanoic acid***

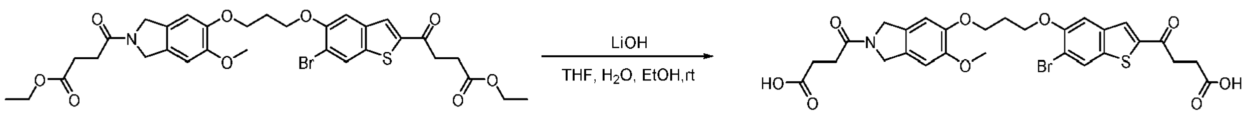


LiOH (0.050 g, 2.0878 mmol) was added to a solution of ethyl 4-(5-(3-((6-bromo-2-(4-ethoxy-4-oxobutanoyl)benzo[b]thiophen-5-yl)oxy)propoxy)-6-methoxyisoindolin-2-yl)-4-oxobutanoate (0.044 g, 63.7130 μmol) in THF (2 mL), EtOH (1 mL) and Water (2 mL) at 25°C. The reaction mixture was stirred for 2 h at 25°C. The reaction mixture was adjusted to pH=3 with HCl (1 mol/L). The reaction mixture was purified on C18 column ACN/$H_2O$ (0.1%FA)(0-55%). The pure fractions was concentrated and dried by lyophilization. There was 4-(5-(3-((6-bromo-2-(3-carboxypropanoyl)benzo[b]thiophen-5-yl)oxy)propoxy)-6-methoxyisoindolin-2-yl)-4-oxobutanoic acid (0.012 g, 18.9128 μmol, 29.6844% yield) obtained as a white solid. LCMS: (ESI, m/z): $[M+H]^+$ = 634.07. $^1H$ NMR (400 MHz, DMSO-$d_6$) δ 8.37 (s, 1H), 8.24 (d, *J* = 3.1 Hz, 1H), 7.68 (d, *J* = 3.8 Hz, 1H), 7.04 - 6.92 (m, 2H), 4.73 (s, 1H), 4.71 (s, 1H), 4.51 (d, *J* = 4.7 Hz, 2H), 4.29 (s, 2H), 4.23 - 4.15 (m, 2H), 3.73 (s, 3H), 3.33 - 3.25 (m, 2H), 2.63 - 2.54 (m, 4H), 2.53 - 2.50 (m, 2H), 2.31 - 2.20 (m, 2H).

**EXAMPLE 10**

**4-(5-(3-((2-(3-carboxypropanoyl)-4-fluoro-6-methoxyisoindolin-5-yl)oxy)propoxy)-6-methoxybenzo[b]t hiophen-2-yl)-4-oxobutanoic acid**

***Step a: ethyl 4-(5-(3-((2-(4-ethoxy-4-oxobutanoyl)-4-fluoro-6-methoxyisoindolin-5-yl)oxy)propoxy)-6-methoxybenzo[b] thiophen-2-yl)-4-oxobutanoate***

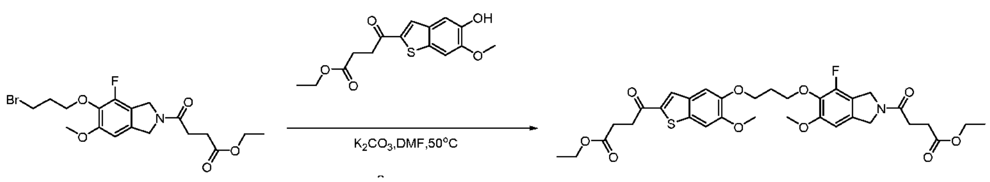


Potassium carbonate (0.099 g, 716.3246 μmol) was added to a solution of ethyl 4-(5-hydroxy-6-methoxybenzo[b]thiophen-2-yl)-4-oxobutanoate (0.062 g, 201.0709 μmol) and ethyl 4-(5-(3-bromopropoxy)-4-fluoro-6-methoxyisoindolin-2-yl)-4-oxobutanoate (0.069 g, 159.6186 μmol) in DMF (3 mL) at 25°C under $N_2$ atmosphere. The reaction mixture was stirred overnight at 50°C. The mixture was purified on C18 column ACN/$H_2O$ (0.1%FA)(0-50%). The pure fractions was concentrated and dried further in lyophilization. There was ethyl 4-(5-(3-((2-(4-ethoxy-4-oxobutanoyl)-4-fluoro-6-methoxyisoindolin-5-yl)oxy)propoxy)-6-methoxybenzo[b]t hiophen-2-yl)-4-oxobutanoate (0.071 g, 107.6218 μmol, 67.4244% yield) obtained as a white solid. LCMS: (ESI, m/z): $[M+H]^+$ = 660.220. $^1H$ NMR (400 MHz, DMSO-$d_6$) δ 8.22 (s, 1H), 7.59 (s, 1H), 7.50 (d, *J* = 3.4 Hz, 1H), 6.92 (s, 0.5H), 6.88 (s, 0.5H), 4.80 (s, 1H), 4.78 (s, 1H), 4.57 (s, 2H), 4.27 - 4.20 (m, 2H), 4.17 (t, *J* = 6.0 Hz, 2H), 4.10 - 4.02 (m, 4H), 3.85 (s, 3H), 3.76 (s, 3H), 3.31 - 3.28 (m, 2H), 2.69 - 2.53 (m, 6H), 2.20 - 2.09 (m, 2H), 1.18 (t, *J* = 7.1 Hz, 6H).

***Step b: 4-(5-(3-((2-(3-carboxypropanoyl)-4-fluoro-6-methoxyisoindolin-5-yl)oxy)propoxy)-6-methoxybenzo[b] thio phen-2-yl)-4-oxobutanoic acid***

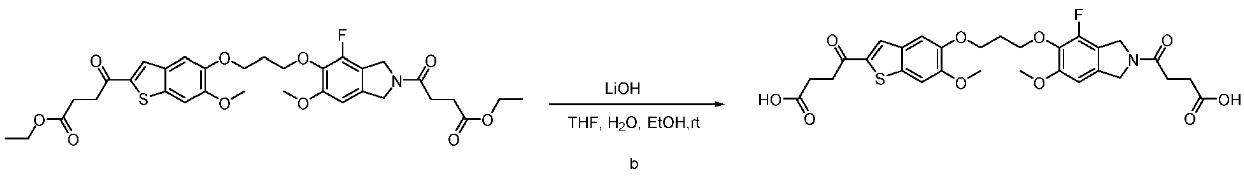


LiOH (0.044 g, 1.8373 mmol) was added to a mixture of ethyl 4-(5-(3-((2-(4-ethoxy-4-oxobutanoyl)-4-fluoro-6-methoxyisoindolin-5-yl)oxy)propoxy)-6-methoxybenzo[b]t hiophen-2-yl)-4-oxobutanoate (0.100 g, 155.1150 μmol) in THF (2 mL), EtOH (2 mL) and Water (2 mL) at 25°C. The reaction mixture was stirred for 2 h at 25°C. The reaction mixture was adjusted to pH=3 with HCl (1 mol/L), The mixture was evaporated under reduced pressure. The residue was purified on C18 column ACN/$H_2O$ (0.1%FA)(0-40%). The pure fractions was concentrated and dried under vacuo. There was 4-(5-(3-((2-(3-carboxypropanoyl)-4-fluoro-6-methoxyisoindolin-5-yl)oxy)propoxy)-6-methoxybenzo[b]thiop hen-2-yl)-4-oxobutanoic acid (0.026 g, 43.0741 μmol, 66.0854% yield) obtained as a white solid. LCMS: (ESI, m/z): $[M+H]^+$ = 604.160. $^1H$ NMR (400 MHz, DMSO-$d_6$) δ 8.21 (s, 1H), 7.59 (s, 1H), 7.50 (d, *J* = 3.0 Hz, 1H), 6.91 (s, 0.5H), 6.88 (s, 0.5H), 4.79 (s, 1H), 4.77 (s, 1H), 4.56 (s, 2H), 4.27 - 4.20 (m, 2H), 4.17 (t, *J* = 6.0 Hz, 2H), 3.85 (s, 3H), 3.76 (s, 3H), 3.26 - 3.24 (m, 2H), 2.63 - 2.54 (m, 4H), 2.48 - 2.44 (m, 2H), 2.19 - 2.10 (m, 2H).

**EXAMPLE 11 (not part of the invention)**

**4-(5-(3-((6-(3-carboxypropanoyl)-3-methoxy-6,7-dihydro-5H-pyrrolo[3,4-b]pyridin-2-yl)oxy)propoxy)-6-methoxythieno[3,2-b]pyridin-2-yl)-4-oxobutanoic acid**

***Step a: 3-methoxy-2-(3-tetrahydropyran-2-yloxypropoxy)-6,7-dihydro-5H-pyrrolo[3,4-b]pyridine***

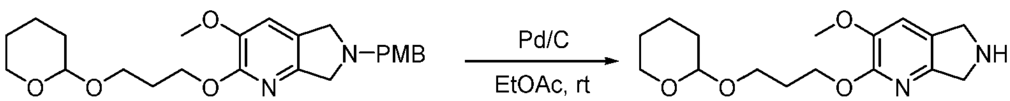


Pd/C (2.553 g, 2.3990 mmol) was added to a solution of 3-methoxy-6-(4-methoxybenzyl)-2-(3-((tetrahydro-2H-pyran-2-yl)oxy)propoxy)-6,7-dihydro-5H-pyrrolo[3,4 -b]pyridine (2.464 g, 5.7500 mmol) in Ethyl acetate (60 mL) at 25°C. The reaction mixture was stirred overnight at 25°C under $H_2$ atmosphere. The resulting mixture was filtered. The filter cake was washed with EA (3 x 100 mL). The filtrate was concentrated under reduced pressure. There was 3-methoxy-2-(3-tetrahydropyran-2-yloxypropoxy)-6,7-dihydro-5H-pyrrolo[3,4-b]pyridine (1.936 g, 4.3947 mmol, 76.4291 % yield ) obtained as a yellow oli. LCMS: (ESI, m/z): $[M+H]^+$ = 309.174.

***Step b: 3-((3-methoxy-6,7-dihydro-5H-pyrrolo[3,4-b]pyridin-2-yl)oxy)propan-1-ol***

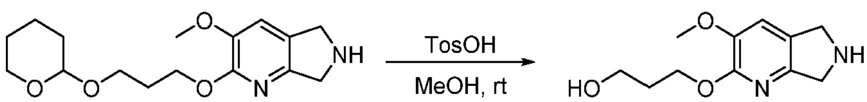


4-methylbenzenesulfonic acid (0.69 g, 2.5430 mmol) was added to a solution of 3-methoxy-2-(3-tetrahydropyran-2-yloxypropoxy)-6,7-dihydro-5H-pyrrolo[3,4-b]pyridine (1.91 g, 4.3357 mmol) in MeOH (40 mL) at 25°C. The reaction mixture was stirred for 2 h at 25°C. The reaction mixture was evaporated under reduced pressure. The residue was purified on C18 column MeCN/water (0-50%). The pure fractions was concentrated and dried under vacuo. There was 3-((3-methoxy-6,7-dihydro-5H-pyrrolo[3,4-b]pyridin-2-yl)oxy)propan-1-ol (0.814 g, 3.6298 mmol, 83.7191% yield) obtained as a yellow oil. LCMS: (ESI, m/z): $[M+H]^+$ = 225.116.

***Step c: ethyl 4-(2-(3-hydroxypropoxy)-3-methoxy-5,7-dihydro-6H-pyrrolo[3,4-b]pyridin-6-yl)-4-oxobutanoate***

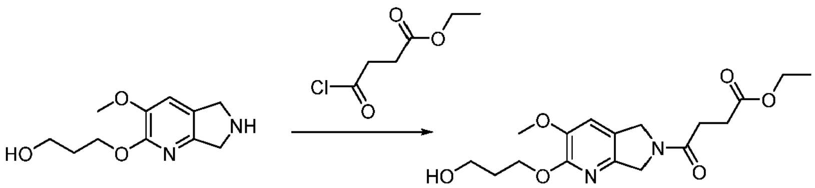

Ethyl Succinyl Chloride (0.232 g, 1.4096 mmol) was added to a solution of 3-((3-methoxy-6,7-dihydro-5H-pyrrolo [3,4-b]pyridin-2-yl)oxy)propan-1-ol (0.366 g, 1.6321 mmol) and Triethylamine (0.363 g, 3.5873 mmol) in Tetrahydrofuran (20 mL) at 0°C under $N_2$ atmosphere. The reaction mixture was stirred at 25°C for 1 h. The reaction mixture was purified on C-18 column MeCN/water (0-100%). The pure fractions was concentrated and dried under vacuo. There was ethyl 4-(2-(3-hydroxypropoxy)-3-methoxy-5,7-dihydro-6H-pyrrolo[3,4-b]pyridin-6-yl)-4-oxobutanoate (224 mg, 635.6750 μmol, 38.9491 % yield) obtained as a white solid. LCMS: (ESI, m/z): $[M+H]^+$ = 353.163. $^1$H NMR (400 MHz, DMSO-$d_6$) δ 7.38 - 7.26 (m, 1H), 4.81 - 4.73 (m, 1H), 4.68 (s, 1H), 4.54 (s, 2H), 4.42 (s, 1H), 4.30 (t, *J* = 6.3 Hz, 2H), 4.11 - 3.99 (m, 2H), 3.78 (s, 3H), 3.57 - 3.50 (m, 2H), 2.66 - 2.59 (m, 2H), 2.58 - 2.52 (m, 2H), 1.91 - 1.80 (m, 2H), 1.18 (t, *J* = 7.1 Hz, 3H).

***Step d: ethyl 4-(3-methoxy-2-(3-((6-methoxy-2-(4-methoxy-4-oxobutanoyl)thieno[3,2-b]pyridin-5-yl)oxy)propoxy)-5,7-d ihydro-6H-pyrrolo[3,4-b]pyridin-6-yl)-4-oxobutanoate***

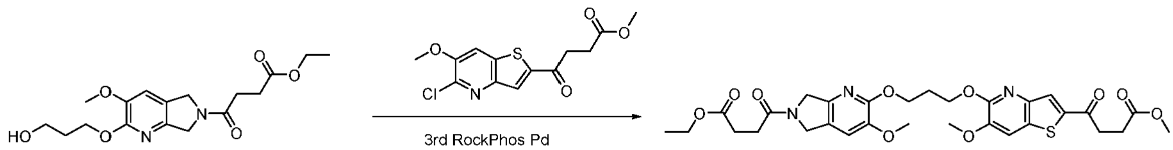


Potassium phosphate tribasic (0.212 g, 998.7454 μmol) and RockPhos Palladacycle Gen.3 (0.022 g, 26.2082 μmol) was added to a solution of ethyl 4-(2-(3-hydroxypropoxy)-3-methoxy-5,7-dihydro-6H-pyrrolo[3,4-b]pyridin-6-yl)-4-oxobutanoate (0.087 g, 246.8916 μmol) and methyl 4-(5-chloro-6-methoxy-thieno[3,2-b]pyridin-2-yl)-4-oxo-butanoate (0.120 g, 382.4626 μmol) in Toluene (20 mL) at 25°C. The reaction mixture was stirred overnight at 25°C under $N_2$ atmosphere. The reaction mixture was evaporated under reduced pressure. The residue was purified on C-18 column MeCN /water (0-100%). The pure fractions was concentrated and dried under vacuo.The residue was purified by HPLC. The pure fractions was concentrated and lyophilization. There was ethyl 4-(3-methoxy-2-(3-((6-methoxy-2-(4-methoxy-4-oxobutanoyl)thieno[3,2-b]pyridin-5-yl)oxy)propoxy)-5,7-di hydro-6H-pyrrolo[3,4-b]pyridin-6-yl)-4-oxobutanoate (20 mg, 31.7623 μmol, 12.8649% yield) obtained as a white solid. LCMS: (ESI, m/z): $[M+H]^+$ = 630.204. $^1$H NMR (400 MHz, DMSO-$d_6$) δ 8.22 (d, *J* = 3.6 Hz, 1H), 7.97 (d, *J* = 4.8 Hz, 1H), 7.37 - 7.28 (m, 1H), 4.73 (s, 1H), 4.61 (s, 1H), 4.55 - 4.48 (m, 3H), 4.47 - 4.36 (m, 3H), 4.09 - 4.01 (m, 2H), 3.89 (s, 3H), 3.78 (s, 3H), 3.61 (d, *J* = 5.6 Hz, 3H), 2.68 (d, *J* = 6.5 Hz, 2H), 2.62 - 2.54 (m, 3H), 2.25 (d, *J* = 4.2 Hz, 2H), 1.18 (t, *J* = 7.1 Hz, 3H).

***Step e: 4-(5-(3-((6-(3-carboxypropanoyl)-3-methoxy-6,7-dihydro-5H-pyrrolo[3,4-b]pyridin-2-yl)oxy)propoxy)-6-m ethoxythieno[3,2-b]pyridin-2-yl)-4-oxobutanoic acid***

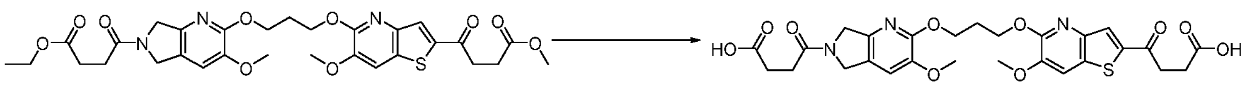

LiOH (0.004 g, 167.0265 μmol) was added to a solution of ethyl 4-(3-methoxy-2-(3-((6-methoxy-2-(4-methoxy-4-oxobutanoyl)thieno[3,2-b]pyridin-5-yl)oxy)propoxy)-5,7-di hydro-6H-pyrrolo[3,4-b]pyridin-6-yl)-4-oxobutanoate (0.010 g, 15.8812 μmol) in Tetrahydrofuran (4 mL) and Water (1 mL) at 25°C. The reaction mixture was stirred at 25°C for 2 h. The reaction mixture was evaporated under reduced pressure. The residue was concentrated and diluted with water (2 mL). The resulting mixture was adjusted to pH=7 with HCl (1 M) at 25°C and evaporated under reduced pressure. The

residue was purified by HPLC. The pure fractions was concentrated and lyophilization. There was 4-(5-(3-((6-(3-carboxypropanoyl)-3-methoxy-6,7-dihydro-5H-pyrrolo[3,4-b]pyridin-2-yl)oxy)propoxy)-6-me thoxythieno[3,2-b]pyridin-2-yl)-4-oxobutanoic acid (2.6 mg, 4.4248 μmol, 27.8619% yield) obtained as a white solid. LCMS: (ESI, m/z): $[M+H]^+$ = 588.157. $^1H$ NMR (400 MHz, DMSO-$d_6$) δ 8.20 (s, 1H), 7.97 (d, *J* = 3.4 Hz, 1H), 7.35 - 7.28 (m, 1H), 4.73 (s, 2H), 4.61 (s, 2H), 4.51 (s, 4H), 4.47 - 4.33 (m, 4H), 3.88 (s, 3H), 3.78 (s, 3H), 2.71 - 2.61 (m, 4H), 2.26 (s, 2H).

**EXAMPLE 12**

**4-(5-(3-((2-(4-ethoxy-4-oxobutanoyl)-6-methoxybenzo[b]thiophen-5-yl)oxy)propoxy)-6-methoxyisoindo lin-2-yl)-4-oxobutanoic acid**

***Step a: tert-butyl 5-methoxyisoindoline-2-carboxylate***

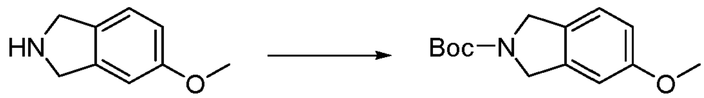

TEA (33.12 g, 327.3070 mmol) was added to a solution of 5-Methoxyisoindoline hydrochloride (20.01 g, 107.7833 mmol) and Di-tert-butyl dicarbonate (35.28 g, 161.6523 mmol) in DCM (500 mL) at 20°C. The reaction mixture was stirred overnight at 20°C. The reaction mixture was evaporated under reduced pressure. The residue was purified on silica gel column EA/n-Hexane (0-50%). The pure fractions was concentrated and dried under vacuo. There was tert-butyl *5-methoxyisoindoline-2-carboxylate* (30.66 g, 104.5347 mmol, 96.9859% yield) obtained as a white solid. LCMS: (ESI, m/z): $[M+H-tBu+ACN]^+$ = 235.136. $^1H$ NMR (400 MHz, DMSO-$d_6$) δ 7.24 - 7.18 (m, 1H), 6.90 (d, *J* = 4.5 Hz, 1H), 6.85 (s, 0.5H), 6.83 (s, 0.5H), 4.58 - 4.44 (m, 4H), 3.74 (s, 3H), 1.45 (s, 9H).

***Step b: tert-butyl 5-bromo-6-methoxy-isoindoline-2-carboxylate***

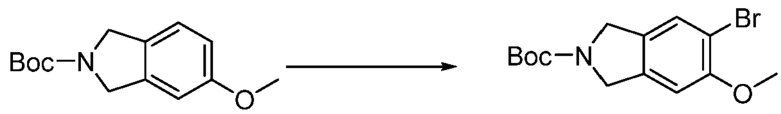

NBS (43.64 g, 245.1906 mmol) was added to a solution of tert-butyl 5-methoxyisoindoline-2-carboxylate (30.40 g, 121.9390 mmol) in Tetrahydrofuran (300 mL) and Acetonitrile (300 mL) at 20°C. The reaction mixture was stirred overnight at 20°C. The reaction mixture was evaporated under reduced pressure. The reaction mixture was concentrated and diluted with EA (1000 mL), washed with $NaHCO_3$ (aq)(3 x 200 mL) and brine (300 mL). The organics dried over $Na_2SO_4$, filtered and evaporated under reduced pressure. The residue was purified on silica gel column EA/n-Hexane (0-50%). The pure fractions was concentrated and dried under vacuo. There was tert-butyl 5-bromo-6-methoxy-isoindoline-2-carboxylate (17.61 g, 53.6562 mmol, 44.0025% yield) obtained as a yellow solid. LCMS: (ESI, m/z): $[M+H-tBu+ACN]^+$ = 313.047. $^1H$ NMR (400 MHz, DMSO-$d_6$) δ 7.54 (d, *J* = 5.3 Hz, 1H), 7.11 (d, *J* = 4.2 Hz, 1H), 4.51 (t, *J* = 9.8 Hz, 4H), 3.82 (d, *J* = 3.7 Hz, 3H), 1.45 (s, 9H).

***Step c: tert-butyl 5-methoxy-6-(4,4,5,5-tetramethyl-1,3,2-dioxaborolan-2-yl)isoindoline-2-carboxylate***

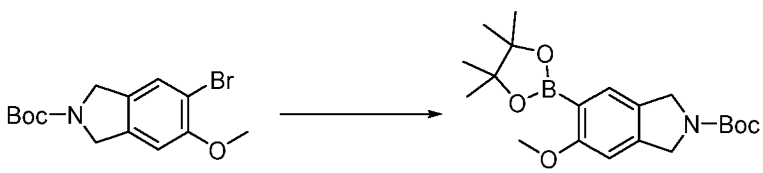

[1,1'-Bis(diphenylphosphino)ferrocene]dichloropalladium(II) (2.10 g, 2.8700 mmol), 4,4,4',4',5,5,5',5'-Octamethyl-2,2'-bi(1,3,2-dioxaborolane) (11.71 g,46.1137 mmol) and Potassium Acetate (9.76 g, 99.4475 mmol) was added

to a solution of tert-butyl 5-bromo-6-methoxy-isoindoline-2-carboxylate (10.04 g, 30.5910 mmol) in 1,4-Dioxane (200 mL) at 20°C. The reaction mixture was heated to 100°C and stirred overnight under $N_2$ atmosphere. The reaction mixture was concentrated and diluted with EA (500 mL), washed with water (200 mL) and brine (200 mL). The organics dried over $Na_2SO_4$, filtered and evaporated under reduced pressure. The residue was purified on silica gel column EA/n-Hexane (0-50%). The pure fractions was concentrated and dried under vacuo. There was tert-butyl 5-methoxy-6-(4,4,5,5-tetramethyl-1,3,2-dioxaborolan-2-yl)isoindoline-2-carboxylate (11.00 g, 29.3126 mmol, 95.8208% yield) obtained as a oil. LCMS: (ESI, m/z): [M+H-tBu+ACN] $^+$ = 361.222.

***Step d: tert-butyl 5-hydroxy-6-methoxy-isoindoline-2-carboxylate***

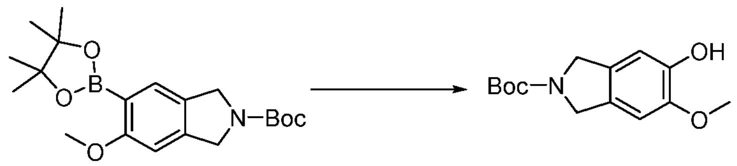

Sodium perborate tetrahydrate (6.28 g, 40.8164 mmol) was added to a solution of tert-butyl 5-methoxy-6-(4,4,5,5-tetramethyl-1,3,2-dioxaborolan-2-yl)isoindoline-2-carboxylate (9.75 g, 25.9816 mmol) in Tetrahydrofura (150 mL) and Water (150 mL) at 20°C. The reaction mixture was stirred for 2 h at 20°C. The reaction mixture was concentrated and diluted with EA (600 mL), washed with water (300 mL) and brine (300 mL). The organics dried over $Na_2SO_4$, filtered and evaporated under reduced pressure. The residue was purified on silica gel column EA/DCM (0-100%). The pure fractions was concentrated and dried under vacuo. There was tert-butyl 5-hydroxy-6-methoxy-isoindoline-2-carboxylate (6.58 g, 24.8017 mmol, 95.4589% yield) obtained as a yellow oil. LCMS: (ESI, m/z): [M+H-tBu+ACN] $^+$ = 251.131. $^1$H NMR (400 MHz, DMSO-*d*$_6$) $\delta$ 8.92 (s, 1H), 6.88 (d, *J* = 5.8 Hz, 1H), 6.70 (d, *J* = 2.3 Hz, 1H), 4.44 (t, *J* = 9.6 Hz, 4H), 3.74 (d, *J* = 3.4 Hz, 3H), 1.44 (s, 9H).

***Step e: tert-butyl 5-(3-bromopropoxy)-6-methoxy-isoindoline-2-carboxylate***

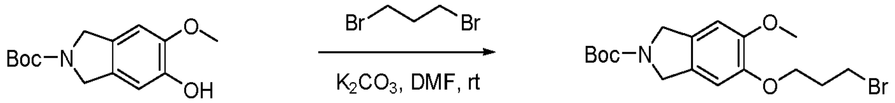


1,3-Dibromopropane (0.564 g, 2.7936 mmol) was added to a mixture of Potassium carbonate (0.286 g, 2.0694 mmol) and tert-butyl 5-hydroxy-6-methoxyisoindoline-2-carboxylate (0.179 g, 674.6977 μmol) in DMF (3 mL) at 20°C under $N_2$ atmosphere. The reaction mixture was stirred for 5 h at 20°C. The mixture was purified on C18 column ACN/$H_2O$ (0.1%FA)(0-50%). The pure fractions was concentrated and dried under vacuo. There was tert-butyl 5-(3-bromopropoxy)-6-methoxy-isoindoline-2-carboxylate (0.154 g, 398.6747 μmol, 59.0894% yield) obtained as a white oil. LCMS: (ESI, m/z): [M+H] $^+$ = 386.090. $^1$H NMR (400 MHz, DMSO-*d*$_6$) $\delta$ 6.96 (t, *J* = 5.8 Hz, 2H), 4.50 (s, 2H), 4.48 (s, 2H), 4.03 (q, *J* = 5.5 Hz, 2H), 3.74 (d, *J* = 3.4 Hz, 3H), 3.66 (t, *J* = 6.5 Hz, 2H), 2.27 - 2.19 (m, 2H), 1.45 (s, 9H).

***Step f: tert-butyl 5-(3-((2-(4-ethoxy-4-oxobutanoyl)-6-methoxybenzo[b]thiophen-5-yl)oxy)propoxy)-6-methoxyisoindoline-2 -carboxylate***

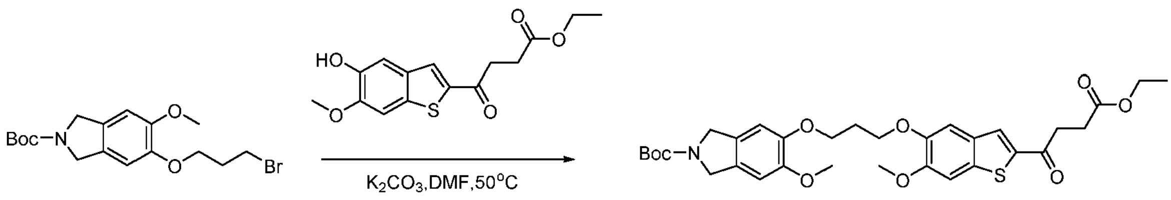


Potassium carbonate (0.079 g, 571.6126 μmol) was added to a solution of tert-butyl 5-(3-bromopropoxy)-6-methoxyisoindoline-2-carboxylate (0.079 g, 204.5150 μmol) and ethyl 4-(5-hydroxy-6-methoxybenzo[b]thiophen-2-yl)-4-oxobutanoate (0.064 g, 207.5571 μmol) in DMF (3 mL) at 20°C under $N_2$ atmosphere. The reaction mixture was stirred overnight at 50°C. The reaction mixture was quenched with adding of water (50 mL) at 20°C, extracted with EA (3 x 50 mL) and washed with brine (50 mL). The organics dried over $Na_2SO_4$, filtered and evaporated under reduced pressure. There was a crude tert-butyl 5-(3-((2-(4-ethoxy-4-oxobutanoyl)-6-methoxybenzo[b]thiophen-5-yl)oxy)propoxy)-6-methoxyisoindoline-2-carboxylate (0.088 g, 143.3886 μmol, 70.1115% yield) obtained as a yellow oil which was used directly in the next step. LCMS: (ESI, m/z): [M+H] $^+$ = 614.230.

***Step g: 4-(5-(3-((2-(4-ethoxy-4-oxobutanoyl)-6-methoxybenzo[b]thiophen-5-yl)oxy)propoxy)-6-methoxyisoindolin -2-yl)-4-oxobutanoic acid***

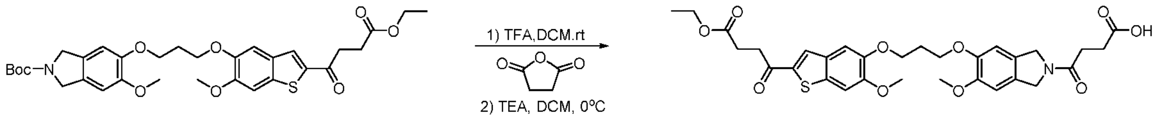


Trifluoroacetic acid (1 mL) was added to a solution of tert-butyl 5-(3-((2-(4-ethoxy-4-oxobutanoyl)-6-methoxybenzo[b]thiophen-5-yl)oxy)propoxy)-6-methoxyisoindoline-2-carboxylate (0.084 g, 136.8709 μmol) in DCM (3mL) at 20°C. The reaction mixture was stirred for 1 h at 20°C. The reaction mixture was evaporated under reduced pressure. Dihydrofuran-2,5-dione (0.050 g, 499.6373 μmol) and TEA (0.195 g, 1.9271 mmol) was added to a solution of the residue in DCM (5 mL) at 0°C under $N_2$ atmosphere. The reaction mixture was stirred and warmed up to 20°C naturally. The reaction mixture was quenched with adding of water (50 mL) at 20°C and adjusted to pH=3 with HCl (1 mol/L), extracted with EA (3 x 50 mL) and washed with brine (50 mL). The organics dried over $Na_2SO_4$, filtered and evaporated under reduced pressure. The residue was purified on C18 column ACN/$H_2O$ (0.1%FA)(0-40%). The pure fractions was concentrated and dried under vacuo. There was 4-(5-(3-((2-(4-ethoxy-4-oxobutanoyl)-6-methoxybenzo[b]thiophen-5-yl)oxy)propoxy)-6-methoxyisoindolin-2-yl)-4-oxobutanoic acid (0.038 g, 61.9221 μmol, 45.2413% yield) obtained as a white solid. LCMS: (ESI, m/z): [M+H] $^+$ = 614.200. $^1$H NMR (400 MHz, DMSO-$d_6$) δ 8.18 (s, 1H), 7.60 (s, 1H), 7.53 (s, 1H), 7.02 (s, 0.5H), 6.98 (s, 0.5H), 6.97 (s, 0.5H), 6.95 (s, 0.5H), 4.74 (s, 1H), 4.72 (s, 1H), 4.52 (s, 2H), 4.20 (t, *J* = 6.3 Hz, 2H), 4.14 (t, *J* = 6.3 Hz, 2H), 4.06 (q, *J* = 7.1 Hz, 2H), 3.86 (s, 3H), 3.75 (s, 3H), 3.30 - 3.28 (m, 2H), 2.70 - 2.64 (m, 2H), 2.58 - 2.52 (m, 4H), 2.27 - 2.18 (m, 2H), 1.17 (t, *J* = 7.1 Hz, 3H).

**EXAMPLE 13 (not part of the invention)**

**sodium 4-(5-(3-((2-(3-carboxylatopropanoyl)-5-methoxythieno[2,3-b]pyridin-6-yl)oxy)propoxy)-6-methoxyisoi ndolin-2-yl)-4-oxobutanoate**

***Step a: 2,3-dimethoxy-5-vinyl-pyridine***

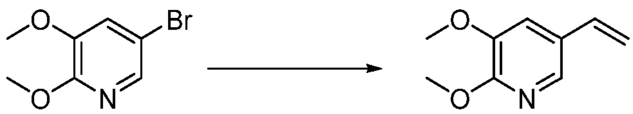

2-ethenyl-4,4,5,5-tetramethyl-1,3,2-dioxaborolane (9.25 g, 60.0595 mmol), Potassium carbonate (18.91 g, 136.8252 mmol) and Pd(dppf)$Cl_2$ (1.97 g, 2.6923 mmol) was added to a solution of 5-Bromo-2,3-dimethoxypyridine (9.88 g, 45.3112 mmol) in 1,4-Dioxane (100 mL) and Water (20 mL) at 20°C. The reaction mixture was stirred at 80°C for 2 h under nitrogen atmosphere. The reaction mixture was concentrated and diluted with EA (100 mL), washed with $NaHCO_3$ (aq)(100 mL) and brine (50 mL). The organics dried over $Na_2SO_4$, filtered and evaporated under reduced pressure. The residue was purified on silica gel column Ethyl acetate/Heptane (0-20%). The pure fractions was concentrated and dried under vacuo. There was 2,3-dimethoxy-5-vinyl-pyridine (5.22 g, 31.6002 mmol, 69.7404% yield) obtained as a colorless oil. LCMS: (ESI, m/z): [M+H] $^+$ = 166.079. $^1$H NMR (400 MHz, DMSO-$d_6$) δ 7.70 (d, *J* = 1.9 Hz, 1H), 7.46 (t, *J* = 5.8 Hz, 1H), 6.73 - 6.63 (m, 1H), 5.83 (dd, *J* = 17.6, 0.8 Hz, 1H), 5.24 (dd, *J* = 11.0, 0.8 Hz, 1H), 3.86 (s, 3H), 3.82 (s, 3H).

***Step b: 5,6-dimethoxypyridine-3-carbaldehyde***

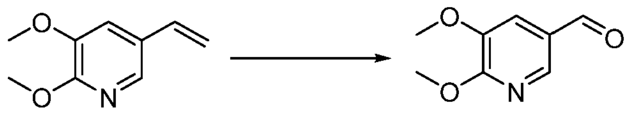

Potassium osmate(VI) dehydrate (0.55 g, 1.4927 mmol) in Water (25 mL) was added to a solution of 2,3-dimethoxy-5-vinyl-pyridine (5.17 g, 31.2975 mmol) and 4-Methylmorpholine N-oxide (7.44 g, 63.5105 mmol) in Tetrahydrofuran (100 mL) at 20°C. The reaction mixture was stirred at 20°C for 30 min. Sodium periodate (6.82 g, 31.8854 mmol) was added to the mixture at 20°C. The reaction mixture was stirred overnight at 20°C. The reaction mixture was concentrated and diluted with EA (500 mL), washed with water (200 mL) and brine (50 mL). The organics dried over $Na_2SO_4$, filtered and evaporated under reduced pressure. The residue was purified on silica gel column EA/Hept (0-20%). The pure fractions was concentrated and dried under vacuo. There was 5,6-dimethoxypyridine-3-carbaldehyde (3.51 g, 20.9976 mmol, 67.0904% yield) obtained as a white solid. LCMS: (ESI, m/z): $[M+H]^+$ = 168.058. $^1H$ NMR (400 MHz, DMSO-$d_6$) $\delta$ 9.95 (s, 1H), 8.34 (d, *J* = 1.9 Hz, 1H), 7.53 (d, *J* = 1.8 Hz, 1H), 3.99 (s, 3H), 3.87 (s, 3H).

***Step c: (5,6-dimethoxy-3-pyridyl)methanol***

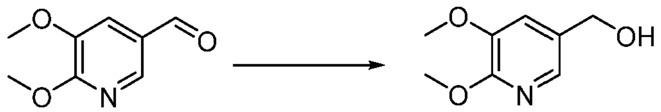

NaBH4 (0.4 g, 10.5729 mmol) was added to a solution of 5,6-dimethoxypyridine-3-carbaldehyde (3.51 g, 20.9976 mmol) in methanol (100 mL) at 20°C. The reaction mixture was stirred at 20°C for 1 h. The reaction mixture was concentrated and diluted with EA (500 mL), washed with water (200 mL) and brine (200 mL). The organics dried over $Na_2SO_4$, filtered and evaporated under reduced pressure. The pure fractions was concentrated and dried under vacuo. There was (5,6-dimethoxy-3-pyridyl)methanol (3.33 g, 19.6835 mmol, 93.7418% yield) obtained as a yellow oil. LCMS: (ESI, m/z): $[M+H]^+$ = 170.074. $^1H$ NMR (400 MHz, DMSO-$d_6$) $\delta$ 7.61 (d, *J* = 1.6 Hz, 1H), 7.23 (t, *J* = 6.4 Hz, 1H), 5.13 (t, *J* = 5.7 Hz, 1H), 4.43 (d, *J* = 5.7 Hz, 2H), 3.84 (s, 3H), 3.77 (s, 3H).

***Step d: (2-bromo-5,6-dimethoxy-3-pyridyl)methanol***

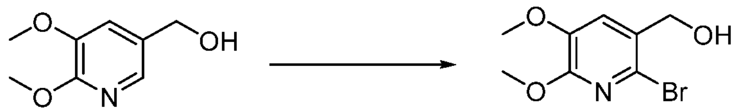

NBS (4.26 g, 23.9347 mmol) and Acetic acid (0.5 mL) was added to a solution of (5,6-dimethoxy-3-pyridyl) methanol (3.30 g, 19.5062 mmol) in Acetonitrile (100 mL) at 20°C. The reaction mixture was stirred at 20°C for 2 h. The reaction mixture was evaporated under reduced pressure. The resdue was diluted with EA (200 mL), washed with $NaHCO_3$ (aq)(2 x 100 mL) and brine (100 mL). The organics dried over $Na_2SO_4$, filtered and evaporated under reduced pressure. The residue was purified on silica gel column EA/Hept (0-100%). The pure fractions was concentrated and dried under vacuo. There was (2-bromo-5,6-dimethoxy-3-pyridyl)methanol (2.91 g, 11.7304 mmol, 60.1369% yield) obtained as a white solid. LCMS: (ESI, m/z): $[M+H]^+$ = 247.984. $^1H$ NMR (400 MHz, DMSO-$d_6$) $\delta$ 7.40 (s, 1H), 5.45 (t, *J* = 5.4 Hz, 1H), 4.42 (d, *J* = 5.5 Hz, 2H), 3.85 (s, 3H), 3.80 (s, 3H).

***Step e: 2-bromo-5,6-dimethoxy-pyridine-3-carbaldehyde***

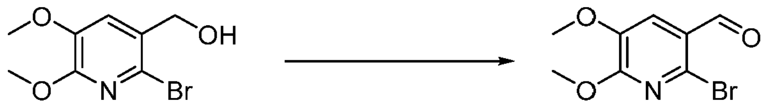

Dess-Martin periodinane (7.3 g, 17.2113 mmol) was added to a solution of (2-bromo-5,6-dimethoxy-3-pyridyl) methanol (2.91 g,11.7304 mmol) in DCM (100 mL) at 20°C. The reaction mixture was stirred at 20°C for 1 h. The reaction mixture was evaporated under reduced pressure. The reaction mixture was concentrated and diluted with EA (200 mL), washed with $NaHCO_3$ (aq)(2 x 100 mL) and brine (100 mL). The organics dried over $Na_2SO_4$, filtered and evaporated under reduced pressure. The residue was purified on silica gel column EA/Hept (0-100%). The pure fractions was concentrated and dried under vacuo. There was 2-bromo-5,6-dimethoxy-pyridine-3-carbaldehyde (2.7 g, 10.9730 mmol, 93.5437% yield) obtained as a white solid. LCMS: (ESI, m/z): $[M+H]^+$ = 245.969. $^1$H NMR (400 MHz, DMSO-$d_6$) $\delta$ 10.04 (s, 1H), 7.52 (s, 1H), 3.99 (s, 3H), 3.87 (s, 3H).

***Step f: 5,6-dimethoxythieno[2,3-b]pyridine-2-carboxylic acid***

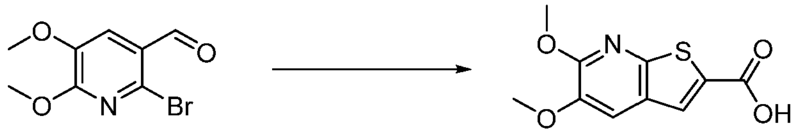

Methyl thioglycolate (1.1870 g, 11.1830 mmol) was added to a mixture of 2-bromo-5,6-dimethoxy-pyridine-3-carbaldehyde (2.68 g, 10.8918 mmol) and Potassium carbonate (4.65 g, 33.6455 mmol) in *N*,*N*-Dimethylformamide (50 mL) at 20°C. The reaction mixture was stirred overnight at 60°C under $N_2$ atmosphere. The reaction mixture was concentrated and diluted with water (500 mL). The precipitate was collected by filtration, washed with water (100 mL). LiOH (0.40 g, 16.7026 mmol) was added to a solution of the filtrate cake in Tetrahydrofuran (50 mL) and Water (10 mL) at 20°C. The reaction mixture was stirred at 20°C for 2 h. The reaction mixture was concentrated and diluted with water (200 mL). The mixture was adjusted to pH=3 with HCl (1 M). The precipitate was collected by filtration, washed with water (100 mL). The pure fractions was dried under vacuo. There was 5,6-dimethoxythieno[2,3-b]pyridine-2-carboxylic acid (2.32 g, 9.6971 mmol, 89.0313% yield) obtained as a white solid. LCMS: (ESI, m/z): $[M+H]^+$ = 240.065. $^1$H NMR (400 MHz, DMSO-$d_6$) $\delta$ 7.91 (s, 1H), 7.80 (s, 1H), 3.98 (s, 3H), 3.85 (s, 3H).

***Step g: tert-butyl 3-(5,6-dimethoxythieno[2,3-b]pyridin-2-yl)-3-oxo-propanoate***

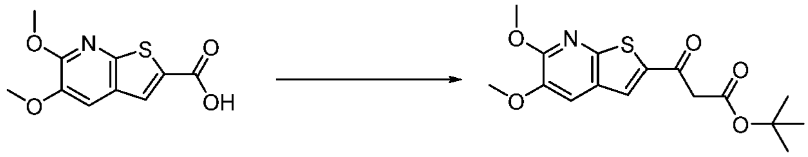

Carbonyl diimidazole (3.11 g, 19.1799 mmol) was added to a solution of 5,6-dimethoxythieno[2,3-b]pyridine-2-carboxylic acid (2.29 g, 9.5717 mmol) in *N*,*N*-Dimethylformamide (50 mL) at 20°C. The reaction mixture was stirred for 30 min at 20°C under nitrogen atmosphere. 3-tert-Butoxy-3-oxopropanoic acid (3.16 g, 19.7294 mmol), Magnesium chloride (1.87 g, 19.6406 mmol) and Triethylamine (2.96 g, 29.2521 mmol) were added to the mixture at 20°C. The reaction mixture was stirred overnight at 20°C. The reaction mixture was concentrated and diluted with EA (500 mL), washed with $NaHCO_3$ (aq)(2 x 300 mL) and brine (200 mL). The organics dried over $Na_2SO_4$, filtered and evaporated under reduced pressure. The residue was purified on silica gel column EA/n-Hexane (0-50%). The pure fractions was concentrated and dried under vacuo. There was tert-butyl 3-(5,6-dimethoxythieno[2,3-b]pyridin-2-yl)-3-oxo-propanoate (2.37 g, 7.0245 mmol, 73.3885% yield) obtained as a white solid. LCMS: (ESI, m/z): $[M+H]^+$ = 338.098. $^1$H NMR (400 MHz, DMSO-$d_6$) $\delta$ 8.15 (d, *J* = 9.8 Hz, 1H), 7.83 (d, *J* = 7.7 Hz, 1H), 4.06 (d, *J* = 8.5 Hz, 2H), 3.98 (s, 3H), 3.88 (s, 3H), 1.41 (s, 9H).

***Step h: O1-tert-butyl O4-ethyl 2-(5,6-dimethoxythieno[2,3-b]pyridine-2-carbonyl)butanedioate***

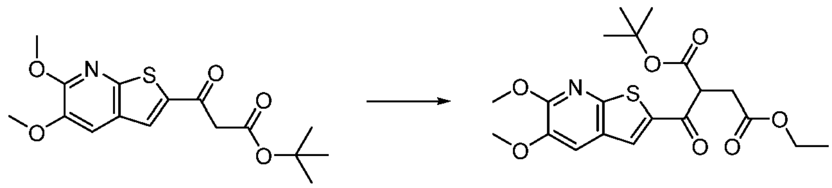

Ethyl bromoacetate (0.63 g, 3.7724 mmol) was added to a mixture of tert-butyl 3-(5,6-dimethoxythieno[2,3-b] pyridin-2-yl)-3-oxo-propanoate (1.2 g, 3.5567 mmol) and Potassium carbonate (0.80 g, 5.7885 mmol) in N,N-Dimethylformamide (20 mL) at 20°C. The reaction mixture was stirred for 6 h at 20°C. The reaction mixture was concentrated and diluted with EA (200 mL), washed with $NaHCO_3$ (aq)(200 mL) and brine (200 mL). The organics dried over $Na_2SO_4$, filtered and evaporated under reduced pressure. The residue was purified on silica gel column EA/n-Hexane (0-50%). The pure fractions was concentrated and dried under vacuo. There was O1-tert-butyl O4-ethyl 2-(5,6-dimethoxythieno[2,3-b] pyridine-2-carbonyl)butanedioate (1.72 g, 4.0616 mmol, 114.1951% yield) obtained as a yellow oil. LCMS: (ESI, m/z): $[M+H]^+$ = 424.135. $^1H$ NMR (400 MHz, DMSO-$d_6$) $\delta$ 8.31 (s, 1H), 7.86 (s, 1H), 4.82 (t, *J* = 7.3 Hz, 1H), 4.05 (m, 2H), 4.00 (s, 3H), 3.87 (s, 3H), 2.92 (d, *J* = 7.2 Hz, 2H), 1.32 (s, 9H), 1.18 - 1.06 (m, 3H).

***Step i: ethyl 4-(5,6-dimethoxythieno[2,3-b]pyridin-2-yl)-4-oxo-butanoate***

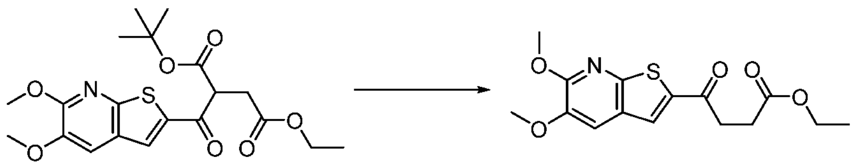

Trifluoroacetic acid (2 mL) was added to a solution of O1-tert-butyl O4-ethyl 2-(5,6-dimethoxythieno[2,3-b] pyridine-2-carbonyl)butanedioate (1.51 g, 3.5657 mmol) in Toluene (10 mL) at 20°C. The reaction mixture was stirred for 1 h at 50°C. The reaction mixture was evaporated under reduced pressure. The residue was purified on C-18 column MeCN/water (0-100%). The pure fractions was concentrated and dried under vacuo. There was ethyl 4-(5,6-dimethoxythieno[2,3-b]pyridin-2-yl)-4-oxo-butanoate (0.94 g, 2.9069 mmol, 81.5252% yield) obtained as a white solid. LCMS: (ESI, m/z): $[M+H]^+$ = 324.083. $^1H$ NMR (400 MHz, DMSO-$d_6$) $\delta$ 8.20 (s, 1H), 7.79 (s, 1H), 4.00-4.08 (m, 2H), 3.99 (s, 3H), 3.87 (s, 3H), 3.30-3.33 (m, 2H), 2.67 (t, *J*= 6.1 Hz, 2H), 1.18 (t, *J* = 7.0 Hz, 3H).

***Step j: ethyl 4-(6-chloro-5-methoxy-thieno[2,3-b]pyridin-2-yl)-4-oxo-butanoate***

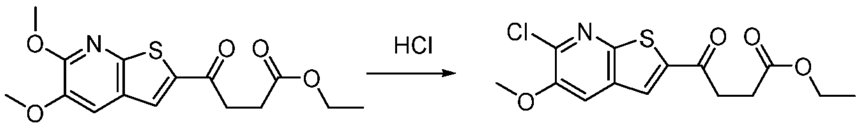


Ethyl 4-(5,6-dimethoxythieno[2,3-b]pyridin-2-yl)-4-oxo-butanoate (0.308 g, 952.4899 μmol) was added to HCl (10 mL) at 20°C. The reaction mixture was stirred at 100°C for 1 h. Cooled the reaction mixture to 20°C, POCl3 (1 mL) was added to the reaction mixture. The reaction mixture was stirred at 100°C for 2 h under $N_2$ atmosphere. The reaction mixture was evaporated under reduced pressure. The residue was purified on C-18 column MeCN/water (0-100%). The pure fractions was concentrated and dried under vacuo. There was ethyl 4-(6-chloro-5-methoxy-thieno[2,3-b]pyridin-2-yl)-4-oxo-butanoate (0.25 g, 762.7004 μmol, 80.0744% yield) obtained as a white solid. LCMS: (ESI, m/z): $[M+H]^+$ = 328.033. $^1H$ NMR (400 MHz, DMSO-$d_6$) $\delta$ 8.31 (s, 1H), 8.14 (s, 1H), 4.00-4.08 (m 2H), 3.99 (s, 3H), 3.44 - 3.36 (m, 2H), 2.70 (t, *J* = 6.2 Hz, 2H), 1.18 (t, *J*= 7.1 Hz, 3H).

***Step k: ethyl 4-(5-(3-((2-(4-ethoxy-4-oxobutanoyl)-5-methoxythieno[2,3-b]pyridin-6-yl)oxy)propoxy)-6-methoxyisoindol in-2-yl)-4-oxobutanoate***

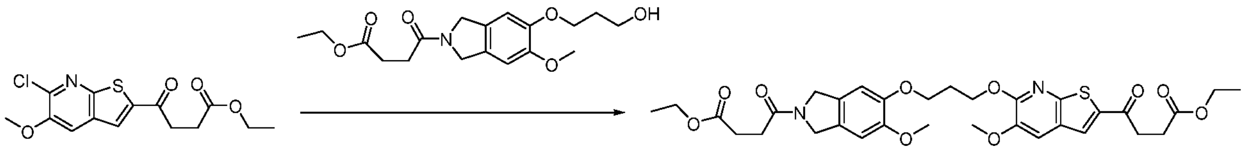

RockPhos Palladacycle Gen.3 (0.03 g, 35.7385 μmol) was added to a mixture of ethyl 4-(6-chloro-5-methoxythieno[2,3-b]pyridin-2-yl)-4-oxo-butanoate (0.10 g, 305.0799 μmol), ethyl 4-(5-(3-hydroxypropoxy)-6-methoxyisoindolin-2-yl)-4-oxobutanoate (0.18 g, 512.2470 μmol) and Potassium phosphate (0.20 g, 942.2127 μmol) in Toluene (10 mL) at 20°C. The reaction mixture was stirred overnight at 130°C under $N_2$ atmosphere. The reaction mixture was evaporated under reduced pressure. The residue was purified on C-18 column MeCN/water (0-100%). The pure fractions was concentrated and dried under vacuo. There was ethyl 4-(5-(3-((2-(4-ethoxy-4-oxobutanoyl)-5-methoxythieno[2,3-b] pyridin-6-yl)oxy)propoxy)-6-methoxyisoindoli n-2-yl)-4-oxobutanoate (0.013 g, 20.2267 μmol, 6.6300% yield) obtained as a yellow oil. LCMS: (ESI, m/z): $[M+H]^+$ = 643.225.

*Step I: sodium 4-(5-(3-((2-(3-carboxylatopropanoyl)-5-methoxythieno[2,3-b]pyridin-6-yl)oxy)propoxy)-6-methoxyisoindol in-2-yl)-4-oxobutanoate*

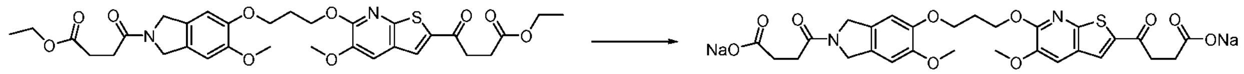

LiOH (3 mg, 125.2699 μmol) was added to a solution of ethyl 4-(5-(3-((2-(4-ethoxy-4-oxobutanoyl)-5-methoxythieno[2,3-b]pyridin-6-yl)oxy)propoxy)-6-methoxyisoindoli n-2-yl)-4-oxobutanoate (0.018 g, 28.0062 μmol) in Tetrahydrofuran (4 mL) and Water (1 mL) at 20°C. The reaction mixture was stirred at 20°C for 1 h. The reaction mixture was purified by Prep-HPLC. The pure fractions was concentrated and dried by lyophilization. $NaHCO_3$ (3.4 mg, 40.4730 μmol) was added to the lyophilized product in Water (5 mL), and stirred at 20°C for 1 h. The solvent was removed by lyophilization. There was sodium 4-(5-(3-((2-(3-carboxylatopropanoyl)-5-methoxythieno[2,3-b]pyridin-6-yl)oxy)propoxy)-6-methoxyisoindoli n-2-yl)-4-oxobutanoate (13 mg, 20.6162 μmol, 73.6129% yield) obtained as a white solid. LCMS: (ESI, m/z): $[M+H]^+$ = 587.162.

**EXAMPLE 14**

**4-(5-(3-((2-(3-carboxypropanoyl)-4-chloro-6-methoxyisoindolin-5-yl)oxy)propoxy)-6-methoxybenzo[b]t hiophen-2-yl)-4-oxobutanoic acid**

*Step a: ethyl 4-[5-(3-bromopropoxy)-6-methoxy-benzothiophen-2-yl]-4-oxo-butanoate*

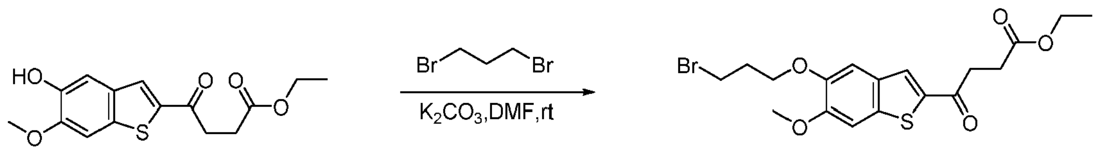


Potassium carbonate (0.113 g, 817.6230 μmol) was added to a solution of ethyl 4-(5-hydroxy-6-methoxybenzo [b]thiophen-2-yl)-4-oxobutanoate (0.081 g, 262.6894 μmol) and 1,3-dibromopropane (0.263 g, 1.3027 mmol) in DMF (5 mL) at 20°C under $N_2$ atmosphere. The reaction mixture was stirred overnight at 20°C. The mixture was purified on C18 column $ACN/H_2O$ (0-60%). The pure fractions was concentrated and dried under vacuo. There was ethyl 4-[5-(3-bromopropoxy)-6-methoxy-benzothiophen-2-yl]-4-oxo-butanoate (0.074 g, 172.3638 μmol, 65.6150% yield) obtained as a white solid. LCMS: (ESI, m/z): $[M+H]^+$ = 309.070. $^1H$ NMR (400 MHz, DMSO-$d_6$) $\delta$ 8.21 (s, 1H), 7.62 (s, 1H), 7.53 (s, 1H), 4.14 (t, $J$ = 6.0 Hz, 2H), 4.06 (q, $J$ = 7.1 Hz, 2H), 3.87 (s, 3H), 3.70 (t, $J$ = 6.5 Hz, 2H), 3.30 (t, $J$ = 6.5 Hz, 2H), 2.66 (t, $J$ = 6.4 Hz, 2H), 2.35 - 2.26 (m, 2H), 1.18 (t, $J$ = 7.1 Hz, 3H).

***Step b: ethyl 4-(5-(3-((4-chloro-2-(4-ethoxy-4-oxobutanoyl)-6-methoxyisoindolin-5-yl)oxy)propoxy)-6-methoxybenzo[b] thiophen-2-yl)-4-oxobutanoate***

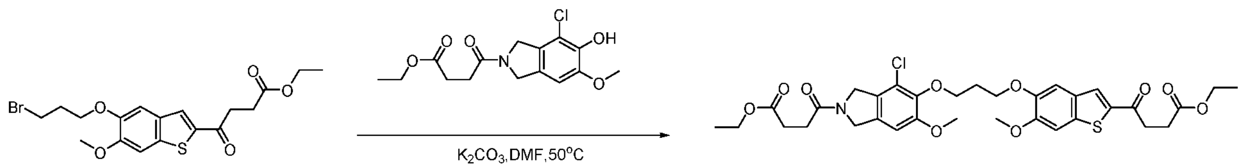


Potassium carbonate (0.094 g, 680.1466 μmol) was added to a solution of ethyl 4-[5-(3-bromopropoxy)-6-methoxy-benzothiophen-2-yl]-4-oxo-butanoate (0.092 g, 214.2904 μmol) and ethyl 4-(4-chloro-5-hydroxy-6-methoxyisoindolin-2-yl)-4-oxobutanoate (0.072 g, 219.6763 μmol) in DMF (3 mL) at 20°C under $N_2$ atmosphere. The reaction mixture was heated to 50°C and stirred overnight. The mixture was purified on C18 column ACN/$H_2O$ (0.1%FA)(0-60%). The pure fractions was concentrated and dried under vacuo. There was ethyl 4-(5-(3-((4-chloro-2-(4-ethoxy-4-oxobutanoyl)-6-methoxyisoindolin-5-yl)oxy)propoxy)-6-methoxybenzo[b]t hiophen-2-yl)-4-oxobutanoate (0.089 g, 131.6233 μmol, 61.4229% yield) obtained as a white solid. LCMS: (ESI, m/z): $[M+H]^+$ = 676.190. $^1H$ NMR (400 MHz, DMSO-$d_6$) $\delta$ 8.23 (s, 1H), 7.60 (s, 1H), 7.51 (d, $J$ = 4.3 Hz, 1H), 7.07 (s, 0.5H), 7.04 (s, 0.5H), 4.83 (s, 1H), 4.75 (s, 1H), 4.61 (s, 1H), 4.50 (s, 1H), 4.26 (s, 2H), 4.15 (t, $J$ = 6.0 Hz, 2H), 4.10 - 4.01 (m, 4H), 3.86 (s, 3H), 3.77 (s, 3H), 3.31 - 3.27 (m, 2H), 2.70 - 2.52 (m, 6H), 2.24 - 2.14 (m, 2H), 1.18 (t, $J$ = 7.1 Hz, 6H).

***Step c: 4-(5-(3-((2-(3-carboxypropanoyl)-4-chloro-6-methoxyisoindolin-5-yl)oxy)propoxy)-6-methoxybenzo[b] thio phen-2-yl)-4-oxobutanoic acid***

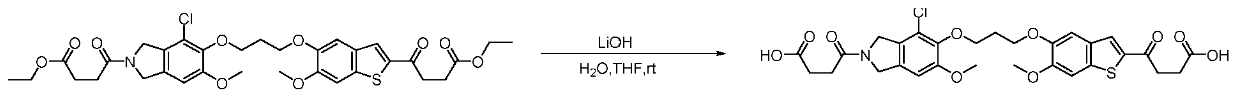


LiOH (0.051 g, 2.1296 mmol) was added to a solution of ethyl 4-(5-(3-((4-chloro-2-(4-ethoxy-4-oxobutanoyl)-6-methoxyisoindolin-5-yl)oxy)propoxy)-6-methoxybenzo[b]t hiophen-2-yl)-4-oxobutanoate (0.040 g, 59.1565 μmol) in THF (2 mL) and $H_2O$ (2 mL) at 20°C. The reaction mixture was stirred for 2 h at 20°C. The reaction mixture was adjusted to pH=3 with HCl (1 mol/L). The mixture was purified on C18 column ACN/$H_2O$ (0.1%FA)(0-45%). The pure fractions was concentrated and dried by lyophilization. There was 4-(5-(3-((2-(3-carboxypropanoyl)-4-chloro-6-methoxyisoindolin-5-yl)oxy)propoxy)-6-methoxybenzo[b]thio phen-2-yl)-4-oxobutanoic acid (0.025 g, 40.3183 μmol, 68.1552% yield) obtained as a white solid. LCMS: (ESI, m/z): $[M+H]^+$ = 620.130. $^1H$ NMR (400 MHz, DMSO-$d_6$) $\delta$ 8.21 (s, 1H), 7.59 (s, 1H), 7.50 (d, $J$ = 3.3 Hz, 1H), 7.06 (s, 0.5H), 7.03 (s, 0.5H), 4.82 (s, 1H), 4.74 (s, 1H), 4.61 (s, 1H), 4.50 (s, 1H), 4.31 - 4.22 (m, 2H), 4.15 (t, $J$ = 6.1 Hz, 2H), 3.86 (s, 3H), 3.77 (s, 3H), 3.26 - 3.24 (m, 2H), 2.61 - 2.55 (m, 4H), 2.54 - 2.44 (m, 2H), 2.19 (t, $J$ = 6.2 Hz, 2H).

**EXAMPLE 15 (not part of the invention)**

**4-(5-(3-((2-(3-carboxypropanoyl)-5-methoxythieno[3,2-b]pyridin-6-yl)oxy)propoxy)-6-methoxyisoindol in-2-yl)-4-oxobutanoic acid**

***Step a: ethyl 4-(5-(3-((2-(4-ethoxy-4-oxobutanoyl)-5-methoxythieno[3,2-b]pyridin-6-yl)oxy)propoxy)-6-methoxyisoindol in-2-yl)-4-oxobutanoate***

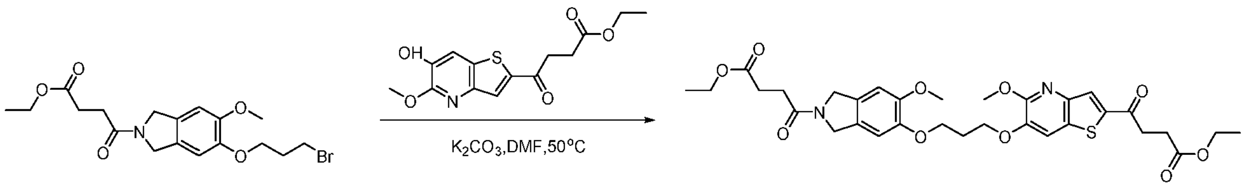


Potassium carbonate (0.089 g, 643.9686 μmol) was added to a solution of ethyl 4-(6-hydroxy-5-methoxythieno[3,2-b]pyridin-2-yl)-4-oxobutanoate (0.069 g, 223.0577 μmol) and ethyl 4-(5-(3-bromopropoxy)-6-methoxyisoindolin-2-yl)-4-oxobutanoate (0.079 g, 190.6877 μmol) in DMF (2 mL) at 20°C under $N_2$ atmosphere. The reaction mixture was heated to 50°C and stirred overnight. The reaction mixture was purified on C18 column ACN/$H_2O$ (0.1%FA)(0-55%). The pure fractions was concentrated and dried by lyophilization. There was ethyl 4-(5-(3-((2-(4-ethoxy-4-oxobutanoyl)-5-methoxythieno[3,2-b]pyridin-6-yl)oxy)propoxy)-6-methoxyisoindoli n-2-yl)-4-oxobutanoate (0.075 g, 116.6925 μmol, 61.1956% yield) obtained as a white solid. LCMS: (ESI, m/z): $[M+H]^+$ = 690.130. $^1H$ NMR (400 MHz, DMSO-$d_6$) $\delta$ 8.27 (s, 1H), 8.03 (s, 1H), 7.06 - 6.89 (m, 2H), 4.74 (s, 1H), 4.72 (s, 1H), 4.51 (s, 2H), 4.27 (t, *J* = 6.2 Hz, 2H), 4.12 (t, *J* = 6.7 Hz, 2H), 4.09 - 4.01 (m, 4H), 3.96 (s, 3H), 3.74 (d, *J* = 2.1 Hz, 3H), 3.40 - 3.34 (m, 2H), 2.68 - 2.54 (m, 6H), 2.29 - 2.19 (m, 2H), 1.18 (t, *J* = 7.1 Hz, 6H).

***Step b: 4-(5-(3-((2-(3-carboxypropanoyl)-5-methoxythieno[3,2-b]pyridin-6-yl)oxy)propoxy)-6-methoxyisoindolin-2-yl)-4-oxobutanoic acid***

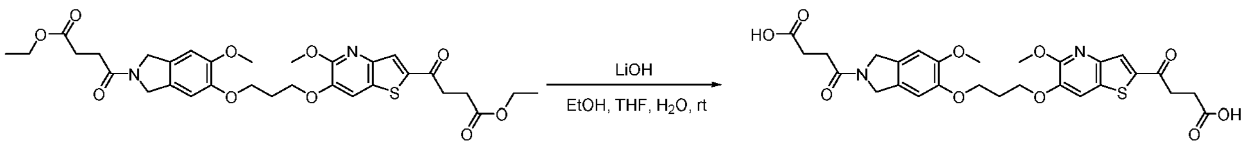


LiOH (0.050 g, 2.0878 mmol) was added to a solution of ethyl 4-(5-(3-((2-(4-ethoxy-4-oxobutanoyl)-5-methoxythieno[3,2-b]pyridin-6-yl)oxy)propoxy)-6-methoxyisoindoli n-2-yl)-4-oxobutanoate (0.051 g, 79.3509 μmol) in THF (2 mL), $H_2O$ (2 mL) and Ethanol (1 mL) at 20°C. The reaction mixture was stirred for 2 h at 20°C. The reaction mixture was adjusted to pH=3 with HCl (1 mol/L), The reaction mixture was purified on C18 column ACN/$H_2O$ (0.1%FA)(0-45%). The pure fractions was concentrated and dried by lyophilization. There was 4-(5-(3-((2-(3-carboxypropanoyl)-5-methoxythieno[3,2-b]pyridin-6-yl)oxy)propoxy)-6-methoxyisoindolin-2 -yl)-4-oxobutanoic acid (0.015 g, 25.5707 μmol, 32.2248% yield) obtained as a white solid. LCMS: (ESI, m/z): $[M+H]^+$ = 586.160. $^1H$ NMR (400 MHz, DMSO-$d_6$) $\delta$ 8.25 (s, 1H), 8.02 (s, 1H), 7.02 - 6.92 (m, 2H), 4.73 (s, 1H), 4.71 (s, 1H), 4.51 (s, 2H), 4.27 (t, *J* = 6.2 Hz, 2H), 4.12 (s, *J* = 4.0 Hz, 2H), 3.96 (s, 3H), 3.74 (s, 3H), 3.40 - 3.34 (m, 2H), 2.63 - 2.46 (m, 6H), 2.24 (t, *J* = 6.2 Hz, 2H).

**EXAMPLE 16 (not part of the invention)**

**4-(5-((2-(((2-(3-carboxypropanoyl)-4-chloro-6-methoxyisoindolin-5-yl)oxy)methyl)allyl)oxy)-4-fluoro-6 -methoxybenzo[b]thiophen-2-yl)-4-oxobutanoic acid**

***Step a: ethyl 4-[4-chloro-5-[2-(chloromethyl)allyloxy]-6-methoxy-isoindolin-2-yl]-4-oxo-butanoate***

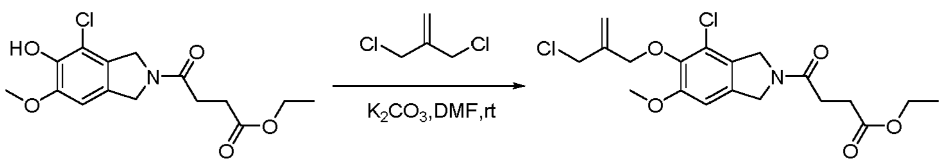


3-Chloro-2-(chloromethyl)prop-1-ene (0.507 g, 2.2635 mmol) was added to a mixture of Potassium carbonate (0.202 g, 1.4616 mmol) and ethyl 4-(4-chloro-5-hydroxy-6-methoxyisoindolin-2-yl)-4-oxobutanoate (0.154 g, 469.8574 μmol) in DMF (5 mL) at 20°C under $N_2$ atmosphere. The reaction mixture was stirred for 3 h at 20°C. The reaction mixture was purified on C18 column ACN/$H_2O$ (0.1%FA)(0-50%). The pure fractions was concentrated and dried under vacuo.

There was ethyl 4-[4-chloro-5-[2-(chloromethyl)allyloxy]-6-methoxy-isoindolin-2-yl]-4-oxo-butanoate (0.151 g, 362.7238 μmol, 77.1987% yield) obtained as a white solid. LCMS: (ESI, m/z): [M+H] + = 416.100.

***Step b: ethyl 4-(5-((2-(((4-chloro-2-(4-ethoxy-4-oxobutanoyl)-6-methoxyisoindolin-5-yl)oxy)methyl)allyl)oxy)-4-fluoro-6-methoxybenzo[b]thiophen-2-yl)-4-oxobutanoate***

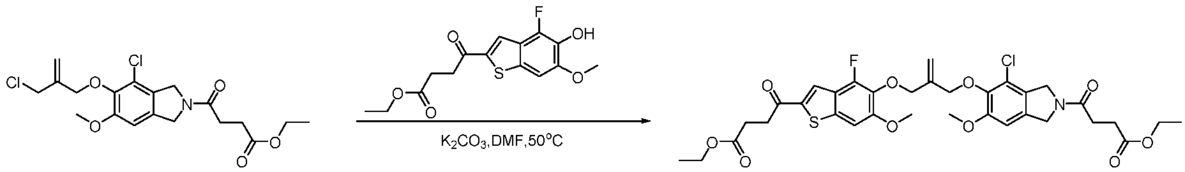


$K_2CO_3$ (0.092 g, 665.6754 μmol) was added to a mixture of ethyl 4-(4-chloro-5-((2-(chloromethyl)allyl)oxy)-6-methoxyisoindolin-2-yl)-4-oxobutanoate (0.068 g, 163.3458 μmol) and ethyl 4-(4-fluoro-5-hydroxy-6-methoxybenzo[b] thiophen-2-yl)-4-oxobutanoate (0.049 g, 150.1504 μmol) in DMF (3 mL) at 20°C under $N_2$ atmosphere. The reaction mixture was stirred overnight at 50°C. The reaction mixture was purified on C18 column ACN/$H_2O$ (0.1%FA)(0-60%). The pure fractions was concentrated and dried by lyophilization. There was ethyl 4-(5-((2-(((4-chloro-2-(4-ethoxy-4-oxobutanoyl)-6-methoxyisoindolin-5-yl)oxy)methyl)allyl)oxy)-4-fluoro-6 -methoxybenzo[b]thiophen-2-yl)-4-oxobutanoate (0.022 g, 31.1538 μmol, 19.0723% yield) obtained as a white solid. LCMS: (ESI, m/z): [M+H] + = 706.180. $^1H$ NMR (400 MHz, DMSO-$d_6$) δ 8.30 (s, 1H), 7.56 (s, 1H), 7.07 (s, 0.5H), 7.04 (s, 0.5H), 5.34 (d, *J* = 10.9 Hz, 2H), 4.83 (s, 1H), 4.77 (s, 2H), 4.70 (s, 1H), 4.66 (s, 2H), 4.61 (s, 1H), 4.47 (s, 1H), 4.06 (q, *J* = 7.1 Hz, 4H), 3.89 (s, 3H), 3.81 (s, 3H), 3.40 - 3.34 (m, 2H), 2.69 - 2.54 (m, 6H), 1.21 - 1.14 (m, 6H).

***Step c: 4-(5-((2-(((2-(3-carboxypropanoyl)-4-chloro-6-methoxyisoindolin-5-yl)oxy)methyl)allyl)oxy)-4-fluoro-6-m ethoxybenzo[b]thiophen-2-yl)-4-oxobutanoic acid***

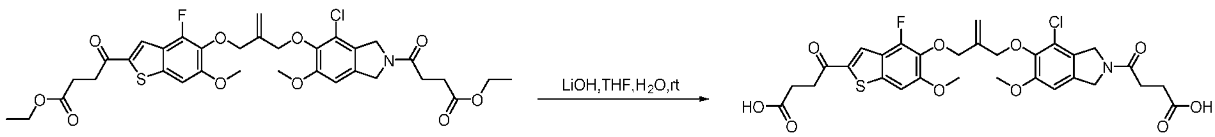


LiOH (0.037 g, 1.5450 mmol) was added to a solution of ethyl 4-(5-((2-(((4-chloro-2-(4-ethoxy-4-oxobutanoyl)-6-methoxyisoindolin-5-yl)oxy)methyl)allyl)oxy)-4-fluoro-6 -methoxybenzo[b]thiophen-2-yl)-4-oxobutanoate (0.012 g, 16.9930 μmol) in THF (1 mL) and $H_2O$ (1 mL) at 20°C. The reaction mixture was stirred for 1 h at 20°C. The reaction mixture was adjusted to pH=3 with HCl (1 mol/L). The reaction mixture was evaporated under reduced pressure. The residue was purified on C18 column ACN/$H_2O$ (0.1%FA)(0-50%). The pure fractions was concentrated and dried by lyophilization. There was 4-(5-((2-(((2-(3-carboxypropanoyl)-4-chloro-6-methoxyisoindolin-5-yl)oxy)methyl)allyl)oxy)-4-fluoro-6-me thoxybenzo[b]thiophen-2-yl)-4-oxobutanoic acid (0.011 g, 16.9213 μmol, 99.5782% yield) obtained as a white solid. LCMS: (ESI, m/z): [M-H] = 648.120. $^1H$ NMR (400 MHz, DMSO-$d_6$) δ 8.27 (d, *J* = 3.0 Hz, 1H), 7.56 (s, 1H), 7.06 (s, 0.5H), 7.03 (s, 0.5H), 5.34 (d, *J* = 10.8 Hz, 2H), 4.82 (s, 1H), 4.77 (d, *J* = 3.3 Hz, 2H), 4.68 (s, 1H), 4.66 (s, 2H), 4.61 (s, 1H), 4.46 (s, 1H), 3.89 (s, 3H), 3.81 (s, 3H), 3.32 - 3.26 (m, 2H), 2.61 - 2.53 (m, 4H), 2.48 - 2.44 (m, 2H).

**EXAMPLE 17**

**(S)-4-(5-(3-((2-((S)-3-carboxybutanoyl)-4-fluoro-6-methoxybenzo[b]thiophen-5-yl)oxy)propoxy)-4-fluo ro-6-methoxyisoindolin-2-yl)-2-methyl-4-oxobutanoic acid**

***Step a: 4-fluoro-6-methoxyisoindolin-5-ol hydrochloride***

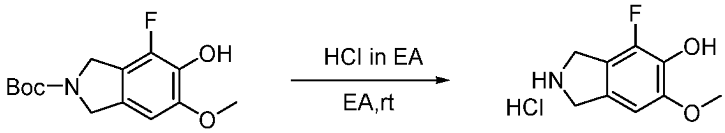


HCl in EA (3 mL, 12 mmol) was added to a solution of tert-butyl 4-fluoro-5-hydroxy-6-methoxyisoindoline-2-carboxylate (0.103 g, 363.5790 μmol) in EA (1 mL) at 20°C. The reaction mixture was stirred for 2 h at 20°C. The reaction mixture was evaporated under reduced pressure. There was 4-fluoro-6-methoxyisoindolin-5-ol hydrochloride (0.12 g, 655.0962 μmol, 180.1799% yield) obtained as a white solid, which was used directly to the next step. LCMS: (ESI, m/z): $[M+H]^+$ = 184.070.

***Step b: methyl (2S)-4-(4-fluoro-5-hydroxy-6-methoxy-isoindolin-2-yl)-2-methyl-4-oxo-butanoate***

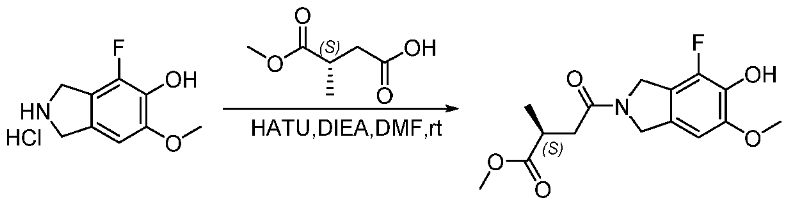


4-fluoro-6-methoxyisoindolin-5-ol hydrochloride (0.112 g, 611.4238 μmol) was added to a solution of (S)-4-methoxy-3-methyl-4-oxobutanoic acid (0.108 g, 739.0133 μmol), HATU (0.197 g, 518.1084 μmol) and DIEA (0.450 g, 3.4818 mmol) in DMF (10 mL) at 20°C under $N_2$ atmosphere. The reaction mixture was stirred for 2 h at 20°C. The reaction mixture was purified on C18 column ACN/$H_2O$ (0.1%FA)(0-55%). The pure fractions was concentrated and dried under vacuo. There was methyl (2S)-4-(4-fluoro-5-hydroxy-6-methoxy-isoindolin-2-yl)-2-methyl-4-oxo-butanoate (0.061 g, 195.9494 μmol, 32.0481% yield) obtained as a white solid. LCMS: (ESI, m/z): $[M+H]^+$ = 312.120.

***Step c: ethyl (2S)-4-[5-(3-bromopropoxy)-4-fluoro-6-methoxy-benzothiophen-2-yl]-2-methyl-4-oxo-butanoate***

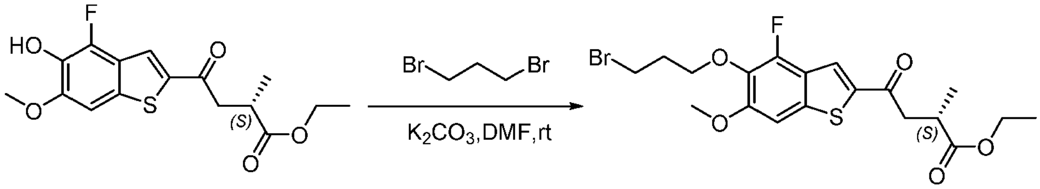


1,3-Dibromopropane (0.255 g, 1.2631 mmol) was added to a mixture of ethyl (S)-4-(4-fluoro-5-hydroxy-6-methoxybenzo[b]thiophen-2-yl)-2-methyl-4-oxobutanoate (0.061 g, 179.2189 μmol) and Potassium carbonate (0.129 g, 933.3927 μmol) in DMF (3 mL) at 20°C under $N_2$ atmosphere. The reaction mixture was stirred for 3 h at 20°C. The mixture was purified on C18 column ACN/$H_2O$ (0.1%FA)(0-70%). The pure fractions was concentrated and dried under vacuo. There was ethyl (2S)-4-[5-(3-bromopropoxy)-4-fluoro-6-methoxy-benzothiophen-2-yl]-2-methyl-4-oxo-butanoate (0.66 g, 1.4306 mmol, 798.2476% yield) obtained as a white solid. LCMS: (ESI, m/z): $[M+H]^+$ = 461.040.

***Step d: ethyl (S)-4-(4-fluoro-5-(3-((4-fluoro-6-methoxy-2-((S)-4-methoxy-3-methyl-4-oxobutanoyl)isoindolin-5-yl)oxy)p ropoxy)-6-methoxybenzo[b]thiophen-2-yl)-2-methyl-4-oxobutanoate***

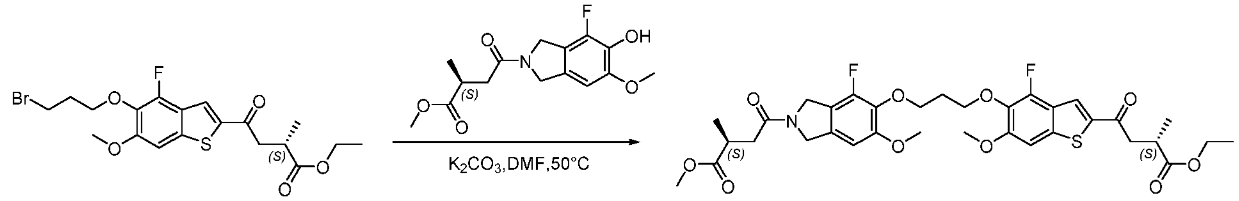


Potassium carbonate (0.074 g, 535.4346 μmol) was added to a solution of ethyl (2S)-4-[5-(3-bromopropoxy)-4-fluoro-6-methoxy-benzothiophen-2-yl]-2-methyl-4-oxo-butanoate (0.071 g, 153.8990 μmol) and methyl (2S)-4-(4-fluoro-5-hydroxy-6-methoxy-isoindolin-2-yl)-2-methyl-4-oxo-butanoate (0.056 g, 179.8880 μmol) in DMF (3 mL) at 20°C. The reaction mixture was stirred overnight at 50°C under $N_2$ atmosphere. The reaction mixture was purified on C18 column ACN/$H_2O$ (0.1%FA)(0-50%). The pure fractions was concentrated and dried under vacuo. There was ethyl (S)-4-(4-fluoro-5-(3-((4-fluoro-6-methoxy-2-((S)-4-methoxy-3-methyl-4-oxobutanoyl)isoindolin-5-yl)oxy)propoxy)-6-methoxybenzo[b]thiophen-2-yl)-2-methyl-4-oxobutanoate (61 mg, 88.1841 μmol, 57.3000% yield ) obtained as a white solid. LCMS: (ESI, m/z): $[M+H]^+$ = 692.230. $^1H$ NMR (400 MHz, DMSO-$d_6$) $\delta$ 8.31 (s, 1H), 7.56 (s, 1H), 6.90 (s, 0.5H), 6.85 (s, 0.5H), 4.77 (s, 2H), 4.56 (s, 1H), 4.54 (s, 1H), 4.25 (t, $J$ = 6.1 Hz, 2H), 4.19 (t, $J$ = 6.1 Hz, 2H), 4.05 (q, $J$ = 7.0 Hz, 2H), 3.88 (s, 3H), 3.77 (d, $J$ = 2.1 Hz, 3H), 3.59 (s, 3H), 3.55 - 3.43 (m, 1H), 3.26 - 3.15 (m, 1H), 3.00 - 2.90 (m, 1H), 2.90 - 2.79 (m, 1H), 2.76 - 2.66 (m, 1H), 2.50 - 2.44 (m, 1H), 2.09 - 1.99 (m, 2H), 1.21 - 1.10 (m, 9H).

***Step e: (S)-4-(5-(3-((2-((S)-3-carboxybutanoyl)-4-fluoro-6-methoxybenzo[b]thiophen-5-yl)oxy)propoxy)-4-fluoro-6-methoxyisoindolin-2-yl)-2-methyl-4-oxobutanoic acid***

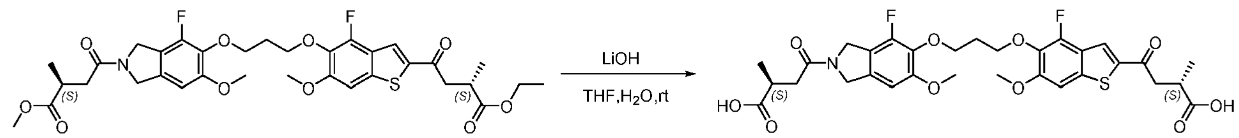


LiOH (0.038 g, 1.5868 mmol) was added to a mixture of ethyl (S)-4-(4-fluoro-5-(3-((4-fluoro-6-methoxy-2-((S)-4-methoxy-3-methyl-4-oxobutanoyl)isoindolin-5-yl)oxy)propoxy)-6-methoxybenzo[b]thiophen-2-yl)-2-methyl-4-oxobutanoate (0.038 g, 54.9344 μmol) in THF (2 mL) and $H_2O$ (2 mL) at 20°C. The reaction mixture was stirred for 2 h at 20°C. The reaction mixture was adjusted to pH=3 with HCl (1 mol/L). The reaction mixture was evaporated under reduced pressure. The residue was purified on C18 column ACN/$H_2O$ (0.1%FA)(0-45%). The pure fractions was concentrated and dried by lyophilization. There was (S)-4-(5-(3-((2-((S)-3-carboxybutanoyl)-4-fluoro-6-methoxybenzo[b]thiophen-5-yl)oxy)propoxy)-4-fluoro-6 -methoxyisoindolin-2-yl)-2-methyl-4-oxobutanoic acid (18 mg, 27.7070 μmol, 50.4365% yield) obtained as a white solid. LCMS: (ESI, m/z): $[M+H]^+$ = 650.18. $^1H$ NMR (400 MHz, DMSO-$d_6$) $\delta$ 12.15 (s, 2H), 8.30 (s, 1H), 7.56 (s, 1H), 6.90 (s, 0.5H), 6.85 (s, 0.5H), 4.76 (s, 2H), 4.56 (s, 1H), 4.54 (s, 1H), 4.26 (t, $J$ = 6.5 Hz, 2H), 4.19 (t, $J$ = 6.1 Hz, 2H), 3.88 (s, 3H), 3.77 (s, 3H), 3.53 - 3.44 (m, 1H), 3.17 - 3.05 (m, 1H), 2.93 - 2.83 (m, 1H), 2.79 - 2.61 (m, 2H), 2.45 - 2.35 (m, 1H), 2.11 - 1.98 (m, 2H), 1.22 - 1.10 (m, 6H).

**EXAMPLE 18**

**4-(5-(3-((2-((2-carboxyethyl)carbamoyl)-6-methoxybenzo[b]thiophen-5-yl)oxy)propoxy)-6-methoxyisoindolin-2-yl)-4-oxobutanoic acid**

***Step a: ethyl 3-[[6-methoxy-5-(methoxymethoxy)benzothiophene-2-carbonyl]amino]propanoate***

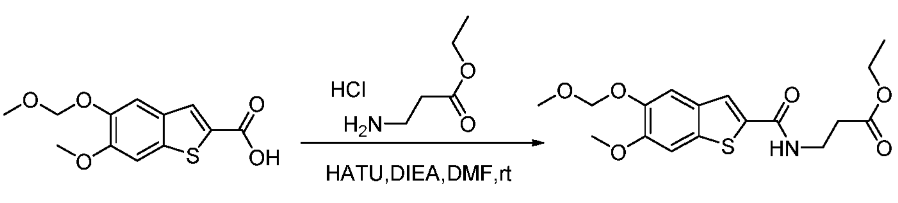

Ethyl 3-aminopropanoate hydrochloride (0.34 g, 2.2134 mmol) was added to a solution of HATU (0.85 g, 2.2355 mmol), DIEA (0.77 g, 5.9578 mmol) and 6-methoxy-5-(methoxymethoxy)benzo[b]thiophene-2-carboxylic acid (0.29 g, 1.0809 mmol) in DMF (15 mL) at 20°C. The reaction mixture was stirred for 2 h at 20°C. The mixture was purified on C18 column ACN/$H_2O$ (0.1%FA)(0-50%). The pure fractions was concentrated and dried under vacuo. There was ethyl 3-[[6-methoxy-5-(methoxymethoxy)benzothiophene-2-carbonyl]amino]propanoate (0.35 g, 952.5987 μmol, 88.1269% yield) obtained as a yellow oil. LCMS: (ESI, m/z): $[M+H]^+$ = 368.110.

***Step b: ethyl 3-[(5-hydroxy-6-methoxy-benzothiophene-2-carbonyl)amino]propanoate***

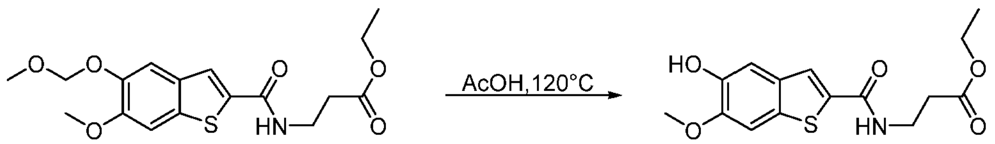


Ethyl 3-[[6-methoxy-5-(methoxymethoxy)benzothiophene-2-carbonyl]amino]propanoate (0.29 g, 789.2963 μmol) was added to Acetic acid (10 mL) at 20°C. The reaction mixture was heated to 120°C and stirred for 4 h. The mixture was purified on C18 column ACN/$H_2O$ (0.1%FA)(0-40%). The pure fractions was concentrated and dried under vacuo. There was ethyl 3-[(5-hydroxy-6-methoxy-benzothiophene-2-carbonyl)amino]propanoate (0.23 g, 711.2738 μmol, 90.1149% yield) obtained as a white solid. LCMS: (ESI, m/z): $[M+H]^+$ = 324.080.

***Step c: ethyl 4-[5-[3-[2-[(3-ethoxy-3-oxo-propyl)carbamoyl]-6-methoxy-benzothiophen-5-yl]oxypropoxy]-6-methoxy-iso indolin-2-yl]-4-oxo-butanoateethyl***

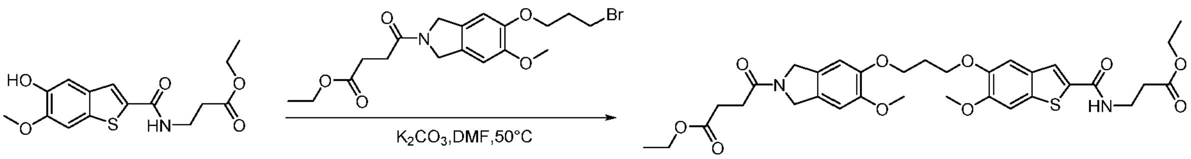


Potassium carbonate (0.25 g,1.8089 mmol) was added to a solution of ethyl 3-[(5-hydroxy-6-methoxy-benzothiophene-2-carbonyl)amino]propanoate (0.21 g, 649.4249 μmol) and ethyl 4-(5-(3-bromopropoxy)-6-methoxyisoindolin-2-yl)-4-oxobutanoate (0.21 g, 506.8913 μmol) in DMF (5 mL) at 20°C under $N_2$ atmosphere. The reaction mixture was heated to 50°C and stirred for 8 h. The reaction mixture was purified on C18 column ACN/ $H_2O$ (0.1%FA)(0-65%). The pure fractions was concentrated and dried under vacuo. There was ethyl 4-[5-[3-[2-[(3-ethoxy-3-oxo-propyl)carbamoyl]-6-methoxy-benzothiophen-5-yl]oxypropoxy]-6-methoxy-iso indolin-2-yl]-4-oxo-butanoate (0.147 g, 223.8324 μmol, 34.4662% yield) obtained as a white solid. LCMS: (ESI, m/z): $[M+H]^+$ = 657.240.

***Step d: 4-(5-(3-((2-((2-carboxyethyl)carbamoyl)-6-methoxybenzo[b]thiophen-5-yl)oxy)propoxy)-6-methoxyisoindo lin-2-yl)-4-oxobutanoic acid***

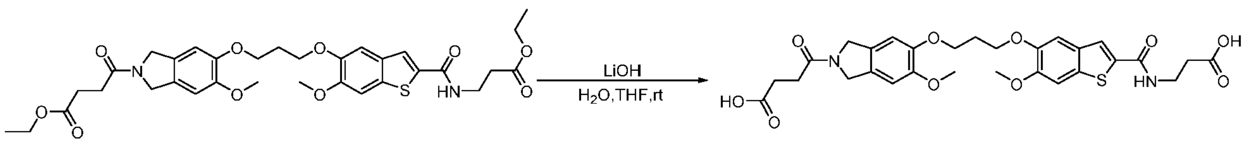


LiOH (0.101 g, 4.2174 mmol) was added to a solution of ethyl 4-[5-[3-[2-[(3-ethoxy-3-oxo-propyl)carbamoyl]-6-methoxy-benzothiophen-5-yl]oxypropoxy]-6-methoxy-iso indolin-2-yl]-4-oxo-butanoate (0.082 g, 124.8589 μmol) in THF (8 mL) and $H_2O$ (8 mL) at 20°C. The reaction mixture was stirred for 2 h at 20°C. The reaction mixture was adjusted to pH=3 with HCl (1 mol/L). The mixture was evaporated under reduced pressure. The residue was purified on C18 column ACN/$H_2O$ (0.1%FA)(0-45%). The pure fractions was concentrated and dried by lyophilization. There was 4-(5-(3-((2-((2-carboxyethyl)carbamoyl)-6-methoxybenzo[b]thiophen-5-yl)oxy)propoxy)-6-methoxyisoindol in-2-yl)-4-oxobutanoic acid

(49 mg, 81.5803 μmol, 65.3380% yield) obtained as a white solid. LCMS: (ESI, m/z): [M+H] $^+$ = 601.180. $^1$H NMR (400 MHz, DMSO-$d_6$) δ 12.20 (s, 2H), 8.69 (t, $J$ = 5.5 Hz, 1H) ,7.88 (s, 1H), 7.56 (s, 1H), 7.44 (d, $J$ = 2.4 Hz, 1H), 7.08 - 6.90 (m, 2H), 4.74 (s, 1H), 4.71 (s, 1H), 4.53 (s, 2H), 4.25 - 4.16 (m, 2H), 4.16 - 4.09 (m, 2H), 3.84 (d, $J$ = 1.5 Hz, 3H), 3.74 (d, $J$ = 2.1 Hz, 3H), 3.50 - 3.42 (m, 2H), 2.60 - 2.52 (m, 4H), 2.50 - 2.47 (m, 2H), 2.27 - 2.17 (m, 2H).

**EXAMPLE 19 (not part of the invention)**

**4-(5-((2-(((2-(3-carboxypropanoyl)-6-methoxybenzo[b]thiophen-5-yl)oxy)methyl)allyl)oxy)-6-methoxyi soindolin-2-yl)-4-oxobutanoic acid**

***Step a: ethyl 4-[5-[2-(chloromethyl)allyloxy]-6-methoxy-isoindolin-2-yl]-4-oxo-butanoate***

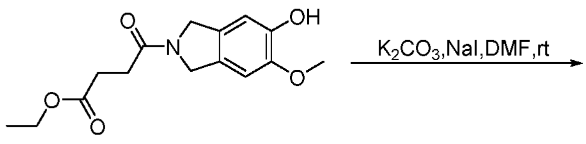


3-Chloro-2-(chloromethyl)prop-1-ene (1.108 g, 8.8643 mmol) was added to a mixture of ethyl 4-(5-hydroxy-6-methoxyisoindolin-2-yl)-4-oxobutanoate (0.507 g, 1.7285 mmol) and Potassium carbonate (0.816 g, 5.9043 mmol) in DMF (20 mL) at 20°C. The reaction mixture was stirred for 8 h at 20°C. The reaction mixture was quenched with adding of water (100 mL) at 20°C, extracted with EA (3 x 50 mL) and washed with brine (50 mL). The organics dried over $Na_2SO_4$, filtered and evaporated under reduced pressure. The residue was purified on silica gel column EA/n-Hex (0-70%). The pure fractions was concentrated and dried under vacuo. There was ethyl 4-[5-[2-(chloromethyl)allyloxy]-6-methoxy-isoindolin-2-yl]-4-oxo-butanoate (0.27 g, 707.0847 μmol, 40.9069% yield) obtained as a yellow oil. LCMS: (ESI, m/z): [M+H] $^+$ = 382.130.

***Step b: ethyl 4-[5-[2-[[2-(4-ethoxy-4-oxo-butanoyl)-6-methoxy-isoindolin-5-yl]oxymethyl]allyloxy]-6-methoxy-benzothi ophen-2-yl]-4-oxo-butanoate***

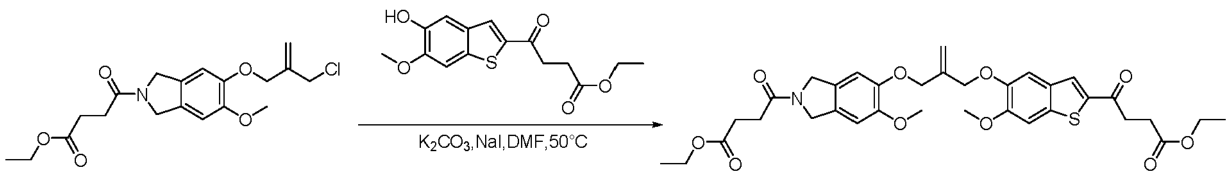


Sodium iodide (0.179 g, 1.1942 mmol) was added to a mixture of ethyl 4-[5-[2-(chloromethyl)allyloxy]-6-methoxy-isoindolin-2-yl]-4-oxo-butanoate (0.216 g, 55.6671 μmol) ethyl 4-(5-hydroxy-6-methoxybenzo[b]thiophen-2-yl)-4-oxobutanoate (0.186 g, 603.2128 μmol) and Potassium carbonate (0.238 g, 1.7221 mmol) in DMF (5 mL) at 20°C. The reaction mixture was stirred for 5 h at 20°C under $N_2$ atmosphere. The mixture was purified on C18 column ACN/$H_2O$ (0.1%FA)(0-70%). The pure fractions was concentrated and dried under vacuo. There was ethyl 4-[5-[2-[[2-(4-ethoxy-4-oxo-butanoyl)-6-methoxy-isoindolin-5-yl]oxymethyl]allyloxy]-6-methoxy-benzothi ophen-2-yl]-4-oxo-butanoate (0.169 g, 258.5135 μmol, 45.7006% yield) obtained as a white solid. LCMS: (ESI, m/z): [M+H] $^+$ = 654.230. $^1$H NMR (400 MHz, DMSO-$d_6$) δ 8.16 (s, 1H), 7.63 (d, $J$ = 4.9 Hz, 1H), 7.53 (s, 1H), 7.05 - 6.87 (m, 2H), 5.45 - 5.32 (m, 2H), 4.81 - 4.72 (m, 3H), 4.66 (s, 3H), 4.52 (s, 1H), 4.50 (s, 1H), 4.10 - 4.00 (m, 4H), 3.87 (d, $J$ = 1.9 Hz, 3H), 3.76 (d, $J$ = 2.8 Hz, 3H), 3.32 - 3.26 (m, 2H), 2.72 - 2.52 (m, 6H), 1.21 - 1.13 (m, 6H).

***Step c: 4-(5-((2-(((2-(3-carboxypropanoyl)-6-methoxybenzo[b]thiophen-5-yl)oxy)methyl)allyl)oxy)-6-methoxyisoi ndolin-2-yl)-4-oxobutanoic acid***

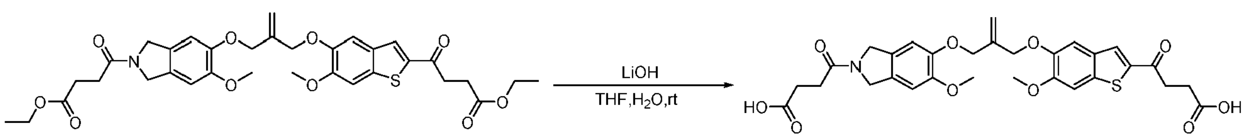


LiOH (0.073 g, 3.0482 mmol) was added to a mixture of ethyl 4-[5-[2-[[2-(4-ethoxy-4-oxo-butanoyl)-6-methoxy-isoindolin-5-yl]oxymethyl]allyloxy]-6-methoxy-benzothi ophen-2-yl]-4-oxo-butanoate (0.075 g, 114.7249 μmol) in THF (4 mL) and $H_2O$ (2 mL) at 20°C. The reaction mixture was stirred for 2 h at 20°C. The reaction mixture was adjusted to pH=3 with HCl (1 mol/L). The mixture was evaporated under reduced pressure. The residue was purified on C18 column ACN/$H_2O$ (0.1%FA)(0-45%). The pure fractions was concentrated and dried by lyophilization. There was 4-(5-((2-(((2-(3-carboxypropanoyl)-6-methoxybenzo[b]thiophen-5-yl)oxy)methyl)allyl)oxy)-6-methoxyisoin dolin-2-yl)-4-oxobutanoic acid (0.0423 g, 70.7794 μmol, 61.6949% yield) obtained as a white solid. LCMS: (ESI, m/z): $[M+H]^+$ = 598.170. $^1H$ NMR (400 MHz, DMSO-$d_6$) δ 12.18 (s, 2H), 8.15 (s, 1H), 7.63 (d, *J* = 4.0 Hz, 1H), 7.53 (s, 1H), 7.06 - 6.90 (m, 2H), 5.45 - 5.32 (m, 2H), 4.78 - 4.70 (m, 3H), 4.66 (t, *J* = 3.3 Hz, 3H), 4.53 (s, 1H), 4.50 (s, 1H), 3.87 (d, *J* = 1.6 Hz, 3H), 3.76 (d, *J* = 2.6 Hz, 3H), 3.29 - 3.24 (m, 2H), 2.65 - 2.50 (m, 6H).

**EXAMPLE 20**

**4-(5-(3-((2-(3-carboxypropanoyl)-4-fluoro-6-methoxybenzo[b]thiophen-5-yl)oxy)propoxy)-6-methoxyis oindolin-2-yl)-4-oxobutanoic acid**

***Step a: ethyl 4-(5-(3-((2-(4-ethoxy-4-oxobutanoyl)-4-fluoro-6-methoxybenzo[b]thiophen-5-yl)oxy)propoxy)-6-methoxyis oindolin-2-yl)-4-oxobutanoate***

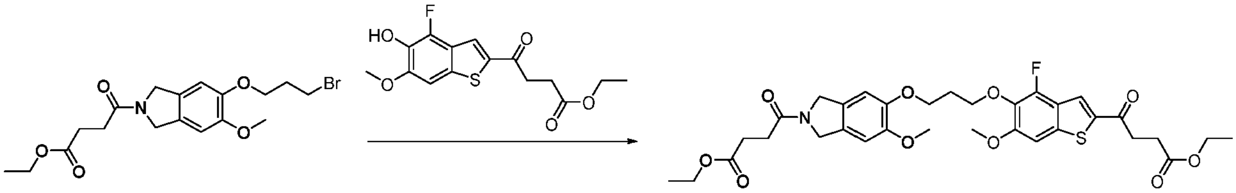

Ethyl 4-(4-fluoro-5-hydroxy-6-methoxybenzo[b]thiophen-2-yl)-4-oxobutanoate (0.081 g, 248.2078 μmol) and Potassium carbonate (0.136 g, 984.0419 μmol) was added to a solution of ethyl 4-(5-(3-bromopropoxy)-6-methoxyisoindolin-2-yl)-4-oxobutanoate (0.103 g, 248.6182 μmol) in N,N-Dimethylformamide (10 mL) at 20°C. The reaction mixture was stirred overnight at 50°C. The reaction mixture was diluted with Ethyl acetate (100 mL), washed sequentially with water (100 mL) and brine (100 mL). The organic layer was dried over $Na_2SO_4$, filtered and evaporated under reduced pressure. The residue was purified on C-18 column MeCN/water (0-100%). The pure fractions was concentrated and dried under vacuo. There was ethyl 4-(5-(3-((2-(4-ethoxy-4-oxobutanoyl)-4-fluoro-6-methoxybenzo[b]thiophen-5-yl)oxy)propoxy)-6-methoxyis oindolin-2-yl)-4-oxobutanoate (66 mg, 100.0428 μmol, 40.2396% yield) obtained as a white solid. LCMS: (ESI, m/z): $[M+H]^+$ = 432.070. $^1H$ NMR (400 MHz, DMSO-$d_6$) δ 8.32 (s, 1H), 7.57 (d, *J* = 3.2 Hz, 1H), 6.98 (s, 0.5H), 6.95 (s, 1H), 6.92 (s, 0.5H), 4.74 (s, 2H), 4.53 (s, 2H), 4.31 - 4.09 (m, 4H), 4.09 - 3.98 (m, 4H), 3.87 (s, 3H), 3.73 (s, 3H), 3.41 - 3.34 (m, 2H), 2.70 - 2.53 (m, 6H), 2.17 - 2.04 (m, 2H), 1.21 - 1.12 (m, 6H).

***Step b: 4-(5-(3-((2-(3-carboxypropanoyl)-4-fluoro-6-methoxybenzo[b]thiophen-5-yl)oxy)propoxy)-6-methoxyisoin dolin-2-yl)-4-oxobutanoic acid***

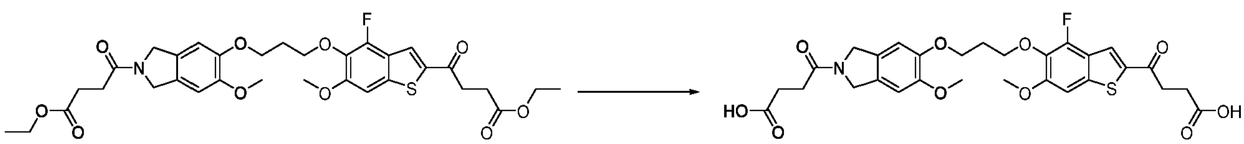

LiOH (0.010 g, 417.5662 μmol) was added to a solution of ethyl 4-(5-(3-((2-(4-ethoxy-4-oxobutanoyl)-4-fluoro-6-methoxybenzo[b]thiophen-5-yl)oxy)propoxy)-6-methoxyis oindolin-2-yl)-4-oxobutanoate (0.052 g, 78.8217 μmol) in

Tetrahydrofuran (10 mL) and Water (2 mL). The reaction mixture was stirred for 2 hours at 50°C. The reaction mixture was acidified pH=4 with HCl (1 M). The mixture was evaporated under reduced pressure. The residue was purified on C-18 column MeCN/water (0-100%). The pure fractions was concentrated and dried under vacuo. There was 4-(5-(3-((2-(3-carboxypropanoyl)-4-fluoro-6-methoxybenzo[b]thiophen-5-yl)oxy)propoxy)-6-methoxyisoind olin-2-yl)-4-oxobutanoic acid (22 mg, 36.4473 μmol, 46.2402% yield) obtained as a white solid. LCMS: (ESI, m/z): [M+H] $^+$ = 604.157. $^1$H NMR (400 MHz, DMSO-$d_6$) δ 12.15 (s, 2H), 8.30 (s, 1H), 7.57 (s, 1H), 6.98 (s, 0.5H), 6.95 (s, 1H), 6.92 (s, 0.5H), 4.74 (s, 2H), 4.53 (s, 2H), 4.33 - 4.06 (m, 4H), 3.91 (s, 3H), 3.68 (s, 3H), 3.30-3.38 (m, 2H), 2.71 - 2.53 (m, 6H), 2.18 - 2.10 (m, 2H).

**EXAMPLE 21 (not part of the invention)**

**trans-2-(5-(3-((2-(3-carboxypropanoyl)-4-fluoro-6-methoxyisoindolin-5-yl)oxy)propoxy)-6-methoxyben zo[b] thiophene-2-carbonyl)cyclopropane-1-carboxylic acid**

***Step a: methyl trans-2-[5-(5,5-dimethyl-1,3,2-dioxaborinan-2-yl)-6-methoxy-benzothiophene-2-carbonyl]cyclopropanecar boxylate***

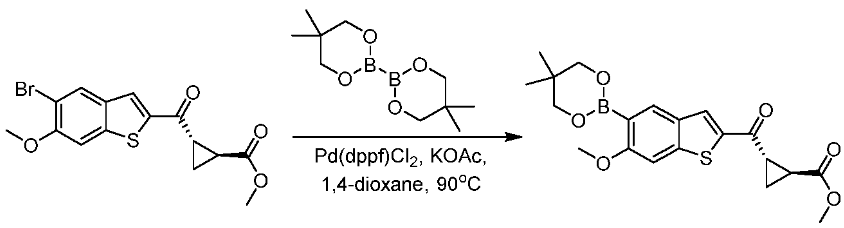


[1,1'-Bis(diphenylphosphino)ferrocene]dichloropalladium(II) (0.203 g, 277.4347 μmol) was added to a mixture of methyl trans-2-(5-bromo-6-methoxybenzo[b]thiophene-2-carbonyl)cyclopropane-1-carboxylate (0.510 g, 1.3813 mmol), bis(neopentyl glycolato)diboron (0.953 g, 4.2190 mmol) and KOAc (0.437 g, 4.4527 mmol) in 1,4-Dioxane (20 mL) at 20°C. The reaction mixture was heated to 90°C and stirred overnight under $N_2$ atmosphere. The reaction mixture was filtered through a celite pad. The filtrate was evaporated under reduced pressure. There was a crude methyl trans-2-[5-(5,5-dimethyl-1,3,2-dioxaborinan-2-yl)-6-methoxy-benzothiophene-2-carbonyl]cyclopropanecarb oxylate (1.61 g, 2.0012 mmol, 144.8795% yield) obtained as a black oil, which was directly used in the next step. LCMS: (ESI, m/z): [M+H] $^+$ = 403.13.

***Step b: methyl trans-2-(5-hydroxy-6-methoxy-benzothiophene-2-carbonyl)cyclopropanecarboxylate***

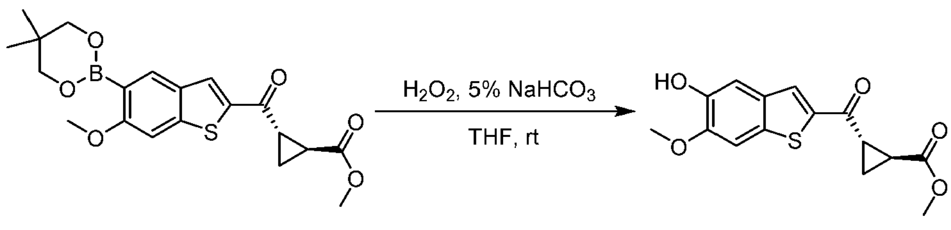


$H_2O_2$ (3.0 mL, 24.7468 mmol, 30% aqueous solution) was added to a mixture of methyl trans-2-[5-(5,5-dimethyl-1,3,2-dioxaborinan-2-yl)-6-methoxy-benzothiophene-2-carbonyl]cyclopropanecarb oxylate (1.56 g, 1.9390 mmol) and $NaHCO_3$ (3.0 mL, 1.6722 mmol, 5% aqueous solution) in THF (30 mL) at 20°C. The reaction mixture was stirred for 1 h at 20°C. The resulting reaction mixture was diluted with brine (150 mL), extracted with EA (150 mL) and washed with brine (150 mL). The organic layer was collected and dried over $Na_2SO_4$, filtered and evaporated under reduced pressure. The residue was purified by silica gel column chromatography, eluting with ethyl acetate/n-hexane (0-100%). The pure fractions was concentrated and dried under reduced pressure. There was methyl trans-2-(5-hydroxy-6-methoxy-benzothiophene-2-carbonyl)cyclopropanecarboxylate (0.30 g, 979.3261 μmol, 71.43% yield) obtained as a reddish brown solid. LCMS: (ESI, m/z): [M+H] $^+$ = 307.05. $^1$H NMR (400 MHz, $CDCl_3$-$d$) δ 7.98 (s, 1H), 7.38 (s, 1H), 7.26 (s, 1H), 4.02 (s, 3H), 3.76 (s, 3H), 3.23 - 3.11 (m, 1H), 2.49 - 2.38 (m, 1H), 1.73 - 1.66 (m, 1H), 1.65 - 1.58 (m, 1H).

***Step c: tert-butyl 4-fluoro-6-methoxy-5-[3-[6-methoxy-2-[trans-2-methoxycarbonylcyclopropanecarbonyl]benzothiophen-5-yl]oxypropoxy]isoindoline-2-carboxylate***

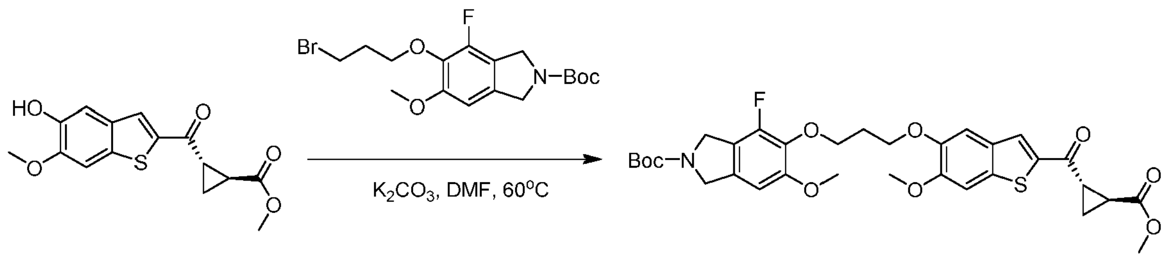


$K_2CO_3$ (0.501 g, 3.6250 mmol) was added to a solution of methyl trans-2-(5-hydroxy-6-methoxy-benzothiophene-2-carbonyl)cyclopropanecarboxylate (0.300 g, 979.3264 μmol) and tert-butyl 5-(3-bromopropoxy)-4-fluoro-6-methoxyisoindoline-2-carboxylate (0.406 g, 1.0043 mmol) in DMF (10 mL) at 20°C. The reaction mixture was heated to 60°C and stirred overnight. The resulting reaction mixture was diluted with $H_2O$ (150 mL), extracted with EA (150 mL) and washed with brine (2 x 150 mL). The organic layer was collected and dried over $Na_2SO_4$, filtered and evaporated under reduced pressure. The residue was purified by silica gel column chromatography, eluting with ethyl acetate/n-hexane (0-50%). The pure fractions was concentrated and dried under reduced pressure. There was tert-butyl 4-fluoro-6-methoxy-5-[3-[6-methoxy-2-[trans-2-methoxycarbonylcyclopropanecarbonyl]benzothiophen-5-yl ]oxypropoxy]isoindoline-2-carboxylate (0.45 g, 714.6357 μmol, 72.9722% yield) obtained as a yellowish solid. LCMS: (ESI, m/z): [M+H-Boc]$^+$ = 530.20. $^1$H NMR (400 MHz, $CDCl_3$-*d*) δ 7.92 (s, 1H), 7.29 (s, 1H), 7.18 (s, 1H), 6.50 (s, 1H), 4.55 (s, 4H), 4.28 (t, *J* = 6.4 Hz, 2H), 4.20 (t, *J* = 5.8 Hz, 2H), 3.87 (s, 3H), 3.71 (s, 3H), 3.67 (s, 3H), 3.11 - 3.04 (m, 1H), 2.38 - 2.31 (m, 1H), 2.30 - 2.20 (m, 2H), 1.65 - 1.58 (m, 1H), 1.57 - 1.50 (m, 1H), 1.44 (s, 9H).

***Step d: methyl trans-2-[5-[3-(4-fluoro-6-methoxy-isoindolin-5-yl)oxypropoxy]-6-methoxy-benzothiophene-2-carbonyl]cyc lopropanecarboxylate***

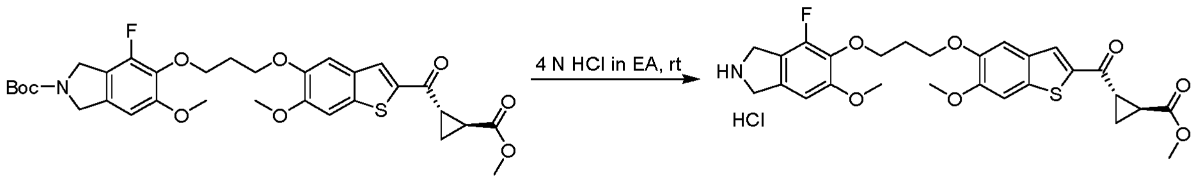


HCl (4 N) in EA (15 mL, 60 mmol) was added to a solution of tert-butyl 4-fluoro-6-methoxy-5-[3-[6-methoxy-2-[trans-2-methoxycarbonylcyclopropanecarbonyl]benzothiophen-5-yl ]oxypropoxy]isoindoline-2-carboxylate (0.45 g, 714.6351 μmol) in EA (5 mL) at 20°C. The reaction mixture was stirred for 2 h at 20°C. The resulting reaction mixture was concentrated under reduced pressure. There was methyl trans-2-[5-[3-(4-fluoro-6-methoxy-isoindolin-5-yl)oxypropoxy]-6-methoxy-benzothiophene-2-carbonyl]cycl opropanecarboxylate (0.42 g, 667.8009 μmol, 93.4464% yield, HCl salt) obtained as a gray solid. LCMS: (ESI, m/z): [M+H]$^+$ = 530.20. $^1$H NMR (400 MHz, DMSO-$d_6$) δ 10.17 (s, 2H), 8.49 (s, 1H), 7.63 (s, 1H), 7.52 (s, 1H), 6.98 (s, 1H), 4.50 (s, 2H), 4.46 (s, 2H), 4.23 (t, *J* = 6.0 Hz, 2H), 4.17 (t, *J* = 5.8 Hz, 2H), 3.86 (s, 3H), 3.77 (s, 3H), 3.68 (s, 3H), 3.30 - 3.26 (m, 1H), 2.27 - 2.20 (m, 1H), 2.19 - 2.11 (m, 2H), 1.54 (t, *J* = 7.1 Hz, 2H).

***Step e: methyl trans-2-[5-[3-[2-(4-ethoxy-4-oxo-butanoyl)-4-fluoro-6-methoxy-isoindolin-5-yl]oxypropoxy]-6-methoxy-be nzothiophene-2-carbonyl]cyclopropanecarboxylate***

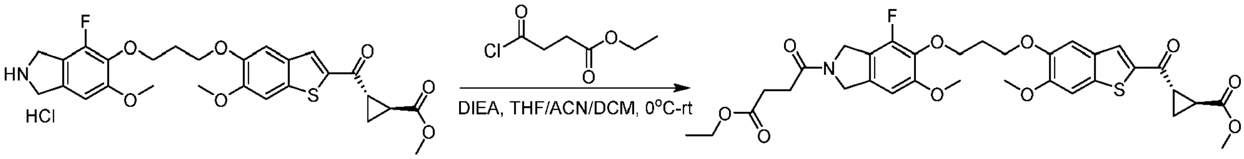


Ethyl 4-chloro-4-oxobutanoate (0.055 mL, 385.9673 μmol) in DCM (2 mL) was added dropwise to a solution of methyl trans-2-[5-[3-(4-fluoro-6-methoxy-isoindolin-5-yl)oxypropoxy]-6-methoxy-benzothiophene-2-carbonyl]cycl opropanecarboxylate (0.202 g, 356.8671 μmol, HCl salt) and N,N-Diisopropylethylamine (0.185 mL, 1.0621 mmol) in THF (4 mL) and ACN (4 mL) at 0°C. The reaction mixture was stirred for 2 h at 20°C. The reaction mixture was concentrated under reduced pressure. The residue was purified by Prep-TLC with the developing solvent of DCM/MeOH (25:1). There was methyl trans-2-[5-[3-[2-(4-ethoxy-4-oxo-butanoyl)-4-fluoro-6-methoxy-isoindolin-5-yl]oxypropoxy]-6-methoxy-be nzothiophene-2-carbonyl]cyclopropanecarboxylate (0.221 g, 336.0184 μmol, 94.1579% yield) obtained as an off-white semi-solid. LCMS: (ESI, m/z): [M+H] + = 658.25. $^1$H NMR (400 MHz, $CDCl_3$-*d*) $\delta$ 7.93 (s, 1H), 7.19 (s, 2H), 6.54 (s, 0.5H), 6.49 (s, 0.5H), 4.74 (d, *J* = 7.7 Hz, 2H), 4.68 (d, *J* = 5.6 Hz, 2H), 4.29 (s, 2H), 4.21 (s, 2H), 4.09 (q, *J* = 7.1 Hz, 2H), 3.88 (s, 3H), 3.73 (s, 3H), 3.68 (s, 3H), 3.15 - 3.02 (m, 1H), 2.72 - 2.54 (m, 4H), 2.40 - 2.31 (m, 1H), 2.30 - 2.18 (m, 2H), 1.65 - 1.51 (m, 2H), 1.20 (t, *J* = 7.0 Hz, 3H).

***Step f: trans-2-(5-(3-((2-(3-carboxypropanoyl)-4-fluoro-6-methoxyisoindolin-5-yl)oxy)propoxy)-6-methoxybenzo[ b]thiophene-2-carbonyl)cyclopropane-1-carboxylic acid***

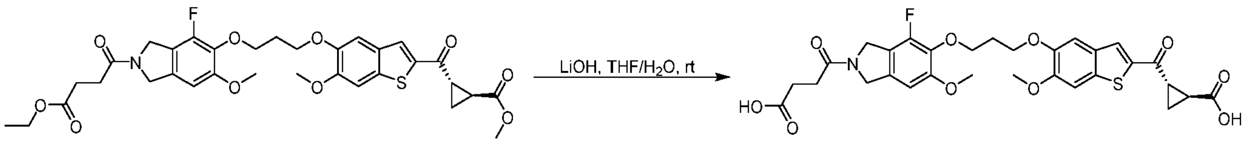


LiOH (0.027 g, 1.1274 mmol) was added to a solution of methyl trans-2-[5-[3-[2-(4-ethoxy-4-oxo-butanoyl)-4-fluoro-6-methoxy-isoindolin-5-yl]oxypropoxy]-6-methoxy-be nzothiophene-2-carbonyl]cyclopropanecarboxylate (0.165 g, 250.8735 μmol) in THF (6 mL) and $H_2O$ (2 mL) at 20°C. The reaction mixture was stirred for 2 h at 20°C. The resulting reaction mixture was adjusted to pH= 2~3 with HCl (6 N) and evaporated under reduced pressure. The residue was purified by reverse C18 column chromatography, eluting with the mobilie phase ACN/$H_2O$ (0.1% FA )(0-100%). The pure fractions was concentrated and lyophilized overnight. There was trans-2-(5-(3-((2-(3-carboxypropanoyl)-4-fluoro-6-methoxyisoindolin-5-yl)oxy)propoxy)-6-methoxybenzo[b ]thiophene-2-carbonyl)cyclopropane-1-carboxylic acid (0.052 g, 84.4674 μmol, 33.6693% yield) obtained as a white solid. LCMS: (ESI, m/z): [M+H] + = 616.20. $^1$H NMR (400 MHz, DMSO-$d_6$) $\delta$ 12.45 (s, 2H), 8.48 (s, 1H), 7.61 (s, 1H), 7.51 (d, *J* = 2.2 Hz, 1H), 6.91 (s, 0.5H), 6.88 (s, 0.5H), 4.78 (d, *J* = 5.5 Hz, 2H), 4.56 (s, 2H), 4.23 (t, *J* = 5.0 Hz, 2H), 4.17 (t, *J* = 5.9 Hz, 2H), 3.86 (s, 3H), 3.76 (s, 3H), 3.27 - 3.19 (m, 1H), 2.60 - 2.53 (m, 2H), 2.49 - 2.44 (m, 2H), 2.21 - 2.07 (m, 3H), 1.49 (t, *J* = 7.1 Hz, 2H).

The following example shown in table 2 was synthesized using the above procedure with the corresponding starting materials.

**Table 2**

| EX No. | Chemical name | Structure | MS: (M+H)$^+$ & $^1$HNMR |
|---|---|---|---|
| 22. | 4-(5-(3-((2-(3-carboxyb utanoyl)-6-methoxybenz o[b]thiophen-5-yl)oxy)p ropoxy)-6-methoxyisoin dolin-2-yl)-2-methyl-4-o xobutanoic acid | 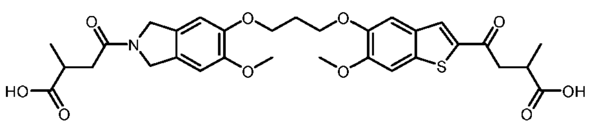 | 614 |

(continued)

| EX No. | Chemical name | Structure | MS: $(M+H)^+$ & $^1$HNMR |
|---|---|---|---|
| 23. | (S)-4-(5-(3-((2-((S)-3-ca rboxybutanoyl)-6-metho xybenzo[b] thiophen-5-y l)oxy)propoxy)-6-metho xyisoindolin-2-yl)-2-met hyl-4-oxobutanoic acid | 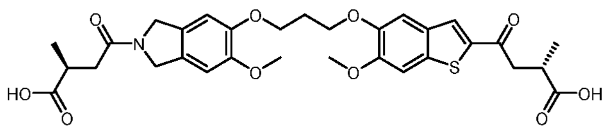 | 614 |
| 24. | (S)-4-(5-(3-((2-(3-carbo xypropanoyl)-6-methox yisoindolin-5-yl) oxy)pro poxy)-6-methoxybenzo [ b]thiophen-2-yl)-2-meth yl-4-oxobutanoic acid | 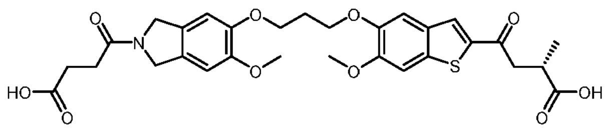 | 600 |
| 25. | (S)-4-(5-(3-((2-(3-carbo xypropanoyl)-6-methox ybenzo[b]thiophen-5-yl) oxy)propoxy)-6-methox yisoindolin-2-yl)-2-meth yl-4-oxobutanoic acid | 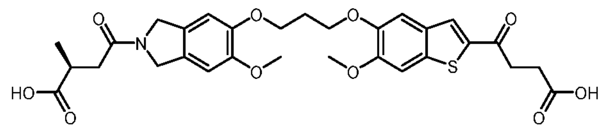 | 600 |
| 26. | (S)-4-(5-(2-((2-((S)-3-ca rboxybutanoyl)-6-metho xybenzo[b] thiophen-5-y l)oxy)ethoxy)-6-methox yisoindolin-2-yl)-2-meth yl-4-oxobutanoic acid | 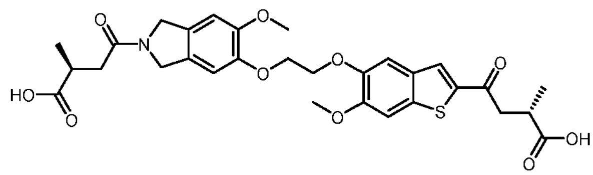 | 600 |
| 27. | (S)-4-(5-(4-((2-((S)-3-ca rboxybutanoyl)-6-metho xybenzo[b] thiophen-5-y l)oxy)butoxy)-6-methox yisoindolin-2-yl)-2-meth yl-4-oxobutanoic acid | 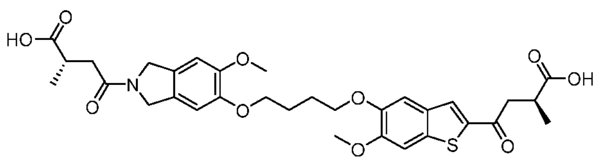 | 628 |
| 28. | (S)-4-(5-((5-((2-((S)-3-c arboxybutanoyl)-6-meth oxybenzo[b] thiophen-5-yl)oxy)pentyl)oxy)-6-m ethoxyisoindolin-2-yl)-2 -methyl-4-oxobutanoic acid | 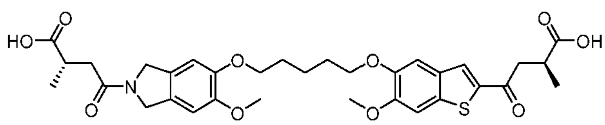 | 642 |
| 29. | (S)-4-(5-(4-(2-((S)-3-car boxybutanoyl)-6-metho xybenzo[b]thiophen-5-y 1)butoxy)-6-methoxyisoi ndolin-2-yl)-2-methyl-4-oxo-butanoic acid (**not part of the invention**) | 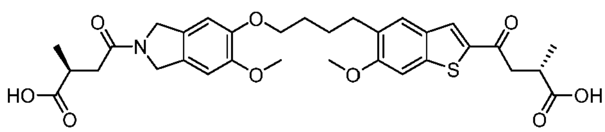 | 612 |
| 30. | (S)-4-(5-(3-((2-((S)-3-ca rboxybutanoyl)-6-metho xybenzo[b] thiophen-5-y 1)amino)propoxy)-6-met hoxyisoindolin-2-yl)-2-methyl-4-oxobutanoic acid (**not part of the invention**) | 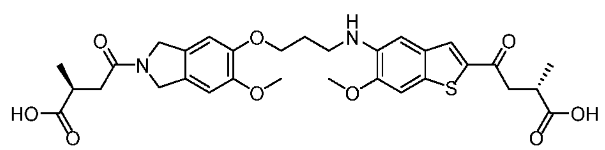 | 613 |

(continued)

| EX No. | Chemical name | Structure | MS: $(M+H)^+$ & $^1$HNMR |
|---|---|---|---|
| 31. | (S)-4-(5-(4-((2-((S)-3-ca rboxybutanoyl)-6-metho xybenzo[b] thiophen-5-y l)amino)butyl)-6-metho xyisoindolin-2-yl)-2-met hyl-4-oxobutanoic acid (**not part of the invention**) | 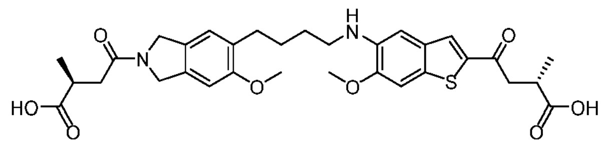 | 611 |
| 32. | (S)-4-(5-(4-(2-((S)-3-car boxybutanoyl)-6-metho xybenzo[b]thiophen-5-y l)butyl)-6-methoxyisoin dolin-2-yl)-2-methyl-4-o xobuta-noic acid (**not part of the invention**) | 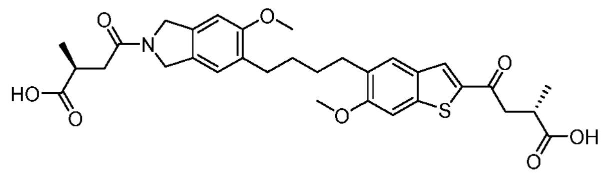 | 596 |
| 33. | (S)-4-(5-(5-(2-((S)-3-car boxybutanoyl)-6-metho xybenzo[b]thiophen-5-y l)pentyl)-6-methoxyisoi ndolin-2-yl)-2-methyl-4-oxobuta-noic acid (**not part of the invention**) | 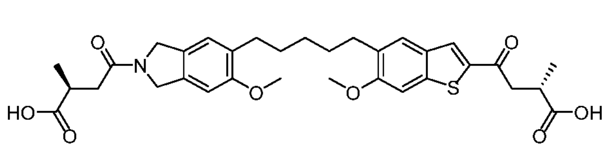 | 610 |
| 34. | (2S)-4-(5-((4-((2-((S)-3-carboxybutanoyl)-6-met hoxybenzo[b] thiophen-5 -yl)oxy)pentan-2-yl) oxy )-6-methoxyisoindolin-2 -yl)-2-methyl-4-oxobuta noic acid | 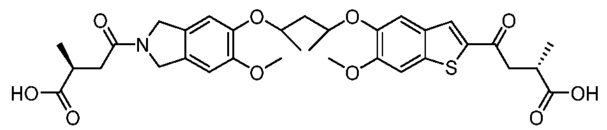 | 642 |
| 35. | (S)-4-(5-(3-((2-((S)-3-ca rboxybutanoyl)-6-metho xybenzo[b] thiophen-5-y l)oxy)-2,2-dimethylprop oxy)-6-methoxyisoindol in-2-yl)-2-methyl-4-oxo butanoic acid | 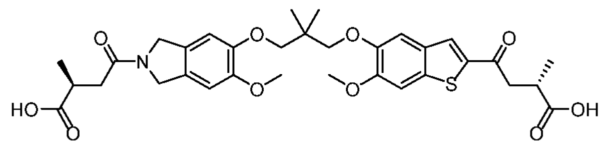 | 642 |
| 36. | (S)-4-(5-((1-(((2-((S)-3-carboxybutanoyl)-6-met hoxybenzo[b] thiophen-5 -yl)oxy)methyl)cyclopro pyl)methoxy)-6-methox yisoindolin-2-yl)-2-meth yl-4-oxo-butanoic acid (**not part of the invention**) | 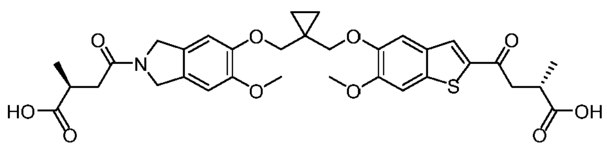 | 640 |
| 37. | 4-(5-(3-((2-(3-carboxypr opanoyl)-6-methoxyisoi ndolin-5-yl) oxy)propox y)-6-methoxybenzo [b]th iophen-2-yl)-2,2-dimeth yl-4-oxobutanoic acid | 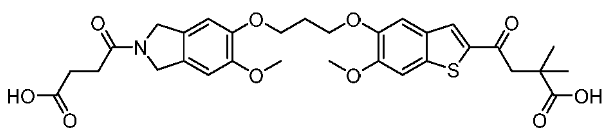 | 614 |
| 38. | 4-(5-(3-((2-(3-carboxypr opanoyl)-6-methoxyben zo[b]thiophen-5-yl)oxy) propoxy)-6-methoxyisoi ndolin-2-yl)-2,2-dimeth yl-4-oxobutanoic acid | 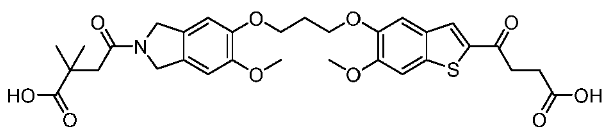 | 614 |

(continued)

| EX No. | Chemical name | Structure | MS: (M+H)+ & 1HNMR |
|---|---|---|---|
| 39. | 4-(5-(3-((2-(3-carboxy-3 -methylbutanoyl)-6-met hoxybenzo[b] thiophen-5 -yl)oxy)propoxy)-6-met hoxyisoindolin-2-yl)-2,2 -dimethyl-4-oxobutanoi c acid | 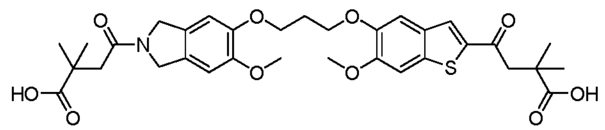 | 642 |
| 40. | (S)-4-(5-((4-(2-((S)-3-ca rboxybutanoyl)-6-metho xybenzo[b] thiophen-5-y l)butyl)amino)-6-metho xyisoindolin-2-yl)-2-met hyl-4-oxobutanoic acid (**not part of the invention**) | 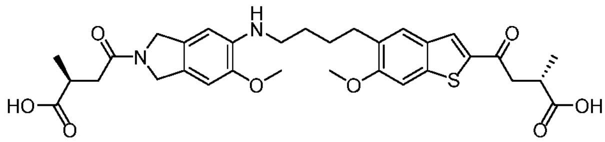 | 611 |
| 41. | (S)-4-(5-((3-((2-((S)-3-c arboxybutanoyl)-6-meth oxybenzo[b] thiophen-5-yl)oxy)propyl)amino)-6-methoxyisoindolin-2-yl) -2-methyl-4-oxobutanoi c acid (**not part of the invention**) | 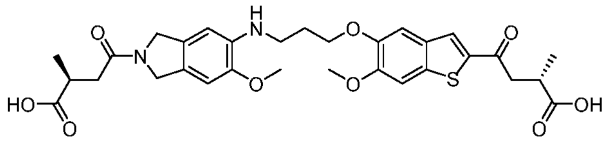 | 613 |
| 42. | (S)-4-(5-(4-((2-((S)-3-ca rboxybutanoyl)-6-metho xybenzo[b] thiophen-5-y l)oxy)butyl)-6-methoxyi soindolin-2-yl)-2-methyl -4-oxobutanoic acid (**not part of the invention**) | 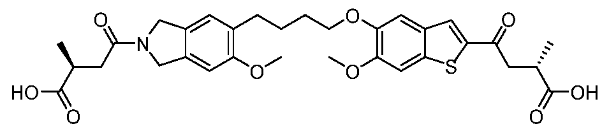 | 612 |
| 43. | (S)-4-(5-(3-((2-((S)-3-ca rboxy-butanoyl)-4-fluoro -6-methoxy-benzo[b]thio phen-5-yl)oxy)propoxy) -6-methoxyisoindolin-2-yl)-2-methyl-4-oxobutan oic acid | 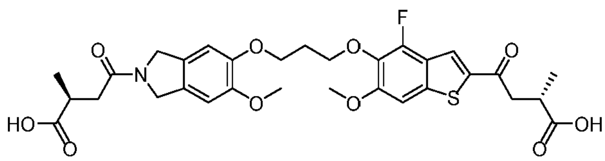 | 632 |
| 44. | (S)-4-(5-(3-((2-((S)-3-ca rboxy-butanoyl)-4-chloro -6-methoxy-benzo[b]thio phen-5-yl)oxy)propoxy) -6-methoxyisoindolin-2-yl)-2-methyl-4-oxobutan oic acid | 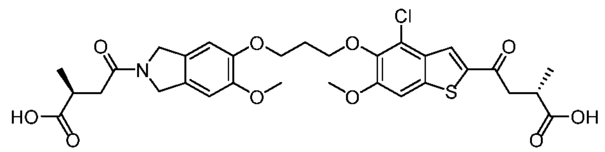 | 648 |
| 45. | (S)-4-(5-(3-((4-bromo-2 -((S)-3-carboxybutanoyl )-6-methoxybenzo[b]thi ophen-5-yl)oxy)propoxy )-6-methoxyisoindolin-2 -yl)-2-methyl-4-oxobuta noic acid | 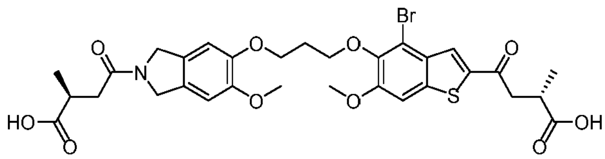 | 692 |
| 46. | (S)-4-(5-(3-((2-((S)-3-ca rboxybutanoyl)-6-metho xybenzo[d] thiazol-5-yl) oxy)propoxy)-6-methox yisoindolin-2-yl)-2-meth yl-4-oxobutanoic acid (**not part of the invention**) | 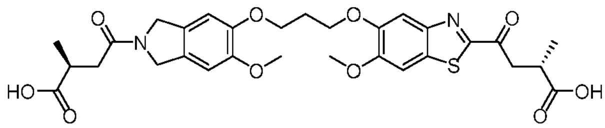 | 615 |

(continued)

| EX No. | Chemical name | Structure | MS: (M+H)+ & 1HNMR |
|---|---|---|---|
| 47. | (S)-4-(5-(3-((2-((S)-3-ca rboxybutanoyl)-6-hydro xybenzo[b] thiophen-5-y l)oxy)propoxy)-6-metho xyisoindolin-2-yl)-2-met hyl-4-oxobutanoic acid | 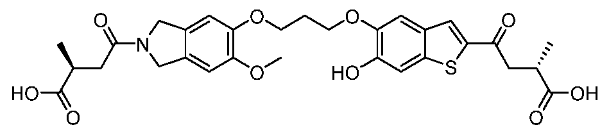 | 600 |
| 48. | (S)-4-(5-(3-((2-((S)-3-ca rboxybutanoyl)-4,7-diflu oro-6-methoxybenzo[b]t hiophen-5-yl)oxy) propo xy)-6-methoxyisoindoli n-2-yl)-2-methyl-4-oxob utanoic acid | 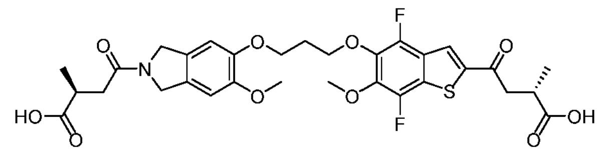 | 650 |
| 49. | (S)-4-(5-(3-((2-((S)-3-ca rboxybutanoyl)-4,7-dich loro-6-methoxybenzo[b] thiophen-5-yl)oxy) propo xy)-6-methoxyisoindoli n-2-yl)-2-methyl-4-oxob utanoic acid | 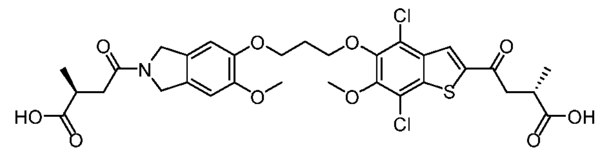 | 682 |
| 50. | (2S)-4-(5-(3-((2-((S)-3-c arboxybutanoyl)-6-meth oxy-3-methylisoindolin-5-yl)oxy)propoxy)-6-me thoxybenzo[b]thiophen-2-yl)-2-methyl-4-oxobut anoic acid (**not part of the invention**) | 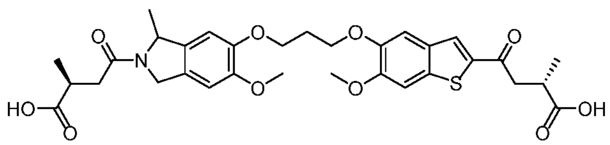 | 628 |
| 51. | (S)-4-(5-(3-((2-((S)-3-ca rboxy-butanoyl)-4-fluoro -6-methoxyi-soindolin-5-yl)oxy)propoxy)-6-meth oxybenzo[b]thiophen-2-yl)-2-methyl-4-oxobutan oic acid | 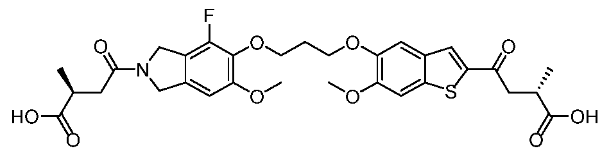 | 632 |
| 52. | (S)-4-(5-(3-((2-((S)-3-ca rboxy-butanoyl)-4-chloro -6-methoxyi-soindolin-5-yl)oxy)propoxy)-6-meth oxybenzo[b]thiophen-2-yl)-2-methyl-4-oxobutan oic acid | 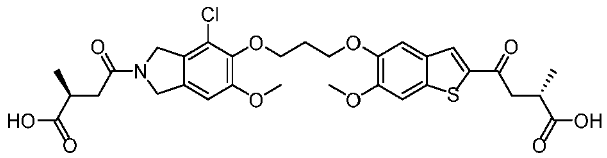 | 648 |
| 53. | (S)-4-(5-(3-((4-bromo-2 -((S)-3-carboxybutanoyl )-6-methoxyisoindolin-5 -yl)oxy)propoxy)-6-met hoxybenzo[b]thiophen-2 -yl)-2-methyl-4-oxobuta noic acid | 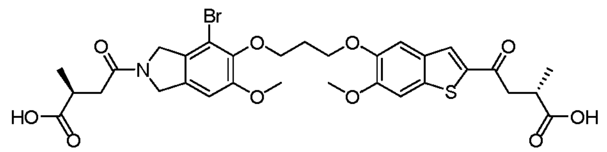 | 692 |
| 54. | (S)-4-(5-(3-((2-((S)-3-ca rboxybutanoyl)-6-hydro xyisoindolin-5-yl)oxy)pr opoxy)-6-methoxybenzo [b]thiophen-2-yl)-2-met hyl-4-oxobutanoic acid (**not part of the invention**) | 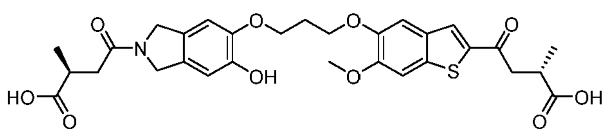 | 600 |

(continued)

| EX No. | Chemical name | Structure | MS: (M+H)$^+$ & $^1$HNMR |
|---|---|---|---|
| 55. | (S)-4-(6-(3-((2-((S)-3-ca rboxybutanoyl)-6-metho xybenzo[b] thiophen-5-y l)oxy)propoxy)-4-fluoro -5-methoxyisoindolin-2-yl)-2-methyl-4-oxobutan oic acid (**not part of the invention**) | 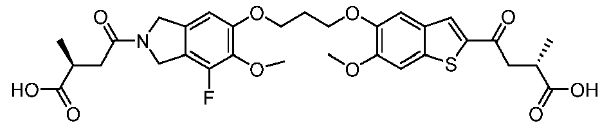 | 632 |
| 56. | (S)-4-(6-(3-((2-((S)-3-ca rboxybutanoyl)-6-metho xybenzo[b] thiophen-5-y l)oxy)propoxy)-4-chloro -5-methoxyisoindolin-2-yl)-2-methyl-4-oxobutan oic acid (**not part of the invention**) | 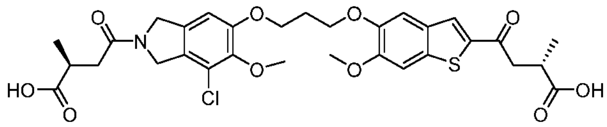 | 648 |
| 57. | (S)-4-(5-(3-((7-bromo-2 -((S)-3-carboxybutanoyl )-6-methoxyisoindolin-5 -yl)oxy)propoxy)-6-met hoxybenzo[b]thiophen-2 -yl)-2-methyl-4-oxobuta noic acid (**not part of the invention**) | 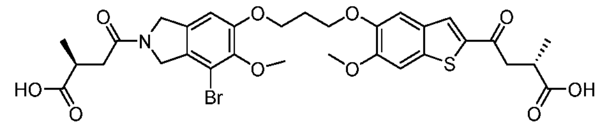 | 692 |
| 58. | (S)-4-(5-(3-((2-((S)-3-ca rboxybutanoyl)-4,7-diflu oro-6-methoxyisoindoli n-5-yl)oxy)propoxy)-6-methoxybenzo[b]thioph en-2-yl)-2-methyl-4-oxo butanoic acid | 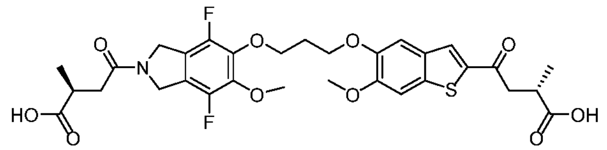 | 650 |
| 59. | (S)-4-(5-(3-((2-((S)-3-ca rboxybutanoyl)-6-metho xybenzo[b] thiophen-5-y l)oxy)propoxy)-7-chloro -4-fluoro-6-methoxyisoi ndolin-2-yl)-2-methyl-4-oxobuta-noic acid | 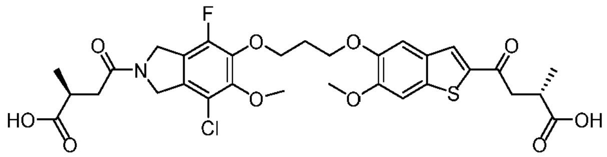 | 666 |
| 60. | (S)-4-(5-(3-((2-((S)-3-ca rboxybutanoyl)-4,7-dich loro-6-methoxyisoindoli n-5-yl)oxy)propoxy)-6-methoxybenzo[b]thioph en-2-yl)-2-methyl-4-oxo butanoic acid | 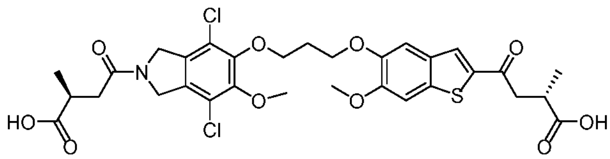 | 682 |
| 61. | (S)-4-(5-(3-((2-((S)-3-ca rboxy-butanoyl)-4-chloro -6-methoxy-benzo[b]thio phen-5-yl)oxy)propoxy) -4-fluoro-6-methoxyisoi ndolin-2-yl)-2-methyl-4-oxobuta-noic acid | 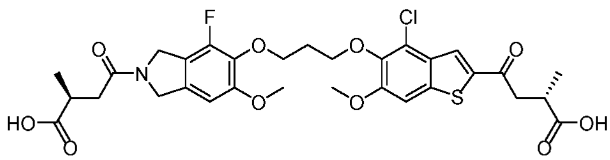 | 666 |
| 62. | (S)-4-(5-(3-((2-((S)-3-ca rboxy-butanoyl)-4-chloro -6-methoxyi-soindolin-5-yl)oxy)propoxy)-4-fluor o-6-methoxybenzo[b]thi ophen-2-yl)-2-methyl-4-oxobuta-noic acid | 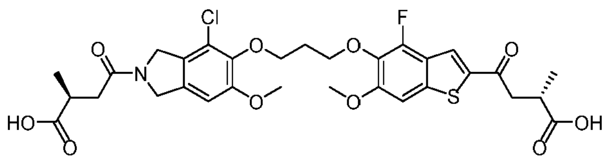 | 666 |

(continued)

| EX No. | Chemical name | Structure | MS: (M+H)+ & 1HNMR |
|---|---|---|---|
| 63. | (S)-4-(5-(3-((2-((S)-3-ca rboxy-butanoyl)-4-chloro -6-methoxy-benzo[b]thio phen-5-yl)oxy)propoxy) -4-chloro-6-methoxyisoi ndolin-2-yl)-2-methyl-4-oxobuta-noic acid | 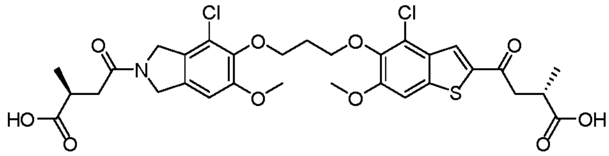 | 682 |
| 64. | (S)-4-(6-(3-((2-((S)-3-ca rboxy-butanoyl)-7-fluoro -6-methoxy-benzo[b]thio phen-5-yl)oxy)propoxy) -4-fluoro-5-methoxyisoi ndolin-2-yl)-2-methyl-4-oxobuta-noic acid (**not part of the invention**) | 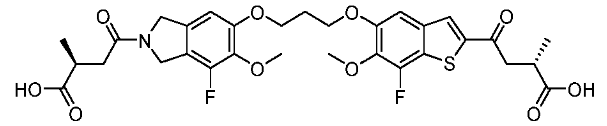 | 650 |
| 65. | (S)-4-(6-(3-((2-((S)-3-ca rboxy-butanoyl)-7-chloro -6-methoxy-benzo[b]thio phen-5-yl)oxy)propoxy) -4-fluoro-5-methoxyisoi ndolin-2-yl)-2-methyl-4-oxobuta-noic acid (**not part of the invention**) | 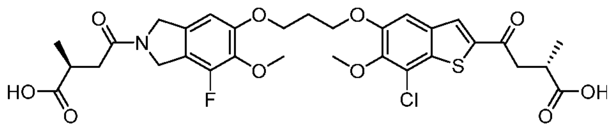 | 666 |
| 66. | (S)-4-(5-(3-((2-((S)-3-ca rboxy-butanoyl)-7-chloro -6-methoxyi-soindolin-5-yl)oxy)propoxy)-7-fluor o-6-methoxybenzo[b]thi ophen-2-yl)-2-methyl-4-oxobuta-noic acid (**not part of the invention**) | 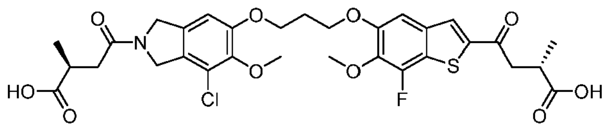 | 666 |
| 67. | (S)-4-(5-(3-((2-((S)-3-ca rboxybutanoyl)-4,7-diflu oro-6-methoxyisoindoli n-5-yl)oxy)propoxy)-7-c hloro-6-methoxybenzo [ b]thiophen-2-yl)-2-meth yl-4-oxobutanoic acid | 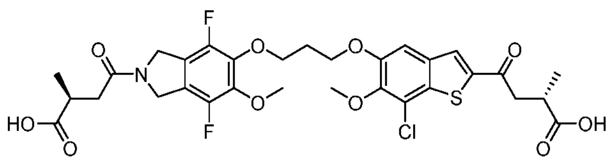 | 684 |
| 68. | (S)-4-(5-(3-((2-((S)-3-ca rboxybutanoyl)-4,7-diflu oro-6-methoxyisoindoli n-5-yl)oxy)propoxy)-7-f luoro-6-methoxybenzo [ b]thiophen-2-yl)-2-meth yl-4-oxobutanoic acid | 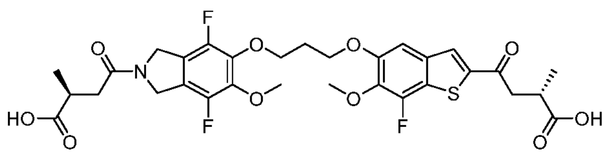 | 668 |
| 69. | (S)-4-(5-(3-((2-((S)-3-ca rboxybutanoyl)-4-chloro -7-fluoro-6-methoxyisoi ndolin-5-yl)oxy)propox y)-7-fluoro-6-methoxyb enzo [b]thiophen-2-yl)-2 -methyl-4-ox-obutanoic acid | 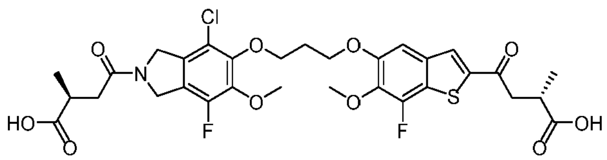 | 684 |

(continued)

| EX No. | Chemical name | Structure | MS: $(M+H)^+$ & $^1$HNMR |
|---|---|---|---|
| 70. | (S)-4-(6-(3-((2-((S)-3-ca rboxybutanoyl)-4,7-diflu oro-6-methoxybenzo[b]t hiophen-5-yl)oxy) propo xy)-4-fluoro-5-methoxyi soindolin-2-yl)-2-methyl -4-oxo-butanoic acid | 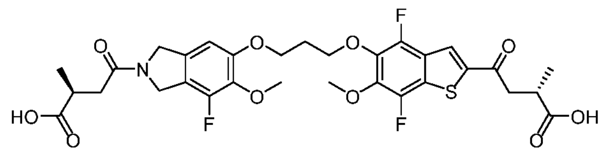 | 668 |
| 71. | (S)-4-(6-(3-((2-((S)-3-ca rboxybutanoyl)-7-chloro -4-fluoro-6-methoxyben zo[b]thiophen-5-yl) oxy) propoxy)-4-fluoro-5-met hoxyisoindolin-2-yl)-2-methyl-4-oxobutanoic acid | 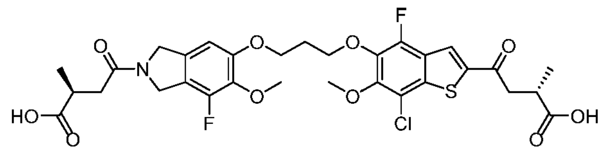 | 684 |
| 72. | (S)-4-(6-(3-((2-((S)-3-ca rboxybutanoyl)-4,7-dich loro-6-methoxybenzo[b] thiophen-5-yl)oxy) propo xy)-4-fluoro-5-methoxyi soindolin-2-yl)-2-methyl -4-oxo-butanoic acid | 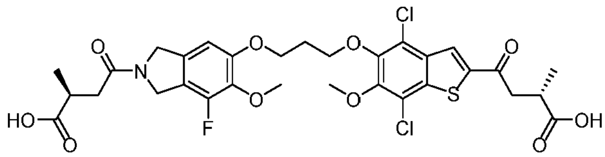 | 700 |
| 73. | (S)-4-(5-(3-((2-((S)-3-ca rboxybutanoyl)-4-chloro -7-fluoro-6-methoxyisoi ndolin-5-yl)oxy)propox y)-7-chloro-6-methoxyb enzo [b]thiophen-2-yl)-2 -methyl-4-ox-obutanoic acid | 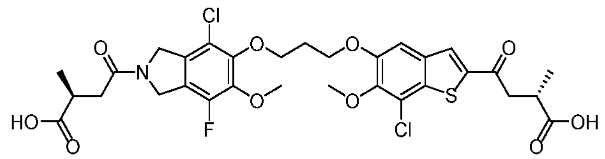 | 700 |
| 74. | (S)-4-(5-(3-((2-((S)-3-ca rboxybutanoyl)-4,7-dich loro-6-methoxyisoindoli n-5-yl)oxy)propoxy)-7-f luoro-6-methoxybenzo [ b]thiophen-2-yl)-2-meth vl-4-oxobutanoic acid | 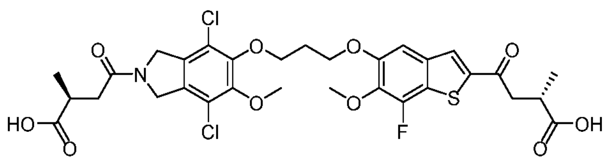 | 700 |
| 75. | (S)-4-(5-(3-((2-((S)-3-ca rboxybutanoyl)-7-chloro -4-fluoro-6-methoxyisoi ndolin-5-yl)oxy)propox y)-7-fluoro-6-methoxyb enzo [b]thiophen-2-yl)-2 -methyl-4-ox-obutanoic acid | 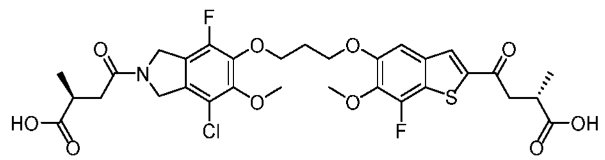 | 684 |
| 76. | (S)-4-(6-(3-((2-((S)-3-ca rboxybutanoyl)-4,7-diflu oro-6-methoxybenzo[b]t hiophen-5-yl)oxy) propo xy)-4-chloro-5-methoxy isoindolin-2-yl)-2-methy l-4-oxo-butanoic acid | 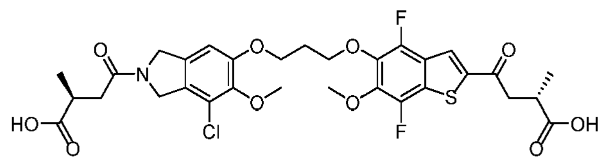 | 684 |
| 77. | (S)-4-(6-(3-((2-((S)-3-ca rboxybutanoyl)-4-chloro -7-fluoro-6-methoxyben zo[b]thiophen-5-yl) oxy) propoxy)-4-chloro-5-me thoxyisoindolin-2-yl)-2-methyl-4-oxobutanoic acid | 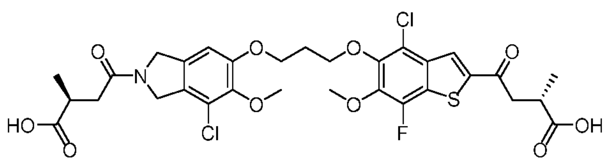 | 700 |

(continued)

| EX No. | Chemical name | Structure | MS: (M+H)+ & 1HNMR |
|---|---|---|---|
| 78. | (S)-4-(5-(3-((2-((S)-3-ca rboxy-butanoyl)-4-fluoro -6-hydroxyi-soindolin-5-yl)oxy)propoxy)-4-fluor o-6-methoxybenzo[b]thi ophen-2-yl)-2-methyl-4-oxobuta-noic acid | 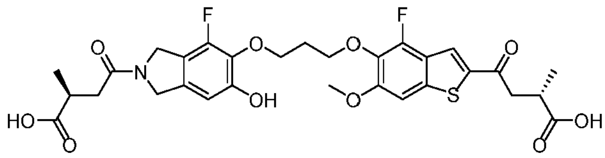 | 636 |
| 79. | (S)-4-(5-(3-((2-((S)-3-ca rboxy-butanoyl)-4-chloro -6-methoxy-benzo[b]thio phen-5-yl)oxy))propoxy) -4-fluoro-6-hydroxyisoi ndolin-2-yl)-2-methyl-4-oxobuta-noic acid | 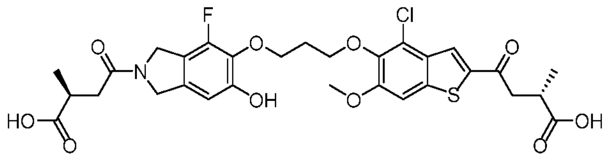 | 652 |
| 80. | (S)-4-(5-(3-((2-((S)-3-ca rboxy-butanoyl)-4-chloro -6-hydroxyi-soindolin-5-yl)oxy)propoxy)-4-fluor o-6-methoxybenzo[b]thi ophen-2-yl)-2-methyl-4-oxobuta-noic acid | 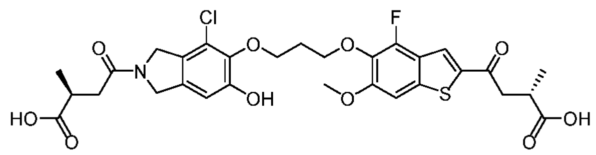 | 652 |
| 81. | (S)-4-(5-(3-((2-((S)-3-ca rboxy-butanoyl)-4-chloro -6-hydroxyi-soindolin-5-yl)oxy)propoxy)-4-chlor o-6-methoxybenzo[b]thi ophen-2-yl)-2-methyl-4-oxobuta-noic acid | 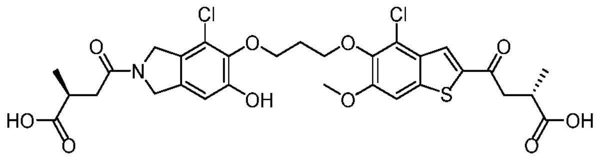 | 668 |
| 82. | (S)-4-(6-(3-((2-((S)-3-ca rboxybutanoyl)-6-metho xybenzo[b] thiophen-5-y l)oxy)propoxy)-4-fluoro -5-hydroxyisoindolin-2-yl)-2-methyl-4-oxobutan oic acid (**not part of the invention**) | 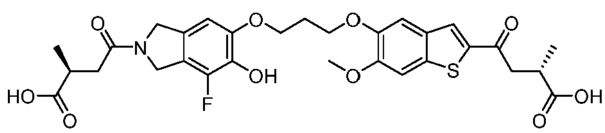 | 618 |
| 83. | (S)-4-(5-(3-((2-((S)-3-ca rboxy-butanoyl)-7-fluoro -6-hydroxyi-soindolin-5-yl)oxy)propoxy)-7-fluor o-6-methoxybenzo[b]thi ophen-2-yl)-2-methyl-4-oxobuta-noic acid (**not part of the invention**) | 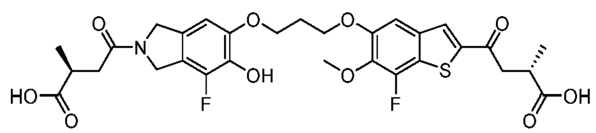 | 636 |
| 84. | (S)-4-(6-(3-((2-((S)-3-ca rboxy-butanoyl)-7-chloro -6-methoxy-benzo[b]thio phen-5-yl)oxy))propoxy) -4-fluoro-5-hydroxyisoi ndolin-2-yl)-2-methyl-4-oxobuta-noic acid (**not part of the invention**) | 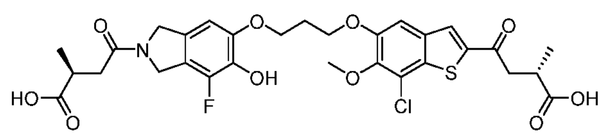 | 652 |

(continued)

| EX No. | Chemical name | Structure | MS: (M+H)+ & 1HNMR |
|---|---|---|---|
| 85. | (S)-4-(5-(3-((2-((S)-3-ca rboxy-butanoyl)-7-chloro -6-hydroxyi-soindolin-5-yl)oxy)propoxy)-7-fluor o-6-methoxybenzo[b]thi ophen-2-yl)-2-methyl-4-oxobuta-noic acid (**not part of the invention**) | 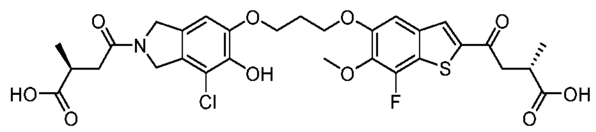 | 652 |
| 86. | (S)-4-(5-(3-((2-((S)-3-ca rboxybutanoyl)-4,7-diflu oro-6-methoxyisoindoli n-5-yl)oxy)propoxy)-4-c hloro-7-fluoro-6-methox ybenzo[b]thiophen-2-yl) -2-methyl-4-oxobutanoi c acid | 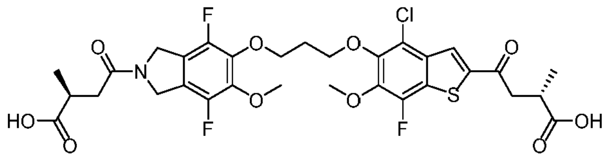 | 702 |
| 87. | (S)-4-(5-(3-((2-((S)-3-ca rboxybutanoyl)-4-chloro -7-fluoro-6-methoxyben zo[b]thiophen-5-yl) oxy) propoxy)-4-chloro-7-flu oro-6-methoxyisoindoli n-2-yl)-2-methyl-4-oxob utanoic acid | 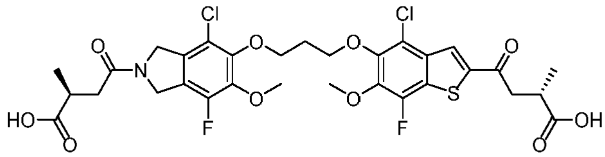 | 718 |
| 88. | (S)-4-(5-(3-((2-((S)-3-ca rboxybutanoyl)-4,7-diflu oro-6-methoxybenzo[b]t hiophen-5-yl)oxy) propo xy)-4,7-difluoro-6-meth oxyisoindolin-2-yl)-2-m ethyl-4-oxobutanoic acid | 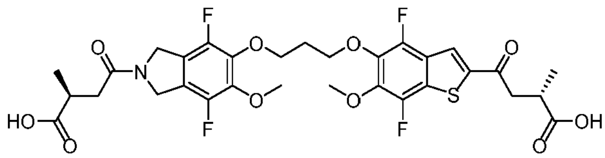 | 686 |
| 89. | (S)-4-(5-(3-((2-((S)-3-ca rboxybutanoyl)-4,7-diflu oro-6-methoxyisoindoli n-5-yl)oxy)propoxy)-7-c hloro-4-fluoro-6-methox ybenzo[b]thiophen-2-yl) -2-methyl-4-oxobutanoi c acid | 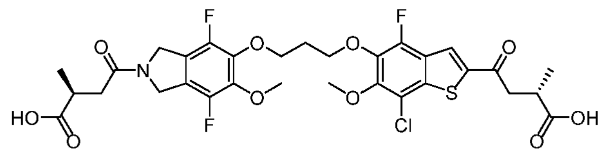 | 702 |
| 90. | (S)-4-(5-(3-((2-((S)-3-ca rboxybutanoyl)-4,7-dich loro-6-methoxybenzo[b] thiophen-5-yl)oxy) propo xy)-4,7-difluoro-6-meth oxyisoindolin-2-yl)-2-m ethyl-4-oxobutanoic acid | 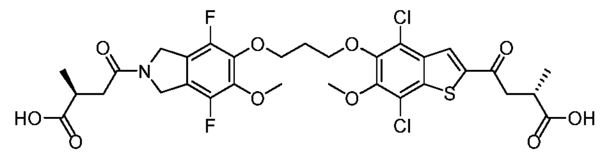 | 718 |
| 91. | (S)-4-(5-(3-((2-((S)-3-ca rboxybutanoyl)-4,7-dich loro-6-methoxybenzo[b] thiophen-5-yl)oxy) propo xy)-4-chloro-7-fluoro-6-methoxyisoindolin-2-yl) -2-methyl-4-oxobutanoi c acid | 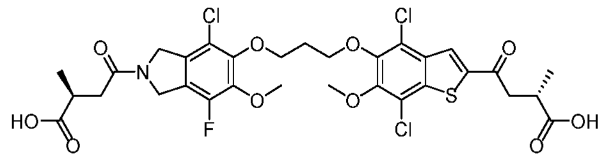 | 734 |
| 92. | (S)-4-(5-(3-((2-((S)-3-ca rboxybutanoyl)-4-chloro -7-fluoro-6-methoxyisoi ndolin-5-yl)oxy)propox y)-7-chloro-4-fluoro-6-methoxybenzo[b]thioph en-2-yl)-2-methyl-4-oxo butanoic acid | 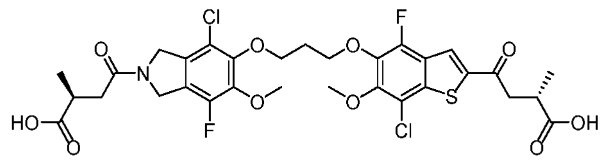 | 718 |

(continued)

| EX No. | Chemical name | Structure | MS: (M+H)+ & 1HNMR |
|---|---|---|---|
| 93. | (S)-4-(5-(3-((2-((S)-3-ca rboxybutanoyl)-4,7-dich loro-6-methoxyisoindoli n-5-yl)oxy)propoxy)-4-c hloro-7-fluoro-6-methox ybenzo[b]thiophen-2-yl) -2-methyl-4-oxobutanoi c acid | 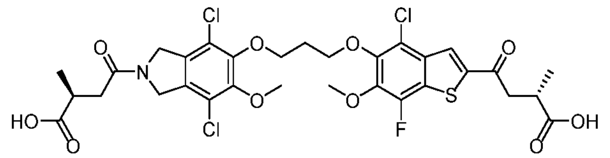 | 734 |
| 94. | (S)-4-(5-(3-((2-((S)-3-ca rboxybutanoyl)-4-chloro -7-fluoro-6-methoxyben zo[b]thiophen-5-yl) oxy) propoxy)-7-chloro-4-flu oro-6-methoxyisoindoli n-2-yl)-2-methyl-4-oxob utanoic acid | 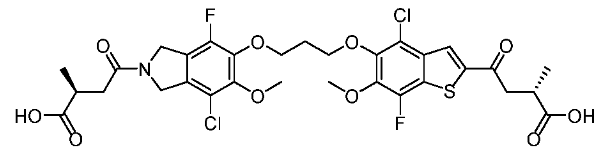 | 718 |
| 95. | (2S,2'S)-4,4'-(7,8,11,13, 17,18-hexahydro-6H,12 H,16H-thieno [2",3":4',5' ]benzo[1',2':9,10] [1,4,8, 11]tetraoxacyclotetradec ino[2,3-f]isoindole-2,12-diyl) bis(2-methyl-4-oxo butanoic acid) **(not part of the invention)** | 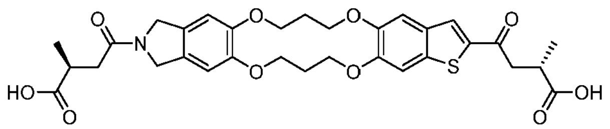 | 626 |
| 96. | (S)-4-(6-methoxy-5-(3-( (6-methoxy-2-((S)-3-me thyl-4-(methylsulfonami do)-4-oxobutanoyl) isoin dolin-5-yl)oxy)propoxy) benzo[b]thiophen-2-yl)-2-methyl-4-oxobutanoic acid **(not part of the invention)** | 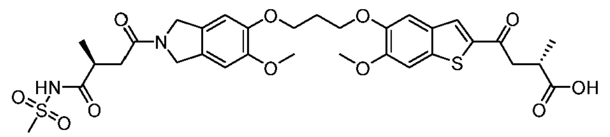 | 691 |
| 97. | (S)-4-(5-(3-((2-((S)-4-(( *N*,*N*-dimethylsulfamoyl) amino)-3-methyl-4-oxob utanoyl)-6-methoxyisoin dolin-5-yl)oxy)propoxy) -6-methoxybenzo[b]thio phen-2-yl)-2-methyl-4-o xobutanoic acid **(not part of the invention)** | 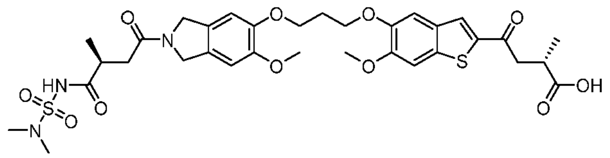 | 720 |
| 98. | (S)-4-(5-(3-((2-(3-cyano propanoyl)-6-methoxyis oindolin-5-yl) oxy)propo xy)-6-methoxybenzo [b]t hiophen-2-yl)-2-methyl-4-ox-obutanoic acid **(not part of the invention)** | 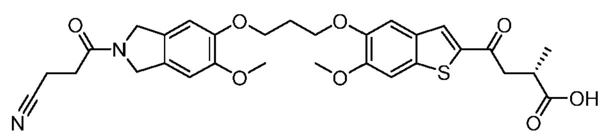 | 581 |
| 99. | 4-(5-(3-((2-(3-cyanopro panoyl)-6-methoxybenz o[b]thiophen-5-yl)oxy)p ropoxy)-6-methoxyisoin dolin-2-yl)-4-oxobutane nitrile **(not part of the invention)** | 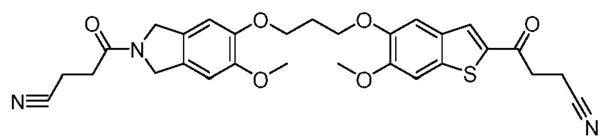 | 548 |

(continued)

| EX No. | Chemical name | Structure | MS: (M+H)+ & 1HNMR |
|---|---|---|---|
| 100. | (S)-4-(5-(3-((2-(3-cyano propanoyl)-6-methoxyb enzo[b]thiophen-5-yl)ox y)propoxy)-6-methoxyis oindolin-2-yl)-2-methyl-4-oxobutanoic acid **(not part of the invention)** | 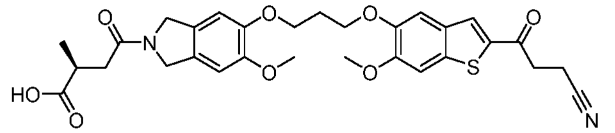 | 581 |
| 101. | (1R,2R)-2-(5-(3-((2-((S) -3-carboxybutanoyl)-6-methoxyisoindolin-5-yl) oxy)propoxy)-6-methox ybenzo[b]thiophene-2-c arbonyl)cyclobutane-1-c arboxylic acid **(not part of the invention)** | 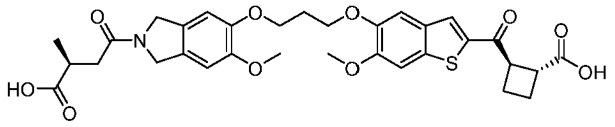 | 626 |
| 102. | (1R,2R)-2-(5-(3-((2-((1 R,2R)-2-carboxycyclobu tane-1-carbonyl)-6-meth oxybenzo[b]thiophen-5-yl)oxy)propoxy)-6-meth oxyisoindoline-2-carbon yl)cyclobutane-1-carbox ylic acid **(not part of the invention)** | 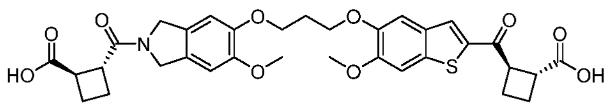 | 638 |
| 103. | (1R,2R)-2-(5-(3-((2-((S) -3-carboxybutanoyl)-6-methoxybenzo [b]thioph en-5-yl)oxy)propoxy)-6-methoxyisoindoline-2-c arbonyl) cyclobutane-1-c arboxylic acid **(not part of the invention)** | 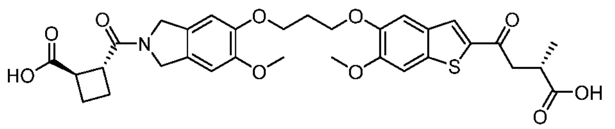 | 626 |
| 104. | (1R,2R)-2-(5-(3-((2-((S) -3-carboxybutanoyl)-6-methoxyisoindolin-5-yl) oxy)propoxy)-6-methox ybenzo[b]thiophene-2-c arbonyl)cyclopropane-1-carboxylic acid | 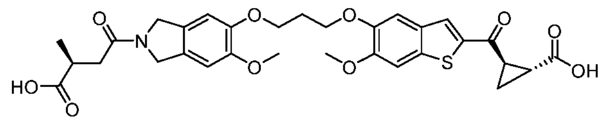 | 612 |
| 105. | (1R,2R)-2-(5-(3-((2-((1 R,2R)-2-carboxycyclopr opane-1-carbonyl)-6-me thoxybenzo[b]thiophen-5-yl)oxy)propoxy)-6-me thoxyisoindoline-2-carb onyl)cyclopropane-1-car boxylic acid | 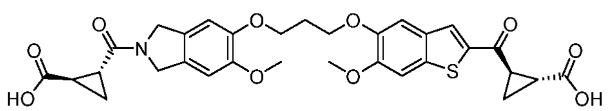 | 610 |
| 106. | (1R,2R)-2-(5-(3-((2-((S) -3-carboxybutanoyl)-6-methoxybenzo [b]thioph en-5-yl)oxy)propoxy)-6-methoxyisoindoline-2-c arbonyl) cyclopropane-1-carboxylic acid | 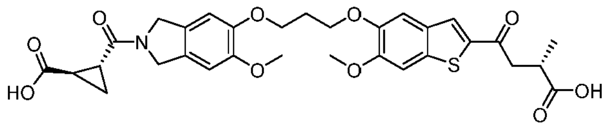 | 612 |

(continued)

| EX No. | Chemical name | Structure | MS: $(M+H)^+$ & $^1$HNMR |
|---|---|---|---|
| 107. | (*S*)-*N*-(*N*,*N*-dimethylsulf amoyl)-4-(5-(3-((2-((S)-4-((*N*,*N*-dimethylsulfam oyl)amino)-3-methyl-4-oxobutanoyl)-6-methox ybenzo[b]thiophen-5-yl) oxy)propoxy)-6-methox yisoindolin-2-yl)-2-meth yl-4-oxobutanamide **(not part of the invention)** | 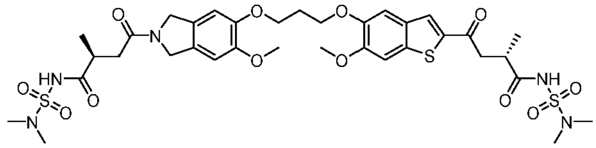 | 826 |
| 108. | (S)-4-(5-(3-((2-((S)-4-(( *N*,*N*-dimethylsulfamoyl) amino)-3-methyl-4-oxob utanoyl)-6-methoxybenz o[b]thiophen-5-yl)oxy)p ropoxy)-6-methoxyisoin dolin-2-yl)-2-methyl-4-o xobutanoic acid **(not part of the invention)** | 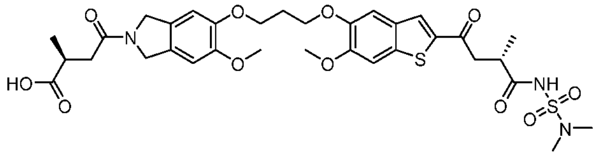 | 720 |
| 109. | (S)-4-(6-methoxy-5-(3-( (6-methoxy-2-((*S*)-3-me thyl-4-(methylsulfonami do)-4-oxobutanoyl) isoin dolin-5-yl)oxy)propoxy) benzo[b]thiophen-2-yl)-2-methyl-*N*-(methylsulf onyl)-4-oxobutanamide **(not part of the invention)** | 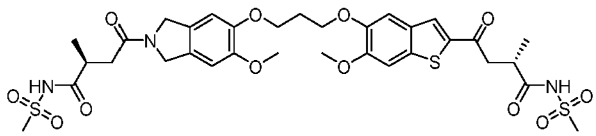 | 768 |
| 110. | (S)-4-(5-methoxy-6-(3-( (6-methoxy-2-((S)-3-me thyl-4-(methylsulfonami do)-4-oxobutanoyl) benz o[b]thiophen-5-yl)oxy)p ropoxy)isoindolin-2-yl)-2-methyl-4-oxobutanoic acid **(not part of the invention)** | 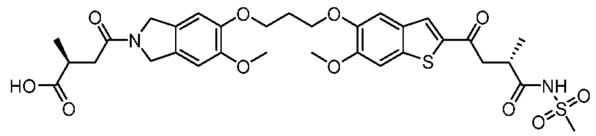 | 691 |
| 111. | (S)-4-(4-(3-((2-((S)-3-ca rboxybutanoyl)-6-metho xybenzo[b] thiophen-5-y l)oxy)propoxy)-6-metho xyisoindolin-2-yl)-2-met hyl-4-oxobutanoic acid | 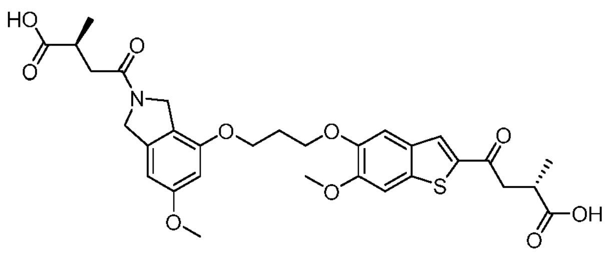 | 614 |
| 112. | (S)-4-(5-(3-((2-((S)-3-ca rboxybutanoyl)-6-metho xybenzo[b] thiophen-4-y l)oxy)propoxy)-6-metho xyisoindolin-2-yl)-2-met hyl-4-oxobutanoic acid | 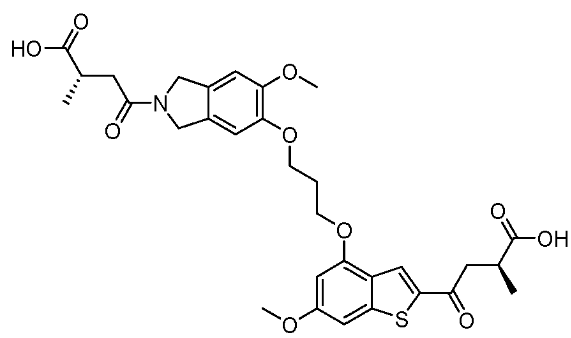 | 614 |

(continued)

| EX No. | Chemical name | Structure | MS: (M+H)+ & 1HNMR |
|---|---|---|---|
| 113. | (S)-4-(4-(4-((2-((S)-3-ca rboxybutanoyl)-6-metho xybenzo[b] thiophen-5-y l)oxy)butyl)-6-methoxyi soindolin-2-yl)-2-methyl -4-oxobutanoic acid **(not part of the invention)** | 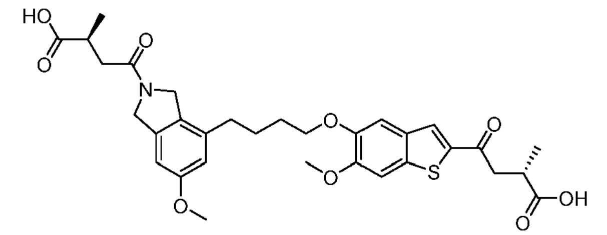 | 612 |
| 114. | (S)-4-(4-(4-(2-((S)-3-car boxybutanoyl)-6-metho xybenzo[b]thiophen-5-y l)butoxy)-6-methoxyisoi ndolin-2-yl)-2-methyl-4-oxobuta-noic acid **(not part of the invention)** | 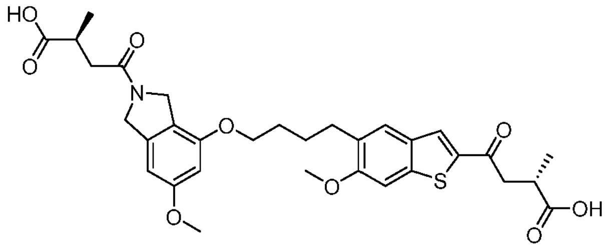 | 612 |
| 115. | (S)-4-(5-(4-((2-((S)-3-ca rboxybutanoyl)-6-metho xybenzo[b] thiophen-4-y l)oxy)butyl)-6-methoxyi soindolin-2-yl)-2-methyl -4-oxobutanoic acid **(not part of the invention)** | 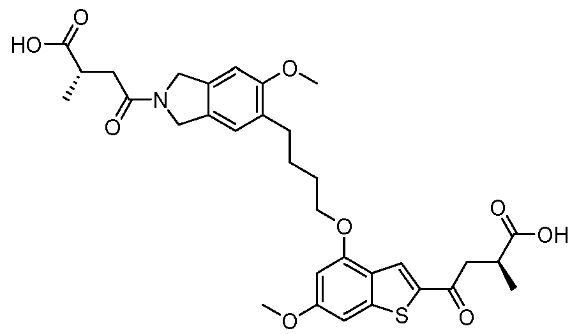 | 612 |
| 116. | (S)-4-(5-(4-(2-((S)-3-car boxybutanoyl)-6-metho xybenzo[b]thiophen-4-y l)butoxy)-6-methoxyisoi ndolin-2-yl)-2-methyl-4-oxobuta-noic acid **(not part of the invention)** | 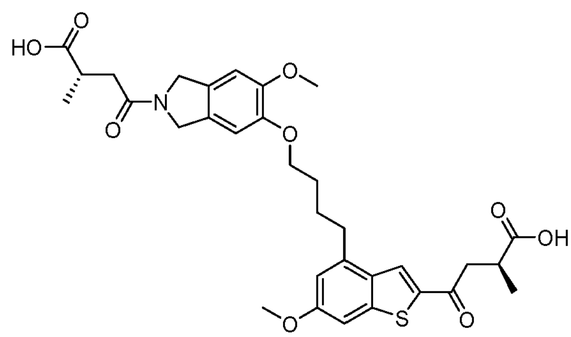 | 612 |
| 117. | (S)-4-(5-(3-((2-((S)-3-ca rboxy-butanoyl)-6-metho xyisoindo-lin-5-yl)oxy)pr opoxy)-6-methylbenzo[ b]thiophen-2-yl)-2-meth yl-4-oxobutanoic acid **(not part of the invention)** | 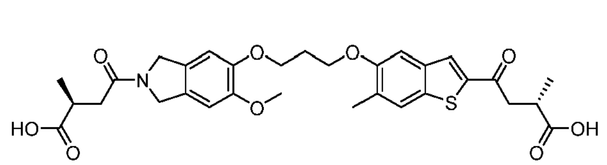 | 598 |
| 118. | (S)-4-(5-(3-((2-((S)-3-ca rboxybutanoyl)-6-ethylb enzo[b]thiophen-5-yl)ox y)propoxy)-6-methoxyis oindolin-2-yl)-2-methyl-4-oxobutanoic acid **(not part of the invention)** | 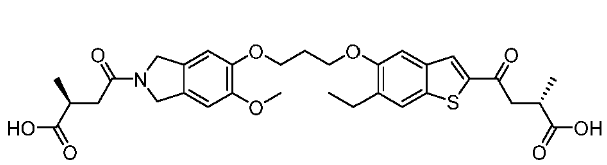 | 612 |

(continued)

| EX No. | Chemical name | Structure | MS: $(M+H)^+$ & $^1HNMR$ |
|---|---|---|---|
| 119. | (S)-4-(5-(3-((2-((S)-3-ca rboxy-butanoyl)-6-metho xyisoindo-lin-5-yl)oxy)pr opoxy)-6-vinylbenzo[b]t hiophen-2-yl)-2-methyl-4-oxobutanoic acid **(not part of the invention)** | 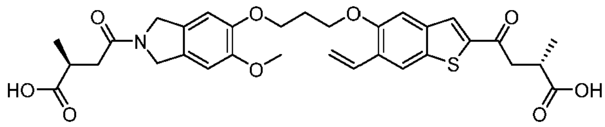 | 610 |
| 120. | (S)-4-(5-(3-((2-((S)-3-ca rboxybutanoyl)-6-ethyn ylbenzo[b] thiophen-5-yl )oxy)propoxy)-6-metho xyisoindolin-2-yl)-2-met hyl-4-oxobutanoic acid **(not part of the invention)** | 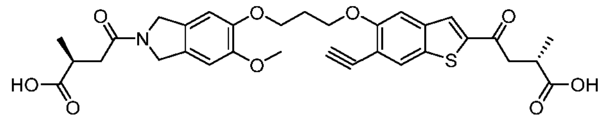 | 608 |
| 121. | (S)-4-(5-(3-((6-amino-2-((S)-3-carboxybutanoyl) benzo[b]thiophen-5-yl)o xy)propoxy)-6-methoxyi soindolin-2-yl)-2-methyl -4-oxobutanoic acid **(not part of the invention)** | 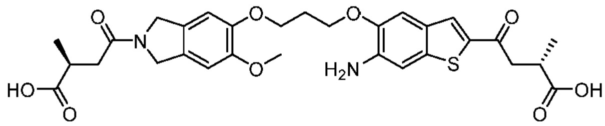 | 599 |
| 122. | (S)-4-(5-(3-((2-((S)-3-ca rboxy-butanoyl)-6-(meth ylamino)ben-zo[b]thioph en-5-yl)oxy)propoxy)-6-methoxyisoindolin-2-yl) -2-methyl-4-oxobutanoi c acid **(not part of the invention)** | 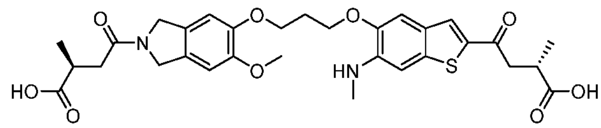 | 613 |
| 123. | (S)-4-(5-(3-((6-bromo-2 -((S)-3-carboxybutanoyl )benzo[b]thiophen-5-yl) oxy)propoxy)-6-methox yisoindolin-2-yl)-2-meth yl-4-oxobutanoic acid | 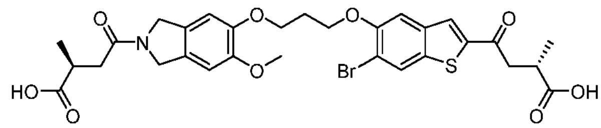 | 662 |
| 124. | (S)-4-(5-(3-((2-((S)-3-ca rboxybutanoyl)-6-metho xybenzo[b] thiophen-5-y 1)oxy)propoxy)-6-methy lisoindolin-2-yl)-2-meth yl-4-oxobutanoic acid **(not part of the invention)** | 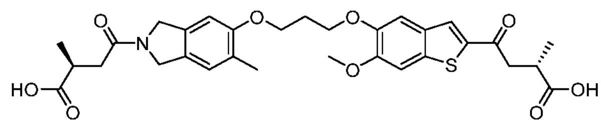 | 598 |
| 125. | (S)-4-(5-(3-((2-((S)-3-ca rboxybutanoyl)-6-ethyli soindolin-5-yl) oxy)prop oxy)-6-methoxybenzo [b ]thiophen-2-yl)-2-methy l-4-oxobutanoic acid **(not part of the invention)** | 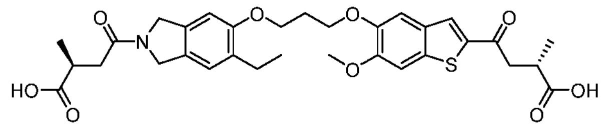 | 612 |
| 126. | (S)-4-(5-(3-((2-((S)-3-ca rboxybutanoyl)-6-metho xybenzo[b] thiophen-5-y l)oxy)propoxy)-6-vinyli soindolin-2-yl)-2-methyl -4-oxobutanoic acid **(not part of the invention)** | 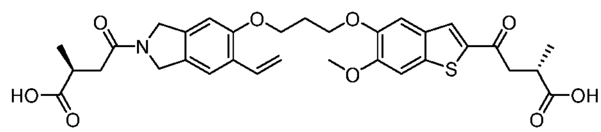 | 610 |

(continued)

| EX No. | Chemical name | Structure | MS: (M+H)+ & 1HNMR |
|---|---|---|---|
| 127. | (S)-4-(5-(3-((2-((S)-3-ca rboxybutanoyl)-6-ethyn ylisoindolin-5-yl)oxy)pr opoxy)-6-methoxybenzo [b]thiophen-2-yl)-2-met hyl-4-oxobutanoic acid **(not part of the invention)** | 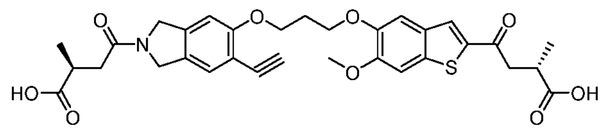 | 608 |
| 128. | (S)-4-(5-(3-((6-amino-2-((S)-3-carboxybutanoyl) isoindolin-5-yl) oxy)prop oxy)-6-methoxybenzo [b ]thiophen-2-yl)-2-methy l-4-oxobutanoic acid **(not part of the invention)** | 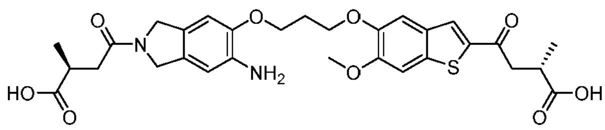 | 599 |
| 129. | (S)-4-(5-(3-((2-((S)-3-ca rboxy-butanoyl)-6-(meth ylamino)isoin-dolin-5-yl) oxy)propoxy)-6-methox ybenzo[b]thiophen-2-yl) -2-methyl-4-oxobutanoi c acid **(not part of the invention)** | 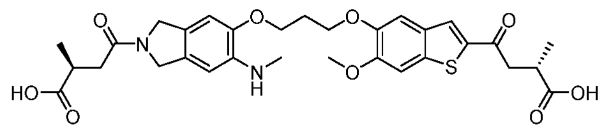 | 613 |
| 130. | (S)-4-(5-(3-((6-bromo-2 -((S)-3-carboxybutanoyl )isoindolin-5-yl) oxy)pro poxy)-6-methoxybenzo [ b]thiophen-2-yl)-2-meth yl-4-oxobutanoic acid | 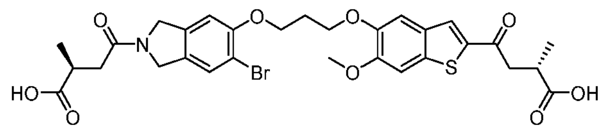 | 662 |
| 131. | (S)-4-(5-(3-((6-((S)-3-ca rboxybutanoyl)-3-metho xy-6,7-dihydro-5H-pyrr olo[3,4-b]pyridin-2-yl)o xy)propoxy)-6-methoxy benzo[b]thiophen-2-yl)-2-methyl-4-oxobutanoic acid **(not part of the invention)** | 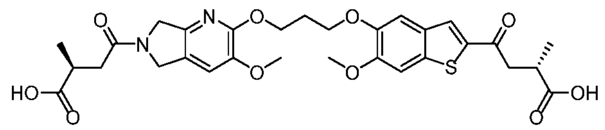 | 615 |
| 132. | (S)-4-(5-(3-((6-((S)-3-ca rboxybutanoyl)-3-metho xy-6,7-dihydro-5H-pyrr olo[3,4-b]pyridin-2-yl)o xy)propoxy)-4-fluoro-6-methoxybenzo[b]thioph en-2-yl)-2-methyl-4-oxo butanoic acid **(not part of the invention)** | 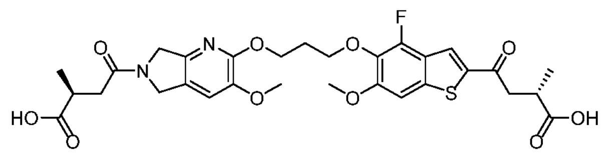 | 633 |
| 133. | (S)-4-(5-(3-((6-((S)-3-ca rboxybutanoyl)-3-metho xy-6,7-dihydro-5H-pyrr olo[3,4-b]pyridin-2-yl)o xy)propoxy)-4-chloro-6-methoxybenzo[b]thioph en-2-yl)-2-methyl-4-oxo butanoic acid **(not part of the invention)** | 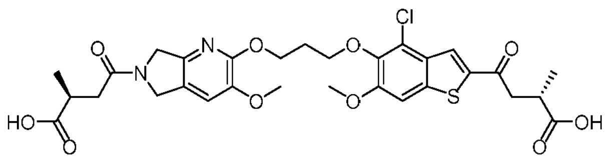 | 649 |

(continued)

| EX No. | Chemical name | Structure | MS: $(M+H)^+$ & $^1$HNMR |
|---|---|---|---|
| 134. | (S)-4-(5-(3-((6-((S)-3-ca rboxybutanoyl)-3-hydro xy-6,7-dihydro-5H-pyrr olo[3,4-b]pyridin-2-yl)o xy)propoxy)-6-methoxyt hieno[3,2-b]pyridin-2-yl )-2-methyl-4-oxobutanoi c acid **(not part of the invention)** | 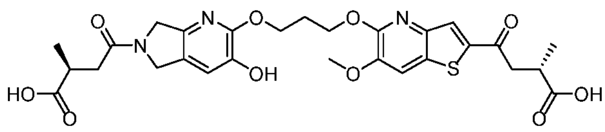 | 602 |
| 135. | (S)-4-(5-(3-((6-((S)-3-ca rboxybutanoyl)-3-metho xy-6,7-dihydro-5H-pyrr olo[3,4-b]pyridin-2-yl)o xy)propoxy)-6-hydroxyt hieno[3,2-b]pyridin-2-yl )-2-methyl-4-oxobutanoi c acid **(not part of the invention)** | 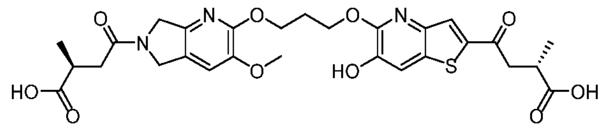 | 602 |
| 136. | (S)-4-(5-(3-((6-((S)-3-ca rboxybutanoyl)-3-metho xy-6,7-dihydro-5H-pyrr olo[3,4-b]pyridin-2-yl)o xy)propoxy)-6-methoxyt hieno[3,2-b]pyridin-2-yl )-2-methyl-4-oxobutanoi c acid **(not part of the invention)** | 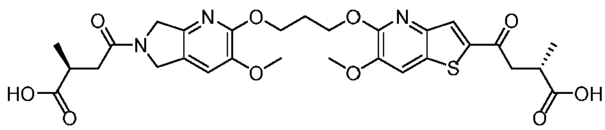 | 616 |
| 137. | (S)-4-(5-(3-((6-((S)-3-ca rboxy-butanoyl)-4-fluoro -3-meth-oxy-6,7-dihydro -5H-pyrrolo[3,4-b]pyridi n-2-yl)oxy)propoxy)-6-methoxythieno[3,2-b]py ridin-2-yl)-2-methyl-4-o xobutanoic acid **(not part of the invention)** | 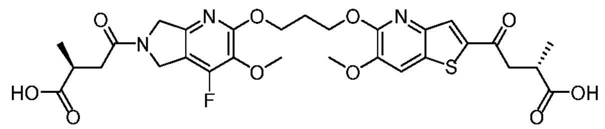 | 634 |
| 138. | (S)-4-(5-(3-((6-((S)-3-ca rboxy-butanoyl)-4-chloro -3-meth-oxy-6,7-dihydro -5H-pyrrolo[3,4-b]pyridi n-2-yl)oxy)propoxy)-6-methoxythieno[3,2-b]py ridin-2-yl)-2-methyl-4-o xobutanoic acid **(not part of the invention)** | 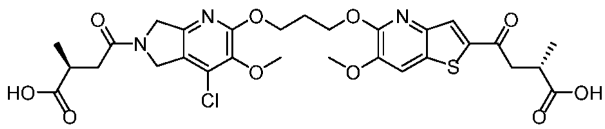 | 650 |
| 139. | (S)-4-(5-(3-((2-((S)-3-ca rboxy-butanoyl)-6-metho xyisoindo-lin-5-yl)oxy)pr opoxy)-6-methoxythien o[3,2-b]pyridin-2-yl)-2-methyl-4-oxobutanoic acid **(not part of the invention)** | 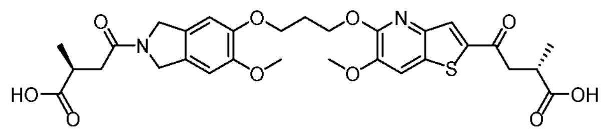 | 615 |
| 140. | (S)-4-(5-(3-((2-((S)-3-ca rboxy-butanoyl)-4-fluoro -6-methoxyi-soindolin-5-yl)oxy)propoxy)-6-meth oxythieno[3,2-b]pyridin-2-yl)-2-methyl-4-oxobut anoic acid **(not part of the invention)** | 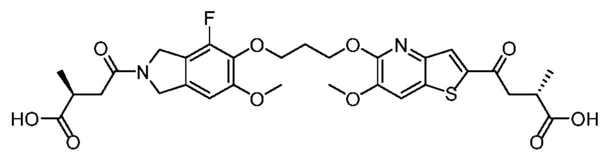 | 633 |

(continued)

| EX No. | Chemical name | Structure | MS: (M+H)+ & 1HNMR |
|---|---|---|---|
| 141. | (S)-4-(5-(3-((2-((S)-3-ca rboxy-butanoyl)-4-chloro -6-methoxyi-soindolin-5-yl)oxy)propoxy)-6-meth oxythieno[3,2-b]pyridin-2-yl)-2-methyl-4-oxobut anoic acid **(not part of the invention)** | 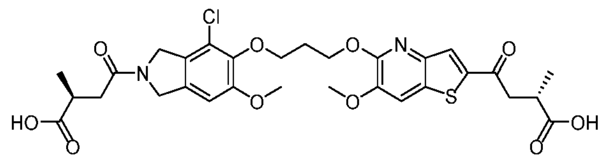 | 649 |
| 142. | (S)-4-(5-(3-((2-((S)-3-ca rboxybutanoyl)-4,7-dich loro-6-methoxyisoindoli n-5-yl)oxy)propoxy)-6-methoxythieno[3,2-b]py ridin-2-yl)-2-methyl-4-o xobuta-noic acid **(not part of the invention)** | 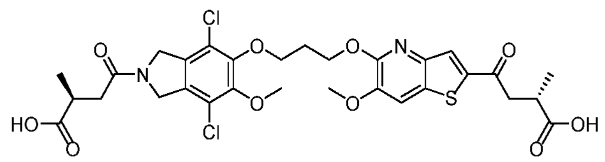 | 683 |
| 143. | (S)-4-(5-(3-((2-((S)-3-ca rboxybutanoyl)-4-chloro -7-fluoro-6-methoxyisoi ndolin-5-yl)oxy)propox y)-6-methoxythieno[3,2-b] pyridin-2-yl)-2-methyl -4-oxobu-tanoic acid **(not part of the invention)** | 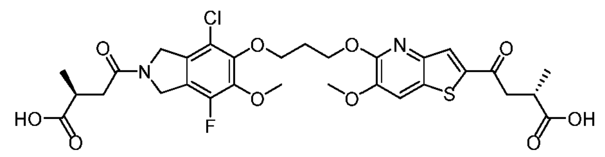 | 667 |
| 144. | (S)-4-(5-(3-((2-((S)-3-ca rboxybutanoyl)-4,7-diflu oro-6-methoxyisoindoli n-5-yl)oxy)propoxy)-6-methoxythieno[3,2-b]py ridin-2-yl)-2-methyl-4-o xobuta-noic acid **(not part of the invention)** | 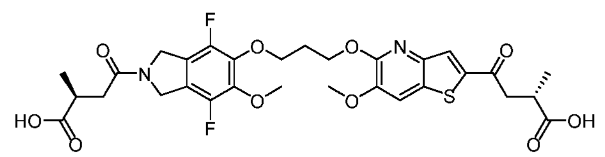 | 651 |
| 145. | (S)-4-(5-(3-((2-((S)-3-ca rboxybutanoyl)-4,7-diflu oro-6-hydro-xyisoindolin -5-yl)oxy)pro-poxy)-6-m ethoxythieno[3,2-b] pyri din-2-yl)-2-methyl-4-ox obu-tanoic acid **(not part of the invention)** | 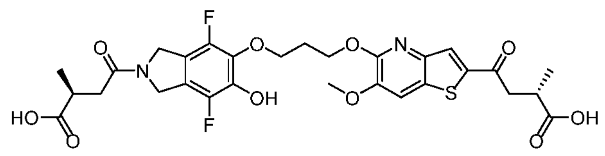 | 637 |
| 146. | (S)-4-(5-(3-((2-((S)-3-ca rboxy-butanoyl)-4-fluoro -6-hydroxy-benzo[b]thio phen-5-yl)oxy)propoxy) -4-fluoro-6-methoxyisoi ndolin-2-yl)-2-methyl-4-oxobuta-noic acid | 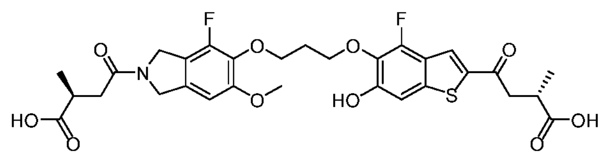 | 636 |
| 147. | (S)-4-(5-(3-((2-((S)-3-ca rboxy-butanoyl)-4-chloro -6-hydroxy-benzo[b]thio phen-5-yl)oxy)propoxy) -4-fluoro-6-methoxyisoi ndolin-2-yl)-2-methyl-4-oxobuta-noic acid | 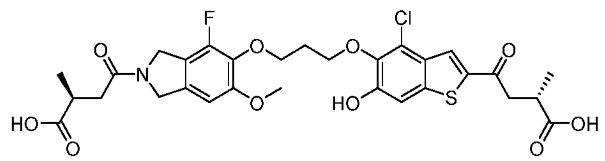 | 652 |

(continued)

| EX No. | Chemical name | Structure | MS: (M+H)+ & 1HNMR |
|---|---|---|---|
| 148. | (S)-4-(5-(3-((2-((S)-3-ca rboxy-butanoyl)-4-chloro -6-methoxyi-soindolin-5-yl)oxy)propoxy)-4-fluor o-6-hydroxybenzo[b]thi ophen-2-yl)-2-methyl-4-oxobuta-noic acid | 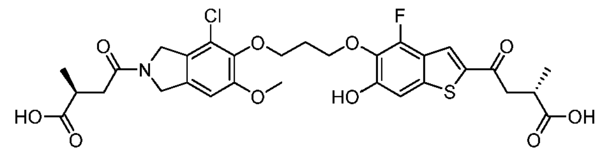 | 652 |
| 149. | (S)-4-(5-(3-((2-((S)-3-ca rboxy-butanoyl)-4-chloro -6-hydroxy-benzo[b]thio phen-5-yl)oxy)propoxy) -4-chloro-6-methoxyisoi ndolin-2-yl)-2-methyl-4-oxobuta-noic acid | 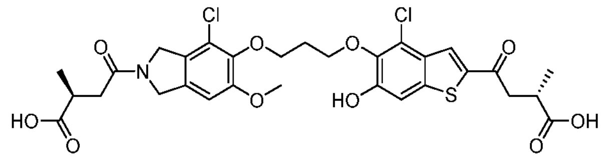 | 668 |
| 150. | (S)-4-(6-(3-((2-((S)-3-ca rboxybutanoyl)-6-hydro xybenzo[b] thiophen-5-y l)oxy)propoxy)-4-fluoro -5-methoxyisoindolin-2-yl)-2-methyl-4-oxobutan oic acid | 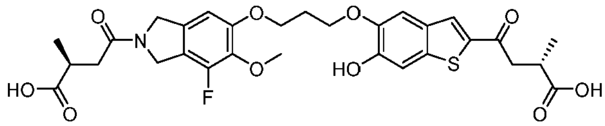 | 618 |
| 151. | (S)-4-(6-(3-((2-((S)-3-ca rboxy-butanoyl)-7-fluoro -6-hydroxy-benzo[b]thio pben-5-yl)oxy)propoxy) -4-fluoro-5-methoxyisoi ndolin-2-yl)-2-methyl-4-oxobuta-noic acid | 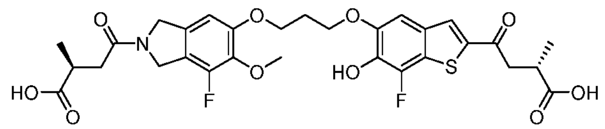 | 636 |
| 152. | (S)-4-(6-(3-((2-((S)-3-ca rboxy-butanoyl)-7-chloro -6-hydroxy-benzo[b]thio pben-5-yl)oxy)propoxy) -4-fluoro-5-methoxyisoi ndolin-2-yl)-2-methyl-4-oxobuta-noic acid | 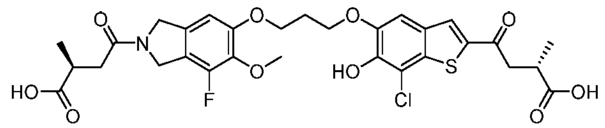 | 652 |
| 153. | (S)-4-(5-(3-((2-((S)-3-ca rboxy-butanoyl)-7-chloro -6-methoxyi-soindolin-5-yl)oxy)propoxy)-7-fluor o-6-hydroxybenzo[b]thi ophen-2-yl)-2-methyl-4-oxobuta-noic acid | 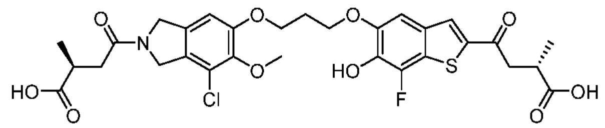 | 652 |
| 154. | (S)-4-(5-(3-((2-((S)-3-ca rboxybutanoyl)-4,7-diflu oro-6-methoxyisoindoli n-5-yl)oxy)propoxy)-7-c hloro-6-hydroxybenzo [b ]thiophen-2-yl)-2-methy l-4-oxobutanoic acid | 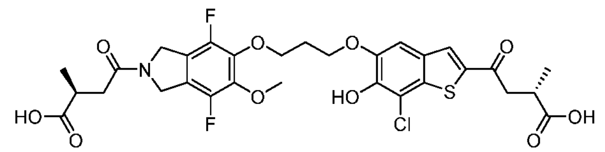 | 670 |
| 155. | (S)-4-(5-(3-((2-((S)-3-ca rboxybutanoyl)-4,7-diflu oro-6-methoxyisoindoli n-5-yl)oxy)propoxy)-7-f luoro-6-hydroxybenzo [b ]thiophen-2-yl)-2-methy l-4-oxobutanoic acid | 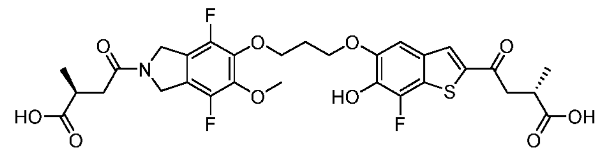 | 654 |

(continued)

| EX No. | Chemical name | Structure | MS: (M+H)+ & 1HNMR |
|---|---|---|---|
| 156. | (S)-4-(5-(3-((2-((S)-3-ca rboxybutanoyl)-4-chloro -7-fluoro-6-methoxyisoi ndolin-5-yl)oxy)propox y)-7-fluoro-6-hydroxybe nzo [b]thiophen-2-yl)-2-methyl-4-ox-obutanoic acid | 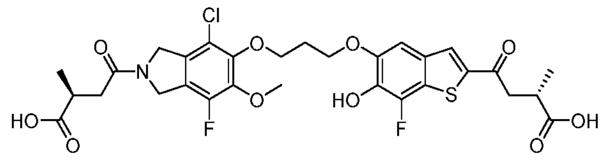 | 670 |
| 157. | (S)-4-(6-(3-((2-((S)-3-ca rboxybutanoyl)-4,7-diflu oro-6-hydroxybenzo[b]t hiophen-5-yl)oxy) propo xy)-4-fluoro-5-methoxyi soindolin-2-yl)-2-methyl -4-oxo-butanoic acid | 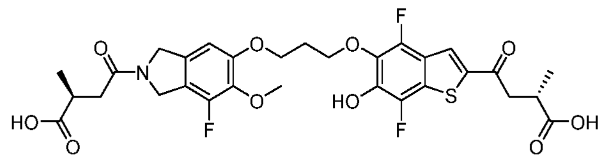 | 654 |
| 158. | (S)-4-(6-(3-((2-((S)-3-ca rboxybutanoyl)-7-chloro -4-fluoro-6-hydroxyben zo[b]thiophen-5-yl) oxy) propoxy)-4-fluoro-5-met hoxyisoindolin-2-yl)-2-methyl-4-oxobutanoic acid | 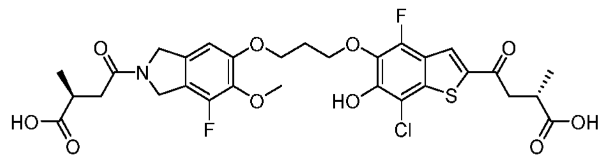 | 670 |
| 159. | (S)-4-(6-(3-((2-((S)-3-ca rboxybutanoyl)-4,7-dich loro-6-hydroxybenzo[b]t hiophen-5-yl)oxy) propo xy)-4-fluoro-5-methoxyi soindolin-2-yl)-2-methyl -4-oxo-butanoic acid | 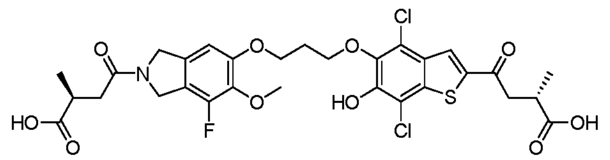 | 686 |
| 160. | (S)-4-(5-(3-((2-((S)-3-ca rboxybutanoyl)-5-metho xybenzo[b] thiophen-6-y 1)oxy)propoxy)-6-metho xyisoindolin-2-yl)-2-met hyl-4-oxobutanoic acid **(not part of the invention)** | 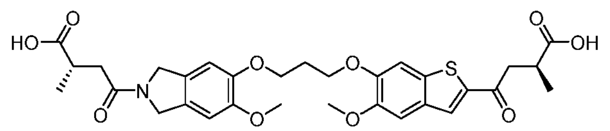 | 614 |
| 161. | 4-(5-(3-((2-(3-carboxypr opanoyl)-6-methoxyisoi ndolin-5-yl) oxy)propox y)-6-methoxythieno [2,3-b]pyridin-2-yl)-4-oxobut anoic acid **(not part of the invention)** | 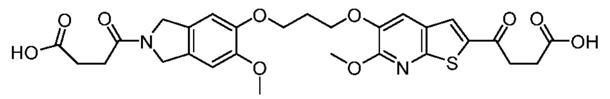 | 587 |
| 162. | 4-(5-(3-((2-(4-(((4R,5R) -5-hydroxy-1,2-dithian-4-yl)oxy)-4-oxobutanoyl )-6-methoxythieno [2,3-b ]pyridin-5-yl)oxy)propo xy)-6-methoxyisoindoli n-2-yl)-4-oxobutanoic acid **(not part of the invention)** | 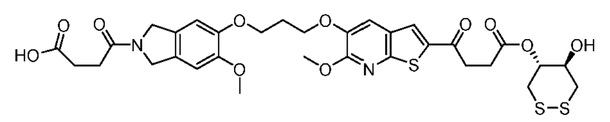 | 721 |

(continued)

| EX No. | Chemical name | Structure | MS: $(M+H)^+$ & $^1$HNMR |
|---|---|---|---|
| 163. | 4-(5-(3-((6-(3-carboxypr opanoyl)-3-methoxy-6,7 -dihydro-5H-pyrrolo[3,4 -b]pyridin-2-yl)oxy)pro poxy)-6-methoxybenzo[ b]thiophen-2-yl)-4-oxob uta-noic acid **(not part of the invention)** | 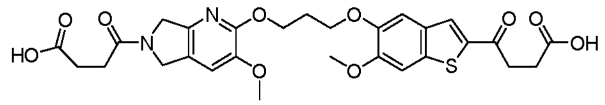 | 587 |
| 164. | 4-(2-(3-((2-(4-(((4R,5R) -5-hydroxy-1,2-dithian-4-yl)oxy)-4-oxobutanoyl )-6-methoxybenzo[b] thi ophen-5-yl)oxy)propoxy )-3-methoxy-5,7-dihydr o-6H-pyrrolo [3,4-b]pyri din-6-yl)-4-oxobuta-noic acid **(not part of the invention)** | 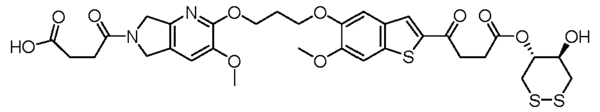 | 721 |
| 165. | 4-(5-(3-((6-(3-carboxypr opanoyl)-2-methoxy-6,7 -dihydro-5H-pyrrolo[3,4 -b]pyridin-3-yl)oxy)pro poxy)-6-methoxybenzo[ b]thiophen-2-yl)-4-oxob uta-noic acid **(not part of the invention)** | 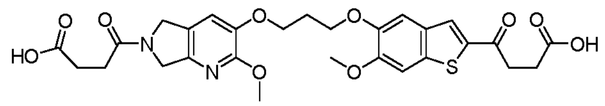 | 587 |
| 166. | 4-(3-(3-((2-(4-(((4R,5R) -5-hydroxy-1,2-dithian-4-yl)oxy)-4-oxobutanoyl )-6-methoxybenzo[b] thi ophen-5-yl)oxy)propoxy )-2-methoxy-5,7-dihydr o-6H-pyrrolo [3,4-b]pyri din-6-yl)-4-oxobuta-noic acid **(not part of the invention)** | 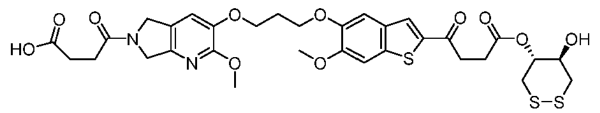 | 721 |
| 167. | 4-(2-(3-((2-(4-(((4R,5R) -5-hydroxy-1,2-dithian-4-yl)oxy)-4-oxobutanoyl )-6-methoxythieno [3,2-b ]pyridin-5-yl)oxy)propo xy)-3-methoxy-5,7-dihy dro-6H-pyrrolo[3,4-b]py ridin-6-yl)-4-ox-obutanoi c acid **(not part of the invention)** | 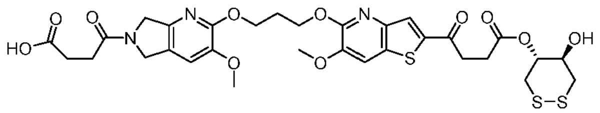 | 722 |
| 168. | 4-(5-(3-((2-(3-carboxypr opanoyl)-5-methoxythie no[2,3-b] pyridin-6-yl)ox y)propoxy)-6-methoxyis oindolin-2-yl)-4-oxo-but anoic acid **(not part of the invention)** | 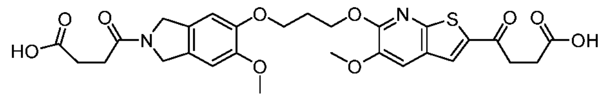 | 587 |

(continued)

| EX No. | Chemical name | Structure | MS: (M+H)+ & 1HNMR |
|---|---|---|---|
| 169. | (S)-4-(5-(3-((2-(3-carbo xypropanoyl)-5-methox ythieno[2,3-b] pyridin-6-yl)oxy)propoxy)-6-meth oxyisoindolin-2-yl)-2-m ethyl-4-oxobutanoic acid **(not part of the invention)** | 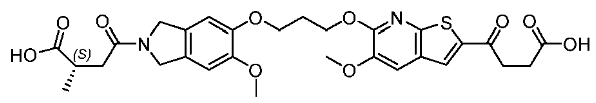 | 601 |
| 170. | (S)-4-(6-(3-((2-(3-carbo xypropanoyl)-6-methox yisoindolin-5-yl) oxy)pro poxy)-5-methoxythieno [ 2,3-b]pyridin-2-yl)-2-me thyl-4-oxobutanoic acid **(not part of the invention)** | 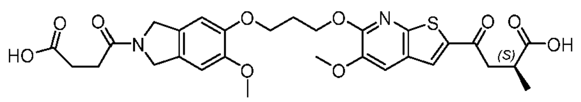 | 601 |
| 171. | (S)-4-(5-(3-((2-((S)-3-ca rboxybutanoyl)-5-metho xythieno[2,3-b]pyridin-6 -yl)oxy)propoxy)-6-met hoxyisoindolin-2-yl)-2-methyl-4-oxobutanoic acid **(not part of the invention)** | 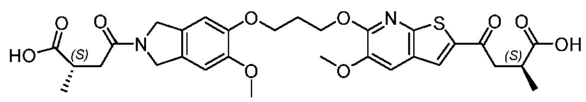 | 615 |
| 172. | 4-(5-(3-((2-(4-(((4R,5R) -5-hydroxy-1,2-dithian-4-yl)oxy)-4-oxobutanoyl )-5-methoxythieno [2,3-b ]pyridin-6-yl)oxy)propo xy)-6-methoxyisoindoli n-2-yl)-4-oxobutanoic acid**(not part of the invention)** | 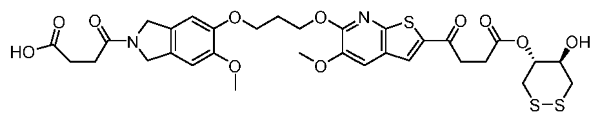 | 721 |

**EXAMPLE A: Human STING WT binding assay**

Cisbio Bioassays' human STING WT binding assay (#64BDSTGPEG & 64BDSTGPEH, Cisbio) is for quantitative measurement of human STING WT ligand using HTRF® technology.

1. **Adding compounds**

Negative control: Dispense 5 μL of diluent into each negative control well. Standard: Dispense 5 μL of each Human STING WT Standard 2'3'-cGAMP (Std 0 - Std 7) into each standard well. Compound: Dispense 5 μL of compound into each compound well.

2. **Adding proteins**

Negative control: Add 5 μL of detection buffer to all wells. Other wells: Add 5 μL of human STING WT protein 6His-tagged protein to all wells.

3. **Adding antibodies**

Add 10 μL of premixed STING WT ligand d2 reagent and 6His Tb antibody working solution to all wells.

4. **RT incubation**

Seal the plate and incubate 3 hours at RT or at Over Night if necessary.

5. **Reading plate**

Remove the plate sealer and read on an HTRF® compatible reader (PerkinElmer, USA). Results were analyzed with a two-wavelength signal ratio: intensity (665 nm)/intensity (620 nm).

6. **Curve fitting**

Calculate HTRF Ratio:

$$\text{Ratio} = \frac{\text{Signal 665 nm}}{\text{Signal 620 nm}} \times 10^4$$

Fit the data in GraphPad to obtain $IC_{50}$ values using equation (2)

$$\text{Equation (2): Y=Bottom + (Top-Bottom) / (1+10^{\wedge} ((LogIC}_{50}\text{-X)*Hill Slope))}$$

Y is HTRF Ratio and X is compound concentration.

$IC_{50}$ value of binding assay for human STING WT:

| Example | hSTING WT binding $IC_{50}$/nM |
|---|---|
| 1 | 0.06 |
| 2 | 11.37 |
| 3 | 8.40 |
| 4 | 0.18 |
| 5 | 0.32 |
| 6 | 0.36 |
| 7 | 1.04 |
| 8 | 0.65 |
| 9 | 2.23 |
| 10 | 0.28 |
| 11 | 2.54 |
| 13 | 12.84 |
| 14 | 46.60 |
| 15 | 92.57 |
| 16 | >150 |
| 17 | 0.19 |
| 18 | 4.59 |
| 19 | 54.62 |
| 20 | 0.19 |
| 21 | 158.60 |

The compounds of the present invention are preferably formulated as pharmaceutical compositions administered by a variety of routes. Most preferably, such compositions are for oral administration. Such pharmaceutical compositions and processes for preparing the same are well known in the art. See, e.g., REMINGTON: THE SCIENCE AND PRACTICE OF PHARMACY (A. Gennaro, et al, eds., 19th ed., Mack Publishing Co., 1995). The compounds of Formula I are generally effective over a wide dosage range.

For example, dosages per day normally fall within the range of about 0.2 mg to about 100 mg total daily dose, preferably 0.2 mg to 50 mg total daily dose, more preferably 0.2 mg to 20 mg total daily dose. In some instances dosage levels below the lower limit of the aforesaid range may be more than adequate, while in other cases still larger doses may be employed. It will be understood that the amount of the compound actually administered will be determined by a physician, in the light of the relevant circumstances, including the condition to be treated, the chosen route of administration, the actual compound or compounds administered, the age, weight, and response of the individual patient, and the severity of the patient's symptoms.

## Claims

1. A compound of Formula I-1 or a pharmaceutically acceptable salt thereof:

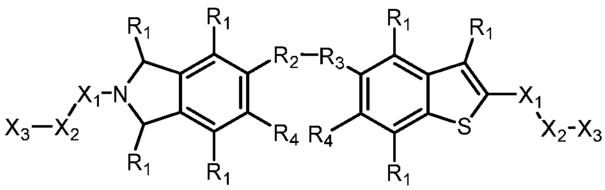

,

I-1

wherein

each $R_1$ is independently selected from H, deuterium, or halogen;
$R_2$ and $R_3$ are independently selected from the group consisting of O-($C_1$-$C_4$ alkylene), -$T_a$-$C_1$-$C_6$alkyl-$T_b$-, or -$T_a$-O-$T_b$, wherein the $C_1$-$C_6$alkyl is optionally substituted with one or more deuterium, or halo;
$T_a$ and $T_b$ each independently are absent, -O-, $C_1$-$C_6$ alkyl, -O-($C_1$-$C_6$ alkyl)-, or -($C_1$-$C_6$alkyl)-O-;
each $R_4$ is independently selected from the group consisting of H, deuterium, halogen, or $OR^6$;
each $R^6$ is independently selected from the group consisting of -H, deuterium, or $C_{1-6}$alkyl, and each of which is independently optionally substituted with deuterium, or -OH;
each $X_1$ is C=O;
each $X_2$ is independently selected from -$(C(R^8)_2)_{(1-3)}$, -$NR^8(C(R^8)_2)_{(1-3)}$, or -$NH(C(R^8)_2)_{(1-3)}$; wherein each $R^8$ is independently selected from the group consisting of H, deuterium, or $C_1$-$C_6$ alkyl; and
each $X_3$ is $COOR^6$, $SO_2R^6$,

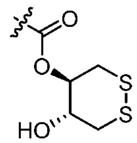

or CN.

2. The compound or pharmaceutically acceptable salt thereof of claim 1, wherein each $R_1$ is independently selected from the group consisting of H, deuterium, F, Cl, or Br.

3. The compound or pharmaceutically acceptable salt thereof of claims 1 or 2, wherein $R_2$-$R_3$ is selected from

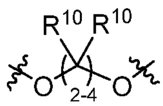

, or

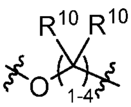

,

wherein:
each $R_{10}$ is independently hydrogen, or $C_{1-2}$ alkyl.

4. The compound or pharmaceutically acceptable salt thereof of claim 3, wherein $R^{10}$ is independently hydrogen, or

methyl.

**5.** The compound or pharmaceutically acceptable salt thereof of any one of claims 1-4, wherein $R_2$-$R_3$ is selected from $-O(CH_2)_2O-$, $-O(CH_2)_3O-$, $-O(CH_2)_4O-$, or $-OCH(CH_3)CH_2CH(CH_3)O-$.

**6.** The compound or pharmaceutically acceptable salt thereof of any one of claims 1-5, wherein each $R_4$ is independently selected from the group consisting of H, deuterium, Br, or $OR^6$.

**7.** The compound or pharmaceutically acceptable salt thereof any one of claims 1-6, wherein each $R_4$ is independently selected from the group consisting of H, deuterium, Br, OH, or $OC_1$-$C_3$ alkyl.

**8.** The compound or pharmaceutically acceptable salt thereof any one of claims 1-7, wherein each $R_4$ independently is selected from the group consisting of H, deuterium, Br, OH, or $OCH_3$.

**9.** The compound or pharmaceutically acceptable salt thereof of any one of claims 1-8, wherein each $X_2$ is $CH_2CHR^8$, wherein $R^8$ is selected from the group consisting of H, deuterium, or $C_1$-$C_3$ alkyl.

**10.** The compound or pharmaceutically acceptable salt thereof of any one of claims 1-9, wherein each $X_2$ is $-CH_2CH_2-$.

**11.** The compound or pharmaceutically acceptable salt thereof of any one of claims 1-10, wherein each $X_3$ is independently selected from COOH, or

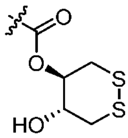
.

**12.** The compound or pharmaceutically acceptable salt thereof of any one of claims 1-11, wherein the compound is

| | |
|---|---|
| 1. | 4-(5-(3-((2-(3-carboxypropanoyl)-6-methoxybenzo[b]thiophen-5-yl)oxy)propoxy)-6 -methoxyisoindolin-2-yl)-4-oxobutanoic acid |
| 2. | 4-(5-(3-((2-(3-carboxypropanoyl)-4-chloro-6-methoxyisoindolin-5-yl)oxy)propoxy) -4-fluoro-6-methoxybenzo[b]thiophen-2-yl)-4-oxobutanoic acid |
| 3. | 4-(5-(3-((2-(3-carboxypropanoyl)-4-chloro-6-methoxybenzo[b]thiophen-5-yl)oxy)pr opoxy)-4-fluoro-6-methoxyisoindolin-2-yl)-4-oxobutanoic acid |
| 4. | 4-(5-(3-((2-(3-carboxypropanoyl)-4-fluoro-6-methoxybenzo[b]thiophen-5-yl)oxy)pr opoxv)-4-fluoro-6-methoxvisoindolin-2-yl)-4-oxobutanoic acid |
| 5. | sodium (S)-4-(5-(3-((2-((S)-3-carboxylatobutanoyl)-6-methoxybenzo[b]thiophen-5-yl)oxy)p ropoxy)-6-methoxyisoindolin-2-yl)-2-methyl-4-oxobutanoate |
| 6. | sodium (S)-4-(5-(3-((2-((S)-3-carboxylatobutanoyl)-4-fluoro-6-methoxybenzo[b]thiophen-5 -yl)oxy)propoxy)-6-methoxyisoindolin-2-yl)-2-methyl-4-oxobutanoate |
| 7. | sodium 4-(5-(3-((2-(3-carboxylatopropanoyl)-6-hydroxybenzo[b]thiophen-5-yl)oxy)propox y)-6-methoxyisoindolin-2-yl)-4-oxobutanoate |
| 9. | 4-(5-(3-((6-bromo-2-(3-carboxypropanoyl)benzo[b]thiophen-5-yl)oxy)propoxy)-6-methoxyisoindolin-2-yl)-4-oxobutanoic acid |
| 10. | 4-(5-(3-((2-(3-carboxypropanoyl)-4-fluoro-6-methoxyisoindolin-5-yl)oxy)propoxy)-6-methoxybenzo[b]thiophen-2-yl)-4-oxobutanoic acid |

(continued)

| | |
|---|---|
| 12. | 4-(5-(3-((2-(4-ethoxy-4-oxobutanoyl)-6-methoxybenzo[b]thiophen-5-yl)oxy)propox y)-6-methoxyisoindolin-2-yl)-4-oxobutanoic acid |
| 14. | 4-(5-(3-((2-(3-carboxypropanoyl)-4-chloro-6-methoxyisoindolin-5-yl)oxy)propoxy) -6-methoxvybenzo[b]thiophen-2-yl)-4-oxobutanoic acid |
| 17. | (S)-4-(5-(3-((2-((S)-3-carboxybutanoyl)-4-fluoro-6-methoxybenzo[b]thiophen-5-yl) oxy)propoxy)-4-fluoro-6-methoxyisoindolin-2-yl)-2-methyl-4-oxobutanoic acid |
| 18. | 4-(5-(3-((2-((2-carboxyethyl)carbamoyl)-6-methoxybenzo[b]thiophen-5-yl)oxy)pro poxy)-6-methoxyisoindolin-2-yl)-4-oxobutanoic acid |
| 20. | 4-(5-(3-((2-(3-carboxypropanoyl)-4-fluoro-6-methoxybenzo[b]thiophen-5-yl)oxy)pr opoxy)-6-methoxyisoindolin-2-yl)-4-oxobutanoic acid |
| 22. | 4-(5-(3-((2-(3-carboxybutanoyl)-6-methoxybenzo[b]thiophen-5-yl)oxy)propoxy)-6-methoxyisoindolin-2-yl)-2-methyl-4-oxobutanoic acid |
| 23. | (S)-4-(5-(3-((2-((S)-3-carboxybutanoyl)-6-methoxybenzo[b]thiophen-5-yl)oxy)prop oxy)-6-methoxyisoindolin-2-yl)-2-methyl-4-oxobutanoic acid |
| 24. | (S)-4-(5-(3-((2-(3-carboxypropanoyl)-6-methoxyisoindolin-5-yl)oxy)propoxy)-6-me thoxybenzo[b]thiophen-2-yl)-2-methyl-4-oxobutanoic acid |
| 25. | (S)-4-(5-(3-((2-(3-carboxypropanoyl)-6-methoxybenzo[b]thiophen-5-yl)oxy)propox y)-6-methoxyisoindolin-2-yl)-2-methyl-4-oxobutanoic acid |
| 26. | (S)-4-(5-(2-((2-((S)-3-carboxybutanoyl)-6-methoxybenzo[b]thiophen-5-yl)oxy)etho xy)-6-methoxyisoindolin-2-yl)-2-methyl-4-oxobutanoic acid |
| 27. | (S)-4-(5-(4-((2-((S)-3-carboxybutanoyl)-6-methoxybenzo[b]thiophen-5-yl)oxy)buto xy)-6-methoxyisoindolin-2-yl)-2-methyl-4-oxobutanoic acid |
| 28. | (S)-4-(5-((5-((2-((S)-3-carboxybutanoyl)-6-methoxybenzo[b]thiophen-5-yl)oxy)pen tyl)oxy)-6-methoxyisoindolin-2-yl)-2-methyl-4-oxobutanoic acid |
| 34. | (2S)-4-(5-((4-((2-((S)-3-carboxybutanoyl)-6-methoxybenzo[b]thiophen-5-yl)oxy)pe ntan-2-yl)oxy)-6-methoxyisoindolin-2-yl)-2-methyl-4-oxobutanoic acid |
| 35. | (S)-4-(5-(3-((2-((S)-3-carboxybutanoyl)-6-methoxybenzo[b]thiophen-5-yl)oxy)-2,2-dimethylpropoxy)-6-methoxyisoindolin-2-yl)-2-methyl-4-oxobutanoic acid |
| 37. | 4-(5-(3-((2-(3-carboxypropanoyl)-6-methoxyisoindolin-5-yl)oxy)propoxy)-6-metho xybenzo[b]thiophen-2-yl)-2,2-dimethyl-4-oxobutanoic acid |
| 38. | 4-(5-(3-((2-(3-carboxypropanoyl)-6-methoxybenzo[b]thiophen-5-yl)oxy)propoxy)-6 -methoxyisoindolin-2-yl)-2,2-dimethyl-4-oxobutanoic acid |
| 39. | 4-(5-(3-((2-(3-carboxy-3-methylbutanoyl)-6-methoxybenzo[b]thiophen-5-yl)oxy)pr opoxy)-6-methoxyisoindolin-2-yl)-2,2-dimethyl-4-oxobutanoic acid |
| 43. | (S)-4-(5-(3-((2-((S)-3-carboxybutanoyl)-4-fluoro-6-methoxybenzo[b]thiophen-5-yl) oxy)propoxy)-6-methoxyisoindolin-2-yl)-2-methyl-4-oxobutanoic acid |
| 44. | (S)-4-(5-(3-((2-((S)-3-carboxybutanoyl)-4-chloro-6-methoxybenzo[b]thiophen-5-yl) oxy)propoxy)-6-methoxyisoindolin-2-yl)-2-methyl-4-oxobutanoic acid |
| 45. | (S)-4-(5-(3-((4-bromo-2-((S)-3-carboxybutanoyl)-6-methoxybenzo[b]thiophen-5-yl) oxy)propoxy)-6-methoxyisoindolin-2-yl)-2-methyl-4-oxobutanoic acid |
| 47. | (S)-4-(5-(3-((2-((S)-3-carboxybutanoyl)-6-hydroxybenzo[b]thiophen-5-yl)oxy)prop oxy)-6-methoxyisoindolin-2-yl)-2-methyl-4-oxobutanoic acid |
| 48. | (S)-4-(5-(3-((2-((S)-3-carboxybutanoyl)-4,7-difluoro-6-methoxybenzo[b]thiophen-5 -yl)oxy)propoxy)-6-methoxyisoindolin-2-yl)-2-methyl-4-oxobutanoic acid |
| 49. | (S)-4-(5-(3-((2-((S)-3-carboxybutanoyl)-4,7-dichloro-6-methoxybenzo[b]thiophen-5 -yl)oxy)propoxy)-6-methoxyisoindolin-2-yl)-2-methyl-4-oxobutanoic acid |

(continued)

| | |
|---|---|
| 51. | (S)-4-(5-(3-((2-((S)-3-carboxybutanoyl)-4-fluoro-6-methoxyisoindolin-5-yl)oxy)pro poxy)-6-methoxybenzo[b]thiophen-2-yl)-2-methyl-4-oxobutanoic acid |
| 52. | (S)-4-(5-(3-((2-((S)-3-carboxybutanoyl)-4-chloro-6-methoxyisoindolin-5-yl)oxy)pro poxy)-6-methoxybenzo[b]thiophen-2-yl)-2-methyl-4-oxobutanoic acid |
| 53. | (S)-4-(5-(3-((4-bromo-2-((S)-3-carboxybutanoyl)-6-methoxyisoindolin-5-yl)oxy)pr opoxy)-6-methoxybenzo[b]thiophen-2-yl)-2-methyl-4-oxobutanoic acid |
| 58. | (S)-4-(5-(3-((2-((S)-3-carboxybutanoyl)-4,7-difluoro-6-methoxyisoindolin-5-yl)oxy )propoxy)-6-methoxybenzo[b]thiophen-2-yl)-2-methyl-4-oxobutanoic acid |
| 59. | (S)-4-(5-(3-((2-((S)-3-carboxybutanoyl)-6-methoxybenzo[b]thiophen-5-yl)oxy)prop oxy)-7-chloro-4-fluoro-6-methoxyisoindolin-2-yl)-2-methyl-4-oxobutanoic acid |
| 60. | (S)-4-(5-(3-((2-((S)-3-carboxybutanoyl)-4,7-dichloro-6-methoxyisoindolin-5-yl)oxy )propoxy)-6-methoxybenzo[b]thiophen-2-yl)-2-methyl-4-oxobutanoic acid |
| 61. | (S)-4-(5-(3-((2-((S)-3-carboxybutanoyl)-4-chloro-6-methoxybenzo[b]thiophen-5-yl) oxy)propoxy)-4-fluoro-6-methoxyisoindolin-2-yl)-2-methyl-4-oxobutanoic acid |
| 62. | (S)-4-(5-(3-((2-((S)-3-carboxybutanoyl)-4-chloro-6-methoxyisoindolin-5-yl)oxy)pro poxy)-4-fluoro-6-methoxybenzo[b]thiophen-2-yl)-2-methyl-4-oxobutanoic acid |
| 63. | (S)-4-(5-(3-((2-((S)-3-carboxybutanoyl)-4-chloro-6-methoxybenzo[b]thiophen-5-yl) oxy)propoxy)-4-chloro-6-methoxyisoindolin-2-yl)-2-methyl-4-oxobutanoic acid |
| 67. | (S)-4-(5-(3-((2-((S)-3-carboxybutanoyl)-4,7-difluoro-6-methoxyisoindolin-5-yl)oxy )propoxy)-7-chloro-6-methoxybenzo[b]thiophen-2-yl)-2-methyl-4-oxobutanoic acid |
| 68. | (S)-4-(5-(3-((2-((S)-3-carboxybutanoyl)-4,7-difluoro-6-methoxyisoindolin-5-yl)oxy )propoxy)-7-fluoro-6-methoxybenzo[b]thiophen-2-yl)-2-methyl-4-oxobutanoic acid |
| 69. | (S)-4-(5-(3-((2-((S)-3-carboxybutanoyl)-4-chloro-7-fluoro-6-methoxyisoindolin-5-y l)oxy)propoxy)-7-fluoro-6-methoxybenzo[b]thiophen-2-yl)-2-methyl-4-oxobutanoic acid |
| 70. | (S)-4-(6-(3-((2-((S)-3-carboxybutanoyl)-4,7-difluoro-6-methoxybenzo[b]thiophen-5 -yl)oxy)propoxy)-4-fluoro-5-methoxyisoindolin-2-yl)-2-methyl-4-oxobutanoic acid |
| 71. | (S)-4-(6-(3-((2-((S)-3-carboxybutanoyl)-7-chloro-4-fluoro-6-methoxybenzo[b]thiop hen-5-yl)oxy)propoxy)-4-fluoro-5-methoxyisoindolin-2-yl)-2-methyl-4-oxobutanoic acid |
| 72. | (S)-4-(6-(3-((2-((S)-3-carboxybutanoyl)-4,7-dichloro-6-methoxybenzo[b]thiophen-5 -yl)oxy)propoxy)-4-fluoro-5-methoxyisoindolin-2-yl)-2-methyl-4-oxobutanoic acid |
| 73. | (S)-4-(5-(3-((2-((S)-3-carboxybutanoy1)-4-chloro-7-fluoro-6-methoxyisoindolin-5-y 1)oxy)propoxy)-7-chloro-6-methoxybenzo[b]thiophen-2-yl)-2-methyl-4-oxobutanoi c acid |
| 74. | (S)-4-(5-(3-((2-((S)-3-carboxybutanoyl)-4,7-dichloro-6-methoxyisoindolin-5-yl)oxy )propoxy)-7-fluoro-6-methoxybenzo[b]thiophen-2-yl)-2-methyl-4-oxobutanoic acid |
| 75. | (S)-4-(5-(3-((2-((S)-3-carboxybutanoyl)-7-chloro-4-fluoro-6-methoxyisoindolin-5-y l)oxy)propoxy)-7-fluoro-6-methoxybenzo[b]thiophen-2-yl)-2-methyl-4-oxobutanoic acid |
| 76. | (S)-4-(6-(3-((2-((S)-3-carboxybutanoyl)-4,7-difluoro-6-methoxybenzo[b]thiophen-5 -yl)oxy)propoxy)-4-chloro-5-methoxyisoindolin-2-yl)-2-methyl-4-oxobutanoic acid |
| 77. | (S)-4-(6-(3-((2-((S)-3-carboxybutanoyl)-4-chloro-7-fluoro-6-methoxybenzo[b]thiop hen-5-yl)oxy)propoxy)-4-chloro-5-methoxyisoindolin-2-yl)-2-methyl-4-oxobutanoi c acid |
| 78. | (S)-4-(5-(3-((2-((S)-3-carboxybutanoyl)-4-fluoro-6-hydroxyisoindolin-5-yl)oxy)pro poxy)-4-fluoro-6-methoxybenzo[b]thiophen-2-yl)-2-methyl-4-oxobutanoic acid |
| 79. | (S)-4-(5-(3-((2-((S)-3-carboxybutanoyl)-4-chloro-6-methoxybenzo[b]thiophen-5-yl) oxy)propoxy)-4-fluoro-6-hydroxyisoindolin-2-yl)-2-methyl-4-oxobutanoic acid |
| 80. | (S)-4-(5-(3-((2-((S)-3-carboxybutanoyl)-4-chloro-6-hydroxyisoindolin-5-yl)oxy)pro poxy)-4-fluoro-6-methoxybenzo[b]thiophen-2-yl)-2-methyl-4-oxobutanoic acid |

(continued)

| | |
|---|---|
| 81. | (S)-4-(5-(3-((2-((S)-3-carboxybutanoyl)-4-chloro-6-hydroxyisoindolin-5-yl)oxy)pro poxy)-4-chloro-6-methoxybenzo[b]thiophen-2-yl)-2-methyl-4-oxobutanoic acid |
| 86. | (S)-4-(5-(3-((2-((S)-3-carboxybutanoyl)-4,7-difluoro-6-methoxyisoindolin-5-yl)oxy )propoxy)-4-chloro-7-fluoro-6-methoxybenzo[b]thiophen-2-yl)-2-methyl-4-oxobuta noic acid |
| 87. | (S)-4-(5-(3-((2-((S)-3-carboxybutanoyl)-4-chloro-7-fluoro-6-methoxybenzo[b]thiop hen-5-yl)oxy)propoxy)-4-chloro-7-fluoro-6-methoxyisoindolin-2-yl)-2-methyl-4-ox obutanoic acid |
| 88. | (S)-4-(5-(3-((2-((S)-3-carboxybutanoyl)-4,7-difluoro-6-methoxybenzo[b]thiophen-5 -yl)oxy)propoxy)-4,7-difluoro-6-methoxyisoindolin-2-yl)-2-methyl-4-oxobutanoic acid |
| 89. | (S)-4-(5-(3-((2-((S)-3-carboxybutanoyl)-4,7-difluoro-6-methoxyisoindolin-5-yl)oxy )propoxy)-7-chloro-4-fluoro-6-methoxybenzo[b]thiophen-2-yl)-2-methyl-4-oxobuta noic acid |
| 90. | (S)-4-(5-(3-((2-((S)-3-carboxybutanoyl)-4,7-dichloro-6-methoxybenzo[b]thiophen-5 -yl)oxy)propoxy)-4,7-difluoro-6-methoxyisoindolin-2-yl)-2-methyl-4-oxobutanoic acid |
| 91. | (S)-4-(5-(3-((2-((S)-3-carboxybutanoyl)-4,7-dichloro-6-methoxybenzo[b]thiophen-5 -yl)oxy)propoxy)-4-chloro-7-fluoro-6-methoxyisoindolin-2-yl)-2-methyl-4-oxobuta noic acid |
| 92. | (S)-4-(5-(3-((2-((S)-3-carboxybutanoy1)-4-chloro-7-fluoro-6-methoxyisoindolin-5-y l)oxy)propoxy)-7-chloro-4-fluoro-6-methoxybenzo[b]thiophen-2-yl)-2-methyl-4-ox obutanoic acid |
| 93. | (S)-4-(5-(3-((2-((S)-3-carboxybutanoyl)-4,7-dichloro-6-methoxyisoindolin-5-yl)oxy )propoxy)-4-chloro-7-fluoro-6-methoxybenzo[b]thiophen-2-yl)-2-methyl-4-oxobuta noic acid |
| 94. | (S)-4-(5-(3-((2-((S)-3-carboxybutanoyl)-4-chloro-7-fluoro-6-methoxybenzo[b]thiop hen-5-yl)oxy)propoxy)-7-chloro-4-fluoro-6-methoxyisoindolin-2-yl)-2-methyl-4-ox obutanoic acid |
| 104. | (1R,2R)-2-(5-(3-((2-((S)-3-carboxybutanoyl)-6-methoxyisoindolin-5-yl)oxy)propox y)-6-methoxybenzo [b]thiophene-2-carbonyl)cyclopropane-1-carboxylic acid |
| 105. | (1R,2R)-2-(5-(3-((2-((1R,2R)-2-carboxycyclopropane- 1 -carbonyl)-6-methoxybenzo [b]thiophen-5-yl) oxy)propoxy)-6-methoxyisoindoline-2-carbonyl)cyclopropane-1-c arboxylic acid |
| 106. | (1R,2R)-2-(5-(3-((2-((S)-3-carboxybutanoyl)-6-methoxybenzo[b]thiophen-5-yl)oxy) propoxy)-6-methoxyisoindoline-2-carbonyl)cyclopropane-1-carboxylic acid |
| 111. | (S)-4-(4-(3-((2-((S)-3-carboxybutanoyl)-6-methoxybenzo[b]thiophen-5-yl)oxy)prop oxy)-6-methoxyisoindolin-2-yl)-2-methyl-4-oxobutanoic acid |
| 112. | (S)-4-(5-(3-((2-((S)-3-carboxybutanoyl)-6-methoxybenzo[b]thiophen-4-yl)oxy)prop oxy)-6-methoxyisoindolin-2-yl)-2-methyl-4-oxobutanoic acid |
| 123. | (S)-4-(5-(3-((6-bromo-2-((S)-3-carboxybutanoyl)benzo[b]thiophen-5-yl)oxy)propox y)-6-methoxyisoindolin-2-yl)-2-methyl-4-oxobutanoic acid |
| 130. | (S)-4-(5-(3-((6-bromo-2-((S)-3-carboxybutanoyl)isoindolin-5-yl)oxy)propoxy)-6-m ethoxybenzo[b]thiophen-2-yl)-2-methyl-4-oxobutanoic acid |
| 146. | (S)-4-(5-(3-((2-((S)-3-carboxybutanoyl)-4-fluoro-6-hydroxybenzo[b]thiophen-5-yl) oxy)propoxy)-4-fluoro-6-methoxyisoindolin-2-yl)-2-methyl-4-oxobutanoic acid |
| 147. | (S)-4-(5-(3-((2-((S)-3-carboxybutanoyl)-4-chloro-6-hydroxybenzo[b]thiophen-5-yl) oxy)propoxy)-4-fluoro-6-methoxyisoindolin-2-yl)-2-methyl-4-oxobutanoic acid |
| 148. | (S)-4-(5-(3-((2-((S)-3-carboxybutanoyl)-4-chloro-6-methoxyisoindolin-5-yl)oxy)pro poxy)-4-fluoro-6-hydroxybenzo[b]thiophen-2-yl)-2-methyl-4-oxobutanoic acid |
| 149. | (S)-4-(5-(3-((2-((S)-3-carboxybutanoyl)-4-chloro-6-hydroxybenzo[b]thiophen-5-yl) oxy)propoxy)-4-chloro-6-methoxyisoindolin-2-yl)-2-methyl-4-oxobutanoic acid |
| 150. | (S)-4-(6-(3-((2-((S)-3-carboxybutanoyl)-6-hydroxybenzo[b]thiophen-5-yl)oxy)prop oxy)-4-fluoro-5-methoxyisoindolin-2-yl)-2-methyl-4-oxobutanoic acid |
| 151. | (S)-4-(6-(3-((2-((S)-3-carboxybutanoyl)-7-fluoro-6-hydroxybenzo[b]thiophen-5-yl) oxy)propoxy)-4-fluoro-5-methoxyisoindolin-2-yl)-2-methyl-4-oxobutanoic acid |

(continued)

| | |
|---|---|
| 152. | (S)-4-(6-(3-((2-((S)-3-carboxybutanoyl)-7-chloro-6-hydroxybenzo[b]thiophen-5-yl) oxy)propoxy)-4-fluoro-5-methoxyisoindolin-2-yl)-2-methyl-4-oxobutanoic acid |
| 153. | (S)-4-(5-(3-((2-((S)-3-carboxybutanoyl)-7-chloro-6-methoxyisoindolin-5-yl)oxy)pro poxy)-7-fluoro-6-hydroxybenzo[b]thiophen-2-yl)-2-methyl-4-oxobutanoic acid |
| 154. | (S)-4-(5-(3-((2-((S)-3-carboxybutanoyl)-4,7-difluoro-6-methoxyisoindolin-5-yl)oxy )propoxy)-7-chloro-6-hydroxybenzo[b]thiophen-2-yl)-2-methyl-4-oxobutanoic acid |
| 155. | (S)-4-(5-(3-((2-((S)-3-carboxybutanoyl)-4,7-difluoro-6-methoxyisoindolin-5-yl)oxy )propoxy)-7-fluoro-6-hydroxybenzo[b]thiophen-2-yl)-2-methyl-4-oxobutanoic acid |
| 156. | (S)-4-(5-(3-((2-((S)-3-carboxybutanoyl)-4-chloro-7-fluoro-6-methoxyisoindolin-5-y 1)oxy)propoxy)-7-fluoro-6-hydroxybenzo[b]thiophen-2-yl)-2-methyl-4-oxobutanoic acid |
| 157. | (S)-4-(6-(3-((2-((S)-3-carboxybutanoyl)-4,7-difluoro-6-hydroxybenzo[b]thiophen-5-yl)oxy)propoxy)-4-fluoro-5-methoxyisoindolin-2-yl)-2-methyl-4-oxobutanoic acid |
| 158. | (S)-4-(6-(3-((2-((S)-3-carboxybutanoyl)-7-chloro-4-fluoro-6-hydroxybenzo[b]thiop hen-5-yl)oxy)propoxy)-4-fluoro-5-methoxyisoindolin-2-yl)-2-methyl-4-oxobutanoic acid |
| 159. | (S)-4-(6-(3-((2-((S)-3-carboxybutanoyl)-4,7-dichloro-6-hydroxybenzo[b]thiophen-5 -yl)oxy)propoxy)-4-fluoro-5-methoxyisoindolin-2-yl)-2-methyl-4-oxobutanoic acid. |

**13.** The compound or a pharmaceutically acceptable salt thereof of any one of claims 1-12, wherein the compound is

| | |
|---|---|
| 1. | 4-(5-(3-((2-(3-carboxypropanoyl)-6-methoxybenzo[b]thiophen-5-yl)oxy)propo xy)-6-methoxyisoindolin-2-yl)-4-oxobutanoic acid |
| 2. | 4-(5-(3-((2-(3-carboxypropanoyl)-4-chloro-6-methoxyisoindolin-5-yl)oxy)prop oxy)-4-fluoro-6-methoxybenzo[b]thiophen-2-yl)-4-oxobutanoic acid |
| 3. | 4-(5-(3-((2-(3-carboxypropanoyl)-4-chloro-6-methoxybenzo[b]thiophen-5-yl)o xy)propoxy)-4-fluoro-6-methoxyisoindolin-2-yl)-4-oxobutanoic acid |
| 4. | 4-(5-(3-((2-(3-carboxypropanoyl)-4-fluoro-6-methoxybenzo[b]thiophen-5-yl)o xy)propoxy)-4-fluoro-6-methoxyisoindolin-2-yl)-4-oxobutanoic acid |
| 5. | sodium (S)-4-(5-(3-((2-((S)-3-carboxylatobutanoyl)-6-methoxybenzo[b]thiophen-5-yl)oxy)p ropoxy)-6-methoxyisoindolin-2-yl)-2-methyl-4-oxobutanoate |
| 6. | sodium (S)-4-(5-(3-((2-((S)-3-carboxylatobutanoyl)-4-fluoro-6-methoxybenzo[b]thiophen-5 -yl)oxy)propoxy)-6-methoxyisoindolin-2-yl)-2-methyl-4-oxobutanoate |
| 7. | sodium 4-(5-(3-((2-(3-carboxylatopropanoyl)-6-hydroxybenzo[b]thiophen-5-yl)oxy)propox y)-6-methoxyisoindolin-2-yl)-4-oxobutanoate |
| 9. | 4-(5-(3-((6-bromo-2-(3-carboxypropanoyl)benzo[b]thiophen-5-yl)oxy)propoxy )-6-methoxyisoindolin-2-yl)-4-oxobutanoic acid |
| 10. | 4-(5-(3-((2-(3-carboxypropanoyl)-4-fluoro-6-methoxyisoindolin-5-yl)oxy)prop oxy)-6-methoxybenzo[b]thiophen-2-yl)-4-oxobutanoic acid |
| 12. | 4-(5-(3-((2-(4-ethoxy-4-oxobutanoyl)-6-methoxybenzo[b]thiophen-5-yl)oxy)pr opoxy)-6-methoxyisoindolin-2-yl)-4-oxobutanoic acid |
| 14. | 4-(5-(3-((2-(3-carboxypropanoyl)-4-chloro-6-methoxyisoindolin-5-yl)oxy)prop oxy)-6-methoxybenzo[b]thiophen-2-yl)-4-oxobutanoic acid |
| 17. | (S)-4-(5-(3-((2-((S)-3-carboxybutanoyl)-4-fluoro-6-methoxybenzo[b]thiophen-5-yl)oxy)propoxy)-4-fluoro-6-methoxyisoindolin-2-yl)-2-methyl-4-oxobutanoic acid |

(continued)

| | |
|---|---|
| 18. | 4-(5-(3-((2-((2-carboxyethyl)carbamoyl)-6-methoxybenzo[b]thiophen-5-yl)oxy )propoxy)-6-methoxyisoindolin-2-yl)-4-oxobutanoic acid |
| 20. | 4-(5-(3-((2-(3-carboxypropanoyl)-4-fluoro-6-methoxybenzo[b]thiophen-5-yl)o xy)propoxy)-6-methoxyisoindolin-2-yl)-4-oxobutanoic acid. |

**14.** A pharmaceutical composition comprising a therapeutically effective amount of at least one compound or pharmaceutically acceptable salt thereof of any one of claims 1-13 and at least one pharmaceutically acceptable excipient.

**15.** At least one compound or pharmaceutically acceptable salt thereof for use as defined in any one of claims 1-13 and/or a pharmaceutical composition of claim 14, which is for use in therapy; or, for use in inducing an immune response in a subject; or, for use in inducing STING-dependent type I interferon production in a subject; or, for use in inducing a STING-dependent cytokine production in a subject; or, for use in treating a cell proliferation disorder in a subject, wherein the cell proliferation disorder is cancer; or, for use as an STING agonists; or, for use as a medicament.

**Patentansprüche**

**1.** Verbindung von Formel I-1 oder pharmazeutisch verträgliches Salz davon:

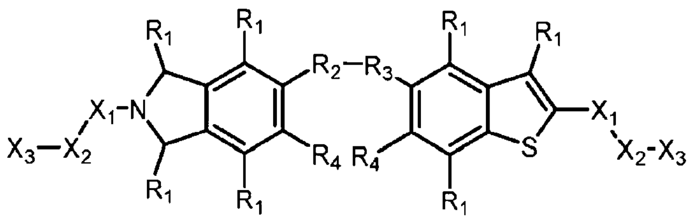
,

I-1

wobei

jedes $R_1$ unabhängig aus H, Deuterium oder Halogen ausgewählt ist;
$R_2$ und $R_3$ unabhängig aus der Gruppe ausgewählt sind, bestehend aus O-($C_1$-$C_4$ Alkylen), -$T_a$-$C_1$-$C_6$-Alkyl-$T_b$-, oder -$T_a$-O-$T_b$, wobei das $C_1$-$C_6$-Alkyl optional mit einem oder mehreren Deuterium oder Halo substituiert ist;
$T_a$ und $T_b$ jeweils unabhängig abwesend, -O-, $C_1$-$C_6$-Alkyl, -O-($C_1$-$C_6$-Alkyl)- oder -($C_1$-$C_6$-Alkyl)-O- sind;
jedes $R_4$ unabhängig aus der Gruppe ausgewählt ist, bestehend aus H, Deuterium, Halogen oder $OR^6$;
jedes $R^6$ unabhängig aus der Gruppe ausgewählt ist, bestehend aus -H, Deuterium oder $C_{1-6}$-Alkyl, und jedes davon unabhängig optional mit Deuterium oder -OH substituiert ist;
jedes $X_1$ C=O ist;
jedes $X_2$ unabhängig aus $-(C(R^8)_2)_{(1-3)}$, $-NR^8(C(R^8)_2)_{(1-3)}$ oder $-NH(C(R^8)_2)_{(1-3)}$ ausgewählt ist; wobei jedes $R^8$ unabhängig aus der Gruppe ausgewählt ist, bestehend aus H, Deuterium oder $C_1$-$C_6$-Alkyl; und
jedes $X_3$ $COOR^6$, $SO_2R^6$,

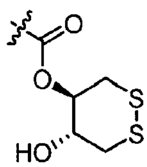
(

oder CN ist.

**2.** Verbindung oder pharmazeutisch verträgliches Salz davon nach Anspruch 1, wobei jedes $R_1$ unabhängig aus der Gruppe ausgewählt ist, bestehend aus H, Deuterium, F, Cl oder Br.

**3.** Verbindung oder pharmazeutisch verträgliches Salz davon nach Anspruch 1 oder 2, wobei $R_2$-$R_3$ aus

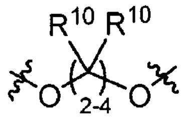

oder

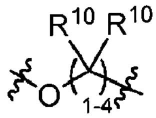

ausgewählt ist, wobei:
jedes $R_{10}$ unabhängig Wasserstoff oder $C_{1-2}$-Alkyl ist.

**4.** Verbindung oder pharmazeutisch verträgliches Salz davon nach Anspruch 3, wobei $R^{10}$ unabhängig Wasserstoff oder Methyl ist.

**5.** Verbindung oder pharmazeutisch verträgliches Salz davon nach einem der Ansprüche 1 bis 4, wobei $R_2$-$R_3$ aus $-O(CH_2)_2O-$, $-O(CH_2)_3O-$, $-O(CH_2)_4O-$ oder $-OCH(CH_3)CH_2CH(CH_3)O-$ ausgewählt ist.

**6.** Verbindung oder pharmazeutisch verträgliches Salz davon nach einem der Ansprüche 1 bis 5, wobei jedes $R_4$ unabhängig aus der Gruppe ausgewählt ist, bestehend aus H, Deuterium, Br oder $OR^6$.

**7.** Verbindung oder pharmazeutisch verträgliches Salz davon nach einem der Ansprüche 1 bis 6, wobei jedes $R_4$ unabhängig aus der Gruppe ausgewählt ist, bestehend aus H, Deuterium, Br, OH oder $OC_1$-$C_3$-Alkyl.

**8.** Verbindung oder pharmazeutisch verträgliches Salz davon nach einem der Ansprüche 1 bis 7, wobei jedes $R_4$ unabhängig aus der Gruppe ausgewählt ist, bestehend aus H, Deuterium, Br, OH oder $OCH_3$.

**9.** Verbindung oder pharmazeutisch verträgliches Salz davon nach einem der Ansprüche 1 bis 8, wobei jedes $X_2$ $CH_2CHR^8$ ist, wobei $R^8$ aus der Gruppe ausgewählt ist, bestehend aus H, Deuterium oder $C_1$-$C_3$-Alkyl.

**10.** Verbindung oder pharmazeutisch verträgliches Salz davon nach einem der Ansprüche 1 bis 9, wobei jedes $X_2$ $-CH_2CH_2-$ ist.

**11.** Verbindung oder pharmazeutisch verträgliches Salz davon nach einem der Ansprüche 1 bis 10, wobei jedes $X_3$ unabhängig aus COOH oder

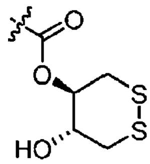

ausgewählt ist.

**12.** Verbindung oder pharmazeutisch verträgliches Salz davon nach einem der Ansprüche 1 bis 11, wobei die Verbindung ist

| | |
|---|---|
| 1. | 4-(5-(3-((2-(3-Carboxypropanoyl)-6-methoxybenzo[b]thiophen-5-yl)oxy)propoxy)-6-methoxyisoindolin-2-yl)-4-oxobutansaure |
| 2. | 4-(5-(3-((2-(3-Carboxypropanoyl)-4-chlor-6-methoxyisoindolin-5-yl)oxy)propoxy)-4-fluor-6-methoxybenzo[b]thiophen-2-yl)-4-oxobutansäure |
| 3. | 4-(5-(3-((2-(3-Carboxypropanoyl)-4-chlor-6-methoxybenzo[b]thiophen-5-yl)oxy)propoxy)-4-fluor-6-methoxyisoindolin-2-yl)-4-oxobutansaure |

(continued)

| | |
|---|---|
| 4. | 4-(5-(3-((2-(3-Carboxypropanoyl)-4-fluor-6-methoxybenzo[b]thiophen-5-yl)oxy)propoxy)-4-fluor-6-methoxyisoindolin-2-yl)-4-oxobutansaure |
| 5. | Natrium (S)-4-(5-(3-((2-((S)-3-Carboxylatobutanoyl)-6-methoxybenzo[b]thiophen-5-yl)oxy)propoxy)-6-methoxyisoindolin-2-yl)-2-methyl-4-oxobutanoat |
| 6. | Natrium (S)-4-(5-(3-((2-((S)-3-Carboxylatobutanoyl)-4-fluor-6-methoxybenzo[b]thiophen-5-yl)oxy)propoxy)-6-methoxyisoindolin-2-yl)-2-methyl-4-oxobutanoat |
| 7. | Natrium 4-(5-(3-((2-(3-Carboxylatpropanoyl)-6-hydroxybenzo[b]thiophen-5-yl)oxy)propoxy)-6-methoxyisoindolin-2-yl)-4-oxobutanoat |
| 9. | 4-(5-(3-((6-Brom-2-(3-carboxypropanoyl)benzo[b]thiophen-5-yl)oxy)propoxy)-6-methoxyisoindolin-2-yl)-4-oxobutansaure |
| 10. | 4-(5-(3-((2-(3-Carboxypropanoyl)-4-fluor-6-methoxyisoindolin-5-yl)oxy)propoxy)-6-methoxybenzo[b]thiophen-2-yl)-4-oxobutansaure |
| 12. | 4-(5-(3-((2-(4-Ethoxy-4-oxobutanoyl)-6-methoxybenzo[b]thiophen-5-yl)oxy)propoxy)-6-methoxyisoindolin-2-yl)-4-oxobutansaure |
| 14. | 4-(5-(3-((2-(3-Carboxypropanoyl)-4-chlor-6-methoxyisoindolin-5-yl)oxy)propoxy)-6-methoxybenzo[b]thiophen-2-yl)-4-oxobutansaure |
| 17. | (S)-4-(5-(3-((2-((S)-3-Carboxybutanoyl)-4-fluor-6-methoxybenzo[b]thiophen-5-yl)oxy)propoxy)-4-fluor-6-methoxyisoindolin-2-yl)-2-methyl-4-oxobutansaure |
| 18. | 4-(5-(3-((2-((2-Carboxyethyl)carbamoyl)-6-methoxybenzo[b]thiophen-5-yl)oxy)propoxy)-6-methoxyisoindolin-2-yl)-4-oxobutansaure |
| 20. | 4-(5-(3-((2-(3-Carboxypropanoyl)-4-fluor-6-methoxybenzo[b]thiophen-5-yl)oxy)propoxy)-6-methoxyisoindolin-2-yl)-4-oxobutansaure |
| 22. | 4-(5-(3-((2-(3-Carboxybutanoyl)-6-methoxybenzo[b]thiophen-5-yl)oxy)propoxy)-6-methoxyisoindolin-2-yl)-2-methyl-4-oxobutansaure |
| 23. | (S)-4-(5-(3-((2-((S)-3-Carboxybutanoyl)-6-methoxybenzo[b]thiophen-5-yl)oxy)propoxy)-6-methoxyisoindolin-2-yl)-2-methyl-4-oxobutansaure |
| 24. | (S)-4-(5-(3-((2-(3-Carboxypropanoyl)-6-methoxyisoindolin-5-yl)oxy)propoxy)-6-methoxybenzo[b]thiophen-2-yl)-2-methyl-4-oxobutansäure |
| 25. | (S)-4-(5-(3-((2-(3-Carboxypropanoyl)-6-methoxybenzo[b]thiophen-5-yl)oxy)propoxy)-6-methoxyisoindolin-2-yl)-2-methyl-4-oxobutansaure |
| 26. | (S)-4-(5-(2-((2-((S)-3-Carboxybutanoyl)-6-methoxybenzo[b]thiophen-5-yl)oxy)ethoxy)-6-methoxyisoindolin-2-yl)-2-methyl-4-oxobutansaure |
| 27. | (S)-4-(5-(4-((2-((S)-3-Carboxybutanoyl)-6-methoxybenzo[b]thiophen-5-yl)oxy)butoxy)-6-methoxyisoindolin-2-yl)-2-methyl-4-oxobutansaure |
| 28. | (S)-4-(5-((5-((2-((S)-3-Carboxybutanoyl)-6-methoxybenzo[b]thiophen-5-yl)oxy)pentyl)oxy)-6-methoxyisoindolin-2-yl)-2-methyl-4-oxobutansaure |
| 34. | (2S)-4-(5-((4-((2-((S)-3-Carboxybutanoyl)-6-methoxybenzo[b]thiophen-5-yl)oxy)pentan-2-yl)oxy)-6-methoxyisoindolin-2-yl)-2-methyl-4-oxobutansäure |
| 35. | (S)-4-(5-(3-((2-((S)-3-Carboxybutanoyl)-6-methoxybenzo[b]thiophen-5-yl)oxy)-2,2-dimethylpropoxy)-6-methoxyisoindolin-2-yl)-2-methyl-4-oxobutansäure |
| 37. | 4-(5-(3-((2-(3-Carboxypropanoyl)-6-methoxyisoindolin-5-yl)oxy)propoxy)-6-methoxybenzo[b]thiophen-2-yl)-2,2-dimethyl-4-oxobutansaure |
| 38. | 4-(5-(3-((2-(3-Carboxypropanoyl)-6-methoxybenzo[b]thiophen-5-yl)oxy)propoxy)-6-methoxyisoindolin-2-yl)-2,2-dimethyl-4-oxobutansaure |

(continued)

| | |
|---|---|
| 39. | 4-(5-(3-((2-(3-Carboxy-3-methylbutanoyl)-6-methoxybenzo[b]thiophen-5-yl)oxy)propoxy)-6-methoxyisoindolin-2-yl)-2,2-dimethyl-4-oxobutansaure |
| 43. | (S)-4-(5-(3-((2-((S)-3-Carboxybutanoyl)-4-fluor-6-methoxybenzo[b]thiophen-5-yl)oxy)propoxy)-6-methoxyisoindolin-2-yl)-2-methyl-4-oxobutansäure |
| 44. | (S)-4-(5-(3-((2-((S)-3-Carboxybutanoyl)-4-chlor-6-methoxybenzo[b]thiophen-5-yl)oxy)propoxy)-6-methoxyisoindolin-2-yl)-2-methyl-4-oxobutansäure |
| 45. | (S)-4-(5-(3-((4-Brom-2-((S)-3-carboxybutanoyl)-6-methoxybenzo[b]thiophen-5-yl)oxy)propoxy)-6-methoxyisoindolin-2-yl)-2-methyl-4-oxobutansäure |
| 47. | (S)-4-(5-(3-((2-((S)-3-Carboxybutanoyl)-6-hydroxybenzo[b]thiophen-5-yl)oxy)propoxy)-6-methoxyisoindolin-2-yl)-2-methyl-4-oxobutansaure |
| 48. | (S)-4-(5-(3-((2-((S)-3-Carboxybutanoyl)-4,7-difluor-6-methoxybenzo[b]thiophen-5-yl)oxy)propoxy)-6-methoxyisoindolin-2-yl)-2-methyl-4-oxobutansäure |
| 49. | (S)-4-(5-(3-((2-((S)-3-Carboxybutanoyl)-4,7-dichlor-6-methoxybenzo[b]thiophen-5-yl)oxy)propoxy)-6-methoxyisoindolin-2-yl)-2-methyl-4-oxobutansäure |
| 51. | (S)-4-(5-(3-((2-((S)-3-Carboxybutanoyl)-4-fluor-6-methoxyisoindolin-5-yl)oxy)propoxy)-6-methoxybenzo[b]thiophen-2-yl)-2-methyl-4-oxobutansäure |
| 52. | (S)-4-(5-(3-((2-((S)-3-Carboxybutanoyl)-4-chlor-6-methoxyisoindolin-5-yl)oxy)propoxy)-6-methoxybenzo[b]thiophen-2-yl)-2-methyl-4-oxobutansäure |
| 53. | (S)-4-(5-(3-((4-Brom-2-((S)-3-carboxybutanoyl)-6-methoxyisoindolin-5-yl)oxy)propoxy)-6-methoxybenzo[b]thiophen-2-yl)-2-methyl-4-oxobutansäure |
| 58. | (S)-4-(5-(3-((2-((S)-3-Carboxybutanoyl)-4,7-difluor-6-methoxyisoindolin-5-yl)oxy)propoxy)-6-methoxybenzo[b]thiophen-2-yl)-2-methyl-4-oxobutansäure |
| 59. | (S)-4-(5-(3-((2-((S)-3-Carboxybutanoyl)-6-methoxybenzo[b]thiophen-5-yl)oxy)propoxy)-7-chlor-4-fluor-6-methoxyisoindolin-2-yl)-2-methyl-4-oxobutansäure |
| 60. | (S)-4-(5-(3-((2-((S)-3-Carboxybutanoyl)-4,7-dichlor-6-methoxyisoindolin-5-yl)oxy)propoxy)-6-methoxybenzo[b]thiophen-2-yl)-2-methyl-4-oxobutansäure |
| 61. | (S)-4-(5-(3-((2-((S)-3-Carboxybutanoyl)-4-chlor-6-methoxybenzo[b]thiophen-5-yl)oxy)propoxy)-4-fluor-6-methoxyisoindolin-2-yl)-2-methyl-4-oxobutansaure |
| 62. | (S)-4-(5-(3-((2-((S)-3-Carboxybutanoyl)-4-chlor-6-methoxyisoindolin-5-yl)oxy)propoxy)-4-fluor-6-methoxybenzo[b]thiophen-2-yl)-2-methyl-4-oxobutansäure |
| 63. | (S)-4-(5-(3-((2-((S)-3-Carboxybutanoyl)-4-chlor-6-methoxybenzo[b]thiophen-5-yl)oxy)propoxy)-4-chlor-6-methoxyisoindolin-2-yl)-2-methyl-4-oxobutansäure |
| 67. | (S)-4-(5-(3-((2-((S)-3-Carboxybutanoyl)-4,7-difluor-6-methoxyisoindolin-5-yl)oxy)propoxy)-7-chlor-6-methoxybenzo[b]thiophen-2-yl)-2-methyl-4-oxobutansäure |
| 68. | (S)-4-(5-(3-((2-((S)-3-Carboxybutanoyl)-4,7-difluor-6-methoxyisoindolin-5-yl)oxy)propoxy)-7-fluor-6-methoxybenzo[b]thiophen-2-yl)-2-methyl-4-oxobutansäure |
| 69. | (S)-4-(5-(3-((2-((S)-3-Carboxybutanoyl)-4-chlor-7-fluor-6-methoxyisoindolin-5-yl)oxy)propoxy)-7-fluor-6-methoxybenzo[b]thiophen-2-yl)-2-methyl-4-oxobutansaure |
| 70. | (S)-4-(6-(3-((2-((S)-3-Carboxybutanoyl)-4,7-difluor-6-methoxybenzo[b]thiophen-5-yl)oxy)propoxy)-4-fluor-5-methoxyisoindolin-2-yl)-2-methyl-4-oxobutansaure |
| 71. | (S)-4-(6-(3-((2-((S)-3-Carboxybutanoyl)-7-chlor-4-fluor-6-methoxybenzo[b]thiophen-5-yl)oxy)propoxy)-4-fluor-5-methoxyisoindolin-2-yl)-2-methyl-4-oxobutansaure |
| 72. | (S)-4-(6-(3-((2-((S)-3-Carboxybutanoyl)-4,7-dichlor-6-methoxybenzo[b]thiophen-5-yl)oxy)propoxy)-4-fluor-5-methoxyisoindolin-2-yl)-2-methyl-4-oxobutansaure |
| 73. | (S)-4-(5-(3-((2-((S)-3-Carboxybutanoyl)-4-chlor-7-fluor-6-methoxyisoindolin-5-yl)oxy)propoxy)-7-chlor-6-methoxybenzo[b]thiophen-2-yl)-2-methyl-4-oxobutansäure |

(continued)

| | |
|---|---|
| 74. | (S)-4-(5-(3-((2-((S)-3-Carboxybutanoyl)-4,7-dichlor-6-methoxyisoindolin-5-yl)oxy)propoxy)-7-fluor-6-methoxybenzo[b]thiophen-2-yl)-2-methyl-4-oxobutansäure |
| 75. | (S)-4-(5-(3-((2-((S)-3-Carboxybutanoyl)-7-chlor-4-fluor-6-methoxyisoindolin-5-yl)oxy)propoxy)-7-fluor-6-methoxybenzo[b]thiophen-2-yl)-2-methyl-4-oxobutansaure |
| 76. | (S)-4-(6-(3-((2-((S)-3-Carboxybutanoyl)-4,7-difluor-6-methoxybenzo[b]thiophen-5-yl)oxy)propoxy)-4-chlor-5-methoxyisoindolin-2-yl)-2-methyl-4-oxobutansäure |
| 77. | (S)-4-(6-(3-((2-((S)-3-Carboxybutanoyl)-4-chlor-7-fluor-6-methoxybenzo[b]thiophen-5-yl)oxy)propoxy)-4-chlor-5-methoxyisoindolin-2-yl)-2-methyl-4-oxobutansäure |
| 78. | (S)-4-(5-(3-((2-((S)-3-Carboxybutanoyl)-4-fluor-6-hydroxyisoindolin-5-yl)oxy)propoxy)-4-fluor-6-methoxybenzo[b]thiophen-2-yl)-2-methyl-4-oxobutansäure |
| 79. | (S)-4-(5-(3-((2-((S)-3-Carboxybutanoyl)-4-chlor-6-methoxybenzo[b]thiophen-5-yl)oxy)propoxy)-4-fluor-6-hydroxyisoindolin-2-yl)-2-methyl-4-oxobutansaure |
| 80. | (S)-4-(5-(3-((2-((S)-3-Carboxybutanoyl)-4-chlor-6-hydroxyisoindolin-5-yl)oxy)propoxy)-4-fluor-6-methoxybenzo[b]thiophen-2-yl)-2-methyl-4-oxobutansäure |
| 81. | (S)-4-(5-(3-((2-((S)-3-Carboxybutanoyl)-4-chlor-6-hydroxyisoindolin-5-yl)oxy)propoxy)-4-chlor-6-methoxybenzo[b]thiophen-2-yl)-2-methyl-4-oxobutansäure |
| 86. | (S)-4-(5-(3-((2-((S)-3-Carboxybutanoyl)-4,7-difluor-6-methoxyisoindolin-5-yl)oxy)propoxy)-4-chlor-7-fluor-6-methoxybenzo[b]thiophen-2-yl)-2-methyl-4-oxobutansäure |
| 87. | (S)-4-(5-(3-((2-((S)-3-Carboxybutanoyl)-4-chlor-7-fluor-6-methoxybenzo[b]thiophen-5-yl)oxy)propoxy)-4-chlor-7-fluor-6-methoxyisoindolin-2-yl)-2-methyl-4-oxobutansäure |
| 88. | (S)-4-(5-(3-((2-((S)-3-Carboxybutanoyl)-4,7-difluor-6-methoxybenzo[b]thiophen-5-yl)oxy)propoxy)-4,7-difluor-6-methoxyisoindolin-2-yl)-2-methyl-4-oxobutansäure |
| 89. | (S)-4-(5-(3-((2-((S)-3-Carboxybutanoyl)-4,7-difluor-6-methoxyisoindolin-5-yl)oxy)propoxy)-7-chlor-4-fluor-6-methoxybenzo[b]thiophen-2-yl)-2-methyl-4-oxobutansäure |
| 90. | (S)-4-(5-(3-((2-((S)-3-Carboxybutanoyl)-4,7-dichlor-6-methoxybenzo[b]thiophen-5-yl)oxy)propoxy)-4,7-difluor-6-methoxyisoindolin-2-yl)-2-methyl-4-oxobutansäure |
| 91. | (S)-4-(5-(3-((2-((S)-3-Carboxybutanoyl)-4,7-dichlor-6-methoxybenzo[b]thiophen-5-yl)oxy)propoxy)-4-chlor-7-fluor-6-methoxyisoindolin-2-yl)-2-methyl-4-oxobutansäure |
| 92. | (S)-4-(5-(3-((2-((S)-3-Carboxybutanoyl)-4-chlor-7-fluor-6-methoxyisoindolin-5-yl)oxy)propoxy)-7-chlor-4-fluor-6-methoxybenzo[b]thiophen-2-yl)-2-methyl-4-oxobutansäure |
| 93. | (S)-4-(5-(3-((2-((S)-3-Carboxybutanoyl)-4,7-dichlor-6-methoxyisoindolin-5-yl)oxy)propoxy)-4-chlor-7-fluor-6-methoxybenzo[b]thiophen-2-yl)-2-methyl-4-oxobutansäure |
| 94. | (S)-4-(5-(3-((2-((S)-3-Carboxybutanoyl)-4-chlor-7-fluor-6-methoxybenzo[b]thiophen-5-yl)oxy)propoxy)-7-chlor-4-fluor-6-methoxyisoindolin-2-yl)-2-methyl-4-oxobutansäure |
| 104. | (1R,2R)-2-(5-(3-((2-((S)-3-Carboxybutanoyl)-6-methoxyisoindolin-5-yl)oxy)propoxy)-6-methoxybenzo [b]thiophen-2-carbonyl)cyclopropan-1-carbonsäure |
| 105. | (1R,2R)-2-(5-(3-((2-((1R,2R)-2-Carboxycyclopropan-1-carbonyl)-6-methoxybenzo[b]thiophen-5-yl)oxy)propoxy)-6-methoxyisoindolin-2-carbonyl)cyclopropan-1-carbonsäure |
| 106. | (1R,2R)-2-(5-(3-((2-((S)-3-Carboxybutanoyl)-6-methoxybenzo[b]thiophen-5-yl)oxy)propoxy)-6-methoxyisoindolin-2-carbonyl)cyclopropan-1-carbonsäure |
| 111. | (S)-4-(4-(3-((2-((S)-3-Carboxybutanoyl)-6-methoxybenzo[b]thiophen-5-yl)oxy)propoxy)-6-methoxyisoindolin-2-yl)-2-methyl-4-oxobutansaure |
| 112. | (S)-4-(5-(3-((2-((S)-3-Carboxybutanoyl)-6-methoxybenzo[b]thiophen-4-yl)oxy)propoxy)-6-methoxyisoindolin-2-yl)-2-methyl-4-oxobutansaure |
| 123. | (S)-4-(5-(3-((6-Brom-2-((S)-3-carboxybutanoyl)benzo[b]thiophen-5-yl)oxy)propoxy)-6-methoxyisoindolin-2-yl)-2-methyl-4-oxobutansäure |

(continued)

| | |
|---|---|
| 130. | (S)-4-(5-(3-((6-Brom-2-((S)-3-carboxybutanoyl)isoindolin-5-yl)oxy)propoxy)-6-methoxybenzo[b]thiophen-2-yl)-2-methyl-4-oxobutansäure |
| 146. | (S)-4-(5-(3-((2-((S)-3-Carboxybutanoyl)-4-fluor-6-hydroxybenzo[b]thiophen-5-yl)oxy)propoxy)-4-fluor-6-methoxyisoindolin-2-yl)-2-methyl-4-oxobutansaure |
| 147. | (S)-4-(5-(3-((2-((S)-3-Carboxybutanoyl)-4-chlor-6-hydroxybenzo[b]thiophen-5-yl)oxy)propoxy)-4-fluor-6-methoxyisoindolin-2-yl)-2-methyl-4-oxobutansaure |
| 148. | (S)-4-(5-(3-((2-((S)-3-Carboxybutanoyl)-4-chlor-6-methoxyisoindolin-5-yl)oxy)propoxy)-4-fluor-6-hydroxybenzo[b]thiophen-2-yl)-2-methyl-4-oxobutansäure |
| 149. | (S)-4-(5-(3-((2-((S)-3-Carboxybutanoyl)-4-chlor-6-hydroxybenzo[b]thiophen-5-yl)oxy)propoxy)-4-chlor-6-methoxyisoindolin-2-yl)-2-methyl-4-oxobutansaure |
| 150. | (S)-4-(6-(3-((2-((S)-3-Carboxybutanoyl)-6-hydroxybenzo[b]thiophen-5-yl)oxy)propoxy)-4-fluor-5-methoxyisoindolin-2-yl)-2-methyl-4-oxobutansäure |
| 151. | (S)-4-(6-(3-((2-((S)-3-Carboxybutanoyl)-7-fluor-6-hydroxybenzo[b]thiophen-5-yl)oxy)propoxy)-4-fluor-5-methoxyisoindolin-2-yl)-2-methyl-4-oxobutansaure |
| 152. | (S)-4-(6-(3-((2-((S)-3-Carboxybutanoyl)-7-chlor-6-hydroxybenzo[b]thiophen-5-yl)oxy)propoxy)-4-fluor-5-methoxyisoindolin-2-yl)-2-methyl-4-oxobutansaure |
| 153. | (S)-4-(5-(3-((2-((S)-3-Carboxybutanoyl)-7-chlor-6-methoxyisoindolin-5-yl)oxy)propoxy)-7-fluor-6-hydroxybenzo[b]thiophen-2-yl)-2-methyl-4-oxobutansäure |
| 154. | (S)-4-(5-(3-((2-((S)-3-Carboxybutanoyl)-4,7-difluor-6-methoxyisoindolin-5-yl)oxy)propoxy)-7-chlor-6-hydroxybenzo[b]thiophen-2-yl)-2-methyl-4-oxobutansäure |
| 155. | (S)-4-(5-(3-((2-((S)-3-Carboxybutanoyl)-4,7-difluor-6-methoxyisoindolin-5-yl)oxy)propoxy)-7-fluor-6-hydroxybenzo[b]thiophen-2-yl)-2-methyl-4-oxobutansäure |
| 156. | (S)-4-(5-(3-((2-((S)-3-Carboxybutanoyl)-4-chlor-7-fluor-6-methoxyisoindolin-5-yl)oxy)propoxy)-7-fluor-6-hydroxybenzo[b]thiophen-2-yl)-2-methyl-4-oxobutansaure |
| 157. | (S)-4-(6-(3-((2-((S)-3-Carboxybutanoyl)-4,7-difluor-6-hydroxybenzo[b]thiophen-5-yl)oxy)propoxy)-4-fluor-5-methoxyisoindolin-2-yl)-2-methyl-4-oxobutansaure |
| 158. | (S)-4-(6-(3-((2-((S)-3-Carboxybutanoyl)-7-chlor-4-fluor-6-hydroxybenzo[b]thiophen-5-yl)oxy)propoxy)-4-fluor-5-methoxyisoindolin-2-yl)-2-methyl-4-oxobutansaure |
| 159. | (S)-4-(6-(3-((2-((S)-3-Carboxybutanoyl)-4,7-dichlor-6-hydroxybenzo[b]thiophen-5-yl)oxy)propoxy)-4-fluor-5-methoxyisoindolin-2-yl)-2-methyl-4-oxobutansäure. |

**13.** Verbindung oder pharmazeutisch verträgliches Salz davon nach einem der Ansprüche 1 bis 12, wobei die Verbindung ist

| | |
|---|---|
| 1. | 4-(5-(3-((2-(3-Carboxypropanoyl)-6-methoxybenzo[b]thiophen-5-yl)oxy)propoxy)-6-methoxyisoindolin-2-yl)-4-oxobutansaure |
| 2. | 4-(5-(3-((2-(3-Carboxypropanoyl)-4-chlor-6-methoxyisoindolin-5-yl)oxy)propoxy)-4-fluor-6-methoxybenzo[b]thiophen-2-yl)-4-oxobutansäure |
| 3. | 4-(5-(3-((2-(3-Carboxypropanoyl)-4-chlor-6-methoxybenzo[b]thiophen-5-yl)oxy)propoxy)-4-fluor-6-methoxyisoindolin-2-yl)-4-oxobutansaure |
| 4. | 4-(5-(3-((2-(3-Carboxypropanoyl)-4-fluor-6-methoxybenzo[b]thiophen-5-yl)oxy)propoxy)-4-fluor-6-methoxyisoindolin-2-yl)-4-oxobutansaure |
| 5. | Natrium<br>(S)-4-(5-(3-((2-((S)-3-Carboxylatobutanoyl)-6-methoxybenzo[b]thiophen-5-yl)oxy)propoxy)-6-methoxyisoindolin-2-yl)-2-methyl-4-oxobutanoat |
| 6. | Natrium<br>(S)-4-(5-(3-((2-((S)-3-Carboxylatobutanoyl)-4-fluor-6-methoxybenzo[b]thiophen-5-yl)oxy)propoxy)-6-methoxyisoindolin-2-yl)-2-methyl-4-oxobutanoat |

(continued)

| | |
|---|---|
| 7. | Natrium 4-(5-(3-((2-(3-Carboxylatpropanoyl)-6-hydroxybenzo[b]thiophen-5-yl)oxy)propoxy)-6-methoxyisoindolin-2-yl)-4-oxobutanoat |
| 9. | 4-(5-(3-((6-Brom-2-(3-carboxypropanoyl)benzo[b]thiophen-5-yl)oxy)propoxy)-6-methoxyisoindolin-2-yl)-4-oxobutansaure |
| 10. | 4-(5-(3-((2-(3-Carboxypropanoyl)-4-fluor-6-methoxyisoindolin-5-yl)oxy)propoxy)-6-methoxybenzo[b]thiophen-2-yl)-4-oxobutansäure |
| 12. | 4-(5-(3-((2-(4-Ethoxy-4-oxobutanoyl)-6-methoxybenzo[b]thiophen-5-yl)oxy)propoxy)-6-methoxyisoindolin-2-yl)-4-oxobutansaure |
| 14. | 4-(5-(3-((2-(3-Carboxypropanoyl)-4-chlor-6-methoxyisoindolin-5-yl)oxy)propoxy)-6-methoxybenzo[b]thiophen-2-yl)-4-oxobutansaure |
| 17. | (S)-4-(5-(3-((2-((S)-3-Carboxybutanoyl)-4-fluor-6-methoxybenzo[b]thiophen-5-yl)oxy)propoxy)-4-fluor-6-methoxyisoindolin-2-yl)-2-methyl-4-oxobutansaure |
| 18. | 4-(5-(3-((2-((2-Carboxyethyl)carbamoyl)-6-methoxybenzo[b]thiophen-5-yl)oxy)propoxy)-6-methoxyisoindolin-2-yl)-4-oxobutansaure |
| 20. | 4-(5-(3-((2-(3-Carboxypropanoyl)-4-fluor-6-methoxybenzo[b]thiophen-5-yl)oxy)propoxy)-6-methoxyisoindolin-2-yl)-4-oxobutansäure. |

**14.** Pharmazeutische Zusammensetzung, umfassend eine therapeutisch wirksame Menge mindestens einer Verbindung oder eines pharmazeutisch verträglichen Salzes davon nach einem der Ansprüche 1 bis 13 und mindestens einen pharmazeutisch geeigneten Arzneistoffträger.

**15.** Mindestens eine Verbindung oder ein pharmazeutisch verträgliches Salz davon zur Verwendung wie in einem der Ansprüche 1 bis 13 definiert und/oder eine pharmazeutische Zusammensetzung nach Anspruch 14, die zur Verwendung in der Therapie bestimmt ist; oder zur Verwendung bei einem Induzieren einer Immunantwort in einem Subjekt; oder zur Verwendung bei dem Induzieren einer STING-abhängigen Typ-I-Interferon-Produktion in einem Subjekt; oder zur Verwendung bei dem Induzieren einer STING-abhängigen Zytokinproduktion in einem Subjekt; oder zur Verwendung bei einem Behandeln einer Zellproliferationsstörung bei einem Subjekt, wobei die Zellproliferationsstörung Krebs ist; oder zur Verwendung als einen STING-Agonisten; oder zur Verwendung als ein Arzneimittel.

## Revendications

**1.** Composé de formule I-1 ou sel pharmaceutiquement acceptable de celui-ci :

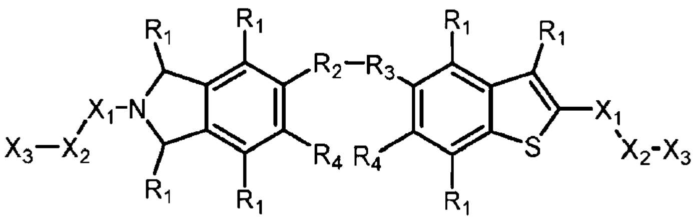

,

I-1

dans lequel

chaque $R_1$ est indépendamment choisi parmi H, deutérium ou halogène ;
$R_2$ et $R_3$ sont indépendamment choisis dans le groupe constitué de O-(alkylène en $C_1$-$C_4$), -$T_a$-alkyle en $C_1$-$C_6$-$T_b$-, ou -$T_a$-O-$T_b$, dans lequel l'alkyle en $C_1$-$C_6$ est facultativement substitué par un ou plusieurs deutérium, ou halo ;
$T_a$ et $T_b$ sont chacun indépendamment absents, -O-, alkyle en $C_1$-$C_6$, -O-(alkyle en $C_1$-$C_6$)- ou -(alkyle en $C_1$-$C_6$)-O- ;

chaque $R_4$ est indépendamment choisi dans le groupe constitué de H, deutérium, halogène, ou $OR^6$ ; chaque $R^6$ est indépendamment choisi dans le groupe constitué de -H, deutérium, ou alkyle en $C_1$-$C_6$, et dont chacun est indépendamment substitué facultativement par deutérium, ou -OH ; chaque $X_1$ est C=O ; chaque $X_2$ est indépendamment choisi parmi $-(C(R^8)_2)_{(1-3)}$, $-NR^8(C(R^8)_2)_{(1-3)}$ ou $-NH(C(R^8)_2)_{(1-3)}$ ; dans lequel chaque $R^8$ est indépendamment choisi dans le groupe constitué de H, deutérium, ou alkyle en $C_1$-$C_6$ ; et chaque $X_3$ est $COOR^6$, $SO_2R^6$,

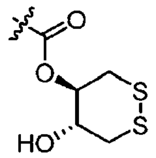

ou CN.

2. Composé ou sel pharmaceutiquement acceptable de celui-ci selon la revendication 1, dans lequel chaque $R_1$ est indépendamment choisi dans le groupe constitué de H, deutérium, F, Cl ou Br.

3. Composé ou sel pharmaceutiquement acceptable de celui-ci selon les revendications 1 ou 2, dans lequel $R_2$-$R_3$ est choisi parmi

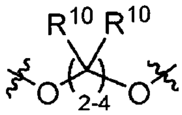

,

ou

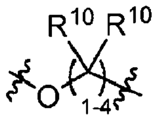

,

dans lequel :
chaque $R^{10}$ est indépendamment hydrogène, ou alkyle en $C_1$-$C_2$.

4. Composé ou sel pharmaceutiquement acceptable de celui-ci selon la revendication 3, dans lequel $R^{10}$ est indépendamment hydrogène, ou méthyle.

5. Composé ou sel pharmaceutiquement acceptable de celui-ci selon l'une quelconque des revendications 1 à 4, dans lequel $R_2$-$R_3$ est choisi parmi - $O(CH_2)_2O$-, $-O(CH_2)_3O$-, $-O(CH_2)_4O$- ou $-OCH(CH_3)CH_2CH(CH_3)O$-.

6. Composé ou sel pharmaceutiquement acceptable de celui-ci selon l'une quelconque des revendications 1 à 5, dans lequel chaque $R_4$ est indépendamment choisi dans le groupe constitué de H, deutérium, Br ou $OR^6$.

7. Composé ou sel pharmaceutiquement acceptable de celui-ci l'une quelconque des revendications 1 à 6, dans lequel chaque $R_4$ est indépendamment choisi dans le groupe constitué de H, deutérium, Br, OH ou O-alkyle en $C_1$-$C_3$.

8. Composé ou sel pharmaceutiquement acceptable de celui-ci selon l'une quelconque des revendications 1 à 7, dans lequel chaque $R_4$ est indépendamment choisi dans le groupe constitué de H, deutérium, Br, OH ou $OCH_3$.

9. Composé ou sel pharmaceutiquement acceptable de celui-ci selon l'une quelconque des revendications 1 à 8, dans lequel chaque $X_2$ est $CH_2CHR^8$, dans lequel $R^8$ est choisi dans le groupe constitué de H, deutérium ou alkyle en $C_1$-$C_3$.

**10.** Composé ou sel pharmaceutiquement acceptable de celui-ci selon l'une quelconque des revendications 1 à 9, dans lequel chaque $X_2$ est $-CH_2CH_2-$.

**11.** Composé ou sel pharmaceutiquement acceptable de celui-ci selon l'une quelconque des revendications 1 à 10, dans lequel chaque $X_3$ est indépendamment choisi parmi COOH ou

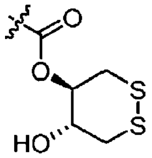

.

**12.** Composé ou sel pharmaceutiquement acceptable de celui-ci selon l'une quelconque des revendications 1 à 11, dans lequel le composé est

| | |
|---|---|
| 1. | acide 4-(5-(3-((2-(3-carboxypropanoyl)-6-méthoxybenzo[b]thiophén-5-yl)oxy)propoxy)-6-méthoxy-iso-indolin-2-yl)-4-oxobutanoïque |
| 2. | acide 4-(5-(3-((2-(3-carboxypropanoyl)-4-chloro-6-méthoxy-iso-indolin-5-yl)oxy)propoxy)-4-fluoro-6-méthoxybenzo[b]thiophén-2-yl)-4-oxobutanoïque |
| 3. | acide 4-(5-(3-((2-(3-carboxypropanoyl)-4-chloro-6-méthoxybenzo[b]thiophén-5-yl)oxy)propoxy)-4-fluoro-6-méthoxy-iso-indolin-2-yl)-4-oxobutanoïque |
| 4. | acide 4-(5-(3-((2-(3-carboxypropanoyl)-4-fluoro-6-méthoxybenzo[b]thiophén-5-yl)oxy)propoxy)-4-fluoro-6-méthoxy-iso-indolin-2-yl)-4-oxobutanoïque |
| 5. | sodium (S)-4-(5-(3-((2-((S)-3-carboxylatobutanoyl)-6-méthoxybenzo[b]thiophén-5-yl)oxy)propoxy)-6-méthoxy-iso-indolin-2-yl)-2-méthyl-4-oxobutanoate |
| 6. | sodium (S)-4-(5-(3-((2-((S)-3-carboxylatobutanoyl)-4-fluoro-6-méthoxybenzo[b]thiophén-5-yl)oxy)propoxy)-6-méthoxy-iso-indolin-2-yl)-2-méthyl-4-oxobutanoate |
| 7. | sodium 4-(5-(3-((2-(3-carboxylatopropanoyl)-6-hydroxybenzo[b]thiophén-5-yl)oxy)propoxy)-6-méthoxy-iso-indolin-2-yl)-4-oxobutanoate |
| 9. | acide 4-(5-(3-((6-bromo-2-(3-carboxypropanoyl)benzo[b]thiophén-5-yl)oxy)propoxy)-6-méthoxy-iso-indolin-2-yl)-4-oxobutanoïque |
| 10. | acide 4-(5-(3-((2-(3-carboxypropanoyl)-4-fluoro-6-méthoxy-iso-indolin-5-yl)oxy)propoxy)-6-méthoxybenzo[b]thiophén-2-yl)-4-oxobutanoïque |
| 12. | acide 4-(5-(3-((2-(4-éthoxy-4-oxobutanoyl)-6-méthoxybenzo[b]thiophén-5-yl)oxy)propoxy)-6-méthoxy-iso-indolin-2-yl)-4-oxobutanoïque |
| 14. | acide 4-(5-(3-((2-(3-carboxypropanoyl)-4-chloro-6-méthoxy-iso-indolin-5-yl)oxy)propoxy)-6-méthoxybenzo[b]thiophén-2-yl)-4-oxobutanoïque |
| 17. | acide (S)-4-(5-(3-((2-((S)-3-carboxybutanoyl)-4-fluoro-6-méthoxybenzo[b]thiophén-5-yl)oxy)propoxy)-4-fluoro-6-méthoxy-iso-indolin-2-yl)-2-méthyl-4-oxobutanoïque |
| 18. | acide 4-(5-(3-((2-((2-carboxyéthyl)carbamoyl)-6-méthoxybenzo[b]thiophén-5-yl)oxy)propoxy)-6-méthoxy-iso-indolin-2-yl)-4-oxobutanoïque |
| 20. | acide 4-(5-(3-((2-(3-carboxypropanoyl)-4-fluoro-6-méthoxybenzo[b]thiophén-5-yl)oxy)propoxy)-6-méthoxy-iso-indolin-2-yl)-4-oxobutanoïque |
| 22. | acide 4-(5-(3-((2-(3-carboxybutanoyl)-6-méthoxybenzo[b]thiophén-5-yl)oxy)propoxy)-6-méthoxy-iso-indolin-2-yl)-2-méthyl-4-oxobutanoïque |
| 23. | acide (S)-4-(5-(3-((2-((S)-3-carboxybutanoyl)-6-méthoxybenzo[b]thiophén-5-yl)oxy)propoxy)-6-méthoxy-iso-indolin-2-yl)-2-méthyl-4-oxobutanoïque |

(continued)

| | |
|---|---|
| 24. | acide (S)-4-(5-(3-((2-(3-carboxypropanoyl)-6-méthoxy-iso-indolin-5-yl)oxy)propoxy)-6-méthoxybenzo [b]thiophén-2-yl)-2-méthyl-4-oxobutanoïque |
| 25. | acide (S)-4-(5-(3-((2-(3-carboxypropanoyl)-6-méthoxybenzo[b]thiophén-5-yl)oxy)propoxy)-6-méthoxy-iso-indolin-2-yl)-2-méthyl-4-oxobutanoïque |
| 26. | acide (S)-4-(5-(2-((2-((S)-3-carboxybutanoyl)-6-méthoxybenzo[b]thiophén-5-yl)oxy)éthoxy)-6-méthoxy-iso-indolin-2-yl)-2-méthyl-4-oxobutanoïque |
| 27. | acide (S)-4-(5-(4-((2-((S)-3-carboxybutanoyl)-6-méthoxybenzo[b]thiophén-5-yl)oxy)butoxy)-6-méthoxy-iso-indolin-2-yl)-2-méthyl-4-oxobutanoïque |
| 28. | acide (S)-4-(5-((5-((2-((S)-3-carboxybutanoyl)-6-méthoxybenzo[b]thiophén-5-yl)oxy)pentyl)oxy)-6-méthoxy-iso-indolin-2-yl)-2-méthyl-4-oxobutanoïque |
| 34. | acide (2S)-4-(5-((4-((2-((S)-3-carboxybutanoyl)-6-méthoxybenzo[b]thiophén-5-yl)oxy)pentan-2-yl) oxy)-6-méthoxy-iso-indolin-2-yl)-2-méthyl-4-oxobutanoïque |
| 35. | acide (S)-4-(5-(3-((2-((S)-3-carboxybutanoyl)-6-méthoxybenzo[b]thiophén-5-yl)oxy)-2,2-diméthylpropoxy)-6-méthoxy-iso-indolin-2-yl)-2-méthyl-4-oxobutanoïque |
| 37. | acide 4-(5-(3-((2-(3-carboxypropanoyl)-6-méthoxy-iso-indolin-5-yl)oxy)propoxy)-6-méthoxybenzo[b] thiophén-2-yl)-2,2-diméthyl-4-oxobutanoïque |
| 38. | acide 4-(5-(3-((2-(3-carboxypropanoyl)-6-méthoxybenzo[b]thiophén-5-yl)oxy)propoxy)-6-méthoxy-iso-indolin-2-yl)-2,2-diméthyl-4-oxobutanoïque |
| 39. | acide 4-(5-(3-((2-(3-carboxy-3-méthylbutanoyl)-6-méthoxybenzo[b]thiophén-5-yl)oxy)propoxy)-6-méthoxy-iso-indolin-2-yl)-2,2-diméthyl-4-oxobutanoïque |
| 43. | acide (S)-4-(5-(3-((2-((S)-3-carboxybutanoyl)-4-fluoro-6-méthoxybenzo[b]thiophén-5-yl)oxy)propoxy)-6-méthoxy-iso-indolin-2-yl)-2-méthyl-4-oxobutanoïque |
| 44. | acide (S)-4-(5-(3-((2-((S)-3-carboxybutanoyl)-4-chloro-6-méthoxybenzo[b]thiophén-5-yl)oxy)propoxy)-6-méthoxy-iso-indolin-2-yl)-2-méthyl-4-oxobutanoïque |
| 45. | acide (S)-4-(5-(3-((4-bromo-2-((S)-3-carboxybutanoyl)-6-méthoxybenzo[b]thiophén-5-yl)oxy)propoxy)-6-méthoxy-iso-indolin-2-yl)-2-méthyl-4-oxobutanoïque |
| 47. | acide (S)-4-(5-(3-((2-((S)-3-carboxybutanoyl)-6-hydroxybenzo[b]thiophén-5-yl)oxy)propoxy)-6-méthoxy-iso-indolin-2-yl)-2-méthyl-4-oxobutanoïque |
| 48. | acide (S)-4-(5-(3-((2-((S)-3-carboxybutanoyl)-4,7-difluoro-6-méthoxybenzo[b]thiophén-5-yl)oxy)propoxy)-6-méthoxy-iso-indolin-2-yl)-2-méthyl-4-oxobutanoïque |
| 49. | acide (S)-4-(5-(3-((2-((S)-3-carboxybutanoyl)-4,7-dichloro-6-méthoxybenzo[b]thiophén-5-yl)oxy)propoxy)-6-méthoxy-iso-indolin-2-yl)-2-méthyl-4-oxobutanoïque |
| 51. | acide (S)-4-(5-(3-((2-((S)-3-carboxybutanoyl)-4-fluoro-6-méthoxy-iso-indolin-5-yl)oxy)propoxy)-6-méthoxybenzo[b]thiophén-2-yl}-2-méthyl-4-oxobutanoïque |
| 52. | acide (S)-4-(5-(3-((2-((S)-3-carboxybutanoyl)-4-chloro-6-méthoxy-iso-indolin-5-yl)oxy)propoxy)-6-méthoxybenzo[b]thiophén-2-yl)-2-méthyl-4-oxobutanoïque |
| 53. | acide (S)-4-(5-(3-((4-bromo-2-((S)-3-carboxybutanoyl)-6-méthoxy-iso-indolin-5-yl)oxy)propoxy)-6-méthoxybenzo[b]thiophén-2-yl)-2-méthyl-4-oxobutanoïque |
| 58. | acide (S)-4-(5-(3-((2-((S)-3-carboxybutanoyl)-4,7-difluoro-6-méthoxy-iso-indolin-5-yl)oxy)propoxy)-6-méthoxybenzo[b]thiophén-2-yl)-2-méthyl-4-oxobutanoïque |
| 59. | acide (S)-4-(5-(3-((2-((S)-3-carboxybutanoyl)-6-méthoxybenzo[b]thiophén-5-yl)oxy)propoxy)-7-chloro-4-fluoro-6-méthoxy-iso-indolin-2-yl)-2-méthyl-4-oxobutanoïque |
| 60. | acide (S)-4-(5-(3-((2-((S)-3-carboxybutanoyl)-4,7-dichloro-6-méthoxy-iso-indolin-5-yl)oxy)propoxy)-6-méthoxybenzo[b]thiophén-2-yl)-2-méthyl-4-oxobutanoïque |
| 61. | acide (S)-4-(5-(3-((2-((S)-3-carboxybutanoyl)-4-chloro-6-méthoxybenzo[b]thiophén-5-yl)oxy)propoxy)-4-fluoro-6-méthoxy-iso-indolin-2-yl)-2-méthyl-4-oxobutanoïque |

(continued)

| | |
|---|---|
| 62. | acide (S)-4-(5-(3-((2-((S)-3-carboxybutanoyl)-4-chloro-6-méthoxy-iso-indolin-5-yl)oxy)propoxy)-4-fluoro-6-méthoxybenzo[b]thiophén-2-yl)-2-méthyl-4-oxobutanoïque |
| 63. | acide (S)-4-(5-(3-((2-((S)-3-carboxybutanoyl)-4-chloro-6-méthoxybenzo[b]thiophén-5-yl)oxy)propoxy)-4-chloro-6-méthoxy-iso-indolin-2-yl)-2-méthyl-4-oxobutanoïque |
| 67. | acide (S)-4-(5-(3-((2-((S)-3-carboxybutanoyl)-4,7-difluoro-6-méthoxy-iso-indolin-5-yl)oxy)propoxy)-7-chloro-6-méthoxybenzo[b]thiophén-2-yl)-2-méthyl-4-oxobutanoïque |
| 68. | acide (S)-4-(5-(3-((2-((S)-3-carboxybutanoyl)-4,7-difluoro-6-méthoxy-iso-indolin-5-yl)oxy)propoxy)-7-fluoro-6-méthoxybenzo[b]thiophén-2-yl)-2-méthyl-4-oxobutanoïque |
| 69. | acide (S)-4-(5-(3-((2-((S)-3-carboxybutanoyl)-4-chloro-7-fluoro-6-méthoxy-iso-indolin-5-yl)oxy)propoxy)-7-fluoro-6-méthoxybenzo[b]thiophén-2-yl)-2-méthyl-4-oxobutanoïque |
| 70. | acide (S)-4-(6-(3-((2-((S)-3-carboxybutanoyl)-4,7-difluoro-6-méthoxybenzo[b]thiophén-5-yl)oxy)propoxy)-4-fluoro-5-méthoxy-iso-indolin-2-yl)-2-méthyl-4-oxobutanoïque |
| 71. | acide (S)-4-(6-(3-((2-((S)-3-carboxybutanoyl)-7-chloro-4-fluoro-6-méthoxybenzo[b]thiophén-5-yl)oxy)propoxy)-4-fluoro-5-méthoxy-iso-indolin-2-yl)-2-méthyl-4-oxobutanoïque |
| 72. | acide (S)-4-(6-(3-((2-((S)-3-carboxybutanoyl)-4,7-dichloro-6-méthoxybenzo[b]thiophén-5-yl)oxy)propoxy)-4-fluoro-5-méthoxy-iso-indolin-2-yl)-2-méthyl-4-oxobutanoïque |
| 73. | acide (S)-4-(5-(3-((2-((S)-3-carboxybutanoyl)-4-chloro-7-fluoro-6-méthoxy-iso-indolin-5-yl)oxy)propoxy)-7-chloro-6-méthoxybenzo[b]thiophén-2-yl)-2-méthyl-4-oxobutanoïque |
| 74. | acide (S)-4-(5-(3-((2-((S)-3-carboxybutanoyl)-4,7-dichloro-6-méthoxy-iso-indolin-5-yl)oxy)propoxy)-7-fluoro-6-méthoxybenzo[b]thiophén-2-yl)-2-méthyl-4-oxobutanoïque |
| 75. | acide (S)-4-(5-(3-((2-((S)-3-carboxybutanoyl)-7-chloro-4-fluoro-6-méthoxy-iso-indolin-5-yl)oxy)propoxy)-7-fluoro-6-méthoxybenzo[b]thiophén-2-yl)-2-méthyl-4-oxobutanoïque |
| 76. | acide (S)-4-(6-(3-((2-((S)-3-carboxybutanoyl)-4,7-difluoro-6-méthoxybenzo[b]thiophén-5-yl)oxy)propoxy)-4-chloro-5-méthoxy-iso-indolin-2-yl)-2-méthyl-4-oxobutanoïque |
| 77. | acide (S)-4-(6-(3-((2-((S)-3-carboxybutanoyl)-4-chloro-7-fluoro-6-méthoxybenzo[b]thiophén-5-yl)oxy)propoxy)-4-chloro-5-méthoxy-iso-indolin-2-yl)-2-méthyl-4-oxobutanoïque |
| 78. | acide (S)-4-(5-(3-((2-((S)-3-carboxybutanoyl)-4-fluoro-6-hydroxy-iso-indolin-5-yl)oxy)propoxy)-4-fluoro-6-methoxybenzo[b]thiophen-2-yl)-2-methyl-4-oxobutanoïque |
| 79. | acide (S)-4-(5-(3-((2-((S)-3-carboxybutanoyl)-4-chloro-6-méthoxybenzo[b]thiophén-5-yl)oxy)propoxy)-4-fluoro-6-hydroxy-iso-indolin-2-yl)-2-méthyl-4-oxobutanoïque |
| 80. | acide (S)-4-(5-(3-((2-((S)-3-carboxybutanoyl)-4-chloro-6-hydroxy-iso-indolin-5-yl)oxy)propoxy)-4-fluoro-6-méthoxybenzo[b]thiophén-2-yl)-2-méthyl-4-oxobutanoïque |
| 81. | acide (S)-4-(5-(3-((2-((S)-3-carboxybutanoyl)-4-chloro-6-hydroxy-iso-indolin-5-yl)oxy)propoxy)-4-chloro-6-méthoxybenzo[b]thiophén-2-yl)-2-méthyl-4-oxobutanoïque |
| 86. | acide (S)-4-(5-(3-((2-((S)-3-carboxybutanoyl)-4,7-difluoro-6-méthoxy-iso-indolin-5-yl)oxy)propoxy)-4-chloro-7-fluoro-6-méthoxybenzo[b]thiophén-2-yl)-2-méthyl-4-oxobutanoïque |
| 87. | acide (S)-4-(5-(3-((2-((S)-3-carboxybutanoyl)-4-chloro-7-fluoro-6-méthoxybenzo[b]thiophén-5-yl)oxy)propoxy)-4-chloro-7-fluoro-6-méthoxy-iso-indolin-2-yl)-2-méthyl-4-oxobutanoïque |
| 88. | acide (S)-4-(5-(3-((2-((S)-3-carboxybutanoyl)-4,7-difluoro-6-méthoxybenzo[b]thiophén-5-yl)oxy)propoxy)-4,7-difluoro-6-méthoxy-iso-indolin-2-yl)-2-méthyl-4-oxobutanoïque |
| 89. | acide (S)-4-(5-(3-((2-((S)-3-carboxybutanoyl)-4,7-difluoro-6-méthoxy-iso-indolin-5-yl)oxy)propoxy)-7-chloro-4-fluoro-6-méthoxybenzo[b]thiophén-2-yl)-2-méthyl-4-oxobutanoïque |
| 90. | acide (S)-4-(5-(3-((2-((S)-3-carboxybutanoyl)-4,7-dichloro-6-méthoxybenzo[b]thiophén-5-yl)oxy)propoxy)-4,7-difluoro-6-méthoxy-iso-indolin-2-yl)-2-méthyl-4-oxobutanoïque |
| 91. | acide (S)-4-(5-(3-((2-((S)-3-carboxybutanoyl)-4,7-dichloro-6-méthoxybenzo[b]thiophén-5-yl)oxy)propoxy)-4-chloro-7-fluoro-6-méthoxy-iso-indolin-2-yl)-2-méthyl-4-oxobutanoïque |

(continued)

| | |
|---|---|
| 92. | acide (S)-4-(5-(3-((2-((S)-3-carboxybutanoyl)-4-chloro-7-fluoro-6-méthoxy-iso-indolin-5-yl)oxy)propoxy)-7-chloro-4-fluoro-6-méthoxybenzo[b]thiophén-2-yl)-2-méthyl-4-oxobutanoïque |
| 93. | acide (S)-4-(5-(3-((2-((S)-3-carboxybutanoyl)-4,7-dichloro-6-méthoxy-iso-indolin-5-yl)oxy)propoxy)-4-chloro-7-fluoro-6-méthoxybenzo[b]thiophén-2-yl)-2-méthyl-4-oxobutanoïque |
| 94. | acide (S)-4-(5-(3-((2-((S)-3-carboxybutanoyl)-4-chloro-7-fluoro-6-méthoxybenzo[b]thiophén-5-yl)oxy)propoxy)-7-chloro-4-fluoro-6-méthoxy-iso-indolin-2-yl)-2-méthyl-4-oxobutanoïque |
| 104. | acide (1R,2R)-2-(5-(3-((2-((S)-3-carboxybutanoyl)-6-méthoxy-iso-indolin-5-yl)oxy)propoxy)-6-méthoxybenzo[b]thiophène-2-carbonyl)cyclopropane-1-carboxylique |
| 105. | acide (1R,2R)-2-(5-(3-((2-((1R,2R)-2-carboxycyclopropane-1-carbonyl)-6-méthoxybenzo[b]thiophén-5-yl)oxy)propoxy)-6-méthoxy-iso-indoline-2-carbonyl)cyclopropane-1-carboxylique |
| 106. | acide (1 R,2R)-2-(5-(3-((2-((S)-3-carboxybutanoyl)-6-méthoxybenzo[b]thiophén-5-yl)oxy)propoxy)-6-méthoxy-iso-indoline-2-carbonyl)cyclopropane-1-carboxylique |
| 111. | acide (S)-4-(4-(3-((2-((S)-3-carboxybutanoyl)-6-méthoxybenzo[b]thiophén-5-yl)oxy)propoxy)-6-méthoxy-iso-indolin-2-yl)-2-méthyl-4-oxobutanoïque |
| 112. | acide (S)-4-(5-(3-((2-((S)-3-carboxybutanoyl)-6-méthoxybenzo[b]thiophén-4-yl)oxy)propoxy)-6-méthoxy-iso-indolin-2-yl)-2-méthyl-4-oxobutanoïque |
| 123. | acide (S)-4-(5-(3-((6-bromo-2-((S)-3-carboxybutanoyl)benzo[b]thiophén-5-yl)oxy)propoxy)-6-méthoxy-iso-indolin-2-yl)-2-méthyl-4-oxobutanoïque |
| 130. | acide (S)-4-(5-(3-((6-bromo-2-((S)-3-carboxybutanoyl)iso-indolin-5-yl)oxy)propoxy)-6-méthoxybenzo[b]thiophén-2-yl)-2-méthyl-4-oxobutanoïque |
| 146. | acide (S)-4-(5-(3-((2-((S)-3-carboxybutanoyl)-4-fluoro-6-hydroxybenzo[b]thiophén-5-yl)oxy)propoxy)-4-fluoro-6-méthoxy-iso-indolin-2-yl)-2-méthyl-4-oxobutanoïque |
| 147. | acide (S)-4-(5-(3-((2-((S)-3-carboxybutanoyl)-4-chloro-6-hydroxybenzo[b]thiophén-5-yl)oxy)propoxy)-4-fluoro-6-méthoxy-iso-indolin-2-yl)-2-méthyl-4-oxobutanoïque |
| 148. | acide (S)-4-(5-(3-((2-((S)-3-carboxybutanoyl)-4-chloro-6-méthoxy-iso-indolin-5-yl)oxy)propoxy)-4-fluoro-6-hydroxybenzo[b]thiophén-2-yl)-2-méthyl-4-oxobutanoïque |
| 149. | acide (S)-4-(5-(3-((2-((S)-3-carboxybutanoyl)-4-chloro-6-hydroxybenzo[b]thiophén-5-yl)oxy)propoxy)-4-chloro-6-méthoxy-iso-indolin-2-yl)-2-méthyl-4-oxobutanoïque |
| 150. | acide (S)-4-(6-(3-((2-((S)-3-carboxybutanoyl)-6-hydroxybenzo[b]thiophén-5-yl)oxy)propoxy)-4-fluoro-5-méthoxy-iso-indolin-2-yl)-2-méthyl-4-oxobutanoïque |
| 151. | acide (S)-4-(6-(3-((2-((S)-3-carboxybutanoyl)-7-fluoro-6-hydroxybenzo[b]thiophén-5-yl)oxy)propoxy)-4-fluoro-5-méthoxy-iso-indolin-2-yl)-2-méthyl-4-oxobutanoïque |
| 152. | acide (S)-4-(6-(3-((2-((S)-3-carboxybutanoyl)-7-chloro-6-hydroxybenzo[b]thiophén-5-yl)oxy)propoxy)-4-fluoro-5-méthoxy-iso-indolin-2-yl)-2-méthyl-4-oxobutanoïque |
| 153. | acide (S)-4-(5-(3-((2-((S)-3-carboxybutanoyl)-7-chloro-6-méthoxy-iso-indolin-5-yl)oxy)propoxy)-7-fluoro-6-hydroxybenzo[b]thiophén-2-yl)-2-méthyl-4-oxobutanoïque |
| 154. | acide (S)-4-(5-(3-((2-((S)-3-carboxybutanoyl)-4,7-difluoro-6-méthoxy-iso-indolin-5-yl)oxy)propoxy)-7-chloro-6-hydroxybenzo[b]thiophén-2-yl)-2-méthyl-4-oxobutanoïque |
| 155. | acide (S)-4-(5-(3-((2-((S)-3-carboxybutanoyl)-4,7-difluoro-6-méthoxy-iso-indolin-5-yl)oxy)propoxy)-7-fluoro-6-hydroxybenzo[b]thiophén-2-yl)-2-méthyl-4-oxobutanoïque |
| 156. | acide (S)-4-(5-(3-((2-((S)-3-carboxybutanoyl)-4-chloro-7-fluoro-6-méthoxy-iso-indolin-5-yl)oxy)propoxy)-7-fluoro-6-hydroxybenzo[b]thiophén-2-yl)-2-méthyl-4-oxobutanoïque |
| 157. | acide (S)-4-(6-(3-((2-((S)-3-carboxybutanoyl)-4,7-difluoro-6-hydroxybenzo[b]thiophén-5-yl)oxy)propoxy)-4-fluoro-5-méthoxy-iso-inddolin-2-yl)-2-méthyl-4-oxobutanoïque |
| 158. | acide (S)-4-(6-(3-((2-((S)-3-carboxybutanoyl)-7-chloro-4-fluoro-6-hydroxybenzo[b]thiophén-5-yl)oxy)propoxy)-4-fluoro-5-méthoxy-iso-indolin-2-yl)-2-méthyl-4-oxobutanoïque |

(continued)

| | |
|---|---|
| 159. | acide (S)-4-(6-(3-((2-((S)-3-carboxybutanoyl)-4,7-dichloro-6-hydroxybenzo[b]thiophén-5-yl)oxy)propoxy)-4-fluoro-5-méthoxy-iso-indolin-2-yl)-2-méthyl-4-oxobutanoïque. |

**13.** Composé ou sel pharmaceutiquement acceptable de celui-ci selon l'une quelconque des revendications 1 à 12, dans lequel le composé est

| | |
|---|---|
| 1. | acide 4-(5-(3-((2-(3-carboxypropanoyl)-6-méthoxybenzo[b]thiophén-5-yl)oxy)propoxy)-6-méthoxy-iso-indolin-2-yl)-4-oxobutanoïque |
| 2. | acide 4-(5-(3-((2-(3-carboxypropanoyl)-4-chloro-6-méthoxy-iso-indolin-5-yl)oxy)propoxy)-4-fluoro-6-méthoxybenzo[b]thiophen-2-yl)-4-oxobutanoique |
| 3. | acide 4-(5-(3-((2-(3-carboxypropanoyl)-4-chloro-6-méthoxybenzo[b]thiophén-5-yl)oxy)propoxy)-4-fluoro-6-méthoxy-iso-indolin-2-yl)-4-oxobutanoïque |
| 4. | acide 4-(5-(3-((2-(3-carboxypropanoyl)-4-fluoro-6-méthoxybenzo[b]thiophén-5-yl)oxy)propoxy)-4-fluoro-6-méthoxy-iso-indolin-2-yl)-4-oxobutanoïque |
| 5. | sodium (S)-4-(5-(3-((2-((S)-3-carboxylatobutanoyl)-6-méthoxybenzo[b]thiophén-5-yl)oxy)propoxy)-6-méthoxy-iso-indolin-2-yl)-2-méthyl-4-oxobutanoate |
| 6. | sodium (S)-4-(5-(3-((2-((S)-3-carboxylatobutanoyl)-4-fluoro-6-méthoxybenzo[b]thiophén-5-yl)oxy)propoxy)-6-méthoxy-iso-indolin-2-yl)-2-méthyl-4-oxobutanoate |
| 7. | sodium 4-(5-(3-((2-(3-carboxylatopropanoyl)-6-hydroxybenzo[b]thiophén-5-yl)oxy)propoxy)-6-méthoxy-iso-indolin-2-yl)-4-oxobutanoate |
| 9. | acide 4-(5-(3-((6-bromo-2-(3-carboxypropanoyl)benzo[b]thiophén-5-yl)oxy)propoxy)-6-méthoxy-iso-indolin-2-yl)-4-oxobutanoïque |
| 10. | acide 4-(5-(3-((2-(3-carboxypropanoyl)-4-fluoro-6-méthoxy-iso-indolin-5-yl)oxy)propoxy)-6-méthoxybenzo[b]thiophén-2-yl)-4-oxobutanoïque |
| 12. | acide 4-(5-(3-((2-(4-éthoxy-4-oxobutanoyl)-6-méthoxybenzo[b]thiophén-5-yl)oxy)propoxy)-6-méthoxy-iso-indolin-2-yl)-4-oxobutanoïque |
| 14. | acide 4-(5-(3-((2-(3-carboxypropanoyl)-4-chloro-6-méthoxy-iso-indolin-5-yl)oxy)propoxy)-6-méthoxybenzo[b]thiophén-2-yl)-4-oxobutanoïque |
| 17. | acide (S)-4-(5-(3-((2-((S)-3-carboxybutanoyl)-4-fluoro-6-méthoxybenzo[b]thiophén-5-yl)oxy)propoxy)-4-fluoro-6-méthoxy-iso-indolin-2-yl)-2-méthyl-4-oxobutanoïque |
| 18. | acide 4-(5-(3-((2-((2-carboxyéthyl)carbamoyl)-6-méthoxybenzo[b]thiophén-5-yl)oxy)propoxy)-6-méthoxy-iso-indolin-2-yl)-4-oxobutanoïque |
| 20. | acide 4-(5-(3-((2-(3-carboxypropanoyl)-4-fluoro-6-méthoxybenzo[b]thiophén-5-yl)oxy)propoxy)-6-méthoxy-iso-indolin-2-yl)-4-oxobutanoïque. |

**14.** Composition pharmaceutique comprenant une quantité thérapeutiquement efficace d'au moins un composé ou sel pharmaceutiquement acceptable de celui-ci selon l'une quelconque des revendications 1 à 13 et d'au moins un excipient pharmaceutiquement acceptable.

**15.** Au moins un composé ou sel pharmaceutiquement acceptable de celui-ci pour utilisation selon l'une quelconque des revendications 1 à 13 et/ou composition pharmaceutique selon la revendication 14, qui est pour utilisation en thérapie ; ou, pour utilisation dans l'induction d'une réponse immunitaire chez un sujet ; ou, pour utilisation dans l'induction de la production d'interféron de type 1 dépendant de STING chez un sujet ; ou, pour utilisation dans l'induction de la production de cytokine dépendante de STING chez un sujet ; ou, pour utilisation dans le traitement d'un trouble de la prolifération cellulaire chez un sujet, dans lequel le trouble de la prolifération cellulaire est un cancer ; ou, pour utilisation en tant qu'agonistes de STING ; ou, pour utilisation en tant que médicament.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2021083783 W
- CN 2021090945 W
- CN 2021111479 W
- CN 2021128942 W
- CN 2021130097 W
- CN 2021138806 W
- CN 2021143043 W
- CN 2022071548 W
- WO 2019195124 A1

### Non-patent literature cited in the description

- **T. W. GREEN** ; **P. G. M. WUTS**. Protective Groups in Organic Synthesis. John Wiley & Sons, 1999